# EUROPEAN PATENT SPECIFICATION

(11) **EP 3 065 775 B1**
(45) Date of publication and mention of the grant of the patent: **30.09.2020**
(21) Application number: 14860352.5
(22) Date of filing: 07.11.2014
(51) Int. Cl.: A61K 39/395, C07K 16/18, A01K 67/027, A61K 49/00, A61K 31/713, G01N 33/68

(54) **VH4 ANTIBODIES AGAINST GRAY MATTER NEURON AND ASTROCYTE**
VH4-ANTIKÖRPER GEGEN UND NEURON UND ASTROZYT DER GRAUEN SUBSTANZ
ANTICORPS VH4 DIRIGÉS CONTRE LES ASTROCYTES ET LES NEURONES DE LA MATIÈRE GRISE

(30) Priority: 08.11.2013 US 201361902004 P
(43) Date of publication of application: 14.09.2016
(73) Proprietor: The Board of Regents of the University of Texas System, Austin, TX 78701 (US)
(72) Inventor: MONSON, Nancy, Dallas, TX 75390 (US)
(74) Representative: Rückerl, Florian
(86) International application number: PCT/US2014/064533
(87) International publication number: WO 2015/070009

(56) References cited:
- WO-A1-2013/059417
- WO-A2-2007/035857
- WO-A2-2008/097439
- US-A1- 2007 081 989
- US-A1- 2013 172 263
- US-B2- 7 101 978
- KLAUS LEHMANN-HORN ET AL: "Targeting B cells in the treatment of multiple sclerosis: recent advances and remaining challenges", THERAPEUTIC ADVANCES IN NEUROLOGICAL DISORDERS, vol. 6, no. 3, 1 May 2013 (2013-05-01), pages 161-173, XP055378035, United Kingdom ISSN: 1756-2856, DOI: 10.1177/1756285612474333
- A J LIGOCKI ET AL: "Expansion of CD27high plasmablasts in transverse myelitis patients that utilize VH4 and JH6 genes and undergo extensive somatic hypermutation", GENES AND IMMUNITY, vol. 14, no. 5, 18 April 2013 (2013-04-18) , pages 291-301, XP055377918, GB ISSN: 1466-4879, DOI: 10.1038/gene.2013.18

### SEQUENCE LISTING

<110> Monson, Nancy
<120> VH4 ANTIBODIES AGAINST GRAY MATTER NEURON AND ASTROCYTE
<130> UTSD.P2730W0
<140> Unknown
   <141> 2014-11-07
<150> 61/902,004
   <151> 2013-11-08
<160> 128
<170> PatentIn version 3.5
<210> 1
   <211> 130
   <212> PRT
   <213> Artificial
<220>
   <223> Synthetic polypeptide (AJL01 Heavy)
<400> 1
   Thr Gly Val His Ser Gln Val Gln Leu Gln Gln Trp Gly Ala Gly Leu
   1 5 10 15
   Leu Lys Pro Ser Glu Thr Leu Ser Leu Thr Cys Ala Val Tyr Gly Gly
   20 25 30
   Ser Phe Asn Glu Phe Tyr Trp Ser Trp Ile Arg Gln Pro Ala Arg Lys
   35 40 45
   Gly Leu Glu Trp Ile Gly Glu Ile Ser His Ser Gly Arg Ala Asn Tyr
   50 55 60
   Asn Pro Ser Leu Lys Ser Arg Val Thr Leu Ser Val Asp Arg Ser Lys
   65 70 75 80
   Asn Gln Phe Ser Leu Asn Leu Ser Pro Val Ala Ala Ala Asp Thr Ala
   85 90 95
   Val Tyr Tyr Cys Ala Arg Arg Glu Ile Val Val Thr Val Arg Gly Arg
   100 105 110
   Arg Ala Phe Asp Ile Trp Gly Gln Gly Thr Met Val Thr Val Ser Ser
   115 120 125
   Ala Ser
   130
<210> 2
   <211> 115
   <212> PRT
   <213> Artificial
<220>
   <223> Synthetic polypeptide (AJL01 Light)
<400> 2
   Thr Gly Val His Ser Glu Ile Val Leu Thr Gln Ser Pro Gly Thr Leu
   1               5                   10                  15
   Ser Leu Ser Pro Gly Glu Arg Ala Ala Leu Ser Cys Arg Ala Ser Gln
               20                  25                  30
   Ser Leu Ile Gly Ser Phe Leu Ala Trp Tyr Gln Gln Lys Pro Gly Gln
           35                  40                  45
   Ala Pro Arg Leu Leu Ile Tyr His Thr Ser Asn Arg Ala Ser Gly Ile
       50                  55                  60
   Pro Asp Arg Phe Ser Gly Gly Gly Phe Gly Thr Asp Phe Thr Leu Thr
   65                  70                  75                  80
   Ile Ser Arg Leu Glu Pro Glu Asp Phe Ala Val Tyr Tyr Cys Gln Gln
                   85                  90                  95
   Tyr Asp Ser Ser Pro Ile Thr Phe Gly Gln Gly Thr Arg Leu Glu Ile
               100                 105                 110
   Lys Arg Thr
           115
<210> 3
   <211> 127
   <212> PRT
   <213> Artificial
<220>
   <223> Synthetic polypeptide (AJL02 Heavy)
<400> 3
   Thr Gly Val His Ser Gln Val Gln Leu Gln Glu Ser Gly Pro Gly Leu
   1               5                   10                  15
   Val Lys Pro Ser Gln Thr Leu Ser Leu Thr Cys Thr Val Ser Gly Gly
               20                  25                  30
   Ser Ile Ser Ser Gly Gly His Tyr Trp Ser Trp Ile Arg Gln Ser Pro
           35                  40                  45
   Gly Lys Gly Leu Glu Trp Ile Gly Asn Val Tyr Tyr Ser Gly Ser Thr
       50                  55                  60
   Tyr Tyr Thr Pro Ser Leu Asp Ser Arg Leu Thr Ile Ser Leu Asp Thr
   65                  70                  75                  80
   Ser Lys Asn Gln Phe Ser Leu Arg Leu Ser Asn Val Thr Val Ala Asp
                   85                  90                  95
   Thr Ala Val Tyr Tyr Cys Ala Arg Gly Arg Asn Trp Glu Gly Glu Phe
               100                 105                 110
   Asp Pro Trp Gly Gln Gly Thr Leu Val Thr Val Ser Ser Ala Ser
           115                 120                 125
<210> 4
   <211> 114
   <212> PRT
   <213> Artificial
<220>
   <223> Synthetic polypeptide (AJL02 Light)
<400> 4
   Thr Gly Val His Ser Asp Ile Gln Met Thr Gln Ser Pro Ser Ser Leu
   1               5                   10                  15
   Ser Ala Ser Leu Gly Asp Arg Val Thr Ile Thr Cys Arg Ala Ser Gln
               20                  25                  30
   Gly Ile Ser Ser Ser Val Asn Trp Phe Gln Gln Lys Pro Gly Lys Ala
           35                  40                  45
   Pro Glu Leu Leu Ile Tyr Ala Ala Ser Thr Leu Gln Ser Gly Val Pro
       50                  55                  60
   Ser Arg Phe Ser Gly Ser Gly Ser Gly Thr Asp Phe Thr Leu Thr Val
   65                  70                  75                  80
   Ser Ser Leu Gln Pro Glu Asp Phe Ala Thr Tyr Tyr Cys Gln Gln Ser
                   85                  90                  95
   Tyr Ser Pro Pro Arg Thr Phe Gly Gln Gly Thr Lys Leu Glu Ile Lys
               100                 105                 110
   Arg Thr
<210> 5
   <211> 134
   <212> PRT
   <213> Artificial
<220>
   <223> Synthetic polypeptide (AJL03 Heavy)
<400> 5
   Thr Gly Val His Ser Gln Leu Gln Leu Gln Glu Ser Gly Pro Gly Leu
   1               5                   10                  15
   Val Lys Pro Ser Glu Thr Leu Ser Leu Thr Cys Thr Val Ser Gly Ala
               20                  25                  30
   Ser Ile Ser Ser Ser Arg Ser Tyr Trp Gly Trp Ile Arg Gln Pro Pro
           35                  40                  45
   Gly Lys Gly Leu Glu Trp Ile Gly Ser Met Tyr Gln Ser Gly Ser Thr
       50                  55                  60
   Tyr Tyr Ser Pro Ser Leu Lys Ser Arg Val Thr Ile Ser Met Asp Thr
   65                  70                  75                  80
   Ser Lys Asn Gln Phe Ser Leu Asn Leu Thr Ser Val Thr Ala Ala Asp
                   85                  90                  95
   Thr Ala Val Tyr Phe Cys Ala Arg His Ser Asn Pro Gly Thr Ala Asn
               100                 105                 110
   Lys Leu Arg Leu Gly Glu Phe Ser Pro Trp Gly Gln Gly Thr Leu Val
           115                 120                 125
   Thr Val Ser Ser Ala Ser
       130
<210> 6
   <211> 114
   <212> PRT
   <213> Artificial
<220>
   <223> Synthetic polypeptide (AJL03 Light)
<400> 6
   Thr Gly Val His Ser Asp Ile Gln Met Thr Gln Ser Pro Ser Ser Leu
   1               5                   10                  15
   Ser Ala Ser Val Gly Asp Arg Val Thr Ile Thr Cys Gln Ala Ser Gln
               20                  25                  30
   Asp Ile Asn Asn Tyr Leu Asn Trp Phe Gln Gln Gln Pro Gly Lys Ala
   35 40 45
   Pro Lys Leu Leu Ile Tyr Asp Ala Ser Lys Leu Gln Met Gly Val Pro
   50 55 60
   Ser Arg Phe Ser Gly Ser Ala Ser Gly Thr Asp Phe Thr Phe Thr Ile
   65 70 75 80
   Ser Ser Leu Gln Pro Glu Asp Ile Gly Thr Tyr Tyr Cys Gln Gln Tyr
   85 90 95
   Tyr Asn Leu Pro Tyr Thr Phe Gly Gln Gly Thr Lys Leu Glu Ile Lys
   100 105 110
   Arg Thr
<210> 7
   <211> 125
   <212> PRT
   <213> Artificial
<220>
   <223> Synthetic polypeptide (AJL04 Heavy)
<400> 7
   Thr Gly Val His Ser Gln Val Gln Leu Gln Glu Ser Gly Pro Gly Leu
   1 5 10 15
   Val Lys Pro Ser Glu Thr Leu Ser Leu Thr Cys Thr Val Ser Gly Asp
   20 25 30
   Ser Ile Arg Ser Asn Phe Trp Thr Trp Ile Arg Gln Ser Pro Gly Arg
   35 40 45
   Gly Leu Glu Trp Ile Gly Tyr Phe Ser Tyr Ser Gly Gly Ile Asn Tyr
   50 55 60
   Ser Pro Ser Leu Lys Ser Arg Val Thr Ile Ser Val Asp Thr Ser Lys
   65 70 75 80
   Asn Gln Phe Ser Leu Lys Leu Thr Ser Val Thr Ala Ala Asp Ala Ala
   85 90 95
   Val Tyr Tyr Cys Ala Arg Asp Pro Asn Gly Asp Tyr Glu Val Asn Trp
   100 105 110
   Trp Gly Gln Gly Thr Leu Val Thr Val Ser Ser Ala Ser
   115 120 125
<210> 8
   <211> 114
   <212> PRT
   <213> Artificial
<220>
   <223> Synthetic polypeptide (AJL04 Light)
<400> 8
   Thr Gly Val His Ser Asp Ile Gln Met Thr Gln Ser Pro Ser Ser Leu
   1 5 10 15
   Ser Ala Ser Val Gly Asp Arg Val Thr Ile Thr Cys Arg Ala Ser Gln
   20 25 30
   Asp Ile Ile Ile Tyr Leu Ala Trp Phe Gln Gln Arg Pro Gly Lys Ala
   35 40 45
   Pro Arg Ser Leu Ile Tyr Ser Ala Ser Thr Leu Gln Ser Gly Val Pro
   50 55 60
   Ser Lys Phe Ser Gly Ser Gly Ser Gly Thr Tyr Phe Thr Leu Thr Ile
   65 70 75 80
   Ser Ser Leu Gln Pro Glu Asp Ser Ala Thr Tyr Tyr Cys Gln Gln Tyr
   85 90 95
   Lys Ser Tyr Pro Ile Thr Phe Gly Gln Gly Thr Arg Leu Glu Ile Lys
   100 105 110
   Arg Thr
<210> 9
   <211> 129
   <212> PRT
   <213> Artificial
<220>
   <223> Synthetic polypeptide (AJL05 Heavy)
<400> 9
   Thr Gly Val His Ser Gln Leu Gln Leu Gln Glu Ser Gly Pro Gly Leu
   1 5 10 15
   Val Lys Pro Ser Glu Thr Leu Ser Leu Thr Cys Thr Val Ser Gly Gly
   20 25 30
   Ser Ile Arg Asn Ser Asn Tyr Tyr Trp Asp Trp Ile Arg Gln Pro Pro
   35 40 45
   Gly Lys Gly Leu Glu Trp Ile Gly Ser Gly Tyr Tyr Ser Gly Ser Ala
   50 55 60
   Tyr Tyr His Ser Ser Leu Lys Ser Arg Val Ser Ile Ser Val Asp Thr
   65 70 75 80
   Ser Lys Asn Gln Phe Ser Leu Asn Leu Thr Ser Val Thr Ala Ala Asp
   85 90 95
   Thr Ala Phe Tyr Tyr Cys Ala Arg Arg Ser Tyr Tyr Tyr Ala Ser Gly
   100 105 110
   Ser His Asp Tyr Trp Gly Gln Gly Thr Leu Val Thr Val Ser Ser Ala
   115 120 125
   Ser
<210> 10
   <211> 114
   <212> PRT
   <213> Artificial
<220>
   <223> Synthetic polypeptide (AJL05 Light)
<400> 10
   Thr Gly Val His Ser Asp Ile Gln Met Thr Gln Ser Pro Ser Ser Leu
   1 5 10 15
   Ser Ala Ser Val Gly Asp Arg Val Thr Ile Thr Cys Arg Ala Ser Gln
   20 25 30
   Asp Ile Ser Ser Tyr Leu Ala Trp Phe Gln Gln Lys Pro Gly Lys Ala
   35 40 45
   Pro Lys Ser Leu Ile Tyr Gly Ala Ser Ser Leu Gln Ser Gly Val Pro
   50 55 60
   Ser Lys Phe Ser Gly Ser Gly Ser Gly Thr Asp Phe Thr Leu Thr Ile
   65 70 75 80
   Ser Gly Leu Gln Pro Glu Asp Phe Ala Thr Tyr His Cys Gln Gln Tyr
   85 90 95
   Arg Ser Phe Pro Ile Thr Phe Gly Gln Gly Thr Arg Leu Glu Ile Lys
   100 105 110
   Arg Thr
<210> 11
   <211> 131
   <212> PRT
   <213> Artificial
<220>
   <223> Synthetic polypeptide (AJL06 heavy)
<400> 11
   Thr Gly Val His Ser Gln Leu Gln Leu Gln Glu Ser Gly Ser Gly Leu
   1 5 10 15
   Val Lys Pro Ser Gln Thr Leu Ser Leu Thr Cys Ala Val Ser Gly Gly
   20 25 30
   Ser Ile Asn Thr Gly Asn Tyr Phe Trp Ser Trp Ile Arg Gln Pro Pro
   35 40 45
   Gly Lys Gly Leu Glu Trp Ile Gly Tyr Met Phe Arg Ser Thr Ser Thr
   50 55 60
   Tyr Tyr Asn Pro Ser Leu Lys Gly Arg Val Thr Ile Ser Gly Gly Thr
   65 70 75 80
   Ser Leu Ser Gln Phe Ser Leu Arg Leu Asp Ser Val Thr Ala Ala Asp
   85 90 95
   Thr Ala Ile Tyr Tyr Cys Ala Arg Gly Arg Tyr Tyr Cys Gly Val Asn
   100 105 110
   Cys His Pro Phe Asp Ser Trp Gly Gln Gly Thr Leu Val Thr Val Ser
   115 120 125
   Ser Ala Ser
   130
<210> 12
   <211> 113
   <212> PRT
   <213> Artificial
<220>
   <223> Synthetic polypeptide (AJL06 light)
<400> 12
   Thr Gly Val His Ser Asp Ile Gln Met Thr Gln Ser Pro Ser Ser Leu
   1               5                   10                  15
   Ser Ala Ser Val Gly Asp Arg Val Ile Ile Thr Cys Gln Ala Ser Gln
               20                  25                  30
   Asp Ile Ser Ile Tyr Leu Asn Trp Tyr Gln Val Lys Pro Gly Lys Ala
           35                  40                  45
   Pro Lys Leu Leu Ile Tyr Asp Ala Ser Asn Leu Gln Ala Gly Val Pro
       50                  55                  60
   Ser Arg Phe Ser Gly Ser Gly Ser Gly Thr Asp Phe Ser Phe Thr Ile
   65                  70                  75                  80
   Ser Ser Leu Gln Pro Glu Asp Val Ala Ala Tyr Tyr Cys His Gln Tyr
                   85                  90                  95
   Asp Ser Leu Pro Ser Phe Gly Gln Gly Thr Lys Leu Glu Ile Lys Arg
               100                 105                 110
   Thr
<210> 13
   <211> 127
   <212> PRT
   <213> Artificial
<220>
   <223> Synthetic polypeptide (AJL07 heavy)
<400> 13
   Thr Gly Val His Ser Gln Val Gln Leu Gln Glu Ser Gly Pro Gly Leu
   1               5                   10                  15
   Val Lys Pro Ser Glu Thr Leu Ser Leu Thr Cys Asn Val Ser Gly Gly
               20                  25                  30
   Ser Ile Asn Asn Tyr Tyr Trp Ser Trp Ile Arg Gln Pro Pro Gly Lys
           35                  40                  45
   Gly Leu Glu Trp Ile Gly Tyr Ile Tyr Tyr Asn Gly Asn Ile Asn Tyr
       50                  55                  60
   Asn Pro Ser Leu Lys Ser Arg Val Thr Ile Ser Arg Asp Met Ser Lys
   65                  70                  75                  80
   Asn Gln Phe Ser Leu Asn Leu Arg Ser Val Thr Ala Ala Asp Thr Ala
                   85                  90                  95
   Val Tyr Tyr Cys Gly Ile Gly Tyr Ser Ala Val Ala Ala Gly Thr Val
               100                 105                 110
   Asp Tyr Trp Gly His Gly Thr Leu Val Thr Val Ser Ser Ala Ser
           115                 120                 125
<210> 14
   <211> 114
   <212> PRT
   <213> Artificial
<220>
   <223> Synthetic polypeptide (AJL07 light)
<400> 14
   Thr Gly Val His Ser Ala Ile Gln Leu Thr Gln Ser Pro Ser Ser Leu
   1               5                   10                  15
   Ser Ala Ser Val Gly Asp Arg Val Thr Ile Thr Cys Arg Ala Ser Gln
               20                  25                  30
   Gly Ile Ser Ser Gly Leu Ala Trp Tyr Gln Gln Glu Pro Gly Lys Ala
           35                  40                  45
   Pro Lys Leu Leu Ile Tyr Asp Ala Ser Thr Leu Glu Ser Gly Val Pro
       50                  55                  60
   Ser Arg Phe Ser Gly Ser Gly Ser Ala Ile Asp Phe Thr Leu Thr Ile
   65                  70                  75                  80
   Ser Ser Leu Gln Pro Glu Asp Phe Ala Thr Tyr Tyr Cys Gln Gln Phe
                   85                  90                  95
   Asn Thr Phe Pro Tyr Thr Phe Gly Gln Gly Thr Lys Leu Glu Ile Lys
               100                 105                 110
   Arg Thr
<210> 15
   <211> 131
   <212> PRT
   <213> Artificial
<220>
   <223> Synthetic polypeptide (AJL08 heavy)
<400> 15
   Thr Gly Val His Ser Gln Val Gln Leu Gln Glu Ser Gly Pro Gly Leu
   1               5                   10                  15
   Val Lys Pro Ser Glu Thr Leu Ser Leu Thr Cys Ala Val Ser Gly Phe
               20                  25                  30
   Ser Ile Thr Ser Gly Tyr Tyr Trp Gly Trp Ile Arg Gln Pro Pro Gly
           35                  40                  45
   Lys Gly Leu Glu Trp Ile Gly Ser Ile Tyr His Thr Gly Thr Thr Tyr
       50                  55                  60
   Tyr Asn Pro Ser Leu Lys Ser Arg Val Thr Ile Ser Val Asp Thr Ser
   65                  70                  75                  80
   Lys Asn Gln Phe Ser Leu Asn Leu Asn Ser Val Thr Ala Ala Asp Thr
                   85                  90                  95
   Ala Phe Tyr Tyr Cys Ala Arg Asp Pro Leu Phe Pro Gly Arg Asn Leu
               100                 105                 110
   Leu Ser Val Phe Asp Asn Trp Gly Gln Gly Thr Leu Val Thr Val Ser
           115                 120                 125
   Ser Ala Ser
       130
<210> 16
   <211> 114
   <212> PRT
   <213> Artificial
<220>
   <223> Synthetic polypeptide (AJL08 light)
<400> 16
   Thr Gly Val His Ser Asp Ile Gln Met Thr Gln Ser Pro Ser Thr Leu
   1               5                   10                  15
   Ser Ala Ser Val Gly Asp Arg Val Thr Ile Thr Cys Arg Ala Ser Gln
               20                  25                  30
   Ser Ile Ser Thr Trp Leu Ala Trp Tyr Gln Gln Lys Pro Gly Lys Ala
           35                  40                  45
   Pro Arg Leu Leu Ile Tyr Lys Ala Ser Ser Leu Glu Ser Gly Val Pro
       50                  55                  60
   Ser Arg Phe Ser Gly Ser Gly Ser Gly Thr Glu Phe Thr Leu Thr Ile
   65                  70                  75                  80
   Ser Gly Leu Gln Pro Asp Asp Phe Ala Asn Tyr Ser Cys Gln Gln Tyr
                   85                  90                  95
   Asn Ile Tyr Pro Phe Thr Phe Gly Gln Gly Thr Lys Leu Glu Ile Lys
               100                 105                 110
   Arg Thr
<210> 17
   <211> 128
   <212> PRT
   <213> Artificial
<220>
   <223> Synthetic polypeptide (AJL09 heavy)
<400> 17
   Thr Gly Val His Ser Gln Val Gln Leu Gln Glu Ser Gly Pro Gly Leu
   1               5                   10                  15
   Val Lys Pro Ser Glu Thr Leu Ser Leu Thr Cys Ser Val Ser Gly Gly
               20                  25                  30
   Ser Ile Ser Ser Tyr Tyr Trp Gly Trp Ile Arg Gln Ser Pro Gly Lys
           35                  40                  45
   Gly Leu Glu Cys Ile Gly Tyr Ile Tyr Phe Ser Gly Ser Thr Ser Tyr
       50                  55                  60
   Asn Pro Ser Leu Lys Ser Arg Val Thr Ile Ser Val Asp Thr Ala Lys
   65                  70                  75                  80
   Asn Gln Ile Ser Leu Asn Leu Thr Ser Val Thr Ala Ala Asp Thr Ala
                   85                  90                  95
   Val Tyr Phe Cys Ala Arg Val Trp Gly Ser Ser Trp Tyr Ala Asn Trp
               100                 105                 110
   Phe Asp Pro Trp Gly Gln Gly Thr Leu Val Thr Val Ser Ser Ala Ser
           115                 120                 125
<210> 18
   <211> 114
   <212> PRT
   <213> Artificial
<220>
   <223> Synthetic polypeptide (AJL09 light)
<400> 18
   Thr Gly Val His Ser Ala Ile Gln Leu Thr Gln Ser Pro Ser Ser Leu
   1               5                   10                  15
   Ser Ala Ser Val Gly Asp Arg Val Thr Ile Thr Cys Arg Ala Ser Gln
               20                  25                  30
   Gly Ile Ser Ser Ala Leu Ala Trp Tyr Gln His Lys Pro Gly Lys Ala
           35                  40                  45
   Pro Lys Leu Leu Ile Phe Asp Ala Ser Thr Leu Ala Ala Gly Val Pro
       50                  55                  60
   Ser Arg Phe Ser Gly Ser Gly Ser Gly Thr Asp Phe Thr Leu Thr Ile
   65                  70                  75                  80
   Ser Ser Leu Gln Pro Glu Asp Phe Ala Thr Tyr Tyr Cys Gln Gln Tyr
                   85                  90                  95
   Asn Thr Tyr Val Leu Thr Phe Gly Gln Gly Thr Arg Leu Glu Ile Lys
               100                 105                 110
   Arg Thr
<210> 19
   <211> 129
   <212> PRT
   <213> Artificial
<220>
   <223> Synthetic polypeptide (AJL10 heavy)
<400> 19
   Thr Gly Val His Ser Gln Val Gln Leu Gln Glu Ser Gly Pro Gly Leu
   1               5                   10                  15
   Val Lys Pro Ser Glu Thr Leu Ser Leu Thr Cys Ser Val Ser Gly Gly
               20                  25                  30
   Ala Val Ser Asn Tyr Tyr Trp Ser Trp Ile Arg Gln Ser Ala Gly Lys
           35                  40                  45
   Gly Leu Glu Trp Leu Gly Arg Ile Tyr Ile Asn Gly Thr Thr Tyr Tyr
       50                  55                  60
   Asn Pro Ser Leu Arg Ser Arg Val Ser Met Ser Val Asp Thr Ser Lys
   65 70 75 80
   Gly Gln Phe Ser Leu Arg Leu Thr Ser Val Thr Ala Ala Asp Thr Ala
   85 90 95
   Ile Tyr Tyr Cys Ala Arg Trp Gly Ala Leu Leu Gly Asp Tyr Tyr Tyr
   100 105 110
   Gly Leu Asp Val Trp Gly Gln Gly Thr Thr Val Thr Val Ser Ser Ala
   115 120 125
   Ser
<210> 20
   <211> 119
   <212> PRT
   <213> Artificial
<220>
   <223> Synthetic polypeptide (AJL10 light)
<400> 20
   Thr Gly Val His Gly Asp Ile Val Met Thr Gln Ser Pro Leu Ser Leu
   1 5 10 15
   Pro Val Thr Pro Gly Glu Pro Ala Ser Ile Ser Cys Arg Ser Thr Gln
   20 25 30
   Ser Leu Leu His Ser Asn Glu Tyr Ile Tyr Leu Asp Trp Tyr Val Gln
   35 40 45
   Lys Pro Gly Gln Ser Pro Gln Leu Leu Ile Phe Leu Ala Ser Asn Arg
   50 55 60
   Ala Ser Gly Val Pro Asp Arg Phe Ser Gly Ser Ala Ser Gly Thr Asp
   65 70 75 80
   Phe Thr Leu Lys Ile Ser Arg Val Glu Ala Glu Asp Val Gly Val Tyr
   85 90 95
   Tyr Cys Met Gln Ala Leu Glu Ala Pro Trp Thr Phe Gly Gln Gly Thr
   100 105 110
   Arg Leu Glu Ile Lys Arg Thr
   115
<210> 21
   <211> 126
   <212> PRT
   <213> Artificial
<220>
   <223> Synthetic polypeptide (AJL11 heavy)
<400> 21
   Thr Gly Val His Ser Gln Leu Gln Leu Gln Glu Ser Gly Pro Gly Leu
   1               5                   10                  15
   Val Lys Pro Ser Glu Thr Leu Ser Leu Thr Cys Ser Val Ser Gly Gly
               20                  25                  30
   Ser Ile Thr Ser Thr Ser Tyr Tyr Trp Gly Trp Ile Arg Gln Ser Pro
           35                  40                  45
   Gly Lys Gly Leu Glu Trp Ile Gly Ser Val Tyr Tyr Ser Gly Asn Thr
       50                  55                  60
   Phe Tyr Asn Ala Ser Leu Lys Ser Arg Val Thr Ile Ser Ile Asp Thr
   65                  70                  75                  80
   Ser Lys Tyr Gln Phe Ser Leu Met Leu Arg Ser Val Thr Ala Ala Asp
                   85                  90                  95
   Thr Ala Val Tyr Tyr Cys Ala Arg Arg His Asp Trp Phe Trp Phe Asp
               100                 105                 110
   Pro Trp Gly Gln Gly Thr Leu Val Thr Val Ser Ser Ala Ser
           115                 120                 125
<210> 22
   <211> 115
   <212> PRT
   <213> Artificial
<220>
   <223> Synthetic polypeptide (AJL11 light)
<400> 22
   Thr Gly Val His Ser Glu Ile Val Leu Thr Gln Ser Pro Gly Thr Leu
   1               5                   10                  15
   Ser Leu Ser Pro Gly Glu Arg Val Thr Leu Ser Cys Arg Ala Ser Gln
               20                  25                  30
   Ser Val Ser Ser Ser Tyr Leu Ala Trp Tyr Gln Gln Lys Pro Gly Gln
           35                  40                  45
   Ala Pro Arg Leu Leu Ile Tyr Asp Ser Ser Ser Arg Ala Thr Gly Ile
       50                  55                  60
   Pro Asp Arg Phe Ser Gly Ser Gly Ser Gly Thr Asp Phe Thr Leu Thr
   65                  70                  75                  80
   Ile Ser Arg Leu Asp Pro Glu Asp Phe Ala Val Tyr Tyr Cys Gln Gln
                   85                  90                  95
   Tyr Gly Ser Ser Pro Ser Thr Phe Gly Gln Gly Thr Lys Leu Glu Ile
               100                 105                 110
   Lys Arg Thr
           115
<210> 23
   <211> 128
   <212> PRT
   <213> Artificial
<220>
   <223> Synthetic polypeptide (AJL12 heavy)
<400> 23
   Thr Gly Val His Ser Gln Leu Gln Leu Gln Glu Ser Gly Pro Gly Leu
   1               5                   10                  15
   Val Lys Pro Ser Glu Thr Leu Ser Leu Thr Cys Thr Val Ser Gly Gly
               20                  25                  30
   Ser Val Thr Ser Ser Asp Tyr Tyr Trp Ala Trp Ile Arg Gln Pro Pro
           35                  40                  45
   Gly Lys Gly Pro Glu Trp Ile Gly Ser Ile Ser Asn Ser Gly Asn Thr
       50                  55                  60
   Tyr Tyr Ser Pro Ser Leu Lys Ser Arg Val Ser Ile Ser Gly Asp Thr
   65                  70                  75                  80
   Ser Lys Lys Gln Phe Ser Leu Asn Leu Ser Ser Val Thr Asp Ala Asp
                   85                  90                  95
   Thr Ala Val Tyr Tyr Cys Thr Arg His Gly His Tyr Val Ser Gly Gly
               100                 105                 110
   Leu Gly Pro Trp Gly Gln Gly Thr Leu Val Thr Val Ser Ser Ala Ser
           115                 120                 125
<210> 24
   <211> 115
   <212> PRT
   <213> Artificial
<220>
   <223> Synthetic polypeptide (AJL12 light)
<400> 24
   Thr Gly Val His Ser Glu Ile Val Leu Thr Gln Ser Pro Gly Thr Leu
   1               5                   10                  15
   Ser Leu Ser Pro Gly Glu Arg Ala Thr Leu Ser Cys Arg Ala Ser Gln
               20                  25                  30
   Ser Val Gly Ser Tyr Tyr Leu Ala Trp Tyr Gln Gln Lys Pro Gly Gln
           35                  40                  45
   Ala Pro Arg Leu Leu Ile His Gly Ala Ser Ser Arg Ala Thr Gly Thr
       50                  55                  60
   Pro Asp Arg Phe Ser Gly Ser Gly Ser Gly Thr Asp Phe Thr Leu Thr
   65                  70                  75                  80
   Ile Ser Lys Leu Glu Pro Glu Asp Phe Ala Leu Tyr Tyr Cys Gln Gln
                   85                  90                  95
   Tyr Gly Pro Ser Pro Trp Thr Phe Gly Gln Gly Thr Lys Val Glu Ile
               100                 105                 110
   Lys Arg Thr
           115
<210> 25
   <211> 127
   <212> PRT
   <213> Artificial
<220>
   <223> Synthetic polypeptide (AJL13 heavy)
<400> 25
   Thr Gly Val His Ser Gln Val Gln Leu Gln Glu Ser Gly Pro Gly Leu
   1               5                   10                  15
   Val Lys Pro Ser Glu Thr Leu Ser Leu Thr Cys Asn Val Ser Gly Gly
               20                  25                  30
   Ser Ile Asn Asn Tyr Tyr Trp Ser Trp Ile Arg Gln Pro Pro Gly Lys
           35                  40                  45
   Gly Leu Glu Trp Ile Gly Tyr Ile Tyr Tyr Asn Gly Asn Ile Asn Tyr
       50                  55                  60
   Asn Pro Ser Leu Lys Ser Arg Val Thr Ile Ser Arg Asp Met Ser Lys
   65                  70                  75                  80
   Asn Gln Phe Ser Leu Asn Leu Arg Ser Val Thr Ala Ala Asp Thr Ala
                   85                  90                  95
   Val Tyr Tyr Cys Gly Ile Gly Tyr Ser Ala Val Ala Ala Gly Thr Val
               100                 105                 110
   Asp Tyr Trp Gly Gln Gly Thr Leu Val Thr Val Ser Ser Ala Ser
           115                 120                 125
<210> 26
   <211> 119
   <212> PRT
   <213> Artificial
<220>
   <223> Synthetic polypeptide (AJL13 light)
<400> 26
   Thr Gly Val His Gly Asp Val Val Met Thr Gln Ser Pro Leu Ser Leu
   1               5                   10                  15
   Pro Val Thr Leu Gly Gln Pro Ala Ser Ile Ser Cys Arg Ser Ser Gln
               20                  25                  30
   Ser Leu Val His Arg Asn Gly Asn Thr Tyr Leu Asn Trp Phe Gln Gln
           35                  40                  45
   Arg Pro Gly Gln Ser Pro Arg Arg Leu Ile Tyr Thr Val Ser Lys Arg
       50                  55                  60
   Gly Ser Gly Val Pro Asp Arg Phe Ser Gly Ser Gly Ser Gly Thr Asp
   65                  70                  75                  80
   Phe Thr Leu Lys Ile Ser Arg Val Glu Ala Glu Asp Val Gly Asp Tyr
                   85                  90                  95
   Tyr Cys Met Gln Gly Thr His Trp Pro Trp Thr Ser Gly Gln Gly Thr
               100                 105                 110
   Arg Leu Glu Ile Lys Arg Thr
           115
<210> 27
   <211> 135
   <212> PRT
   <213> Artificial
<220>
   <223> Synthetic polypeptide (AJL14 heavy)
<400> 27
   Thr Gly Val His Ser Gln Val Gln Leu Gln Glu Ser Gly Pro Gly Leu
   1               5                   10                  15
   Val Lys Pro Ser Gln Thr Leu Ser Leu Thr Cys Thr Val Ser Gly Gly
               20                  25                  30
   Ser Leu Ser Ser Val Asn Tyr Tyr Trp Asn Trp Ile Arg Gln Pro Ala
           35                  40                  45
   Gly Lys Gly Leu Glu Trp Met Gly Arg Ile Tyr Ala Ser Gly Tyr Thr
       50                  55                  60
   Thr Tyr Asn Pro Ser Phe Gln Ser Arg Val Thr Ile Ser Leu Asp Pro
   65                  70                  75                  80
   Ser Lys Asn Gln Ile Ser Leu Lys Val Thr Ser Leu Thr Ala Ala Asp
                   85                  90                  95
   Thr Ala Ile Tyr Tyr Cys Ala Arg His Asp Leu Gly His Cys Ser Ser
               100                 105                 110
   Thr Ser Cys Tyr Leu Ser Trp Phe Asp Ala Trp Gly Gln Gly Thr Thr
           115                 120                 125
   Val Thr Val Ser Ser Ala Ser
       130                 135
<210> 28
   <211> 120
   <212> PRT
   <213> Artificial
<220>
   <223> Synthetic polypeptide (AJL14 light)
<400> 28
   Thr Gly Val His Ser Asp Ile Val Met Thr Gln Ser Pro Asp Ser Leu
   1               5                   10                  15
   Ala Val Ser Leu Gly Glu Arg Ala Thr Ile Asn Cys Arg Ser Ser Gln
               20                  25                  30
   Thr Ile Phe Phe Ser Pro Asn Asn Asn Asn His Leu Ala Trp Tyr Gln
           35                  40                  45
   Gln Lys Pro Gly Gln Pro Pro Arg Leu Leu Ile Tyr Trp Ala Ser Thr
       50                  55                  60
   Arg Glu Ser Gly Val Pro Asp Arg Phe Ser Gly Ser Gly Ser Gly Thr
   65                  70                  75                  80
   Asp Phe Thr Leu Thr Ile Ser Gly Leu Gln Ala Glu Asp Val Ala Val
                   85                  90                  95
   Tyr Tyr Cys Gln Gln Tyr Tyr Ser Leu Pro Tyr Thr Phe Gly Gln Gly
               100                 105                 110
   Thr Lys Leu Glu Ile Lys Arg Thr
           115                 120
<210> 29
   <211> 134
   <212> PRT
   <213> Artificial
<220>
   <223> Synthetic polypeptide (AJL15 heavy)
<400> 29
   Thr Gly Val His Ser Gln Leu Gln Leu Gln Glu Ser Gly Pro Gly Leu
   1               5                   10                  15
   Val Lys Pro Ser Glu Thr Leu Ser Leu Thr Cys Thr Val Ser Gly Gly
               20                  25                  30
   Ser Ile Thr Ser Arg Asn Asn Tyr Trp Gly Trp Ile Arg Gln Ser Pro
           35                  40                  45
   Gly Lys Gly Leu Glu Trp Ile Gly Ser Leu Tyr Tyr Thr Gly Ser Asp
       50                  55                  60
   Tyr Tyr Asn Pro Ser Leu Lys Ser Arg Val Thr Ile Ser Val Asp Thr
   65                  70                  75                  80
   Ser Lys Asn Gln Phe Ser Leu Arg Leu Ser Ser Val Thr Ala Ala Asp
                   85                  90                  95
   Thr Ala Val Tyr Tyr Cys Val Arg Val Asn Val Asp Asp Phe Trp Ser
               100                 105                 110
   Gly Leu Gly Gly Ala Trp Phe Asp Pro Trp Gly Gln Gly Thr Leu Val
           115                 120                 125
   Thr Val Ser Ser Ala Ser
       130
<210> 30
   <211> 119
   <212> PRT
   <213> Artificial
<220>
   <223> Synthetic polypeptide (AJL15 light)
<400> 30
   Thr Gly Val His Gly Asp Ile Val Met Thr Gln Thr Pro Leu Ser Leu
   1               5                   10                  15
   Ser Val Thr Pro Gly Gln Pro Ala Ser Ile Ser Cys Lys Ser Ser Gln
               20                  25                  30
   Ser Leu Leu Asp Ser Asp Gly Lys Thr His Leu Tyr Trp Tyr Leu Gln
           35                  40                  45
   Lys Pro Gly Gln Ser Pro Gln Ser Leu Ile Tyr Glu Val Ser Lys Arg
       50                  55                  60
   Phe Ser Gly Val Pro Asp Arg Phe Thr Gly Ser Gly Ser Gly Thr Asp
   65                  70                  75                  80
   Phe Thr Leu Lys Ile Ser Arg Val Glu Ala Glu Asp Val Gly Leu Tyr
                   85                  90                  95
   Tyr Cys Met Gln Ser Ala Gln Leu Pro Tyr Thr Phe Gly Gln Gly Thr
               100                 105                 110
   Lys Leu Glu Ile Lys Arg Thr
           115
<210> 31
   <211> 133
   <212> PRT
   <213> Artificial
<220>
   <223> Synthetic polypeptide (AJL16 heavy)
<400> 31
   Thr Gly Val His Ser Gln Leu Gln Leu Gln Glu Ser Gly Pro Gly Leu
   1               5                   10                  15
   Val Lys Pro Ser Glu Thr Leu Ser Leu Thr Cys Thr Val Ser Gly Gly
               20                  25                  30
   Ser Ile Ser Ser Ser Pro Tyr Tyr Trp Gly Trp Ile Arg Gln Pro Pro
           35                  40                  45
   Gly Lys Gly Leu Glu Trp Ile Gly Ser Ile Tyr Tyr Ser Gly His Thr
       50                  55                  60
   Tyr Tyr Asn Pro Ser Leu Lys Ser Arg Val Thr Ile Ser Val Asp Thr
   65                  70                  75                  80
   Ser Lys Asn Gln Phe Ser Leu Arg Leu Thr Ser Val Thr Ala Ala Asp
                   85                  90                  95
   Thr Ser Val Tyr Tyr Cys Ala Lys Gln Thr Asp Asp Tyr Gly Asp Tyr
               100                 105                 110
   Ala Ser Arg Gly Trp Phe Asp Pro Trp Gly Gln Gly Thr Leu Val Thr
           115                 120                 125
   Val Ser Ser Ala Ser
       130
<210> 32
   <211> 114
   <212> PRT
   <213> Artificial
<220>
   <223> Synthetic polypeptide (AJL16 light)
<400> 32
   Thr Gly Val His Ser Asp Ile Gln Met Thr Gln Ser Pro Ser Ser Leu
   1               5                   10                  15
   Ser Ala Ser Val Gly Asp Arg Val Thr Phe Thr Cys Gln Ala Ser His
               20                  25                  30
   Asp Ile Ser Asn Tyr Leu Asn Trp Tyr Gln Gln Lys Pro Gly Lys Val
           35                  40                  45
   Pro Glu Leu Leu Ile Tyr Asp Ala Ser Asn Leu Lys Thr Gly Val Pro
       50                  55                  60
   Ser Arg Phe Ser Gly Ser Gly Ser Gly Thr Asp Phe Thr Phe Thr Ile
   65                  70                  75                  80
   Ser Ser Leu Gln Pro Glu Asp Ile Ala Thr Tyr Tyr Cys Gln Gln Tyr
   85 90 95
   Asp Asn Leu Pro Ile Thr Phe Gly Gln Gly Thr Arg Leu Glu Ile Lys
   100 105 110
   Arg Thr
<210> 33
   <211> 135
   <212> PRT
   <213> Artificial
<220>
   <223> Synthetic polypeptide (AJL18 heavy)
<400> 33
   Thr Gly Val His Ser Gln Val Gln Leu Gln Glu Ser Gly Pro Gly Leu
   1 5 10 15
   Val Lys Pro Ser Gln Thr Leu Ser Leu Thr Cys Thr Val Ser Gly Asp
   20 25 30
   Ser Ile Ser Ser Asp Lys Tyr Tyr Trp Thr Trp Ile Arg Gln Leu Pro
   35 40 45
   Gly Lys Gly Leu Glu Trp Ile Gly Tyr Ile Ser Tyr Ser Gly Thr Thr
   50 55 60
   Tyr Tyr Asn Pro Ser Leu Lys Ser Arg Val Ser Ile Ser Val Asp Thr
   65 70 75 80
   Ser Gly Asn Gln Phe Ser Leu Arg Leu Ser Ser Val Thr Ala Ala Asp
   85 90 95
   Thr Ala Arg Tyr Tyr Cys Ala Arg Val Tyr Tyr Asp Val Leu Ser Ala
   100 105 110
   Tyr Tyr Asn Met Gly Ser Trp Val Asp Pro Trp Gly Gln Gly Thr Leu
   115 120 125
   Val Thr Val Ser Ser Ala Ser
   130 135
<210> 34
   <211> 114
   <212> PRT
   <213> Artificial
<220>
   <223> Synthetic polypeptide (AJL18 light)
<400> 34
   Thr Gly Val His Ser Asp Ile Gln Met Thr Gln Ser Pro Ser Ser Leu
   1               5                   10                  15
   Pro Ala Ser Val Gly Asp Arg Val Thr Ile Thr Cys Arg Ala Ser Gln
               20                  25                  30
   Ser Ile Ser Ser Tyr Leu Asn Trp Tyr Gln Gln Lys Pro Gly Arg Ala
           35                  40                  45
   Pro Lys Leu Leu Ile Tyr Ala Ala Ser Ser Leu Gln Ser Gly Val Ser
       50                  55                  60
   Ser Arg Phe Ser Gly Ser Gly Ser Gly Thr Asp Phe Thr Leu Thr Ile
   65                  70                  75                  80
   Ser Ser Leu Gln Pro Glu Asp Ser Ala Thr Tyr Tyr Cys Gln Gln Ser
                   85                  90                  95
   Tyr Ser Thr Pro Trp Thr Phe Gly Gln Gly Thr Lys Val Glu Ile Lys
               100                 105                 110
   Arg Thr
<210> 35
   <211> 127
   <212> PRT
   <213> Artificial
<220>
   <223> Synthetic polypeptide (AJL19 heavy)
<400> 35
   Thr Gly Val His Ser Gln Val Gln Leu Gln Gln Trp Gly Ala Gly Leu
   1               5                   10                  15
   Leu Lys Pro Pro Glu Thr Leu Ser Leu Thr Cys Ala Val Phe Gly Gly
               20                  25                  30
   Ser Leu Ser Gly Tyr Tyr Trp Ser Trp Ile Arg Gln Pro Pro Gly Lys
           35                  40                  45
   Gly Pro Glu Trp Ile Ala Glu Ile Asn His Ser Gly Asp Ala Asn Tyr
       50                  55                  60
   Asn Pro Ser Leu Lys Ser Arg Val Thr Ile Ser Val Asp Thr Ser Lys
   65                  70                  75                  80
   Asn Gln Phe Ser Leu Lys Met Ser Ser Val Thr Val Ala Asp Thr Ala
                   85                  90                  95
   Leu Tyr Tyr Cys Ala Thr Gln Gly Ser Arg Leu Thr Thr Phe Ala Phe
               100                 105                 110
   Asp Val Trp Gly Gln Gly Thr Met Val Thr Val Ser Ser Ala Ser
           115                 120                 125
<210> 36
   <211> 116
   <212> PRT
   <213> Artificial
<220>
   <223> Synthetic polypeptide (AJL19 light)
<400> 36
   Thr Gly Val His Ser Glu Ile Val Leu Thr Gln Ser Pro Gly Thr Leu
   1               5                   10                  15
   Ser Leu Ser Pro Gly Glu Arg Val Thr Leu Ser Cys Arg Thr Ser Gln
               20                  25                  30
   Ser Val Ser Ser Asp Ser Leu Ala Trp Tyr Gln Gln Lys Pro Gly Gln
           35                  40                  45
   Thr Pro Arg Leu Leu Ile Tyr His Thr Ser Thr Arg Ala Ala Gly Ile
       50                  55                  60
   Pro Asp Arg Phe Ser Gly Thr Gly Ser Gly Thr Asp Phe Thr Leu Thr
   65                  70                  75                  80
   Ile Ala Arg Leu Glu Pro Glu Asp Phe Ala Val Tyr Tyr Cys Gln His
                   85                  90                  95
   Tyr Gly Arg Ser Ser Leu Phe Thr Phe Gly Gln Gly Thr Lys Leu Glu
               100                 105                 110
   Ile Lys Arg Thr
           115
<210> 37
   <211> 132
   <212> PRT
   <213> Artificial
<220>
   <223> Synthetic polypeptide (AJL20 heavy)
<400> 37
   Thr Gly Val His Ser Gln Leu Gln Leu Gln Glu Ser Gly Pro Gly Leu
   1               5                   10                  15
   Val Lys Pro Ser Glu Thr Leu Ser Leu Thr Cys Thr Val Ser Gly Gly
               20                  25                  30
   Ser Ile Ser Gly Ser Ser Phe Tyr Trp Gly Trp Val Arg Gln Pro Pro
           35                  40                  45
   Gly Arg Gly Leu Glu Trp Ile Gly Thr Ile Tyr Tyr Arg Gly Thr Thr
       50                  55                  60
   Tyr Tyr Thr Pro Ser Leu Lys Ser Arg Val Thr Ile Ser Val Asp Thr
   65                  70                  75                      80
   Ser Lys Asn Gln Phe Ser Leu Arg Leu Asn Ser Val Thr Ala Ala Asp
                   85                  90                  95
   Thr Ala Ile Tyr Tyr Cys Ala Ser Leu Pro His Tyr Asp Phe Trp Ser
               100                 105                 110
   Gly Ser Val Phe Phe Asp Tyr Trp Gly Gln Gly Thr Leu Val Thr Val
           115                 120                 125
   Ser Ser Ala Ser
       130
<210> 38
   <211> 114
   <212> PRT
   <213> Artificial
<220>
   <223> Synthetic polypeptide (AJL20 light)
<400> 38
   Thr Gly Val His Ser Asp Ile Gln Met Thr Gln Ser Pro Ser Ser Leu
   1               5                   10                  15
   Ser Ala Ser Val Gly Asp Arg Val Thr Ile Thr Cys Arg Ala Ser Gln
               20                  25                  30
   Gly Ile Ala Asn Tyr Leu Ala Trp Tyr Gln Gln Lys Pro Gly Asn Ile
           35                  40                  45
   Pro Lys Leu Leu Ile Tyr Ala Ala Ser Thr Leu Gln Ser Gly Val Pro
       50                  55                  60
   Ser Arg Phe Ser Gly Ser Gly Ser Gly Thr Asp Phe Ala Leu Thr Ile
   65                  70                  75                  80
   Ser Cys Leu Gln Pro Glu Asp Val Ala Thr Tyr Tyr Cys Gln Lys Tyr
                   85                  90                  95
   Asn Ser Ala Pro Leu Thr Phe Gly Gly Gly Thr Lys Val Glu Ile Lys
               100                 105                 110
   Arg Thr
<210> 39
   <211> 126
   <212> PRT
   <213> Artificial
<220>
   <223> Synthetic polypeptide (WR01 heavy)
<400> 39
   Thr Gly Val His Ser Gln Val Gln Leu Gln Glu Ser Gly Pro Gly Leu
   1               5                   10                  15
   Val Lys Pro Ser Glu Thr Leu Ser Leu Thr Cys Thr Val Ser Gly Gly
               20                  25                  30
   Ser Ile Asp Thr Tyr Tyr Trp Thr Trp Ile Arg Gln Pro Pro Gly Lys
           35                  40                  45
   Gly Leu Glu Trp Ile Gly Tyr Ile Tyr Ser Thr Gly Ser Pro Lys Tyr
       50                  55                  60
   Lys Pro Ser Leu Lys Ser Arg Val Val Met Ser Val Asp Thr Ser Thr
   65                  70                  75                  80
   Asn Glu Phe Ala Leu Arg Leu Ser Ser Val Thr Ala Ala Asp Thr Ala
                   85                  90                  95
   Val Tyr Tyr Cys Ala Arg Ser Ser Gly Phe Tyr Val Glu His Leu Glu
               100                 105                 110
   Lys Trp Gly Gln Gly Thr Thr Val Thr Val Ser Ser Ala Ser
           115                 120                 125
<210> 40
   <211> 115
   <212> PRT
   <213> Artificial
<220>
   <223> Synthetic polypeptide (WR01 light)
<400> 40
   Thr Gly Val His Ser Glu Ile Val Leu Thr Gln Ser Pro Ala Thr Leu
   1               5                   10                  15
   Ser Leu Ser Pro Gly Glu Arg Ala Thr Leu Ser Cys Arg Ala Ser Gln
               20                  25                  30
   Thr Val Ser Ser Ser Tyr Leu Asp Trp Phe Gln Gln Lys Pro Gly Gln
           35                  40                  45
   Ala Pro Arg Leu Leu Ile Tyr Gly Ala Ser Ser Arg Ala Thr Gly Ile
       50                  55                  60
   Pro Asp Arg Phe Ser Gly Ser Gly Ser Gly Thr Asp Phe Thr Leu Thr
   65                  70                  75                  80
   Ile Ser Arg Leu Glu Pro Glu Asp Phe Ala Val Tyr Tyr Cys Gln Gln
                   85                  90                  95
   Phe Ala Thr Ser Pro Tyr Thr Phe Gly Gln Gly Thr Lys Leu Glu Ile
               100                 105                 110
   Lys Arg Thr
           115
<210> 41
   <211> 131
   <212> PRT
   <213> Artificial
<220>
   <223> Synthetic polypeptide (WR02 heavy)
<400> 41
   Thr Gly Val His Ser Gln Val Gln Leu Gln Glu Ser Gly Pro Gly Leu
   1               5                   10                  15
   Val Lys Pro Ser Gln Thr Leu Ser Leu Thr Cys Thr Val Ser Gly Gly
               20                  25                  30
   Ser Ile Ser Ser Gly Asp Tyr His Trp Ser Trp Ile Arg Gln Pro Pro
           35                  40                  45
   Gly Lys Gly Leu Glu Trp Ile Gly Asn Ile Asn Tyr Asn Gly Gly Ala
   50 55 60
   Tyr His Asn Pro Ser Leu Thr Asn Arg Val Ile Met Ser Val Asp Thr
   65 70 75 80
   Ser Lys Asn His Phe Ser Leu Lys Leu Thr Ser Val Thr Ala Ala Asp
   85 90 95
   Thr Ala Val Tyr Tyr Cys Ala Arg Glu Ser Gln Trp Leu Arg Tyr Gly
   100 105 110
   Ala Phe Gly Met Asp Val Trp Gly Gln Gly Thr Thr Val Thr Val Ser
   115 120 125
   Ser Ala Ser
   130
<210> 42
   <211> 115
   <212> PRT
   <213> Artificial
<220>
   <223> Synthetic polypeptide (WR02 light)
<400> 42
   Thr Gly Val His Ser Glu Ile Val Leu Thr Gln Ser Pro Gly Thr Leu
   1               5                   10                  15
   Ser Leu Ser Pro Gly Glu Arg Ala Thr Leu Ser Cys Arg Ala Ser Gln
               20                  25                  30
   Ser Val Ser Ser Ser His Leu Val Trp Tyr Gln Gln Lys Ala Gly Gln
           35                  40                  45
   Ala Pro Arg Leu Val Ile Tyr Gly Ala Asn Arg Arg Ala Ser Gly Thr
       50                  55                  60
   Pro Asp Arg Phe Ser Gly Ser Gly Ser Gly Thr Asp Phe Thr Leu Thr
   65                  70                  75                  80
   Ile Ser Arg Leu Glu Pro Glu Asp Phe Ala Val Tyr Tyr Cys Gln Gln
                   85                  90                  95
   Tyr Gly Ser Ser Pro Tyr Thr Phe Gly Gln Gly Thr Lys Leu Glu Ile
               100                 105                 110
   Lys Arg Thr
                              115
<210> 43
   <211> 131
   <212> PRT
   <213> Artificial
<220>
   <223> Synthetic polypeptide (WR03 heavy)
<400> 43
   Thr Gly Val His Ser Gln Val Gln Leu Gln Glu Ser Gly Pro Gly Leu
   1               5                   10                  15
   Val Lys Pro Ser Gly Thr Leu Ser Leu Thr Cys Ala Val Ser Gly Gly
               20                  25                  30
   Ser Ile Ser Ser Ser Tyr Trp Trp Ser Trp Val Arg Gln Pro Pro Gly
           35                  40                  45
   Lys Gly Leu Glu Trp Val Gly Glu Ile Tyr His Ser Gly Gly Ala Asn
       50                  55                  60
   Tyr Ser Pro Ser Leu Lys Ser Arg Val Thr Ile Ser Val Asp Lys Ser
   65                  70                  75                  80
   Lys Asn Gln Phe Ser Leu Asn Leu Ile Ser Val Thr Ala Ala Asp Thr
                   85                  90                  95
   Ala Val Tyr Phe Cys Ala Arg Ser Arg Met Leu Val Gly Ala Asp Gly
               100                 105                 110
   Gly Gly Ala Phe Asp Ile Trp Gly Gln Gly Thr Met Val Thr Val Ser
           115                 120                 125
   Ser Ala Ser
       130
<210> 44
   <211> 115
   <212> PRT
   <213> Artificial
<220>
   <223> Synthetic polypeptide (WR03 light)
<400> 44
   Thr Gly Val His Ser Glu Ile Val Leu Thr Gln Ser Pro Gly Thr Leu
   1               5                   10                  15
   Ser Leu Ser Pro Gly Glu Arg Ala Thr Leu Ser Cys Gly Ala Ser Gln
               20                  25                  30
   Ser Val Ser Ser Ser Tyr Leu Ala Trp Tyr Gln Gln Lys Pro Gly Leu
           35                  40                  45
   Ala Pro Arg Leu Leu Ile Tyr Asp Ala Ser Ser Arg Ala Thr Gly Ile
       50                  55                  60
   Pro Asp Arg Phe Ser Gly Ser Gly Ser Gly Thr Asp Phe Thr Leu Thr
   65                  70                  75                  80
   Ile Ser Arg Leu Glu Pro Glu Asp Phe Ala Val Tyr Tyr Cys Gln Gln
                   85                  90                  95
   Tyr Gly Ser Ser Pro Gln Thr Phe Gly Gln Gly Thr Lys Val Glu Ile
               100                 105                 110
   Lys Arg Thr
           115
<210> 45
   <211> 126
   <212> PRT
   <213> Artificial
<220>
   <223> Synthetic polypeptide (WR04 heavy)
<400> 45
   Thr Gly Val His Ser Gln Leu Gln Leu Gln Gln Ser Gly Pro Gly Leu
   1 5 10 15
   Val Lys Pro Ser Glu Thr Leu Ser Leu Thr Cys Thr Val Ser Gly Gly
   20 25 30
   Ser Ile Ser Ser Gly Gly Ser Tyr Trp Gly Trp Ile Arg Gln Ala Pro
   35 40 45
   Gly Lys Gly Leu Glu Trp Ile Gly Ser Met Tyr Tyr Ser Gly Ser Thr
   50 55 60
   Phe Tyr Asn Pro Ser Val Lys Ser Arg Val Thr Ile Ser Val Asp Arg
   65 70 75 80
   Ser Lys Glu Gln Phe Ser Leu Asn Leu Asn Ala Val Thr Ala Ala Asp
   85 90 95
   Thr Ala Val Tyr Tyr Cys Val Arg His Arg Arg Ser Glu Pro Ser Asp
   100 105 110
   Ser Trp Gly Gln Gly Thr Leu Val Thr Val Ser Ser Ala Ser
   115 120 125
<210> 46
   <211> 115
   <212> PRT
   <213> Artificial
<220>
   <223> Synthetic polypeptide (WR04 light)
<400> 46
   Thr Gly Val His Ser Glu Ile Val Met Thr Gln Ser Pro Ala Thr Leu
   1               5                   10                  15
   Ser Val Ser Pro Gly Glu Arg Ala Thr Leu Ser Cys Arg Ala Ser Gln
               20                  25                  30
   Ser Val Ser Ser Asn Leu Ala Trp Tyr Gln Gln Lys Pro Gly Gln Ala
           35                  40                  45
   Pro Arg Leu Leu Ile Tyr Gly Thr Ser Thr Arg Ala Thr Gly Ile Pro
       50                  55                  60
   Ala Arg Phe Ser Gly Ser Gly Ser Gly Thr Glu Phe Thr Leu Thr Ile
   65                  70                  75                  80
   Ser Ser Leu Gln Ser Glu Asp Ser Ala Val Tyr Phe Cys Gln Gln Tyr
                   85                  90                  95
   Asn Asn Trp Pro Leu Tyr Thr Phe Gly Gln Gly Thr Lys Val Glu Ile
               100                 105                 110
   Lys Arg Thr
           115
<210> 47
   <211> 126
   <212> PRT
   <213> Artificial
<220>
   <223> Synthetic polypeptide (WR05 heavy)
<400> 47
   Thr Gly Val His Ser Gln Val Gln Leu Gln Glu Ser Gly Pro Gly Leu
   1               5                   10                  15
   Val Lys Pro Ser Glu Thr Leu Ser Leu Thr Cys Ser Val Ser Gly Gly
   20 25 30
   Ser Val Ser Ser Asn Gly His Phe Trp Ser Trp Ile Arg Leu Pro Pro
   35 40 45
   Gly Lys Gly Leu Glu Trp Ile Gly Tyr Val Tyr Asn Thr Gly Thr Ser
   50 55 60
   Gly Tyr Ser Pro Ser Leu Lys Ser Arg Val Thr Ile Ser Val Asp Thr
   65 70 75 80
   Ser Lys Asn Gln Phe Ser Leu Thr Leu Arg Ser Val Thr Ala Ala Asp
   85 90 95
   Thr Ala Ile Tyr Tyr Cys Ala Arg Gly Leu Thr Gly Asn Tyr Pro Ser
   100 105 110
   His Trp Gly Gln Gly Thr Leu Val Thr Val Ser Ser Ala Ser
   115 120 125
<210> 48
   <211> 114
   <212> PRT
   <213> Artificial
<220>
   <223> Synthetic polypeptide (WR05 light)
<400> 48
   Thr Gly Val His Ser Asp Ile Gln Met Thr Gln Ser Pro Ser Ser Val
   1               5                   10                  15
   Ser Ala Ser Val Gly Asp Arg Val Thr Ile Thr Cys Arg Ala Ser Arg
               20                  25                  30
   Gly Val Ser Thr Trp Leu Ala Trp Tyr Gln Gln Lys Pro Gly Glu Ala
           35                  40                  45
   Pro Lys Leu Leu Ile Tyr Ala Ser Ser Arg Leu Glu Gly Gly Val Pro
       50                  55                  60
   Ala Arg Phe Ser Gly Ser Gly Ser Gly Thr Asp Phe Thr Leu Thr Ile
   65                  70                  75                  80
   Ser Ser Leu Gln Pro Glu Asp Phe Ala Thr Tyr Tyr Cys Gln Gln Gly
                   85                  90                  95
   Asn Ser Phe Pro Leu Thr Phe Gly Gly Gly Thr Lys Val Glu Ile Lys
                   100                 105                 110
   Arg Thr
<210> 49
   <211> 126
   <212> PRT
   <213> Artificial
<220>
   <223> Synthetic polypeptide (WR06 heavy)
<400> 49
   Thr Gly Val His Ser Gln Leu Gln Leu Gln Glu Ser Gly Pro Gly Leu
   1               5                   10                  15
   Val Lys Pro Ser Glu Thr Leu Ser Leu Thr Cys Thr Val Ser Gly Gly
               20                  25                  30
   Ser Val Ser Ser Ser Ala Tyr Trp Trp Ala Trp Ile Arg Gln Pro Pro
           35                  40                  45
   Gly Gly Gly Leu Glu Trp Ile Gly His Ile Tyr Tyr Phe Gly Asn Lys
       50                  55                  60
   Tyr Tyr Lys Ser Ser Leu Glu Ser Arg Val Thr Ile Ser Leu Asp Ala
   65                  70                  75                  80
   Ser Gln Asn Gln Phe Ser Leu Lys Leu Thr Ser Val Thr Ala Ala Asp
                   85                  90                  95
   Thr Ala Leu Tyr Tyr Cys Ala Arg Val Asp Thr Ala Leu Ala Phe Asp
               100                 105                 110
   Phe Trp Gly Gln Gly Thr Met Val Thr Val Ser Ser Ala Ser
           115                 120                 125
<210> 50
   <211> 114
   <212> PRT
   <213> Artificial
<220>
   <223> Synthetic polypeptide (WR06 light)
<400> 50
   Thr Gly Val His Ser Asp Ile Gln Met Thr Gln Ser Pro Ser Ser Leu
   1               5                   10                  15
   Ser Ala Ser Val Gly Asp Arg Val Thr Ile Thr Cys Arg Ala Ser Gln
               20                  25                  30
   Tyr Val Ser Ser Ser Leu Asn Trp Tyr Gln Gln Lys Pro Gly Lys Ala
           35                  40                  45
   Pro Thr Leu Leu Ile Tyr Leu Ala Ser Asn Leu Arg Ser Gly Val Pro
       50                  55                  60
   Ser Arg Phe Ser Gly Ser Glu Ser Gly Thr Asp Phe Thr Leu Thr Ile
   65                  70                  75                  80
   Asn Ser Leu Gln Pro Glu Asp Val Ala Thr Tyr Phe Cys Gln Gln Ser
                   85                  90                  95
   Tyr Ser Leu Pro Arg Thr Phe Gly Pro Gly Thr Lys Val Asp Ile Lys
               100                 105                 110
   Arg Thr
<210> 51
   <211> 135
   <212> PRT
   <213> Artificial
<220>
   <223> Synthetic polypeptide (WR07 heavy)
<400> 51
   Thr Gly Val His Ser Gln Leu Gln Leu Gln Glu Ser Gly Pro Gly Leu
   1               5                   10                  15
   Val Lys Pro Ser Glu Thr Leu Ser Leu Thr Cys Ser Val Ser Gly Gly
               20                  25                  30
   Ser Val Ser Ser Thr Thr Tyr Tyr Trp Gly Trp Ile Arg Gln Ser Pro
           35                  40                  45
   Gly Lys Gly Leu Glu Trp Ile Gly Ser Ile Tyr His Ser Gly Lys Thr
       50                  55                  60
   Tyr Tyr Asn Pro Ser Leu Lys Ser Arg Val Thr Ile Ser Val Asp Thr
   65                  70                  75                  80
   Ser Lys Asn Gln Phe Ser Leu Asn Leu Ser Ser Val Thr Ala Ala Asp
                   85                  90                  95
   Thr Ala Val Tyr Tyr Cys Ala Arg Glu Asn Ser His His Tyr Asp Ser
               100                 105                 110
   Ser Gly Tyr Tyr Leu Gly Gly Phe Asp Tyr Trp Gly Gln Gly Thr Leu
           115                 120                 125
   Val Thr Val Ser Ser Ala Ser
       130                 135
<210> 52
   <211> 114
   <212> PRT
   <213> Artificial
<220>
   <223> Synthetic polypeptide (WR07 light)
<400> 52
   Thr Gly Val His Ser Glu Ile Val Leu Thr Gln Ser Pro Gly Thr Leu
   1               5                   10                  15
   Ser Leu Ser Pro Gly Glu Arg Ala Thr Leu Ser Cys Arg Ala Ser Gln
               20                  25                  30
   Ser Val Ser Ser Ser Tyr Leu Ala Trp Tyr Gln Gln Lys Pro Gly Gln
           35                  40                  45
   Ala Pro Arg Leu Leu Ile Tyr Gly Ala Ser Ser Arg Ala Thr Gly Ile
       50                  55                  60
   Pro Asp Arg Phe Ser Gly Ser Gly Ser Gly Thr Asp Phe Thr Leu Thr
   65                  70                  75                  80
   Ile Ser Arg Leu Glu Pro Glu Asp Phe Ala Val Tyr Phe Cys Gln His
                   85                  90                  95
   Tyr Gly Ser Pro Ser Thr Phe Gly Gln Gly Thr Lys Val Glu Ile Lys
               100                 105                 110
   Arg Thr
<210> 53
   <211> 126
   <212> PRT
   <213> Artificial
<220>
   <223> Synthetic polypeptide (WR08 heavy)
<400> 53
   Thr Gly Val His Ser Gln Val Gln Leu Gln Glu Ser Gly Pro Gly Leu
   1               5                   10                  15
   Val Lys Pro Ser Gln Thr Leu Ser Leu Thr Cys Thr Val Ser Gly Gly
               20                  25                  30
   Ser Ile Ser Ser Gly Tyr Tyr Trp Ser Trp Ile Arg Gln His Pro Gly
           35                  40                  45
   Lys Gly Leu Asp Cys Ile Gly Tyr Ile His Tyr Thr Gly Thr Thr Tyr
       50                  55                  60
   Tyr Asn Pro Ser Leu Lys Ser Arg Leu Thr Ile Ser Val Asp Thr Ser
   65                  70                  75                  80
   Lys Asn Gln Phe Ser Leu Asn Leu Thr Ser Val Thr Ala Ala Asp Thr
                   85                  90                  95
   Ala Val Tyr Tyr Cys Ala Arg Glu Glu Tyr Thr Thr Ser Ser Val Asp
               100                 105                 110
   Tyr Trp Gly Arg Gly Thr Leu Val Thr Val Ser Ser Ala Ser
           115                 120                 125
<210> 54
   <211> 119
   <212> PRT
   <213> Artificial
<220>
   <223> Synthetic polypeptide (WR08 light)
<400> 54
   Thr Gly Val His Gly Asp Val Val Met Thr Gln Ser Pro Leu Ser Leu
   1               5                   10                  15
   Pro Val Thr Leu Gly Gln Pro Ala Ser Ile Ser Cys Arg Ser Ser Glu
               20                  25                  30
   Ser Leu Val Ser Val Asp Gly Asn Thr Tyr Leu Asn Trp Phe His Gln
           35                  40                  45
   Arg Pro Gly Gln Ser Pro Arg Arg Leu Ile Tyr Lys Val Ser Asn Arg
       50                  55                  60
   Asp Ser Gly Val Pro Asp Arg Phe Ser Gly Ser Gly Ser Gly Thr Asp
   65                  70                  75                  80
   Phe Thr Leu Lys Ile Ser Arg Val Glu Ala Glu Asp Val Gly Ile Tyr
                   85                  90                  95
   Tyr Cys Met Gln Ala Thr His Trp Leu Arg Thr Phe Gly Gln Gly Thr
               100                 105                 110
   Arg Leu Glu Ile Lys Arg Thr
           115
<210> 55
   <211> 126
   <212> PRT
   <213> Artificial
<220>
   <223> Synthetic polypeptide (WR09 heavy)
<400> 55
   Thr Gly Val His Ser Gln Val Gln Leu Gln Glu Ser Gly Pro Gly Leu
   1               5                   10                  15
   Val Lys Pro Ser Gln Thr Leu Ser Leu Thr Cys Thr Val Ser Gly Gly
               20                  25                  30
   Ser Ile Ser Ser Gly Tyr Tyr Trp Ser Trp Ile Arg Gln His Pro Gly
           35                  40                  45
   Lys Gly Leu Glu Trp Ile Gly Tyr Ile His Tyr Thr Gly Thr Thr Tyr
       50                  55                  60
   Tyr Asn Pro Ser Leu Lys Ser Arg Val Thr Ile Ser Val Asp Thr Ser
   65                  70                  75                  80
   Lys Asn Gln Phe Ser Leu Asn Leu Thr Ser Val Thr Ala Ala Asp Thr
                   85                  90                  95
   Ala Val Tyr Tyr Cys Ala Arg Glu Glu Tyr Thr Thr Ser Ser Val Asp
               100                 105                 110
   Tyr Trp Gly Arg Gly Thr Leu Val Thr Val Ser Ser Ala Ser
           115                 120                 125
<210> 56
   <211> 117
   <212> PRT
   <213> Artificial
<220>
   <223> Synthetic polypeptide (WR09 light)
<400> 56
   Thr Gly Val His Ser Glu Ile Val Leu Thr Gln Ser Pro Gly Thr Leu
   1               5                   10                  15
   Ser Leu Ser Pro Gly Glu Arg Ala Thr Leu Ser Cys Arg Ala Ser Gln
               20                  25                  30
   Arg Val Ser Ser Gly Phe Leu Ala Trp Tyr Gln Gln Lys Pro Gly Gln
           35                  40                  45
   Ala Pro Arg Leu Leu Ile Tyr Gly Ala Ser Ser Arg Ala Thr Gly Ile
       50                  55                  60
   Pro Asp Arg Phe Ser Gly Ser Gly Ser Gly Thr Asp Phe Thr Leu Thr
   65                  70                  75                  80
   Ile Ser Arg Leu Glu Pro Glu Asp Phe Ala Val Tyr Tyr Cys Gln Gln
                   85                  90                  95
   Tyr Glu Ser Ser Pro Ser Pro Tyr Asn Phe Gly Gln Gly Thr Lys Leu
               100                 105                 110
   Glu Ile Lys Arg Thr
           115
<210> 57
   <211> 133
   <212> PRT
   <213> Artificial
<220>
   <223> Synthetic polypeptide (WR10 heavy)
<400> 57
   Thr Gly Val His Ser Gln Val Gln Leu Gln Glu Ser Gly Pro Gly Leu
   1 5 10 15
   Val Lys Pro Ser Gly Thr Leu Ser Leu Thr Cys Ala Val Ser Gly Gly
   20 25 30
   Ser Ile Ser Asn Asn Lys Trp Trp Asn Trp Val Arg Gln Ser Pro Gly
   35 40 45
   Lys Gly Leu Glu Trp Ile Gly Glu Ile Tyr His Ser Gly Gly Thr Asn
   50 55 60
   Tyr Asn Pro Ser Leu Lys Ser Arg Val Thr Ile Ser Val Asp Lys Ser
   65 70 75 80
   Lys Asn Leu Phe Ser Leu Lys Leu Ser Ser Val Thr Ala Ala Asp Thr
   85 90 95
   Ala Val Tyr Tyr Cys Ala Ser Ala Thr Thr Met Val Arg Gly Leu Ser
   100 105 110
   Leu Tyr Tyr Tyr Gly Leu Asp Val Trp Gly Pro Gly Thr Thr Val Thr
   115 120 125
   Val Ser Ser Ala Ser
   130
<210> 58
   <211> 115
   <212> PRT
   <213> Artificial
<220>
   <223> Synthetic polypeptide (WR10 light)
<400> 58
   Thr Gly Val His Ser Glu Ile Val Leu Thr Gln Ser Pro Gly Thr Leu
   1 5 10 15
   Ser Leu Ser Pro Gly Glu Arg Ala Ala Leu Ser Cys Arg Ala Ser Gln
   20 25 30
   Ser Leu Ile Gly Ser Phe Leu Ala Trp Tyr Gln Gln Lys Pro Gly Gln
   35 40 45
   Ala Pro Arg Leu Leu Ile Tyr His Thr Ser Asn Arg Ala Ser Gly Ile
   50 55 60
   Pro Asp Arg Phe Ser Gly Gly Gly Phe Gly Thr Asp Phe Thr Leu Thr
   65 70 75 80
   Ile Ser Arg Leu Glu Pro Glu Asp Phe Ala Val Tyr Tyr Cys Gln Gln
   85 90 95
   Tyr Asp Ser Ser Pro Ile Thr Phe Gly Gln Gly Thr Arg Leu Glu Ile
   100 105 110
   Lys Arg Thr
   115
<210> 59
   <211> 133
   <212> PRT
   <213> Artificial
<220>
   <223> Synthetic polypeptide (WR11 heavy)
<400> 59
   Thr Gly Val His Ser Gln Val Gln Leu Gln Glu Ser Gly Pro Gly Leu
   1               5                   10                  15
   Val Lys Pro Ser Gly Thr Leu Ser Leu Thr Cys Ala Val Ser Gly Gly
               20                  25                  30
   Ser Ile Ser Asn Asn Lys Trp Trp Asn Trp Val Arg Gln Ser Pro Gly
           35                  40                  45
   Lys Gly Leu Glu Trp Ile Gly Glu Ile Tyr His Ser Gly Gly Thr Asn
       50                  55                  60
   Tyr Asn Pro Ser Leu Lys Ser Arg Val Thr Ile Ser Val Asp Lys Ser
   65                  70                  75                  80
   Lys Asn Leu Phe Ser Leu Lys Leu Ser Ser Val Thr Ala Ala Asp Thr
                   85                  90                  95
   Ala Val Tyr Tyr Cys Ala Ser Ala Thr Thr Met Val Arg Gly Leu Ser
               100                 105                 110
   Leu Tyr Tyr Tyr Gly Leu Asp Val Trp Gly Gln Gly Thr Thr Val Thr
           115                 120                 125
   Val Ser Ser Ala Ser
       130
<210> 60
   <211> 116
   <212> PRT
   <213> Artificial
<220>
   <223> Synthetic polypeptide (WR11 light)
<400> 60
   Thr Gly Val His Ser Glu Ile Val Leu Thr Gln Ser Pro Gly Thr Leu
   1               5                   10                  15
   Ser Leu Ser Pro Gly Glu Arg Ala Thr Leu Ser Cys Arg Ala Ser Gln
               20                  25                  30
   Ser Val Ser Ser Phe Leu Ala Trp Tyr Gln Gln Lys Pro Gly Gln Ala
           35                  40                  45
   Pro Arg Leu Leu Ile Tyr Asp Ala Ser Asn Arg Ala Thr Gly Ile Pro
           50                  55                  60
   Ala Arg Phe Ser Gly Ser Gly Ser Gly Thr Asp Phe Thr Leu Thr Ile
   65                  70                  75                  80
   Ser Ser Leu Glu Pro Glu Asp Phe Ala Val Tyr Tyr Cys Gln Gln Arg
                   85                  90                  95
   Thr Asn Trp Pro Pro Ser Leu Thr Phe Gly Gly Gly Thr Lys Val Glu
               100                 105                 110
   Ile Lys Arg Thr
           115
<210> 61
   <211> 136
   <212> PRT
   <213> Artificial
<220>
   <223> Synthetic polypeptide (WR12 heavy)
<400> 61
   Thr Gly Val His Ser Gln Val Gln Leu Gln Glu Ser Gly Pro Gly Leu
   1 5 10 15
   Val Lys Pro Ser Gln Thr Leu Ser Leu Thr Cys Thr Val Ser Gly Asp
   20 25 30
   Ser Val Ser Ser Asn Asp His Tyr Trp Ser Trp Ile Arg Gln Pro Pro
   35 40 45
   Gly Gln Gly Leu Glu Trp Ile Gly Tyr Ile Ser His Gly Gly Thr Thr
   50 55 60
   Tyr Tyr Asn Pro Ser Leu Lys Ser Arg Val Thr Met Ser Ile Asp Thr
   65 70 75 80
   Ser Thr Asn Gln Phe Ser Leu Arg Val Thr Ser Val Arg Ala Ala Asp
   85 90 95
   Met Ala Val Tyr Phe Cys Ala Arg Ala Pro Ala Pro Ile Thr Thr Phe
   100 105 110
   Gly Met Val Thr Pro Val Pro Tyr Phe His Ser Trp Gly Gln Gly Thr
   115 120 125
   Leu Val Thr Val Ser Ser Ala Ser
   130 135
<210> 62
   <211> 119
   <212> PRT
   <213> Artificial
<220>
   <223> Synthetic polypeptide (WR12 light)
<400> 62
   Thr Gly Val His Gly Asp Ile Val Met Thr Gln Ser Pro Leu Ser Leu
   1               5                   10                  15
   Pro Val Ser Pro Gly Glu Pro Ala Ser Ile Ser Cys Arg Ser Ser Gln
               20                  25                  30
   Ser Leu Leu His Ser Asn Gly Tyr Asn Tyr Leu Ser Trp Tyr Leu Gln
           35                  40                  45
   Lys Pro Gly Gln Ser Pro Gln Leu Leu Ile Phe Ser Ser Ser Ile Arg
       50                  55                  60
   Ala Ser Gly Val Pro Asp Arg Phe Ser Gly Ser Gly Ser Gly Thr Asp
   65                  70                  75                  80
   Phe Thr Leu Thr Ile Asn Arg Val Glu Ala Glu Asp Val Gly Val Tyr
                   85                  90                  95
   Tyr Cys Met Gln Ala Leu Gln Thr Pro Leu Thr Phe Gly Gly Gly Thr
               100                 105                 110
   Lys Leu Glu Ile Lys Arg Thr
           115
<210> 63
   <211> 136
   <212> PRT
   <213> Artificial
<220>
   <223> Synthetic polypeptide (WR13 heavy)
<400> 63
   Thr Gly Val His Ser Gln Val Gln Leu Gln Glu Ser Gly Pro Gly Leu
   1               5                   10                  15
   Val Lys Pro Ser Gln Thr Leu Ser Leu Thr Cys Thr Val Ser Gly Asp
               20                  25                  30
   Ser Val Ser Ser Asn Asp His Tyr Trp Ser Trp Ile Arg Gln Pro Pro
   35 40 45
   Gly Gln Gly Leu Glu Trp Ile Gly Tyr Ile Ser His Gly Gly Thr Thr
   50 55 60
   Tyr Tyr Asn Pro Ser Leu Lys Ser Arg Val Thr Met Ser Ile Asp Thr
   65 70 75 80
   Ser Thr Asn Gln Phe Ser Leu Arg Val Thr Ser Val Arg Ala Ala Asp
   85 90 95
   Met Ala Val Tyr Phe Cys Ala Arg Ala Pro Ala Pro Ile Thr Thr Phe
   100 105 110
   Gly Met Val Thr Pro Val Pro Tyr Phe His Ser Trp Gly Gln Gly Thr
   115 120 125
   Leu Val Thr Val Ser Ser Ala Ser
   130 135
<210> 64
   <211> 119
   <212> PRT
   <213> Artificial
<220>
   <223> Synthetic polypeptide (WR13 light)
<400> 64
   Thr Gly Val His Gly Asp Ile Val Met Thr Gln Ser Pro Leu Ser Leu
   1               5                   10                  15
   Pro Val Ser Pro Gly Glu Ser Ala Ser Ile Ser Cys Arg Ser Ser Gln
               20                  25                  30
   Ser Leu Leu His Ser Asn Gly Tyr Asn Tyr Leu Ser Trp Tyr Leu Gln
           35                  40                  45
   Lys Pro Gly Gln Ser Pro Gln Leu Leu Ile Phe Ser Ser Ser Ile Arg
       50                  55                  60
   Ala Ser Gly Val Pro Asp Arg Phe Ser Gly Ser Gly Ser Gly Thr Asp
   65                  70                  75                  80
   Phe Thr Leu Thr Ile Asn Arg Val Glu Ala Glu Asp Val Gly Val Tyr
                   85                  90                  95
   Tyr Cys Met Gln Ala Leu Gln Thr Pro Leu Thr Phe Gly Gln Gly Thr
               100                 105                 110
   Lys Leu Glu Ile Lys Arg Thr
           115
<210> 65
   <211> 392
   <212> DNA
   <213> Artificial
<220>
   <223> Synthetic oligonucleotide (AJL01 heavy)
<400> 65
   accggtgtac attcccaggt gcagctacag cagtggggcg caggactgtt gaagccttcg 60
   gagaccctgt ccctcacctg cgctgtctat ggtgggtcct tcaatgaatt ctactggagc 120
   tggatccgtc agcccgcacg gaagggcctg gagtggattg gagaaatcag tcatagcgga 180
   agagccaact acaacccgtc cctcaagagt cgcgtcaccc tgtctgtaga caggtccaag 240
   aaccagttct ccctgaacct gagccctgtg gccgccgcgg acacagctgt ctattactgt 300
   gcgcgacggg agatagtcgt aactgttcgg gggcgtcgtg cttttgatat ctggggccaa 360
   gggacaatgg tcaccgtctc ttcagcgtcg ac 392
<210> 66
   <211> 345
   <212> DNA
   <213> Artificial
<220>
   <223> Synthetic oligonucleotide (AJL01 light)
<400> 66
   accggtgtac attcagaaat tgtgttgacg cagtctccag gcaccctgtc tttgtctcca      60
   ggggaaagag ccgccctctc ctgcagggcc agtcagagtc ttatcggcag cttcttagcc     120
   tggtaccagc agaaacctgg ccaggctccc aggctcctca tctatcatac atccaacagg     180
   gcctctggca tcccagacag gttcagtggc ggtgggtttg ggacagactt cactctcacc     240
   atcagcagac tggagcctga agattttgca gtttattact gtcaacagta tgatagctca     300
   ccgatcacct tcggccaagg gacacgactg gagattaaac gtacg                     345
<210> 67
   <211> 383
   <212> DNA
   <213> Artificial
<220>
   <223> Synthetic oligonucleotide (AJL02 heavy)
<400> 67
   accggtgtac attcccaggt gcagctgcag gagtcgggcc caggactggt gaagccttca      60
   cagaccctgt ccctcacctg tactgtctct ggtggctcca tcagcagtgg tggtcactac     120
   tggagctgga tccgccagtc cccagggaag ggcctggagt ggattgggaa cgtctattat     180
   agtggaagca cctactacac cccgtccctc gacagccgac ttaccatatc attagacacg     240
   tctaagaacc agttctccct gaggctgagt aatgtgactg tcgcggacac ggccgtctat     300
   tactgtgcga gaggtagaaa ttgggagggc gaattcgacc cctggggcca aggaaccctg     360
   gtcaccgtct cctcagcgtc gac                                             383
<210> 68
   <211> 342
   <212> DNA
   <213> Artificial
<220>
   <223> Synthetic oligonucleotide (AJL02 light)
<400> 68
   accggtgtac attctgacat ccagatgacc cagtctccgt cctccctgtc tgcgtcttta 60
   ggagacagag tcaccatcac ttgccgggca agtcagggca ttagcagctc tgtaaattgg 120
   tttcagcaga aaccagggaa agcccctgaa ctcctgatct atgctgcatc cactttgcaa 180
   agtggggtcc catcaagatt cagtggcagt ggatctggga cagatttcac tctcaccgtc 240
   agcagtctgc aacctgaaga ttttgcaact tactactgtc agcagagtta cagtccccct 300
   cgaacttttg gccaggggac caagctggaa atcaaacgta cg 342
<210> 69
   <211> 404
   <212> DNA
   <213> Artificial
<220>
   <223> Synthetic oligonucleotide (AJL03 heavy)
<400> 69
   accggtgtac attcccagct gcagctgcag gagtcgggcc caggactggt gaagccttcg 60
   gagaccctgt ccctcacgtg cactgtctct ggtgcctcca tcagcagtag tcgttcctac 120
   tggggctgga tccgccagcc cccagggaag gggctggagt ggattgggag tatgtatcaa 180
   agtgggagca cttactacag tccgtccctc aagagtcgag tcaccatatc catggacacg 240
   tccaagaacc agttctccct aaacctgacg tctgtgaccg ccgcggacac ggctgtgtat 300
   ttctgtgcga gacattcgaa ccccggaacg gcgaacaaat tgcgtttggg ggagttttcg 360
   ccctggggcc agggaaccct ggtcaccgtc tcctcagcgt cgac 404
<210> 70
   <211> 342
   <212> DNA
   <213> Artificial
<220>
   <223> Synthetic oligonucleotide (AJL03 light)
<400> 70
   accggtgtac attctgacat ccagatgacc cagtctccat cctccctgtc tgcatctgta 60
   ggagacagag tcaccatcac ttgccaggcg agtcaggaca ttaacaacta tttaaattgg 120
   tttcagcagc aaccagggaa agcccctaag ctgctgatct acgatgcatc caaattgcaa 180
   atgggggtcc catcaaggtt cagtggaagt gcatctggga cagattttac ttttaccatc 240
   agcagcctgc agcctgaaga tattggcaca tattactgtc aacagtatta taatctcccg 300
   tacacttttg gccaggggac caagctggag atcaaacgta cg 342
<210> 71
   <211> 377
   <212> DNA
   <213> Artificial
<220>
   <223> Synthetic oligonucleotide (AJL04 heavy)
<400> 71
   accggtgtac attcccaggt gcagctgcag gagtcgggcc caggactggt gaagccttcg        60
   gagaccctgt ccctcacctg cactgtctct ggtgactcca tcagaagtaa cttctggacc       120
   tggatccggc agtccccagg gaggggactg gagtggattg ggtatttctc ttacagtggg       180
   ggcatcaact acagcccctc cctcaagagt cgcgtcacca tttcagtgga cacatccaag       240
   aaccagttct ccctaaaact gacctctgtg accgcggcgg acgcggccgt atattactgt       300
   gcgagagatc ccaacggtga ctacgaagtt aactggtggg gccaaggaac cctggtcacc       360
   gtctcctcag cgtcgac                                                      377
<210> 72
   <211> 342
   <212> DNA
   <213> Artificial
<220>
   <223> Synthetic oligonucleotide (AJL04 light)
<400> 72
   accggtgtac attctgacat ccagatgacc cagtctccat cttcactgtc tgcatctgta        60
   ggagacagag tcaccatcac ttgtcgggcg agtcaggaca ttattattta tttagcctgg       120
   tttcagcaga gaccagggaa agcccctagg tccctgatct attctgcatc cactttgcag       180
   agtggggtcc catcaaaatt cagcggcagt ggatctggga catatttcac tctcaccatc       240
   agcagcctgc agcctgaaga ttctgcaact tattactgcc aacaatataa aagttatccc       300
   atcaccttcg gccaagggac acgactggag attaaacgta cg                          342
<210> 73
   <211> 389
   <212> DNA
   <213> Artificial
<220>
   <223> Synthetic oligonucleotide (AJL05 heavy)
<400> 73
   accggtgtac attcccagct gcagctgcag gagtcgggcc caggactggt gaagccttcg 60
   gagaccctgt ccctcacctg cactgtctct ggtggctcca tcagaaatag taattactac 120
   tgggactgga tccgccagcc cccaggaaag gggctggagt ggattgggag tggctattat 180
   agtgggagcg cctactacca ttcgtccctc aagagtcgag tcagcatatc cgtagacacg 240
   tccaagaacc agttttccct taatctgacc tctgtgaccg ccgcagacac ggctttttat 300
   tactgtgcga gacgttccta ttattatgct tcggggagcc acgactactg gggccaggga 360
   accctggtca ccgtctcctc agcgtcgac 389
<210> 74
   <211> 342
   <212> DNA
   <213> Artificial
<220>
   <223> Synthetic oligonucleotide (AJL05 light)
<400> 74
   accggtgtac attctgacat ccagatgacc cagtctccat cctcactgtc tgcatctgta 60
   ggagacagag tcaccatcac ttgtcgggcg agtcaggaca ttagcagtta cttagcctgg 120
   tttcagcaga aaccagggaa agcccctaag tccctgatct atggtgcttc cagtttgcag 180
   agtggggtcc catcaaaatt cagcggcagt ggatctggga cagatttcac tctcaccatc 240
   agcggcctgc agcctgaaga ttttgcaact tatcactgcc aacaatacag gagtttccct 300
   atcactttcg gccaagggac acgactggag attaaacgta cg 342
<210> 75
   <211> 395
   <212> DNA
   <213> Artificial
<220>
   <223> Synthetic oligonucleotide (AJL06 heavy)
<400> 75
   accggtgtac attcccagct gcagctgcag gagtccggct caggactggt gaagccttca 60
   cagaccctgt ccctcacctg cgctgtctct ggtggctcca tcaacactgg caattacttc 120
   tggagctgga tccggcagcc accagggaag ggcctggagt ggattgggta catgtttcga 180
   agtacgagta cctactacaa cccgtccctc aagggtcgag tcaccatttc aggaggcacg 240
   tccctgagcc agttctccct gagactggac tctgtgactg ccgcggacac ggccatctac 300
   tactgtgcga gaggacgtta ttattgtggt gttaattgcc atccctttga ctcctggggc 360
   caaggaaccc tggtcaccgt ctcctcagcg tcgac 395
<210> 76
   <211> 339
   <212> DNA
   <213> Artificial
<220>
   <223> Synthetic oligonucleotide (AJL06 light)
<400> 76
   accggtgtac attctgacat ccagatgacc cagtctccat cctccctgtc tgcatctgta 60
   ggagacagag tcatcatcac ttgccaggcg agccaagaca ttagcatcta tttaaattgg 120
   tatcaggtga aaccagggaa agcccctaaa ctcctgatct acgatgcctc caatttgcaa 180
   gcaggggtcc catcaaggtt cagtggaagt ggatcaggga cagattttag tttcaccatc 240
   agcagcctgc agcctgaaga tgtggcagca tattactgtc atcagtatga ttctttaccg 300
   agttttggcc aggggaccaa gctggaaatc aaacgtacg 339
<210> 77
   <211> 383
   <212> DNA
   <213> Artificial
<220>
   <223> Synthetic oligonucleotide (AJL07 heavy)
<400> 77
   accggtgtac attcccaggt gcagctgcag gagtcgggcc caggactggt gaagccttcg 60
   gagaccctgt ccctcacctg caatgtctct ggtggctcca tcaacaacta ttactggagt 120
   tggatccggc agcccccagg gaagggactg gagtggattg gttatatcta ttacaatggg 180
   aatattaatt acaacccttc cctcaagagt cgagtcacca tatcaagaga catgtccaag 240
   aaccagttct ccctgaacct gcggtctgtg accgctgcgg acacggccgt gtattactgt 300
   ggaattggat atagtgcggt ggcagctggt acagttgact actggggcca tggcaccctg 360
   gtcaccgtct cctcagcgtc gac 383
<210> 78
   <211> 342
   <212> DNA
   <213> Artificial
<220>
   <223> Synthetic oligonucleotide (AJL07 light)
<400> 78
   accggtgtac attcagccat ccagttgacc cagtctccat cctccctgtc tgcatctgtg      60
   ggagacagag tcaccatcac ttgccgggca agtcagggca ttagcagtgg tttagcctgg     120
   tatcagcagg aaccagggaa agctcctaaa ctcctgatct atgatgcctc cactttggaa     180
   agtggggtcc catcaagatt cagcggcagt ggatctgcaa tagatttcac tctcaccatc     240
   agcagtctgc agcctgaaga ttttgcaact tattactgtc aacagtttaa tactttcccg     300
   tacacttttg gccaggggac caagctggag atcaaacgta cg                        342
<210> 79
   <211> 395
   <212> DNA
   <213> Artificial
<220>
   <223> Synthetic oligonucleotide (AJL08 heavy)
<400> 79
   accggtgtac attcccaggt gcagctgcag gagtcgggcc caggactggt gaagccttcg      60
   gagaccctgt ccctcacctg cgctgtctct ggtttctcca tcaccagtgg ttactactgg     120
   ggctggatcc ggcagccccc cgggaagggg ctggagtgga tagggagtat ctatcatact     180
   gggactacct actacaaccc gtccctcaag agtcgagtca ccatatcagt agacacgtcc     240
   aagaaccagt tctccctgaa cctaaactct gtgaccgccg cagacacggc cttttattac     300
   tgtgcgagag atcctctatt cccggggcgg aacctactct ccgtttttga caactggggc     360
   cagggcaccc tggtcaccgt ctcctcagcg tcgac                               395
<210> 80
   <211> 342
   <212> DNA
   <213> Artificial
<220>
   <223> Synthetic oligonucleotide (AJL08 light)
<400> 80
   accggtgtac attctgacat ccagatgacc cagtctcctt ccaccctgtc tgcatctgta 60
   ggagacagag tcaccatcac ttgccgggcc agtcagagta ttagtacctg gttggcctgg 120
   tatcagcaga aaccagggaa agcccctagg ctcctgatct ataaggcatc tagtttagaa 180
   agtggggtcc catcacgttt cagcggcagt ggatctggga cagaattcac tctcaccatc 240
   agcggcctgc agcctgatga ctttgcaaat tattcctgcc aacaatataa tatttacccg 300
   ttcacttttg gccaggggac caagctggag atcaaacgta cg 342
<210> 81
   <211> 386
   <212> DNA
   <213> Artificial
<220>
   <223> Synthetic oligonucleotide (AJL09 heavy)
<400> 81
   accggtgtac attcccaggt gcagctgcag gagtcgggcc caggactggt gaagccttcg 60
   gagaccctgt ccctcacctg cagtgtctct ggtggctcca tcagtagtta ctactggggc 120
   tggatccggc agtccccagg gaaaggactg gagtgcattg gatacatcta tttcagtggg 180
   agcaccagct acaacccctc cctcaagagt cgagtcacca tatcagtaga cacggccaag 240
   aaccagatct ccctgaacct gacctctgtg accgctgcgg acacggccgt gtatttttgt 300
   gcgagagttt ggggcagcag ctggtacgct aactggttcg acccctgggg ccagggcacc 360
   ctggtcaccg tctcctcagc gtcgac 386
<210> 82
   <211> 342
   <212> DNA
   <213> Artificial
<220>
   <223> Synthetic oligonucleotide (AJL09 light)
<400> 82
   accggtgtac attcagccat ccagttgacc cagtctccat cctccctgtc tgcgtctgtt 60
   ggagacagag tcaccatcac ttgccgggca agtcagggca ttagcagtgc cttagcctgg 120
   tatcagcaca aaccagggaa ggctcctaaa ctcctgatct ttgatgcctc cactttggca 180
   gctggggtcc catccaggtt cagcggcagt ggatctggga cagatttcac tctcaccatc 240
   agcagtctgc agcctgaaga ttttgctact tattactgtc agcagtataa tacttacgtt 300
   ctcactttcg gccaagggac acgactggag attaaacgta cg 342
<210> 83
   <211> 389
   <212> DNA
   <213> Artificial
<220>
   <223> Synthetic oligonucleotide (AJL10 heavy)
<400> 83
   accggtgtac attcccaggt gcagctgcag gagtcgggcc caggactggt gaagccttcg      60
   gagaccctgt ccctcacctg cagtgtctct ggtggcgccg tcagtaatta ctactggagt     120
   tggatccggc agtccgccgg gaagggactg gagtggcttg ggcggatcta tatcaatgga     180
   actacttact acaacccctc cctcaggagc cgggtctcca tgtcagttga cacgtccaag     240
   ggccagttct ccctgaggtt gacctctgtg accgccgcgg acacggccat atattattgt     300
   gcgagatggg gtgccctatt gggcgactac tattacggtt tggacgtctg gggccaaggg     360
   accacggtca ccgtctcctc agcgtcgac                                       389
<210> 84
   <211> 357
   <212> DNA
   <213> Artificial
<220>
   <223> Synthetic oligonucleotide (AJL10 light)
<400> 84
   accggtgtac atggggatat tgtgatgact cagtctccac tctccctgcc cgtcacccct      60
   ggagagccgg cctccatctc ctgcaggtct actcagagcc tcctacacag taatgaatac     120
   atttatttgg attggtacgt gcagaagcca gggcagtctc cacaactcct gatctttttg    180
   gcttctaatc gggcctccgg ggtccctgac aggttcagtg gcagtgcatc aggcacagat     240
   tttacactga aaatcagcag agtggaggct gaggatgttg gggtttatta ctgcatgcaa      300
   gctctagaag ctccgtggac gttcggccaa gggacacgac tggagattaa acgtacg       357
<210> 85
   <211> 380
   <212> DNA
   <213> Artificial
<220>
   <223> Synthetic oligonucleotide (AJL11 heavy)
<400> 85
   accggtgtac attcccagct gcagctgcag gagtcgggcc caggactggt gaagccttcg 60
   gagaccctgt ccctcacctg ctctgtctct ggtggctcca tcaccagtac ttcttattac 120
   tgggggtgga tccgccagtc cccagggaag ggactggagt ggattggcag tgtctattac 180
   agtgggaaca ccttctacaa cgcgtccctc aagagtcgag tcaccatatc catagacacg 240
   tccaagtacc agttctccct gatgctgagg tctgtgaccg ccgcagacac ggctgtgtat 300
   tactgtgcga gacgacatga ttggttctgg ttcgacccct ggggccaagg aaccctggtc 360
   accgtctcct cagcgtcgac 380
<210> 86
   <211> 345
   <212> DNA
   <213> Artificial
<220>
   <223> Synthetic oligonucleotide (AJL11 light)
<400> 86
   accggtgtac attcagaaat tgtgttgacg cagtctccag gcaccctgtc tttgtctcca 60
   ggggaaagag tcaccctctc ctgcagggcc agtcagagtg ttagcagcag ctacttagcc 120
   tggtaccagc agaaacctgg ccaggctccc aggctcctca tctatgattc atccagcagg 180
   gccactggca tcccagacag gttcagtggc agtgggtctg ggacagactt cactctcacc 240
   atcagcagac tggaccctga agattttgcc gtgtattact gtcagcagta tggtagctca 300
   ccaagcactt ttggccaggg gaccaagctg gagatcaaac gtacg 345
<210> 87
   <211> 386
   <212> DNA
   <213> Artificial
<220>
   <223> Synthetic oligonucleotide (AJL12 heavy)
<400> 87
   accggtgtac attcccagct gcagctgcag gagtcgggcc caggactggt gaagccttcg 60
   gagaccctgt ccctcacctg cactgtctct ggtggctccg tcaccagtag tgattactac 120
   tgggcctgga tccgccagcc cccagggaag gggccggagt ggattgggag tatctctaat 180
   agtgggaaca cgtattacag tccgtccctc aagagtcgag tctccatatc gggagacacg 240
   tccaagaagc agttctccct gaacctgagc tctgtgaccg acgcagacac ggctgtgtat 300
   tactgtacga gacacggtca ctacgtttca ggggggctgg gcccctgggg ccaaggaacc 360
   ctggtcaccg tctcctcagc gtcgac 386
<210> 88
   <211> 345
   <212> DNA
   <213> Artificial
<220>
   <223> Synthetic oligonucleotide (AJL12 light)
<400> 88
   accggtgtac attcagaaat tgtgttgacg cagtctccag gcaccctgtc tttgtctcca 60
   ggggaaagag ccaccctctc ctgcagggcc agtcagagtg ttggcagtta ctacttagcc 120
   tggtaccagc agaaacctgg ccaggctccc aggctcctca tccacggtgc atccagcagg 180
   gccactggca ccccagacag gttcagtggc agtgggtctg ggacagactt cactctcacc 240
   atcagcaaac tggagcctga agattttgca ctgtattact gtcagcagta tggtccctca 300
   ccttggacgt tcggccaagg gaccaaggtg gaaatcaaac gtacg 345
<210> 89
   <211> 383
   <212> DNA
   <213> Artificial
<220>
   <223> Synthetic oligonucleotide (AJL13 heavy)
<400> 89
   accggtgtac attcccaggt gcagctgcag gagtcgggcc caggactggt gaagccttcg 60
   gagaccctgt ccctcacctg caatgtctct ggtggctcca tcaacaacta ttactggagt 120
   tggatccggc agcccccagg gaagggactg gagtggattg gttatatcta ttacaatggg 180
   aatattaatt acaacccttc cctcaagagt cgagtcacca tatcaagaga catgtccaag 240
   aaccagttct ccctgaacct gcggtctgtg accgctgcgg acacggccgt gtattactgt 300
   ggaattggat atagtgcggt ggcagctggt acagttgact actggggcca gggaaccctg 360
   gtcaccgtct cctcagcgtc gac 383
<210> 90
   <211> 357
   <212> DNA
   <213> Artificial
<220>
   <223> Synthetic oligonucleotide (AJL13 light)
<400> 90
   accggtgtac atggggatgt tgtgatgact cagtctccac tctccctgcc cgtcaccctt 60
   ggacaaccgg cctccatctc ctgcaggtct agtcaaagcc tcgtacacag gaatggaaac 120
   acctacttga attggtttca gcagaggcca ggccaatcgc caaggcgcct aatttataca 180
   gtttctaagc ggggctctgg ggtcccagac agattcagcg gcagtgggtc aggcactgat 240
   ttcacactga aaatcagcag ggtggaggct gaagatgttg gggactatta ctgcatgcaa 300
   ggtacacact ggccttggac gtccggccaa gggacacgac tggagattaa acgtacg 357
<210> 91
   <211> 407
   <212> DNA
   <213> Artificial
<220>
   <223> Synthetic oligonucleotide (AJL14 heavy)
<400> 91
   accggtgtac attcccaggt gcagctgcag gagtcgggcc caggactggt gaagccttca 60
   cagaccctgt ccctcacctg cactgtctct ggtggctccc tcagtagtgt taattactac 120
   tggaactgga tccggcagcc cgccgggaag ggactggagt ggatggggcg tatctatgcc 180
   agcgggtaca ccacctacaa cccgtccttc cagagtcgag tcaccatatc actggacccg 240
   tccaagaacc agatctccct gaaggtgact tctctgaccg ccgcagacac ggccatctat 300
   tactgtgcga gacacgacct tgggcattgt agtagtacga gctgttacct cagttggttc 360
   gacgcctggg gccaagggac cacggtcacc gtctcctcag cgtcgac 407
<210> 92
   <211> 360
   <212> DNA
   <213> Artificial
<220>
   <223> Synthetic oligonucleotide (AJL14 light)
<400> 92
   accggtgtac attcggacat cgtgatgacc cagtctcccg actccctggc tgtgtctctg 60
   ggcgagaggg ccaccatcaa ctgcaggtcc agccagacta ttttcttcag ccccaacaat 120
   aacaaccact tagcttggta ccagcaaaaa ccagggcagc ctcctaggct gctcatttac 180
   tgggcatcta cccgggaatc cggggtccct gaccgattca gtggcagcgg gtctgggaca 240
   gatttcactc tcaccatcag cggcctgcag gctgaagatg tggcagttta ttactgtcag 300
   caatattata gtcttcccta cacttttggc caggggacca agctggagat caaacgtacg 360
<210> 93
   <211> 404
   <212> DNA
   <213> Artificial
<220>
   <223> Synthetic oligonucleotide (AJL15 heavy)
<400> 93
   accggtgtac attcccagct gcagctgcag gagtcgggcc caggactggt gaagccttcg 60
   gagaccctgt ccctcacctg cactgtctct ggtggctcca tcaccagtag gaataactac 120
   tggggctgga tccgccagtc cccagggaag gggctggagt ggattgggag tctctattat 180
   actgggagcg actactacaa cccgtccctc aagagtcgag tcaccatatc ggtagacaca 240
   tcgaagaacc aattctccct gaggctgagt tctgtgaccg ccgcggacac ggccgtgtat 300
   tattgtgtca gagttaacgt agacgacttt tggagtggtt tagggggggc ctggttcgac 360
   ccctggggcc aaggaaccct ggtcaccgtc tcctcagcgt cgac 404
<210> 94
   <211> 357
   <212> DNA
   <213> Artificial
<220>
   <223> Synthetic oligonucleotide (AJL15 light)
<400> 94
   accggtgtac atggggatat tgtgatgacc cagactccac tctctttgtc cgtcacccct 60
   ggacagccgg cctccatctc ctgcaaatct agtcagagcc tcctggatag tgatggaaag 120
   acccatttgt actggtacct gcagaagcca ggccagtctc cacagtccct gatctatgaa 180
   gtttctaaac ggttctctgg agtgccagat aggttcactg gcagcgggtc agggacagat 240
   ttcacactga aaatcagccg ggtggaggct gaggatgttg ggctttatta ctgcatgcaa 300
   agtgcacagc ttccgtacac ttttggccag gggaccaagc tggaaatcaa acgtacg 357
<210> 95
   <211> 401
   <212> DNA
   <213> Artificial
<220>
   <223> Synthetic oligonucleotide (AJL16 heavy)
<400> 95
   accggtgtac attcccagct gcagctgcag gagtcgggcc caggactggt gaagccttcg        60
   gagaccctgt ccctcacctg cactgtctct ggtggctcca tcagcagtag tccttactac       120
   tggggctgga tccgccagcc cccagggaag gggctggagt ggattgggag tatctattat       180
   agtgggcaca cctattataa cccctccctc aagagtcgag tcaccatatc cgttgacacg       240
   tccaagaacc agttctccct gcggctgacc tctgtgaccg ccgcagacac gtctgtgtat       300
   tactgtgcga aacagaccga tgactacggt gactacgcgt ccaggggctg gttcgacccc       360
   tggggccaag gaaccctggt caccgtctcc tcagcgtcga c                           401
<210> 96
   <211> 342
   <212> DNA
   <213> Artificial
<220>
   <223> Synthetic oligonucleotide (AJL16 light)
<400> 96
   accggtgtac attctgacat ccagatgacc cagtctccat cctccctgtc tgcatctgta        60
   ggagacagag tcaccttcac ttgccaggcg agtcacgaca ttagcaacta tttaaattgg       120
   tatcagcaga aaccagggaa agtccctgag ctcctgatct acgatgcatc caatttgaaa       180
   acaggggtcc catcaaggtt cagtggaagt ggatctggga cagattttac tttcaccatc       240
   agcagcctgc agcctgaaga tattgcaaca tattactgtc aacagtatga taatctccct       300
   atcaccttcg gccaagggac acgactggag attaaacgta cg                          342
<210> 97
   <211> 407
   <212> DNA
   <213> Artificial
<220>
   <223> Synthetic oligonucleotide (AJL18 heavy)
<400> 97
   accggtgtac attcccaggt gcagctgcag gagtcgggcc caggactggt gaagccttca        60
   cagaccctgt ccctcacctg cactgtctct ggtgactcca tcagcagtga taaatactac       120
   tggacctgga tccgccagct cccagggaag ggcctggagt ggattggcta catctcttac       180
   agtgggacca cctactacaa tccgtccctc aagagtcgag tttccatttc agttgacacg 240
   tctgggaacc agttctccct gaggctgagc tctgtgactg ccgcggacac ggcccgctat 300
   tactgtgcgc gagtatatta cgatgttttg tctgcttatt ataatatggg gtcctgggta 360
   gacccctggg gccaaggaac cctggtcacc gtctcctcag cgtcgac 407
<210> 98
   <211> 342
   <212> DNA
   <213> Artificial
<220>
   <223> Synthetic oligonucleotide (AJL18 light)
<400> 98
   accggtgtac attctgacat ccagatgacc cagtctccat cctccctgcc tgcatctgta 60
   ggagacagag tcaccatcac ttgccgggca agtcagagca ttagcagcta tttaaattgg 120
   tatcagcaga aaccagggag agcccctaag ctcctgatct atgctgcatc cagtttacaa 180
   agtggggtct catcaaggtt cagtggcagt ggatctggga cagatttcac tctcaccatc 240
   agcagtctgc aacctgaaga ttctgcaact tactactgtc aacagagtta cagtaccccg 300
   tggacgttcg gccaagggac caaggtggag atcaaacgta cg 342
<210> 99
   <211> 383
   <212> DNA
   <213> Artificial
<220>
   <223> Synthetic oligonucleotide (AJL19 heavy)
<400> 99
   accggtgtac attcccaggt gcagctacag cagtggggcg caggactgtt gaagcctccg 60
   gagaccctgt ccctcacctg cgctgtcttt ggtgggtccc tcagtggtta ctactggagt 120
   tggatccgcc agcccccagg gaaggggccg gagtggattg cggaaatcaa tcatagtgga 180
   gatgccaact acaacccgtc cctcaagagt cgagtcacta tctcagtaga cacgtccaag 240
   aaccagtttt ccctgaagat gagttctgtg accgtcgcag acacggcttt atattactgt 300
   gcgactcaag gctctaggtt gactacattc gcttttgatg tgtggggcca agggacaatg 360
   gtcaccgtct cttcagcgtc gac 383
<210> 100
   <211> 348
   <212> DNA
   <213> Artificial
<220>
   <223> Synthetic Oligonucleotide (AJL19 light)
<400> 100
   accggtgtac attcagaaat tgtgttgacg cagtctccag gcaccctgtc tttgtctcca    60
   ggggaaagag tcaccctctc ctgcaggacc agtcagagtg ttagcagcga ctccttagcc   120
   tggtaccagc agaaacctgg ccagactccc aggctcctca tttatcatac atccaccagg   180
   gccgctggca tcccagacag gttcagtggc actgggtctg ggacagactt cactctcacc   240
   atcgccagac tggagcctga agattttgca gtctattact gtcagcacta tggtcggtca   300
   tccctattca cctttggcca ggggaccaag ctggaaatca aacgtacg                348
<210> 101
   <211> 398
   <212> DNA
   <213> Artificial
<220>
   <223> Synthetic oligonucleotide (AJL20 heavy)
<400> 101
   accggtgtac attcccagct gcagctgcag gagtcgggcc caggactggt gaagccttcg    60
   gagaccctgt ccctcacctg cactgtctct ggtggctcca tcagcggtag ttctttctac   120
   tggggctggg tccgccagcc cccagggagg gggctggagt ggattggcac tatctattat   180
   cgtgggacca cctattacac cccgtctctc aagagtcgag tcaccatatc cgtggacacc   240
   tccaagaacc agttctccct gaggttgaac tctgtgaccg ccgcagacac ggctatatat   300
   tactgtgcga gccttcccca ctacgatttt tggagtggtt ctgttttctt tgactactgg   360
   ggccaaggaa ccctggtcac cgtctcctca gcgtcgac                            398
<210> 102
   <211> 342
   <212> DNA
   <213> Artificial
<220>
   <223> Synthetic oligonucleotide (AJL20 light)
<400> 102
   accggtgtac attctgacat ccagatgacc cagtctccat cctccctgtc tgcatctgta 60
   ggagacagag tcaccatcac ttgccgggcg agtcagggca ttgccaatta tttagcctgg 120
   tatcagcaga aaccagggaa cattcctaag ctcctgatct atgctgcatc cactttgcaa 180
   tcaggggtcc catctcggtt cagtggcagt ggatctggaa cagatttcgc tctcaccatc 240
   agctgcctac agcctgagga tgttgcaact tattactgtc aaaagtataa cagtgcccct 300
   ctcactttcg gcggagggac caaggtggag atcaaacgta cg 342
<210> 103
   <211> 380
   <212> DNA
   <213> Artificial
<220>
   <223> Synthetic oligonucleotide (WR01 heavy)
<400> 103
   accggtgtac attcccaggt gcagctgcag gagtcgggcc caggactggt gaagccttcg 60
   gagaccctgt ccctcacgtg cactgtttct ggtggctcca tcgatactta ctactggacc 120
   tggatccggc agcccccggg gaagggactg gagtggattg ggtatattta ttccactggg 180
   agccccaagt acaagccctc cctcaagagt cgggtcgtca tgtcagtgga cacgtccacg 240
   aacgagttcg ccctgaggct gagctctgtg accgctgcgg acacggccgt gtattactgt 300
   gcgagaagtt cgggatttta cgttgaacac ctggaaaagt ggggccaagg gaccacggtc 360
   accgtctcct cagcgtcgac 380
<210> 104
   <211> 345
   <212> DNA
   <213> Artificial
<220>
   <223> Synthetic oligonucleotide (WR01 light)
<400> 104
   accggtgtac attcagaaat tgtgttgacg cagtctccag ccaccctgtc tttgtctcca 60
   ggggaaagag ccactctctc ctgcagggcc agtcagactg tgagcagcag ctacttagac 120
   tggttccagc agaaacctgg ccaggctccc aggctcctca tctatggtgc atccagcagg 180
   gccactggca tcccagacag gttcagtggc agtgggtctg ggacagactt cactctaact 240
   atcagcagac tggagcctga agattttgca gtgtattatt gtcagcagtt tgctacctca 300
   ccgtacactt ttggccaggg gaccaagctg gagatcaaac gtacg 345
<210> 105
   <211> 395
   <212> DNA
   <213> Artificial
<220>
   <223> Synthetic oligonucleotide (WR02 heavy)
<400> 105
   accggtgtac attcccaggt gcagctgcag gagtcgggcc caggactggt gaagccttca        60
   cagaccctgt ccctcacctg cactgtctct ggtggctcca tcagcagtgg tgattaccac       120
   tggagttgga tccgccagcc cccagggaag ggcctggagt ggattgggaa catcaattat       180
   aatgggggcg cgtaccacaa tccgtccctc acgaatcgag ttatcatgtc agtagacacg       240
   tccaagaatc acttctccct gaaactgacc tctgtgactg ccgcagacac ggccgtgtat       300
   tactgtgcca gagagtctca gtggctgcga tacggcgcgt tcggtatgga cgtctggggc       360
   caagggacca cggtcaccgt ctcctcagcg tcgac                                  395
<210> 106
   <211> 345
   <212> DNA
   <213> Artificial
<220>
   <223> Synthetic oligonucleotide (WR02 light)
<400> 106
   accggtgtac attcagaaat tgtgttgacg cagtctccag gcaccctgtc tttgtctcca        60
   ggggaaagag ccaccctctc ctgcagggcc agtcagagcg ttagcagcag ccacttagtc       120
   tggtaccagc agaaagctgg ccaggctccc aggctcgtca tctatggtgc aaacaggagg       180
   gcctctggca ccccagacag gttcagtggc agtgggtctg ggacagactt cactctcacc       240
   atcagcagac tggagcctga agactttgca gtctattact gtcagcagta tggtagctcg       300
   ccgtacactt ttggccaggg gaccaagctg gagatcaaac gtacg                       345
<210> 107
   <211> 395
   <212> DNA
   <213> Artificial
<220>
   <223> Synthetic oligonucleotide (WR03 heavy)
<400> 107
   accggtgtac attcccaggt gcagctgcag gagtcgggcc caggactggt gaagccttcg 60
   gggaccctgt ccctcacctg cgctgtctct ggtggctcca tcagcagtag ttactggtgg 120
   agttgggtcc gccagccccc cgggaagggg ctggagtggg ttggagaaat ctatcatagt 180
   gggggcgcca actacagccc gtccctcaag agtcgagtca ccatatcagt ggacaagtcc 240
   aagaaccagt tctccctgaa cctgatctct gtgaccgccg cggacacggc cgtgtatttc 300
   tgtgcgagat cacggatgct agtgggtgct gatggtggag gtgcttttga tatctggggc 360
   caagggacaa tggtcaccgt ctcttcagcg tcgac 395
<210> 108
   <211> 345
   <212> DNA
   <213> Artificial
<220>
   <223> Synthetic oligonucleotide (WR03 light)
<400> 108
   accggtgtac attcagaaat tgtgttgacg cagtctccag gcaccctgtc tttgtctcca 60
   ggggaaagag ccaccctctc ctgcggggcc agtcagagtg ttagcagcag ctacttagcc 120
   tggtaccagc agaaacctgg cctggcgccc aggctcctca tctatgatgc atccagcagg 180
   gccactggca tcccagacag gttcagtggc agtgggtctg ggacagactt cactctcacc 240
   atcagcagac tggagcctga agattttgca gtgtattact gtcagcagta tggtagctca 300
   cctcaaacgt tcggccaagg gaccaaggtg gaaatcaaac gtacg 345
<210> 109
   <211> 380
   <212> DNA
   <213> Artificial
<220>
   <223> Synthetic oligonucleotide (WR04 heavy)
<400> 109
   accggtgtac attcccagct gcagctgcag cagtcgggcc caggactggt gaagccttcg        60
   gagaccctgt ccctcacctg cactgtctct ggtggctcca tcagcagtgg tggctcatac       120
   tggggctgga tccgccaggc ccccgggaag ggcctggagt ggattgggag tatgtattat       180
   agtgggagca ccttctacaa cccgtccgtc aagagtcgag tcaccatatc cgtagacagg       240
   tccaaggagc agttctccct caatctgaac gctgtgaccg ccgcagacac ggctgtgtat       300
   tactgtgtga gacataggag atcggaaccc agcgactcct ggggccaagg aaccctggtc       360
   accgtctcct cagcgtcgac                                                   380
<210> 110
   <211> 345
   <212> DNA
   <213> Artificial
<220>
   <223> Synthetic oligonucleotide (WR04 light)
<400> 110
   accggtgtac attcagaaat agtgatgacg cagtctccag ccaccctgtc tgtgtctcca        60
   ggggaaagag ccaccctctc ctgcagggcc agtcagagtg ttagcagcaa cttagcctgg       120
   taccagcaga aacctggcca ggctcccagg ctcctcatct atggtacatc caccagggcc       180
   actggcattc cagccaggtt cagtggcagt gggtctggga cagaattcac tctcaccatc       240
   agcagcctgc agtctgaaga ttctgcagtt tatttctgtc aacagtataa taactggccg       300
   ctgtacactt ttggccaggg gaccaaggtg gagatcaaac gtacg                       345
<210> 111
   <211> 380
   <212> DNA
   <213> Artificial
<220>
   <223> Synthetic oligonucleotide (WR05 heavy)
<400> 111
   accggtgtac attcccaggt gcagctgcag gagtcgggcc caggactggt gaagccttcg 60
   gagaccctgt ctctcacctg cagtgtctct ggtggctccg tcagcagcaa tggtcacttc 120
   tggagctgga tccggctgcc cccagggaag ggactggaat ggattggcta tgtctacaac 180
   actgggacct ccggctacag cccctccctc aagagtcgag tcaccatatc agtagacacg 240
   tccaagaacc agttctccct gacactgagg tctgtgaccg ctgcggacac ggccatttac 300
   tactgcgcga gaggtctcac tgggaactac ccgtctcact ggggccaagg caccctggtc 360
   accgtctcct cagcgtcgac 380
<210> 112
   <211> 342
   <212> DNA
   <213> Artificial
<220>
   <223> Synthetic oligonucleotide (WR05 light)
<400> 112
   accggtgtac attctgacat ccagatgacc cagtctccat cttccgtgtc tgcatctgta 60
   ggagacagag tcaccatcac ttgtcgggcg agtcggggtg ttagtacctg gttagcctgg 120
   tatcagcaga aaccagggga agcccctaag ctcctgatct atgcttcatc ccgtttagaa 180
   ggtggggtcc cagcaaggtt cagcggcagt ggatctggga cagatttcac tctcaccatc 240
   agcagcctgc agcctgaaga ttttgcaacc tactattgtc aacagggtaa cagcttcccc 300
   ctcactttcg gcggagggac caaggtggag atcaaacgta cg 342
<210> 113
   <211> 380
   <212> DNA
   <213> Artificial
<220>
   <223> Synthetic oligonucleotide (WR06 heavy)
<400> 113
   accggtgtac attcccagct gcagctgcag gagtcgggcc caggactggt gaagccttcg 60
   gagaccctgt ccctcacctg cactgtctct ggtggctccg tcagtagtag tgcttactgg 120
   tgggcctgga tccgccagcc cccgggggga ggactggagt ggatcggcca tatctattac 180
   tttgggaaca aatattacaa gtcgtccctc gagagtcgag tcaccatttc actagacgcg 240
   tcccagaacc agttctccct gaagctgacc tccgtgaccg ccgcagacac ggctctgtat 300
   tactgtgcga gagtggacac agctttggct tttgacttct ggggccaagg gacaatggtc 360
   accgtctctt cagcgtcgac 380
<210> 114
   <211> 342
   <212> DNA
   <213> Artificial
<220>
   <223> Synthetic oligonucleotide (WR06 light)
<400> 114
   accggtgtac attctgacat ccagatgacc cagtctccat cctccctgtc tgcatctgta 60
   ggagacagag tcaccatcac ttgccgggca agtcagtatg ttagtagttc tttgaattgg 120
   tatcagcaga aaccagggaa agcccctaca ctcctgatct atcttgcgtc caatttgcga 180
   agtggggtcc catcaaggtt cagtggcagt gaatctggga cagatttcac tctcaccatc 240
   aacagtctgc agcctgaaga tgttgcaact tacttctgtc aacagagtta tagtctccct 300
   cgcactttcg gccctgggac caaagtggat atcaaacgta cg 342
<210> 115
   <211> 407
   <212> DNA
   <213> Artificial
<220>
   <223> Synthetic oligonucleotide (WR07 heavy)
<400> 115
   accggtgtac attcccagct gcagctgcag gagtcgggcc caggactggt gaagccctcg 60
   gagaccctgt ccctcacctg ctctgtctct ggtggctccg tcagtagtac aacttactac 120
   tgggggtgga tccgccagtc cccagggaag gggctggagt ggattgggag catctatcat 180
   agtgggaaaa cctactacaa cccgtccctc aagagtcgag tcaccatatc agtagacacg 240
   tccaagaacc agttctccct gaacctgagc tctgtgaccg ccgcggacac ggccgtgtat 300
   tattgtgcga gagagaattc ccatcactat gatagtagtg gttattactt aggtggattt 360
   gactactggg gccagggaac cctggtcacc gtctcctcag cgtcgac 407
<210> 116
   <211> 342
   <212> DNA
   <213> Artificial
<220>
   <223> Synthetic oligonucleotide (WR07 light)
<400> 116
   accggtgtac attcagaaat tgtgttgacg cagtctccag gcaccctgtc tttgtctcca 60
   ggggaaagag ccaccctctc ctgcagggcc agtcagagtg ttagcagcag ctacttagcc 120
   tggtaccagc agaaacctgg ccaggctccc aggctcctca tctatggtgc atccagcagg 180
   gccactggca tcccagacag gttcagtggc agtgggtctg ggacagactt cactctcacc 240
   atcagcagac tggagcctga agatttcgca gtatatttct gccagcacta tggtagcccc 300
   tccacgtttg gccaggggac caaggtggag atcaaacgta cg 342
<210> 117
   <211> 380
   <212> DNA
   <213> Artificial
<220>
   <223> Synthetic oligonucleotide (WR08 heavy)
<400> 117
   accggtgtac attcccaggt gcagctgcag gagtcgggcc caggactggt gaagccttca 60
   cagaccctgt ccctcacctg cactgtctct ggtggctcca tcagcagtgg ttactactgg 120
   agttggatcc gccagcaccc agggaagggc ctggattgta ttgggtacat ccattacacg 180
   gggaccacct actacaaccc gtccctgaag agtcgactta ccatatcagt ggacacgtct 240
   aagaaccagt tctccctgaa cctgacctct gtgactgccg cggacacggc cgtctattat 300
   tgtgcgagag aagaatatac aacctcgtca gttgattact ggggccgggg caccctggtc 360
   accgtctcct cagcgtcgac 380
<210> 118
   <211> 357
   <212> DNA
   <213> Artificial
<220>
   <223> Synthetic oligonucleotide (WR08 light)
<400> 118
   accggtgtac atggggatgt tgtgatgact cagtctccac tctccctgcc cgtcaccctt 60
   ggacagccgg cctctatctc ctgcaggtct agtgaaagcc tcgtgtctgt tgatggaaat 120
   acctacttga attggtttca ccagaggcca ggccaatctc caaggcgcct aatttataag 180
   gtttctaacc gggactctgg ggtcccagac agattcagcg gcagtgggtc aggcactgat 240
   ttcacactga aaatcagcag ggtggaggct gaggatgttg ggatttatta ctgcatgcaa 300
   gccacacact ggcttcggac gttcggccaa gggacacgac tggagattaa acgtacg 357
<210> 119
   <211> 380
   <212> DNA
   <213> Artificial
<220>
   <223> Synthetic oligonucleotide (WR09 heavy)
<400> 119
   accggtgtac attcccaggt gcagctgcag gagtcgggcc caggactggt gaagccttca 60
   cagaccctgt ccctcacctg cactgtctct ggtggctcca tcagcagtgg ttactactgg 120
   agttggatcc gccagcaccc agggaagggc ctggagtgga ttgggtacat ccattacacg 180
   gggaccacct actacaaccc gtccctgaag agtcgagtta ccatatcagt ggacacgtct 240
   aagaaccagt tctccctgaa cctgacctct gtgactgccg cggacacggc cgtctattat 300
   tgtgcgagag aagaatatac gacctcgtca gttgattact ggggccgtgg caccctggtc 360
   accgtctcct cagcgtcgac 380
<210> 120
   <211> 351
   <212> DNA
   <213> Artificial
<220>
   <223> Synthetic oligonucleotide (WR09 light)
<400> 120
   accggtgtac attcagaaat tgtgttgacg cagtctccag gcaccctgtc tttgtctcca 60
   ggggaaagag ccaccctctc ctgcagggcc agtcagaggg ttagcagcgg ctttttagcc 120
   tggtaccagc agaaacctgg ccaggctccc aggctcctca tctatggtgc atccagcagg 180
   gccactggca tcccagacag gttcagtggc agtggctctg ggacagactt cactctcacc 240
   atcagcagac tggagcctga agattttgca gtgtattact gtcagcagta tgaaagttca 300
   ccgtcaccgt acaactttgg ccaggggacc aagctggaga tcaaacgtac g 351
<210> 121
   <211> 401
   <212> DNA
   <213> Artificial
<220>
   <223> Synthetic oligonucleotide (WR10 heavy)
<400> 121
   accggtgtac attcccaggt gcagctgcag gagtcgggcc caggactggt gaagccttcg 60
   gggaccctgt ccctcacctg cgctgtctct ggtggctcca tcagcaataa taagtggtgg 120
   aattgggtcc gccagtcccc agggaagggg ctggagtgga ttggggaaat ctatcatagt 180
   gggggcacca actacaaccc gtccctcaag agtcgagtca ccatatcggt agacaagtcc 240
   aagaacctgt tctccctgaa gctgagctct gtgaccgccg cggacacggc cgtgtattac 300
   tgtgcgagtg cgactactat ggttcgggga ctgagtcttt actactacgg tctggacgtc 360
   tggggcccag ggaccacggt caccgtctcc tcagcgtcga c 401
<210> 122
   <211> 345
   <212> DNA
   <213> Artificial
<220>
   <223> Synthetic oligonucleotide (WR10 light)
<400> 122
   accggtgtac attcagaaat tgtgttgacg cagtctccag gcaccctgtc tttgtctcca 60
   ggggaaagag ccgccctctc ctgcagggcc agtcagagtc ttatcggcag cttcttagcc 120
   tggtaccagc agaaacctgg ccaggctccc aggctcctca tctatcatac atccaacagg 180
   gcctctggca tcccagacag gttcagtggc ggtgggtttg ggacagactt cactctcacc 240
   atcagcagac tggagcctga agattttgca gtttattact gtcaacagta tgatagctca 300
   ccgatcacct tcggccaagg gacacgactg gagattaaac gtacg 345
<210> 123
   <211> 401
   <212> DNA
   <213> Artificial
<220>
   <223> Synthetic oligonucleotide (WR11 heavy)
<400> 123
   accggtgtac attcccaggt gcagctgcag gagtcgggcc caggactggt gaagccttcg 60
   gggaccctgt ccctcacctg cgctgtctct ggtggctcca tcagcaataa taagtggtgg 120
   aattgggtcc gccagtcccc agggaagggg ctggagtgga ttggggaaat ctatcatagt 180
   gggggcacca actacaaccc gtccctcaag agtcgagtca ccatatcggt agacaagtcc 240
   aagaacctgt tctccctgaa gctgagctct gtgaccgccg cggacacggc cgtgtattac 300
   tgtgcgagtg cgactactat ggttcgggga ctgagtcttt actactacgg tctggacgtc 360
   tggggccaag ggaccacggt caccgtctcc tcagcgtcga c 401
<210> 124
   <211> 348
   <212> DNA
   <213> Artificial
<220>
   <223> Synthetic oligonucleotide (WR11 light)
<400> 124
   accggtgtac attcagaaat tgtgttgacg cagtctccag gcaccctgtc tttgtctcca 60
   ggggaaagag ccaccctctc ctgcagggcc agtcagagtg ttagcagctt cttagcctgg 120
   taccaacaga aacctggcca ggctcccaga ctcctcatct atgatgcatc caacagggcc 180
   actggcatcc cagccaggtt cagtggcagt gggtctggga cagatttcac tctcaccatc 240
   agcagcctag agcctgaaga ttttgcagtt tattactgtc agcagcgtac caactggcct 300
   ccctcgctca ctttcggcgg agggaccaag gtggagatca aacgtacg 348
<210> 125
   <211> 410
   <212> DNA
   <213> Artificial
<220>
   <223> Synthetic oligonucleotide (WR12 heavy)
<400> 125
   accggtgtac attcccaggt gcagctgcag gagtcgggcc caggactggt gaagccttca 60
   cagaccctgt ccctcacctg cactgtctct ggcgactccg tcagcagtaa tgatcactac 120
   tggagttgga tccgccagcc cccagggcag ggcctggagt ggattgggta catctctcac 180
   ggtgggacca cctactacaa cccgtccctc aagagtcgag ttaccatgtc gatcgacacg 240
   tccacgaacc agttctccct gagggtgacc tccgtgcgag ccgcagacat ggccgtctac 300
   ttctgtgcca gggccccggc ccctataacg acttttggaa tggtgacacc agtcccctac 360
   tttcactcct ggggccaagg caccctggtc accgtctcct cagcgtcgac 410
<210> 126
   <211> 357
   <212> DNA
   <213> Artificial
<220>
   <223> Synthetic oligonucleotide (WR12 light)
<400> 126
   accggtgtac atggggatat tgtgatgact cagtctccac tgtccctgcc cgtcagccct 60
   ggagagccgg cctccatctc ctgcaggtct agtcagagcc tcctccatag taatggatac 120
   aactatttga gttggtacct gcagaagcca gggcagtctc cacaactcct gatcttttcg 180
   agttctattc gggcctccgg ggtccctgac aggttcagtg gcagtggatc aggcacagat 240
   tttacactga caatcaacag agtggaggct gaggatgttg gagtttatta ctgcatgcag 300
   gctctacaaa ctccgctcac tttcggcgga gggaccaagc tggaaatcaa acgtacg 357
<210> 127
   <211> 410
   <212> DNA
   <213> Artificial
<220>
   <223> Synthetic oligonucleotide (WR13 heavy)
<400> 127
   accggtgtac attcccaggt gcagctgcag gagtcgggcc caggactggt gaagccttca      60
   cagaccctgt ccctcacctg cactgtctct ggcgactccg tcagcagtaa tgatcactac     120
   tggagttgga tccgccagcc cccagggcag ggcctggagt ggattgggta catctctcac     180
   ggtgggacca cctactacaa cccgtccctc aagagtcgag ttaccatgtc gatcgacacg     240
   tccacgaacc agttctccct gagggtgacc tccgtgcgag ccgcagacat ggccgtctac     300
   ttctgtgcca gggccccggc ccctataacg acttttggaa tggtgacacc agtcccctac     360
   tttcactcct ggggccaagg caccctggtc accgtctcct cagcgtcgac                410
<210> 128
   <211> 357
   <212> DNA
   <213> Artificial
<220>
   <223> Synthetic oligonucleotide (WR13 light)
<400> 128
   accggtgtac atggggatat tgtgatgact cagtctccac tgtccctgcc cgtcagccct      60
   ggagagtcgg cctccatctc ctgcaggtct agtcagagcc tcctccatag taatggatac     120
   aactatttga gttggtacct gcagaagcca gggcagtctc cacaactcct gatcttttcg     180
   agttctattc gggcctccgg ggtccctgac aggttcagtg gcagtggatc aggcacagat     240
   tttaccctga caatcaacag agtggaggct gaggatgttg gagtttatta ctgcatgcag     300
   gctctacaaa ctccgctcac tttcggccag gggaccaagc tggaaatcaa acgtacg        357

## Description

### BACKGROUND OF THE INVENTION

### 1. Field of the Invention

The disclosed subject matter relates to fields of pathology, immunology and molecular biology. More particularly, the present invention relates to an siRNA for use in a method of treating multiple sclerosis (MS) or clinically isolated syndrome in a patient as defined in the claims.

### 2. Background of the Invention

B cells have been implicated in multiple sclerosis (MS) and have been recognized to play a role in MS pathology in addition to the well-accepted pathological role of T cells. B cells and antibodies are present in both the cerebrospinal fluid (CSF) and the central nervous system (CNS) of patients with MS and clinically isolated syndrome (CIS) patients who are at high risk of developing MS. The most common form of MS lesion is characterized by deposition of antibodies and complement (Lucchinetti *et al.,* 2000), and plasmapheresis treatment of patients harboring these lesions leads to symptom improvement (Keegan *et al.,* 2005). In fact, elevated B cells in the CSF correlates with lesion activity on MRI (Cepok *et al.,* 2005) and both increased intrathecal immunoglobulin synthesis (Sellebjerg *et al.,* 2000) and complement activation (Sellebjerg *et al.,* 1998) are also associated with a more aggressive disease course. Collectively these findings implicate a pathological role for antibodies in the pathoetiology of MS.

The inventor's laboratory has previously discovered a biomarker for conversion from CIS to clinically definite MS (CDMS) in the antibody genetics of V_{H}4-utilizing B cells in the CSF, termed the antibody gene signature (AGS) (Cameron *et al.,* 2009). She also found that B cells isolated from CNS lesions harbor the AGS (Ligocki *et al.,* 2010). This shared pattern of somatic hypermutation at 6 codons along the V_{H}4 gene implicates that the B cell pools are recognizing a shared set of antigens in the MS disease state that are not recognized by B cells in healthy individuals. However, the existence of such a phenomenon, and identification of the antigens to which these antibodies bind, remained to be demonstrated.

Lehmann-Horn et al. (Ther Adv Neurol Disord, 2013, 6(3), p. 161-173) summarized findings in support of pathogenic B-cell function in multiple sclerosis and reviewed findings suggestive of regulatory properties of B-cell subsets which may be collaterally abolished by pan-CD20 depletion. Furthermore, the authors provided an outlook on how the concept of pro - and anti-inflammatory B-cell function could be harnessed to further improve safety and effectiveness of B-cell-directed therapeutic approaches in multiple sclerosis.

WO 2007/035857 discloses methods for reducing the number of pathologic antibody producing B cells in a patient suffering from an autoimmune disease by administration of an anti-germline antibody as well as methods for removing pathologic antibodies and B cells and plasma cells producing pathologic antibodies from the body of a patient suffering from autoimmune disease are provided. The methods include contacting the blood or plasma of the patient with an immunoadsorbent having specific binding for an epitope present on germline antibodies, particularly VH4-34 antibodies, wherein said contacting results in the reduction in the amount of germline antibodies present in the blood or bone marrow or lymphoid tissue of the patient or the amount of germline antibody producing B cells present in the blood, lymphoid tissues or bone marrow of the patient.

WO 2008/097439 relates to compositions and methods for the use of anti-autoimmune reagents that specifically bind to anti-desmoglein antibodies, which are responsible for both pemphigus vulgaris and pemphigus foliaceus. In addition, the document discloses methods and compositions for inhibiting the expression or function of a variable region of an anti-desmoglein (anti-Dsg) pathogenic autoantibody.

### SUMMARY OF THE INVENTION

Thus, in accordance with the present invention, there is provided an siRNA for use in a method of treating multiple sclerosis (MS) or clinically isolated syndrome in a patient as defined in the claims.

The present disclosure also discloses a recombinant antibody or antigen-binding fragment thereof, wherein the recombinant antibody or fragment binds to an antigen in human brain gray matter that is recognized by a VH4-comprising antibody having at least two mutations with respect to the germline sequence at codon positions selected from 31B, 32, 40, 56, 57, 60, 81, and 89. The antibody or fragment may be linked to a toxin, a drug or prodrug, or a label, such as a chromophore, fluorophore, chemilluminescent compound, dye, contrast agent, radioabel.

The VH4-comprising antibody may have at least three mutations with respect to the germline sequence at codon positions selected from 31B, 32, 40, 56, 57, 60, 81, and 89, may have at least four mutations with respect to the germline sequence at codon positions selected from 31B, 32, 40, 56, 57, 60, 81, and 89, may at least mutations with respect to the germline sequence at codon positions 31B, 56 and/or 81, may have mutations with respect to the germline sequence at at least at codon positions 31B, 56 and 81, may further have mutations with respect to the germline sequence at one or more codon positions selected from codons 32, 40, 57, 60 and 89, and may even further have mutations with respect to the germline sequence at each of codon positions 31B, 32, 40, 56, 57, 60, 81 and 89. The VH4-comprising antibody may have mutations with respect to the germline sequence at codons 31B, 40, 56, 57, 81 and/or 89, or may have mutations with respect to the germline sequence at each of codons 31B, 40, 56, 57, 81 and 89.

The recombinant antibody or fragment may have at least three mutations with respect to the germline sequence at codon positions selected from 31B, 32, 40, 56, 57, 60, 81, and 89, may have at least four mutations with respect to the germline sequence at codon positions selected from 31B, 32, 40, 56, 57, 60, 81, and 89, may at least mutations with respect to the germline sequence at codon positions 31B, 56 and/or 81, may have mutations with respect to the germline sequence at at least at codon positions 31B, 56 and 81, may further have mutations with respect to the germline sequence at one or more codon positions selected from codons 32, 40, 57, 60 and 89, and may even further have mutations with respect to the germline sequence at each of codon positions 31B, 32, 40, 56, 57, 60, 81 and 89. The recombinant antibody or fragment may have mutations with respect to the germline sequence at codons 31B, 40, 56, 57, 81 and/or 89, or may have mutations with respect to the germline sequence at each of codons 31B, 40, 56, 57, 81 and 89.

The antibody or fragment may have a heavy chain CDRs selected from SEQ ID NOS: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61 and 63, and light chain CDRs selected from SEQ ID NOS: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62 and 64, respectively. Alternatively, the recombinant antibody or fragment may have a heavy chain variable region sequence selected from SEQ ID NOS: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61 and 63, and a light chain variable region sequence selected from SEQ ID NOS: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62 and 64, respectively.

In some embodiments, the VH4 germline is a 4-04, 4-28, 4-30, 4-31, 4-34, 4-39, 4-59, 4-61, or a 4-B4 germline.

In some embodiments, the antibody has a CDR sequence as above, but with from 1 to 5 amino acid substitutions (*e.g*., 1, 2, 3, 4 or 5 substitutions) in the CDR sequence, while maintaining the identity at the AGS codons, or while having amino acid identity at one or more AGS positions as follows. For example, codon 31B may be R, N, D, P, K, G, A, or T. In some embodiments, codon 31 B is a charged amino acid, such as R, N, or D. Codon 40 may be S, L, or A. In some embodiments, codon 40 is S. Codon 56 in some embodiments is R, G, N, T, Y, H, D, or K. For example, codon 56 may be N, T, or G. Codon 57 in various embodiments is A, I, D, S, and K. In some embodiments, codon 57 is A or I. In various embodiments, codon 81 is N, R, or M. For example, codon 81 may be N or R. In some embodiments, codon 89 is F, I, R, or L. In some embodiments, codon 89 is a hydrophobic amino acid, such as F, I, or L. In various embodiments, the antibody contains a set of AGS codons shown in Table 1.

In some embodiments, other properties of the antibodies can be as described in U.S. Patent 8,394,583 or as described herein with respect to preferrential usage of germline sequences..

The disclosure also provides a method of detecting multiple sclerosis (MS) or pre-MS lesion in a subject comprising (a) administering to said subject a recombinant antibody or fragment as defined above, wherein the antibody or fragment carries a label; and (b) detecting the localization of said antibody in a neuronal tissue of said subject. The subject may be a human subject or a non-human mammalian subject.

The disclosure also provides a method of preparing a multiple sclerosis (MS) model comprising (a) providing a non-human mammalian subject; and (b) administering to said subject one or more recombinant antibodies or fragments as defined above. The subject may be a mouse, hamster, rat or rabbit. The method may further comprise repeating step (b) at least once, and even repeating step (b) until an MS-like functional deficit is observed. Step (b) may be continued so long as said animal is alive.

The disclosure also provides a method of preparing a multiple sclerosis (MS) model comprising preparing a non-human mammalian subject that contains a transgenic B cell expressing a recombinant antibody or fragment thereof as defined above. The subject may be a mouse, hamster, rat or rabbit. Preparing may comprise administering a B cell expressing said antibody to said subject, wherein said B cell has been transformed with an expression construct that encodes said antibody under the control of a promoter active in said B cell, such as syngeneic B-cells. Alternatively, preparing may comprise generating a non-human mammalian subject that preparing comprises generating said non-human mammalian subject such that cells of said subject comprise a germ line insert of an exogenous expression construct that encodes said recombinant antibody or fragment thereof under the control of a promoter active in B cells of said subject.

A method of treating multiple sclerosis (MS) or clinically isolated syndrome in a patient comprising administering to said subject an agent or subjecting said subject to a therapy that reduces the amount or function of an antibody having a sequence as defined above. The agent may comprise an anti-idiotypic antibody to said antibody, an antigen fragment that binds to said antibody, an siRNA that reduces said antibody's expression, or a non-Fc containing antibody that competes with said antibody. The therapy may be B-cell ablation that reduces B-cells producing said antibody. The therapy may comprise physical removal of B-cells producing said antibody or physical removal of said antibody. The method may further comprise administering to said subject one or more traditional MS therapies. The agent maybe administered systemically or through a route that targets neuronal tissue.

It is contemplated that any method or composition described herein can be implemented with respect to any other method or composition described herein.

The use of the word "a" or "an" when used in conjunction with the term "comprising" in the claims and/or the specification may mean "one," but it is also consistent with the meaning of "one or more," "at least one," and "one or more than one." The word "about" means plus or minus 5% of the stated number.

Other objects, features and advantages of the present invention will become apparent from the following detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

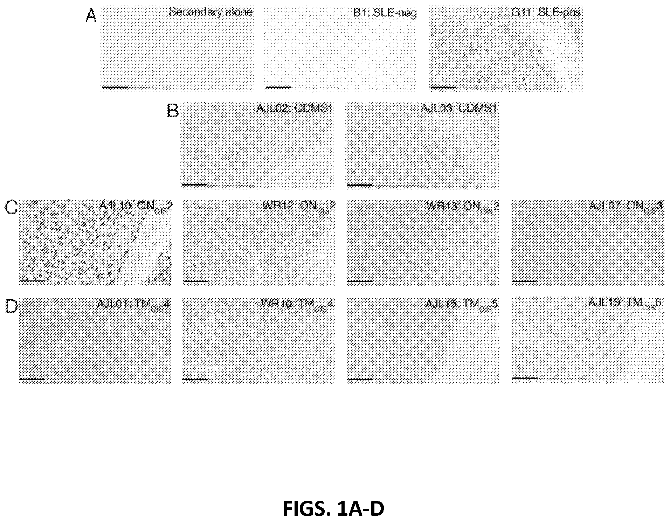

**FIGS. 1A-D****. AGS-enriched rhAbs bind to mouse brain.** DAB images are shown at 20x magnification of the cortex and corpus callosum. The rhAbs in each row are grouped as follows: controls (FIG. 1A), CDMS (FIG. 1B), ON_{CIS} (FIG. 1C), and TM_{CIS} (FIG. 1D). The rhAb designation, patient type, and patient number are shown in the upper right corner of each image. Data are representative of three coronal sections per rhAb. Scale bar represents 100 µm.

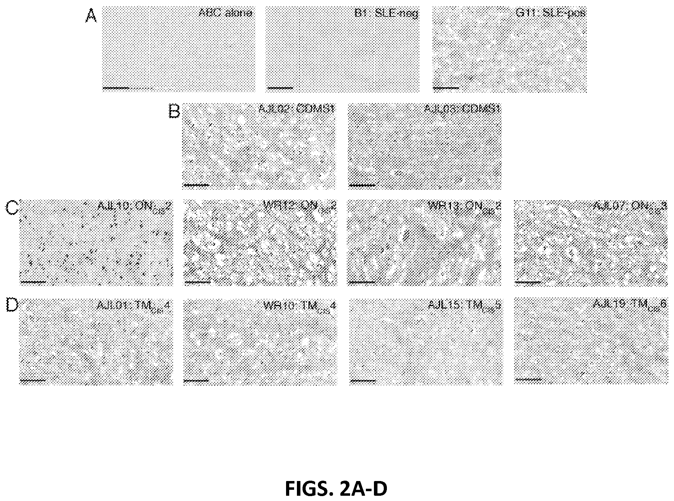

**FIGS. 2A-D****. AGS-enriched rhAbs bind to human MS-GM brain.** DAB images are shown at 20x magnification of MS-GM. The rhAbs in each row are grouped as follows: controls (FIG. 2A), CDMS (FIG. 2B), ON_{CIS} (FIG. 2C), and TM_{CIS} (FIG. 2D). The rhAb designation, patient type, and patient number are shown in the upper right corner of each panel. Data are representative of three MS-GM sections per rhAb. Scale bar represents 100 µm.

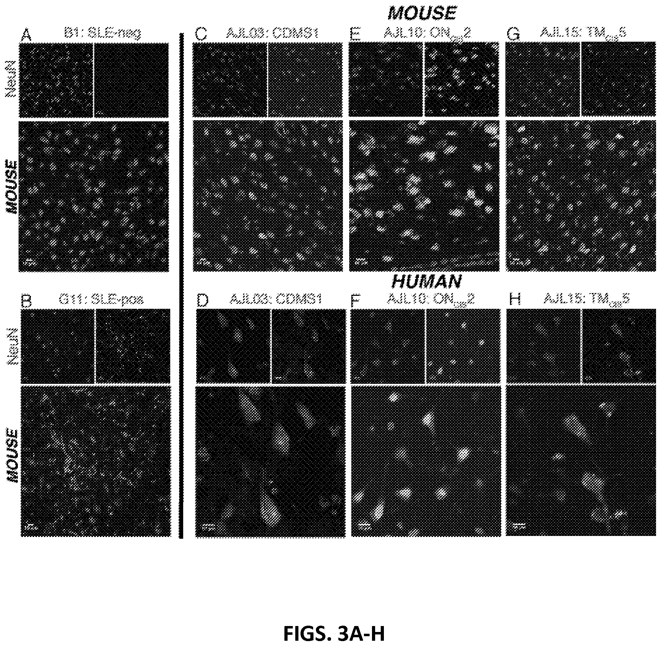

**FIGS. 3A-H****. IFC of AGS-enriched rhAbs targeting neuronal nuclei in both mouse and human MS-GM brain: AJL03, AJL10, and AJL15.** Confocal images are shown at 63x magnification with the colocalization marker for NeuN (for neuronal nuclei) shown as red (Alexa Fluor 594). The primary rhAb is shown as green (Alexa Fluor 488) and nuclei are counterstained blue (DAPI). The images are shown as independent red and green channels above the overlay including DAPI. B1 (- SLE) and G11 (+SLE) negative and positive controls respectively on mouse brain tissue are shown in FIGS. 3A and 3B. Mouse and human (MS-GM) brain tissue IFC are shown for each rhAb labeled above the column: AJL03 (CDMS1) (FIGS. 3C, 3D), AJL10 (ON_{CIS}2) (FIGS. 3E, 3F), and AJL15 (TM_{CIS}5) (FIGS. 3G, 3H). Data are representative of six coronal sections per rhAb on mouse tissue and three sections per rhAb on MS-GM tissue. Scale bar represents 10 µm.

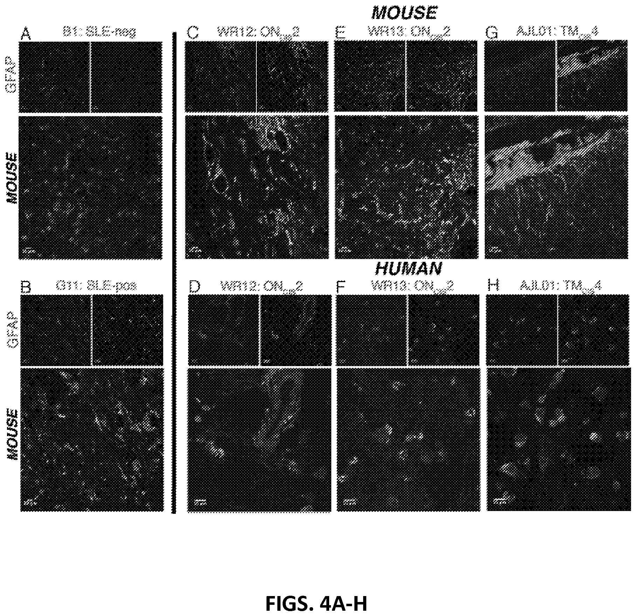

**FIGS. 4A-H****. IFC of AGS-enriched rhAbs targeting astrocytes in both mouse and human MS-GM brain: WR12, WR13, and AJL01.** Confocal images are shown at 63x magnification with the colocalization marker for GFAP (for astrocytes) shown as red (Alexa Fluor 594). The primary rhAb is shown as green (Alexa Fluor 488) and nuclei are counterstained blue (DAPI). The images are shown as independent red and green channels above the overlay including DAPI. B1 (- SLE) and G11 (+SLE) negative and positive controls respectively on mouse brain tissue are shown in FIGS. 4A and 4B. Mouse and human (MS-GM) brain tissue IFC are shown for each rhAb labeled above the column: WR12 (ON_{CIS}2) (FIGS. 4C, 4D), WR13 (ON_{CIS}2) (FIGS. 4E, 4F), and AJL01 (TM_{CIS}4) (FIGS. 4G, 4H). Data are representative of six coronal sections per rhAb on mouse tissue and three sections per rhAb on MS-GM tissue. Scale bar represents 10 µm.

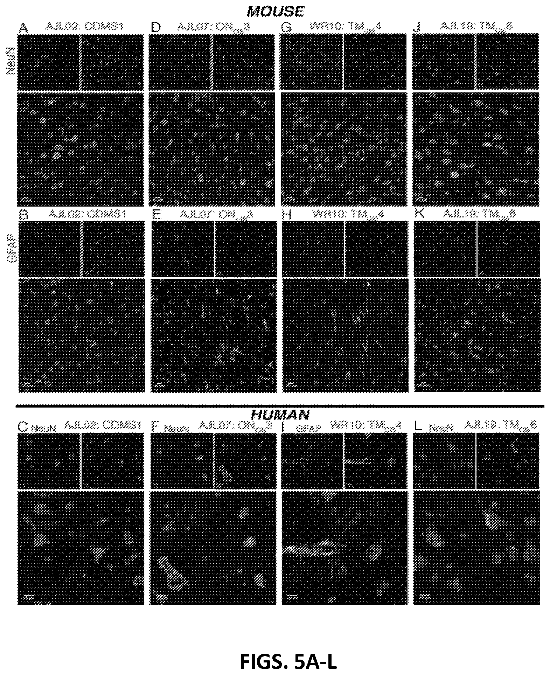

**FIGS. 5A-L****. IFC of AGS-enriched rhAbs targeting both neuronal nuclei and astrocytes in both mouse and human MS-GM brain: AJL02, AJL07, WR10 and AJL19.** Confocal images are shown at 63x magnification with the colocalization marker for NeuN (for neuronal nuclei) across the first row and GFAP (for astrocytes) across the second row shown as red (Alexa Fluor 594). The primary rhAb is shown as green (Alexa Fluor 488) and nuclei are counterstained blue (DAPI). The images are shown as independent red and green channels above the overlay including DAPI. The top two rows are NeuN and GFAP staining on mouse brain tissue with the bottom row showing human brain tissue (MS-GM) with labels of the colocalization marker above the red panel. Each rhAb is labeled above the column: AJL02 (CDMS1) (FIGS. 5A-C), AJL07 (ON_{CIS}3) (FIGS. 5D-F), WR10 (TM_{CIS}4) (FIGS. 5G-I), and AJL19 (TM_{CIS}6) (FIGS. 5J-L). Data are representative of six coronal sections per rhAb on mouse tissue and three sections per rhAb on MS-GM tissue. Scale bar represents 10 µm.

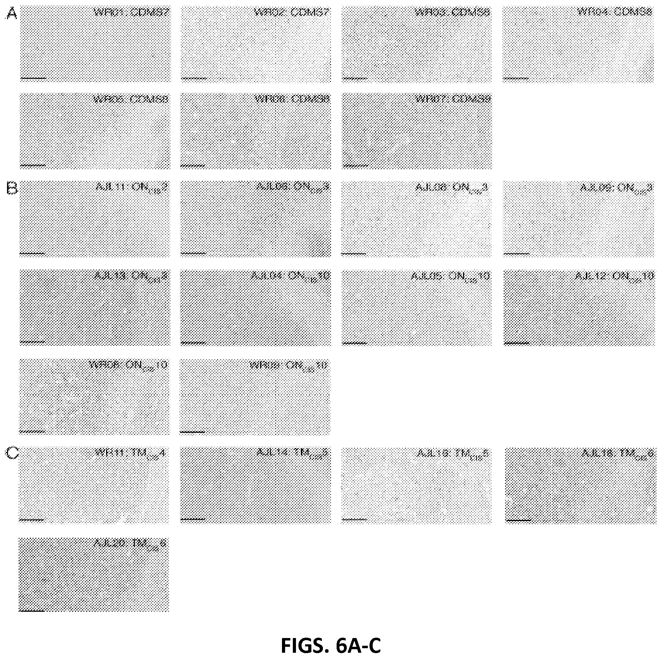

**FIGS. 6A-C****. AGS-enriched rhAbs bind to mouse brain.** The remaining 22 rhAbs not in Figure 1 are shown here. DAB images are shown at 20x magnification of the cortex and corpus callosum. The rhAbs in each section are grouped as follows: CDMS (FIG. 6A), ON_{CIS} (FIG. 6B), and TM_{CIS} (FIG. 6C). The rhAb designation, patient type, and patient number are shown in the upper right corner of each image. Data are representative of three coronal sections per rhAb. Scale bar represents 100 µm.

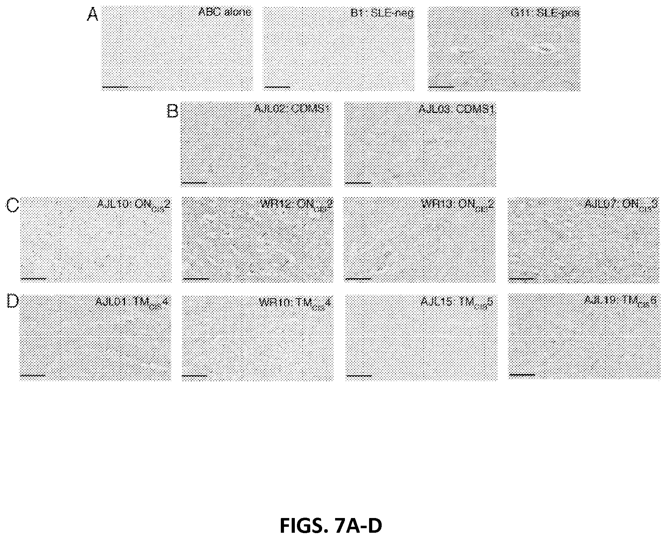

**FIGS. 7A-D****. AGS-enriched rhAb DAB staining on human MS-WM brain.** DAB images are shown at 20x magnification of MS-WM. The rhAbs in each row are grouped as follows: controls (FIG. 7A), CDMS (FIG. 7B), ON_{CIS} (FIG. 7C), and TM_{CIS} (FIG. 7D). The rhAb designation, patient type, and patient number are shown in the upper right corner of each panel. Data are representative of three MS-WM sections per rhAb. Scale bar represents 100 µm.

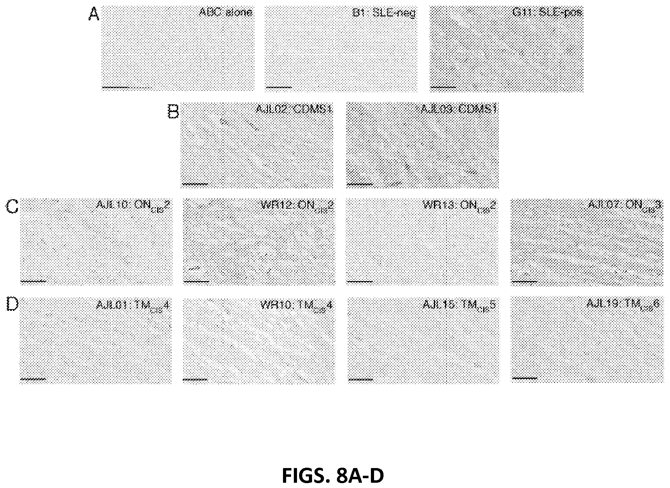

**FIGS. 8A-D****. AGS-enriched rhAb DAB staining on human brain MS MS-P plaque.** DAB images are shown at 20x magnification of MS-P. The rhAbs in each row are grouped as follows: controls (FIG. 8A), CDMS (FIG. 8B), ON_{CIS} (FIG. 8C), and TM_{CIS} (FIG. 8D). The rhAb designation, patient type, and patient number are shown in the upper right corner of each panel. Data are representative of three MS-P sections per rhAb. Scale bar represents 100 µm.

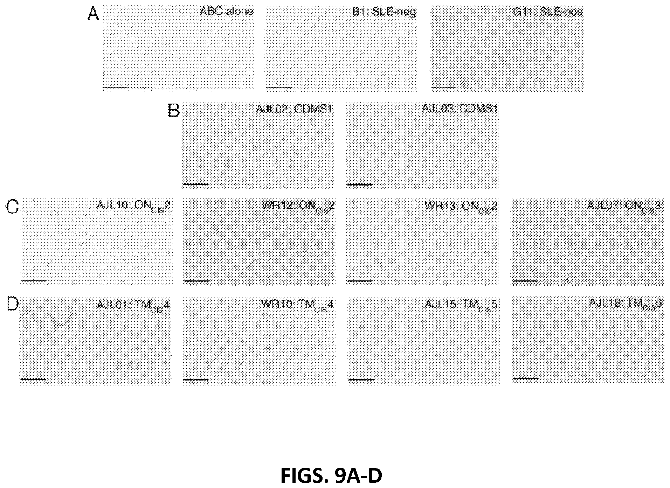

**FIGS. 9A-D****. AGS-enriched rhAb DAB staining on healthy human HC-WM brain.** DAB images are shown at 20x magnification of HC-WM. The rhAbs in each row are grouped as follows: controls (FIG. 9A), CDMS (FIG. 9B), ON_{CIS} (FIG. 9C), and TM_{CIS} (FIG. 9D). The rhAb designation, patient type, and patient number are shown in the upper right corner of each panel. Data are representative of three HC-GM sections per rhAb. Scale bar represents 100 µm.

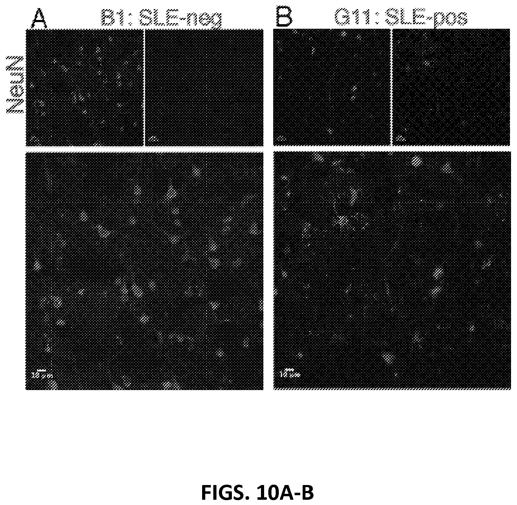

**FIGS. 10A-B****. IFC of control rhAbs on human MS-GM brain: B1 (****FIG. 10A****) and G11 (****FIG. 10B****).** Confocal images are shown at 63x magnification with the colocalization marker for NeuN (for neuronal nuclei) shown as red (Alexa Fluor 594), the primary rhAb as green (Alexa Fluor 488), and nuclei counterstained blue (DAPI). The images are shown as independent red and green channels above the overlay including DAPI with the rhAb label above each panel. Data are representative of three MS-GM sections per rhAb. Scale bar represents 10 µm.

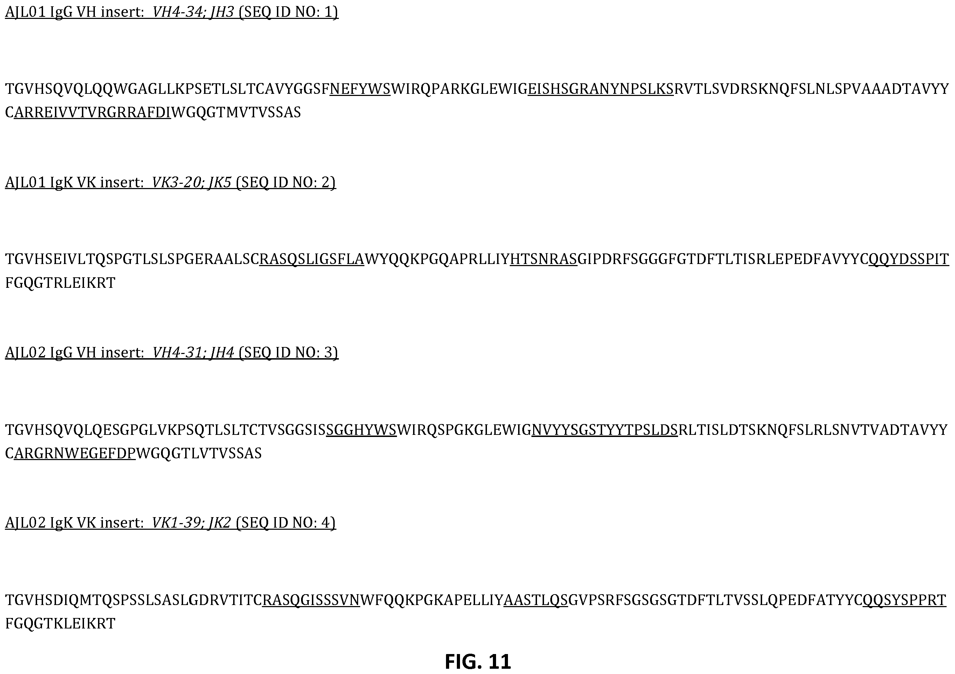

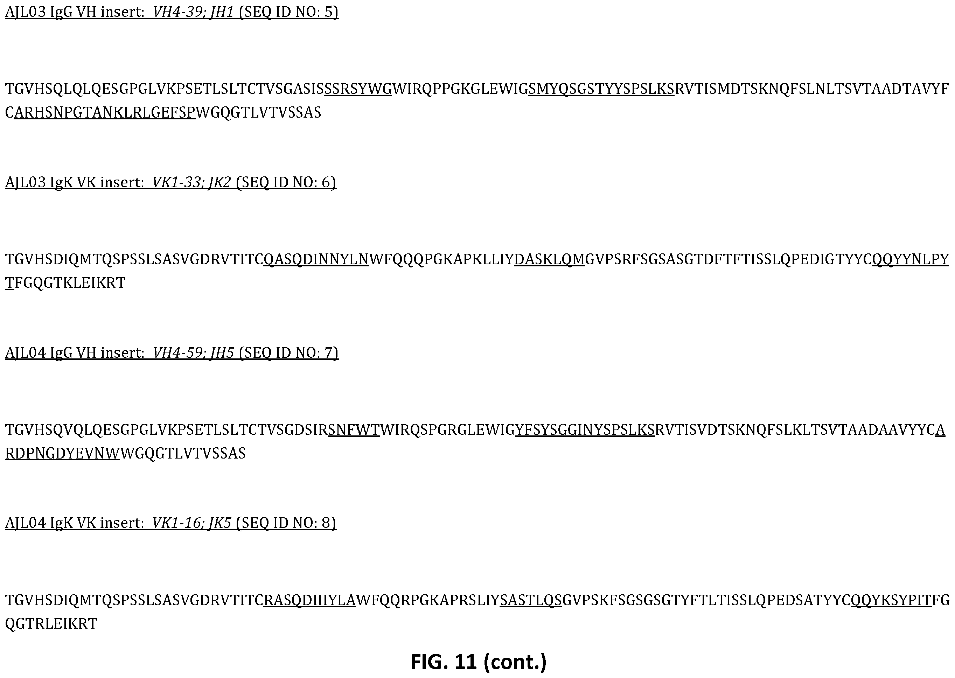

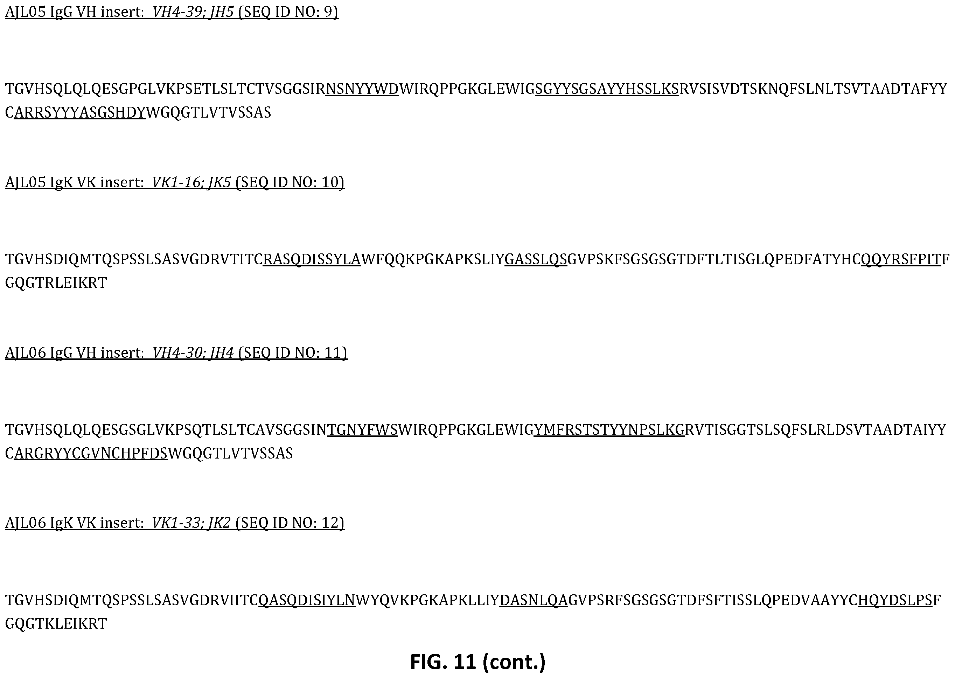

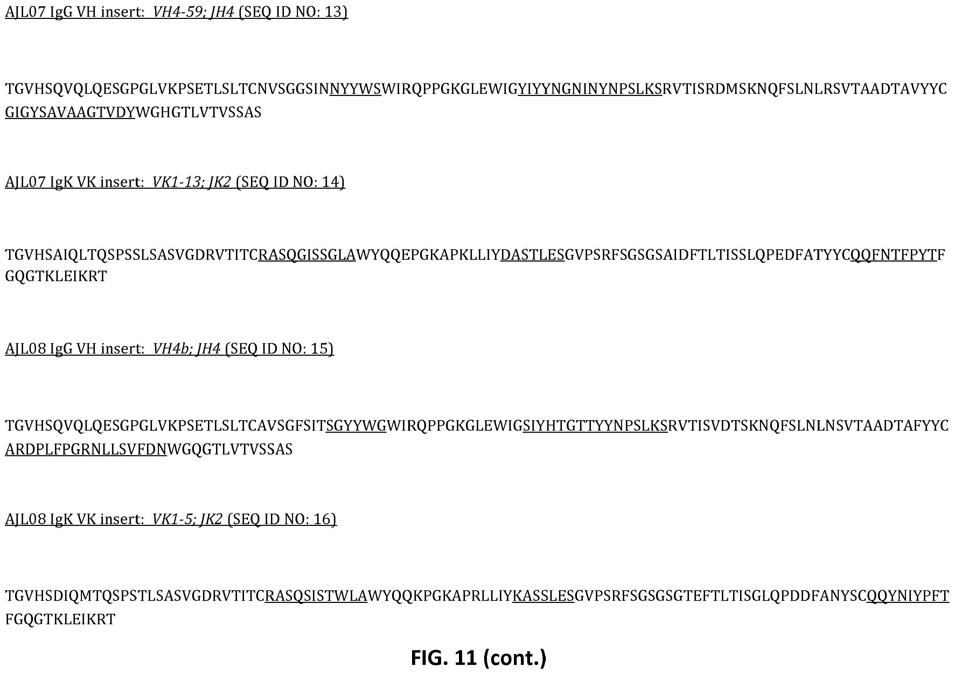

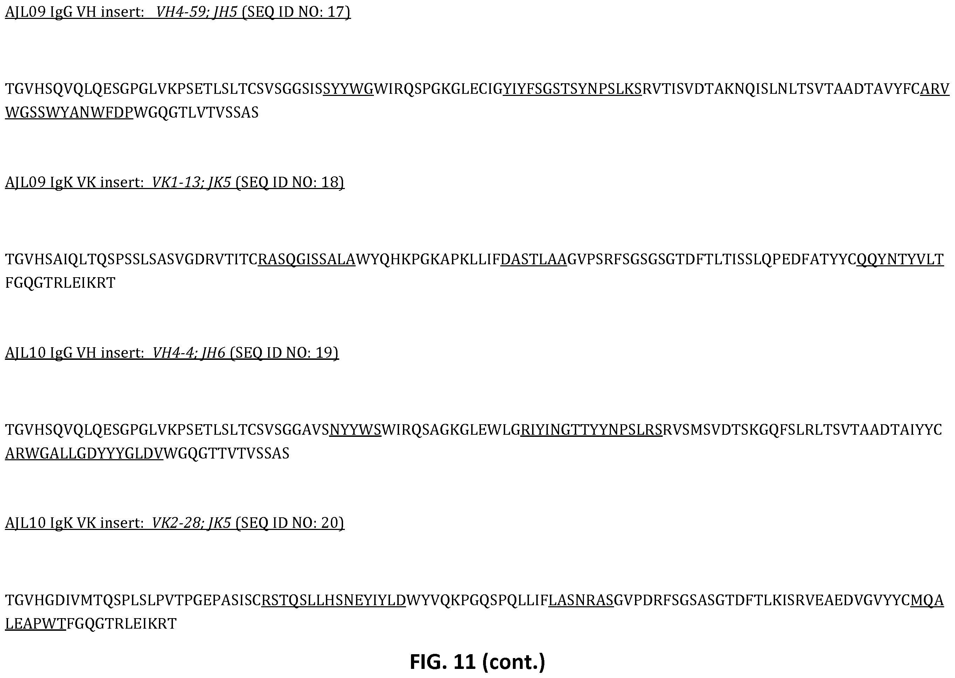

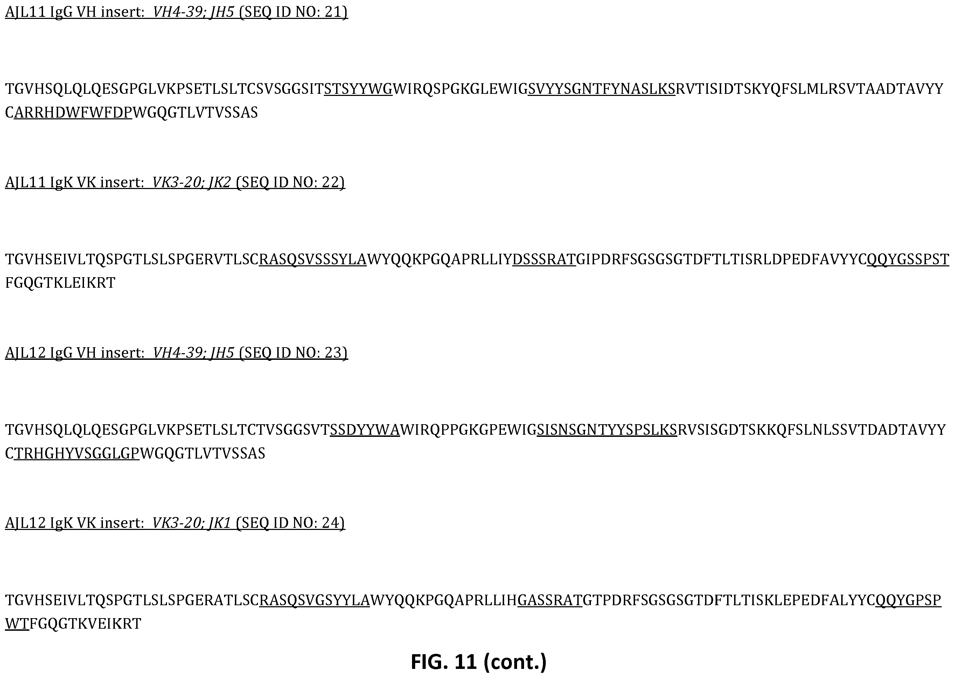

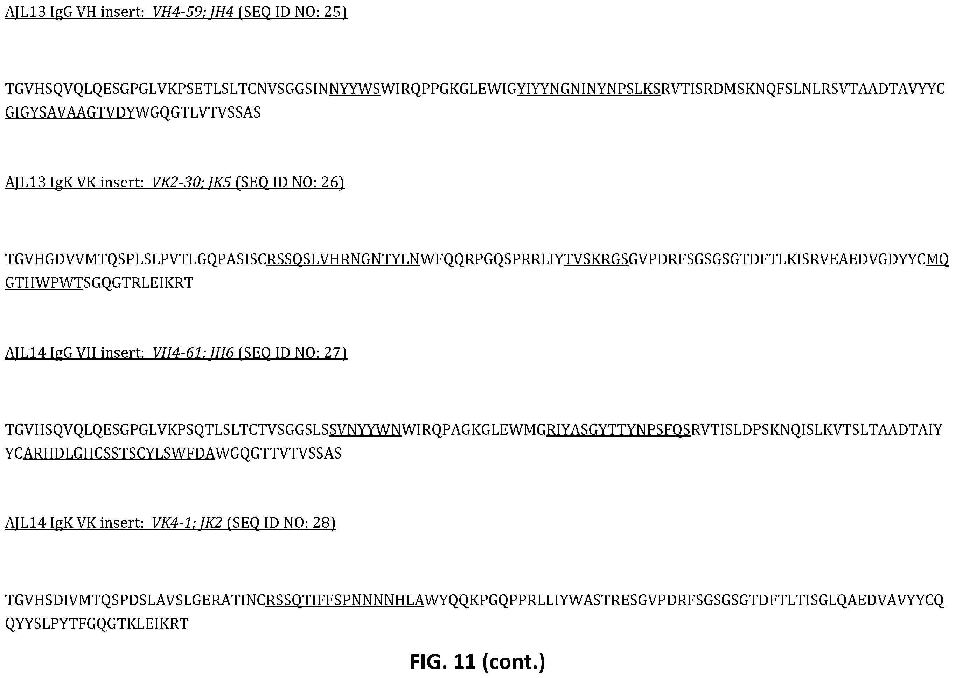

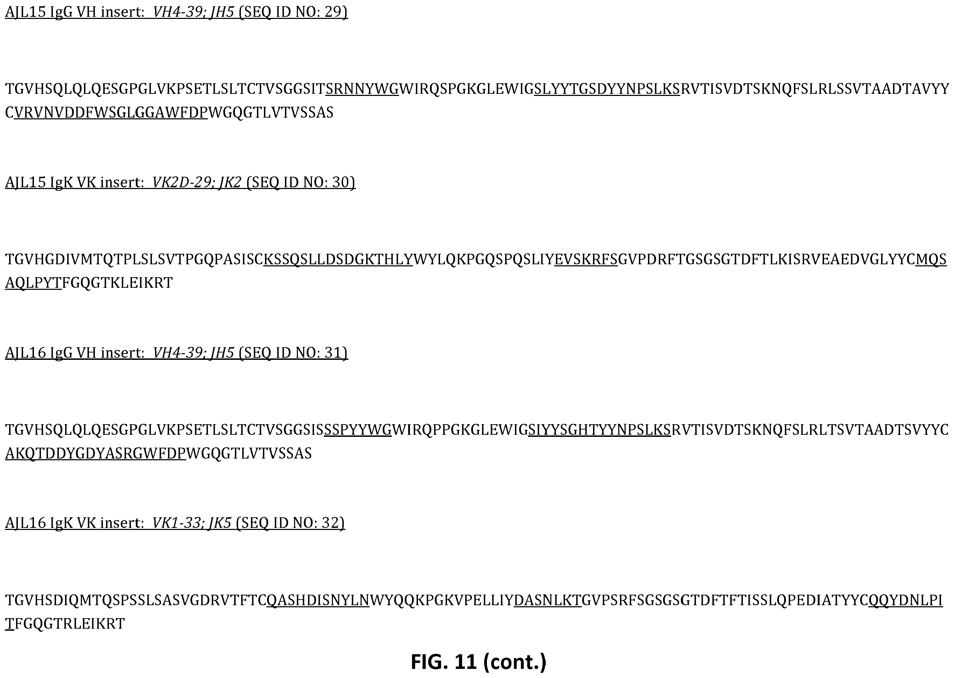

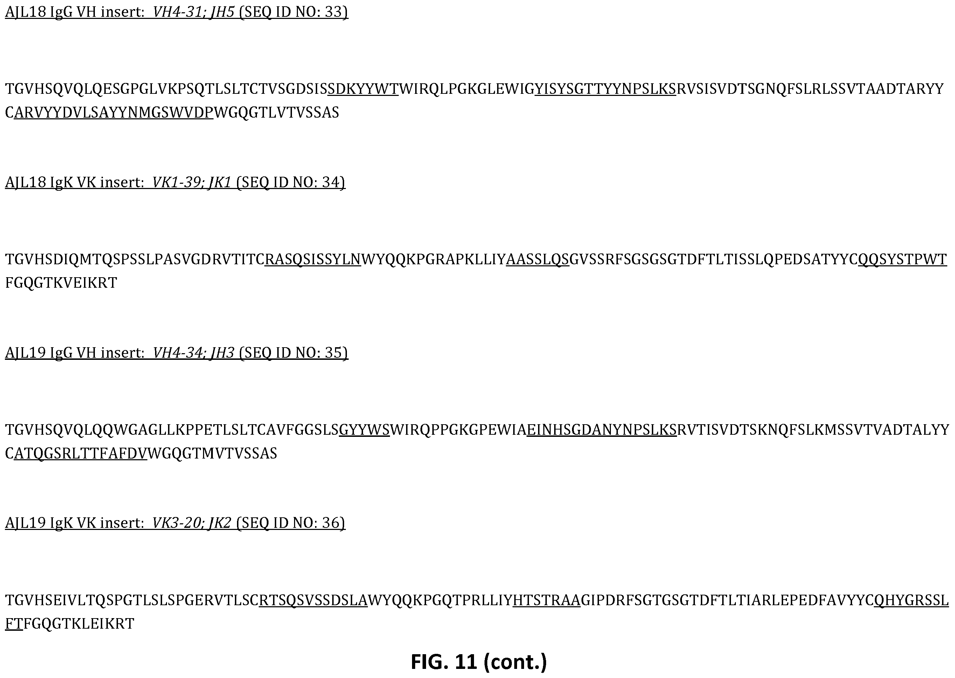

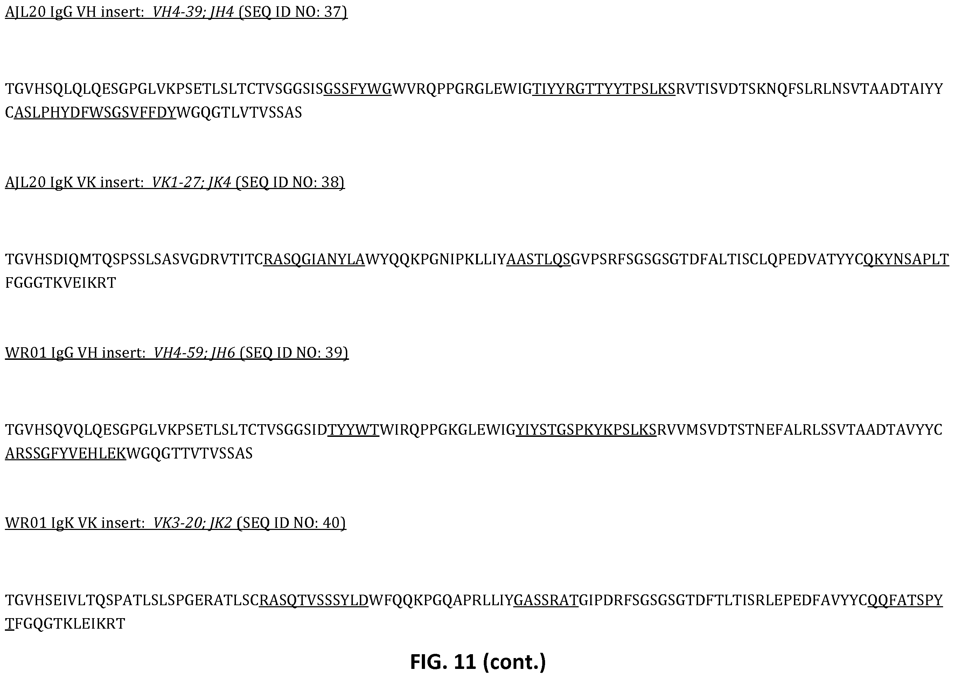

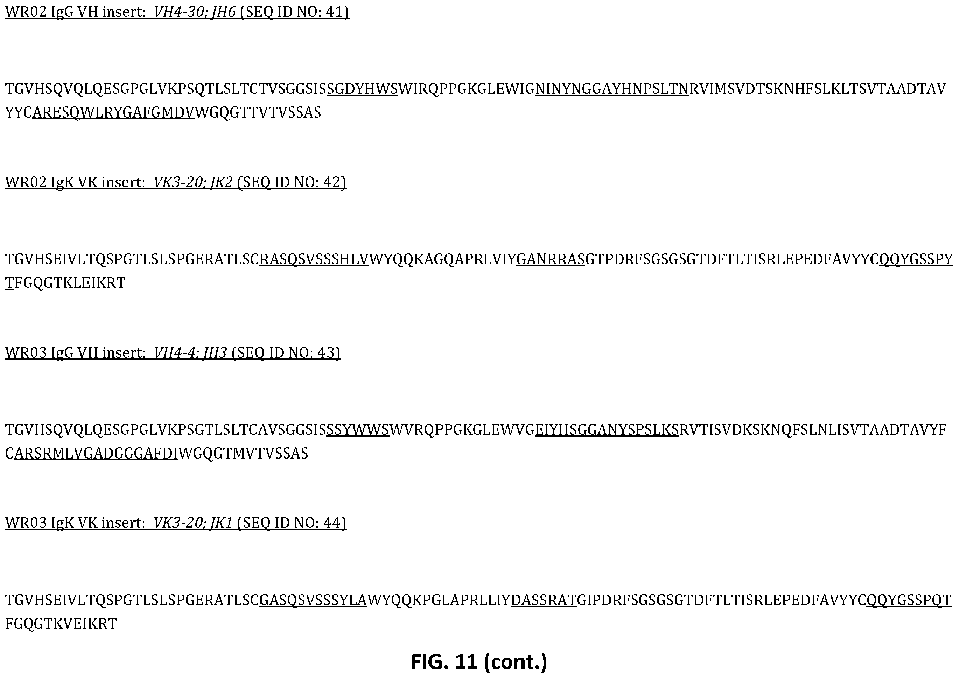

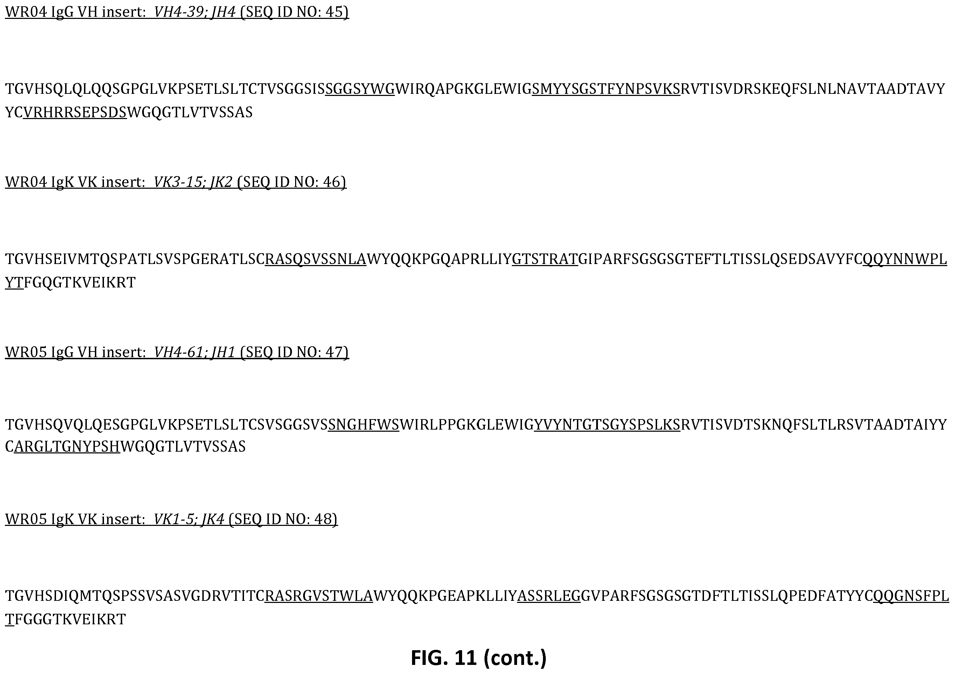

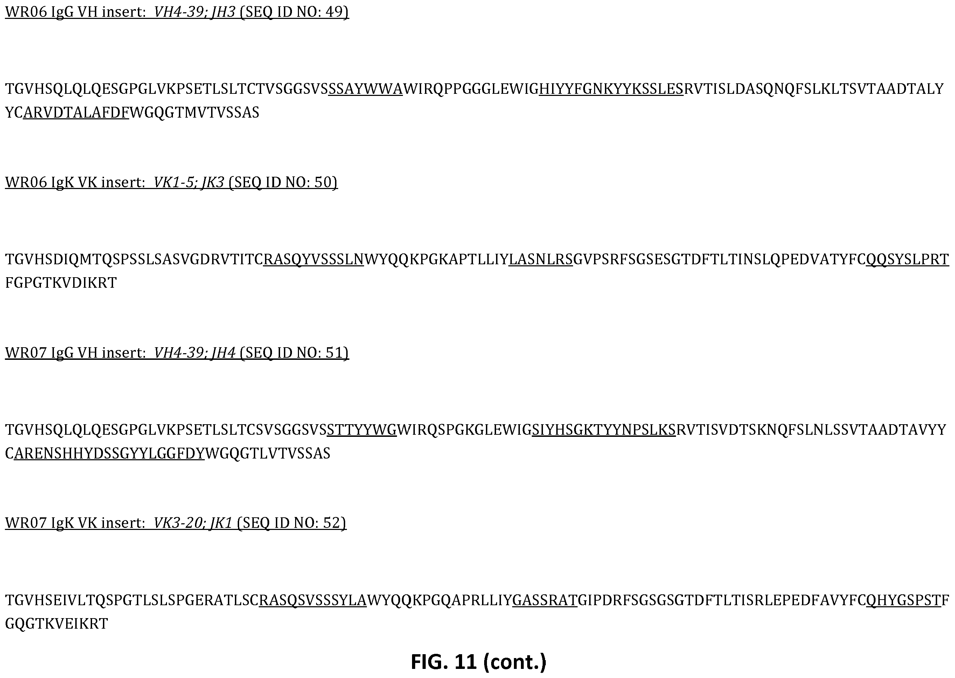

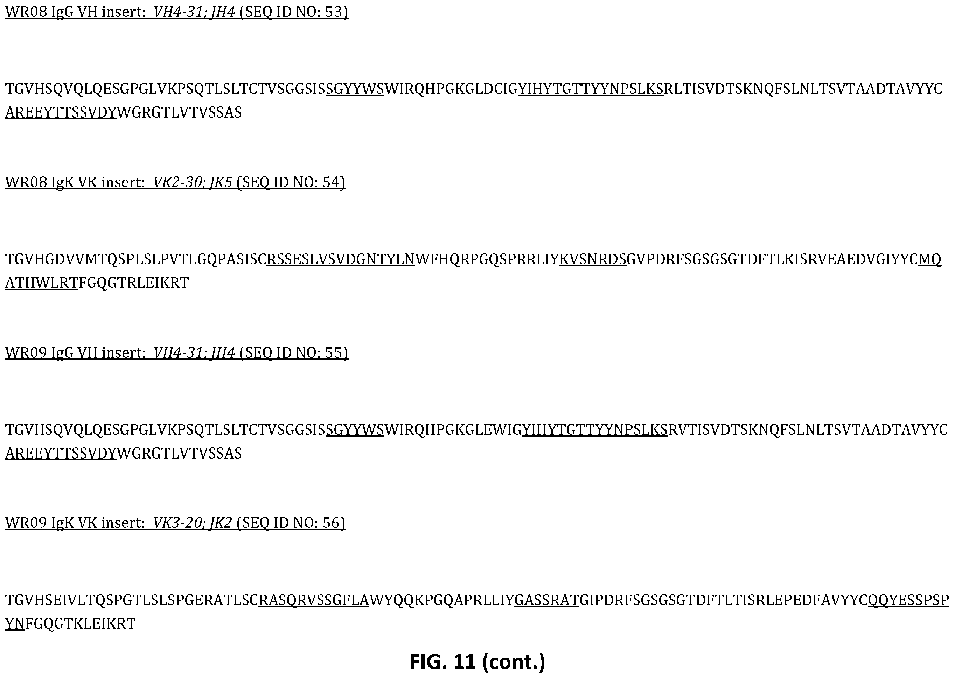

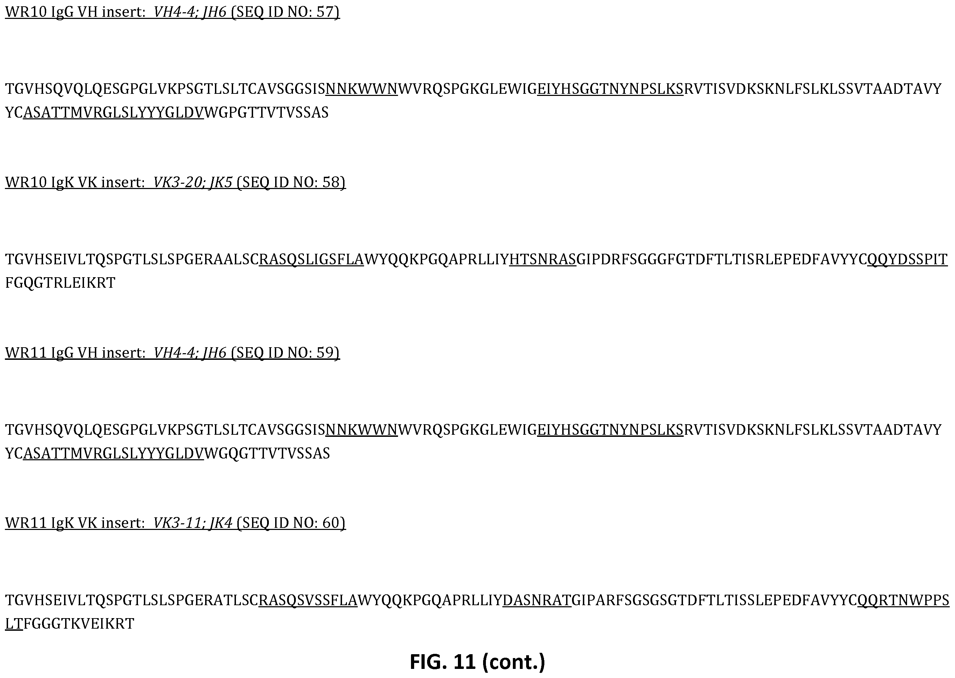

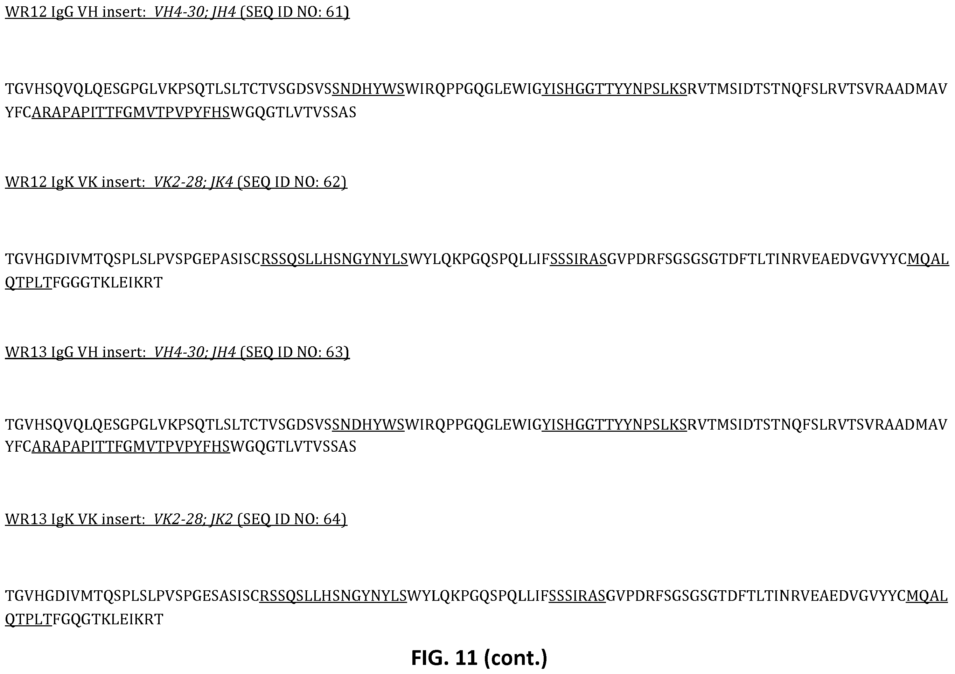

**FIG. 11****. Protein sequences for antibody variable regions.** Each consecutive pair of antibody sequences (heavy and light) present a single antibody. Underlined sequences are CDRs.

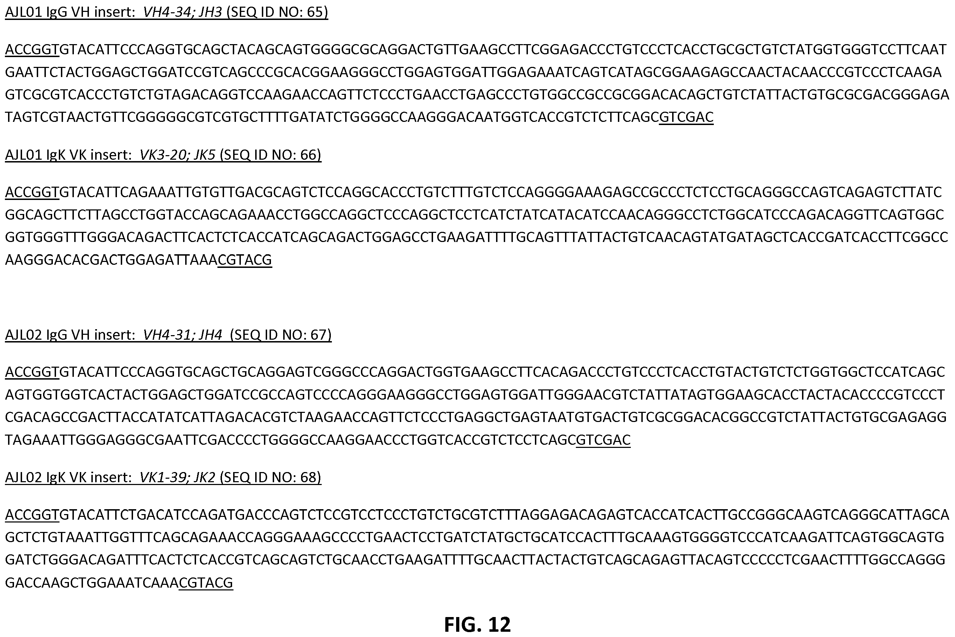

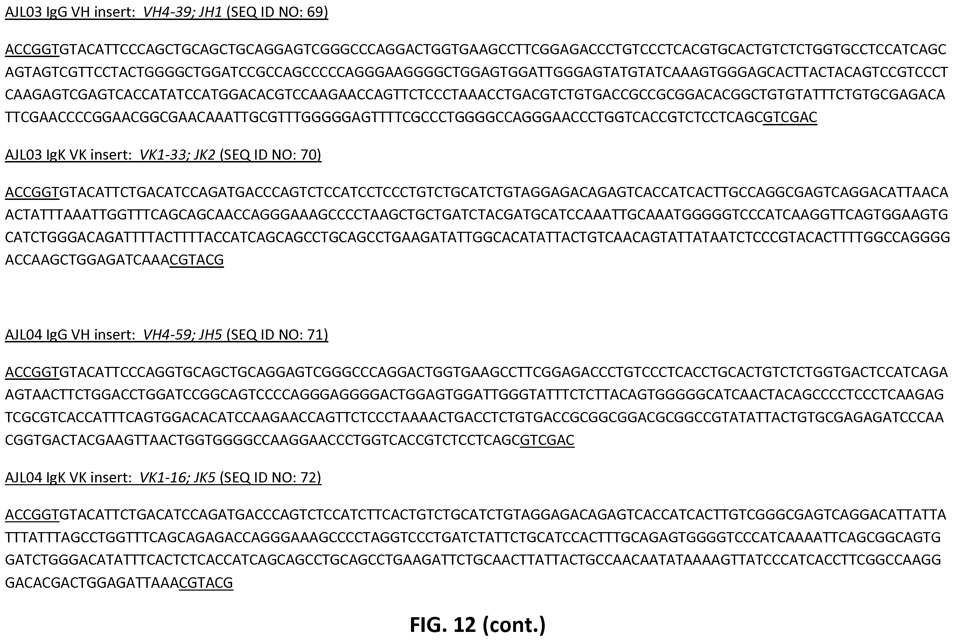

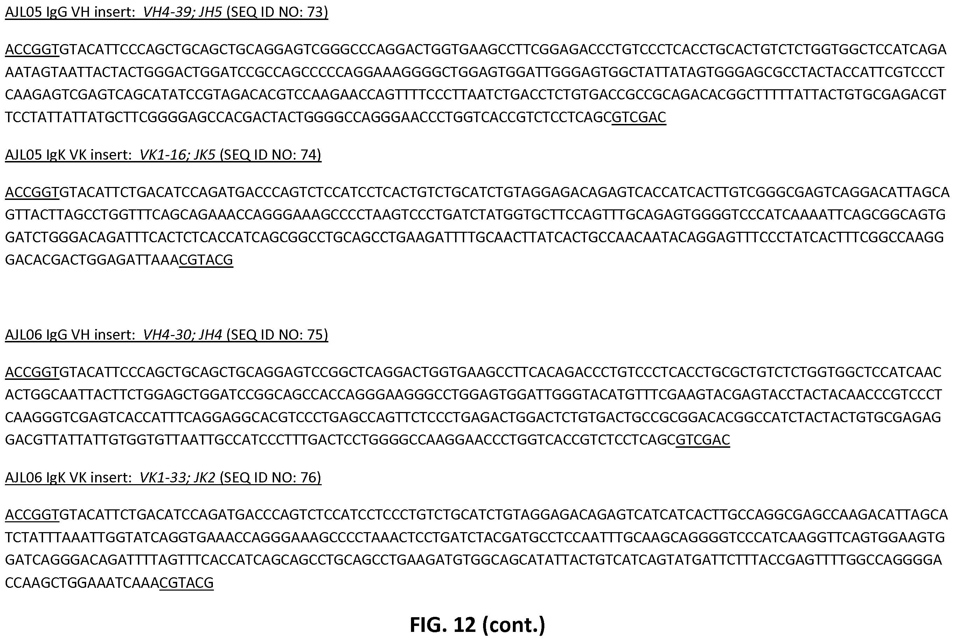

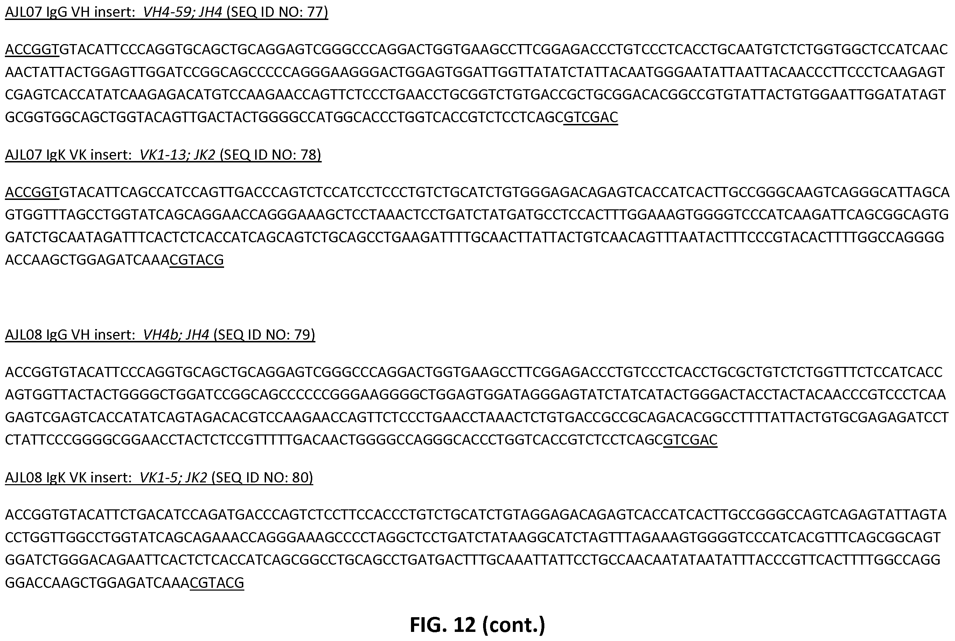

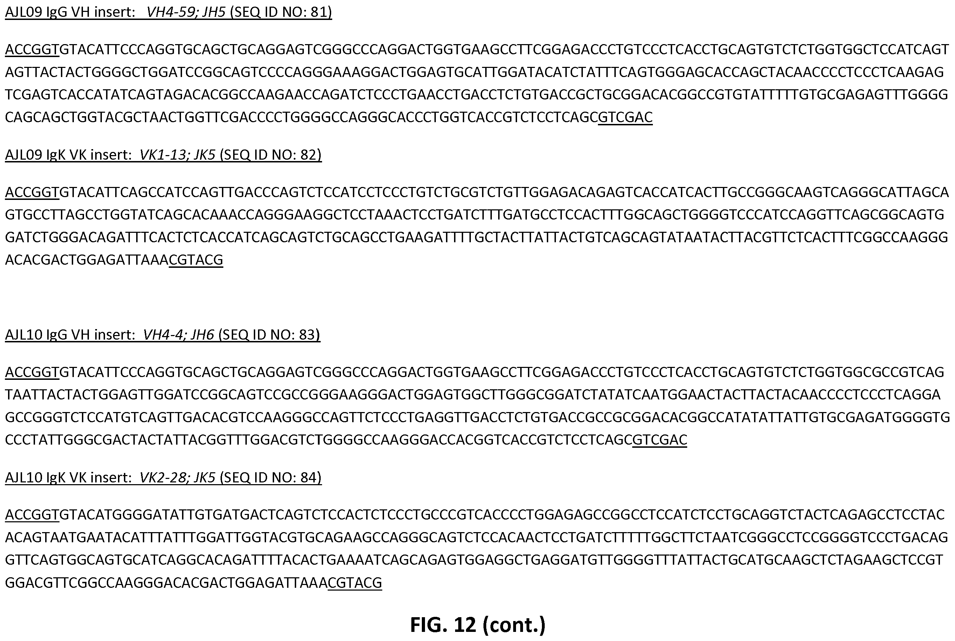

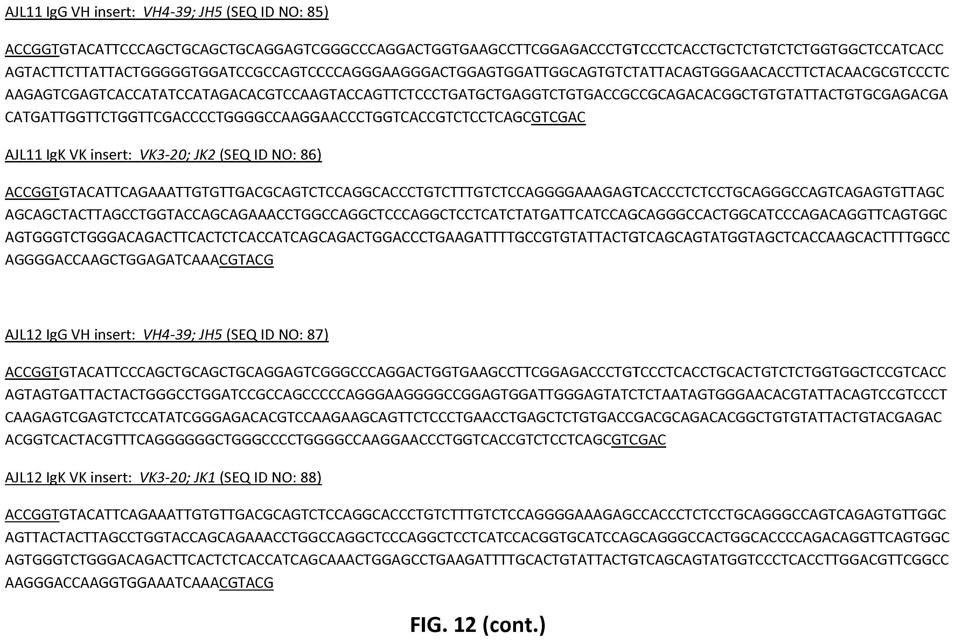

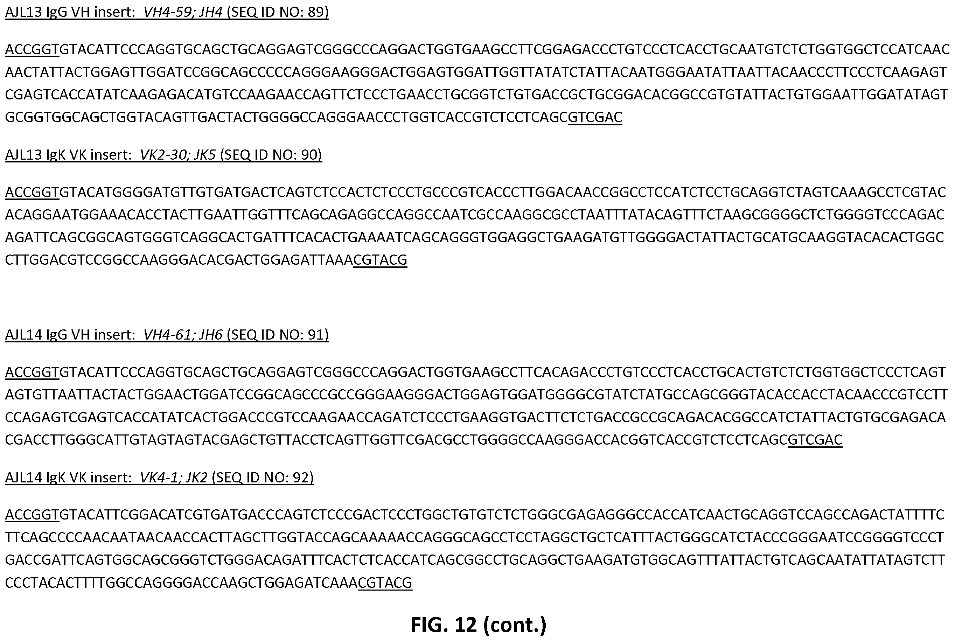

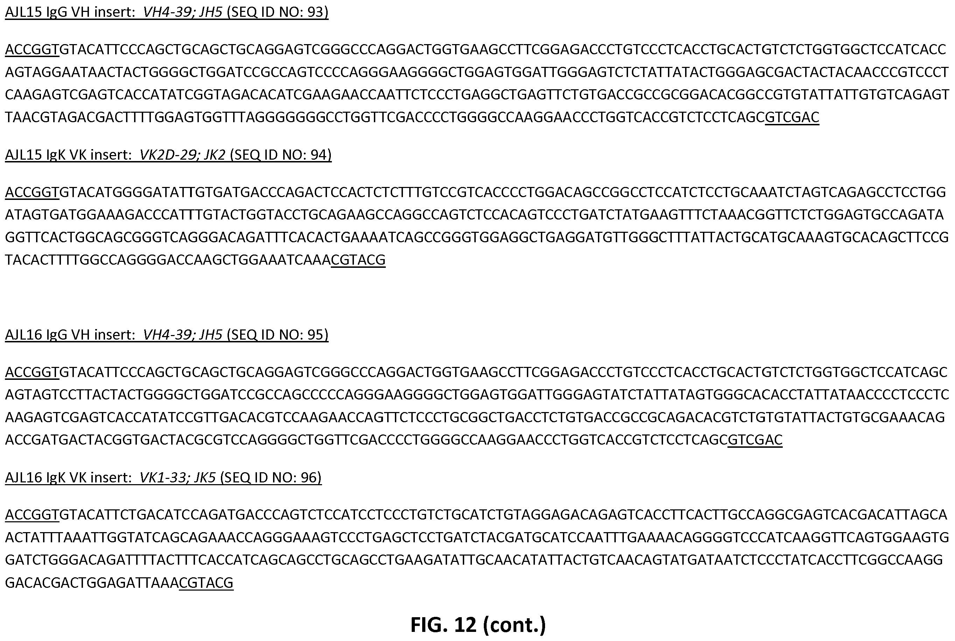

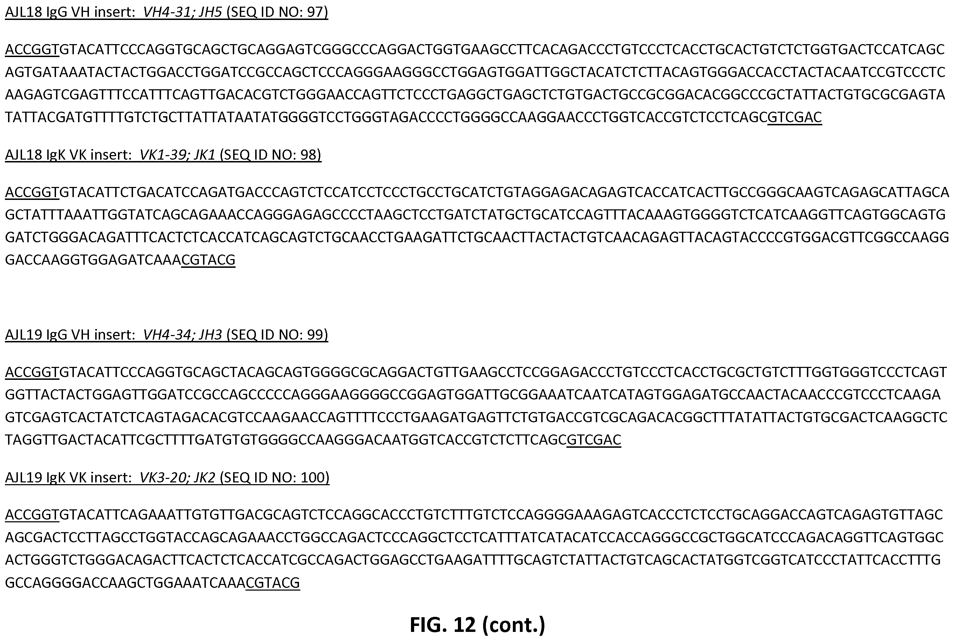

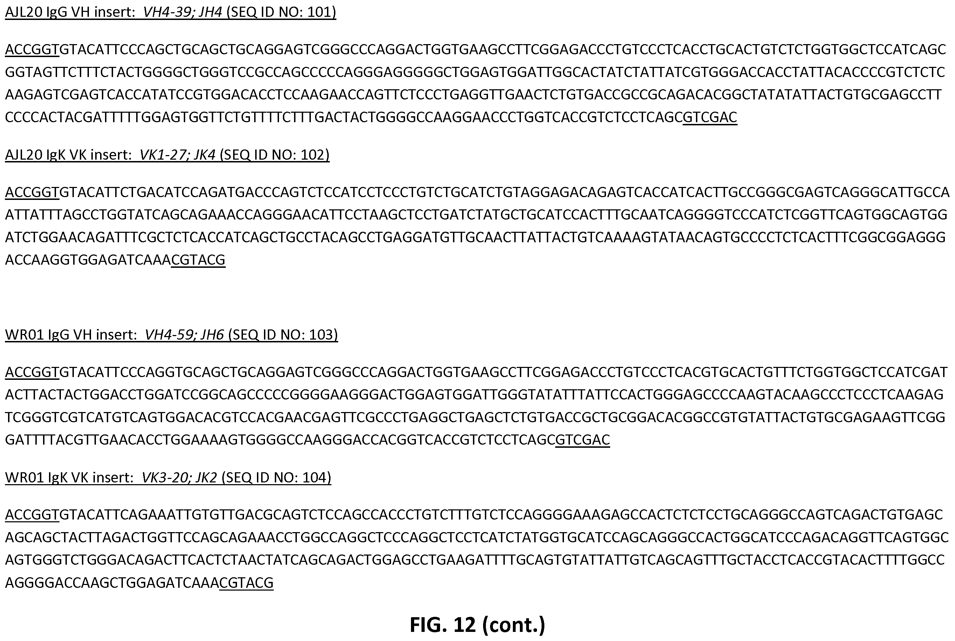

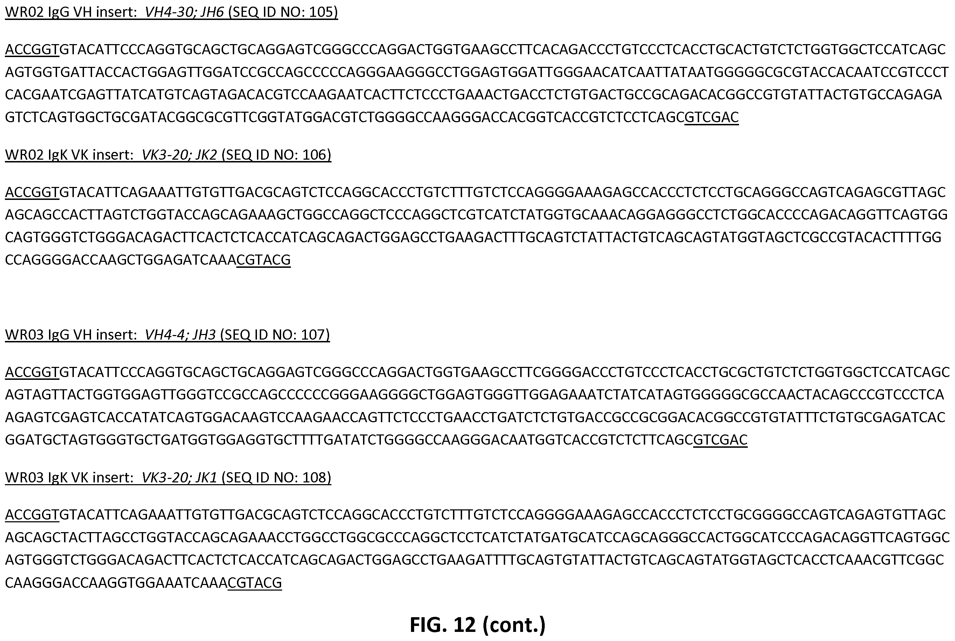

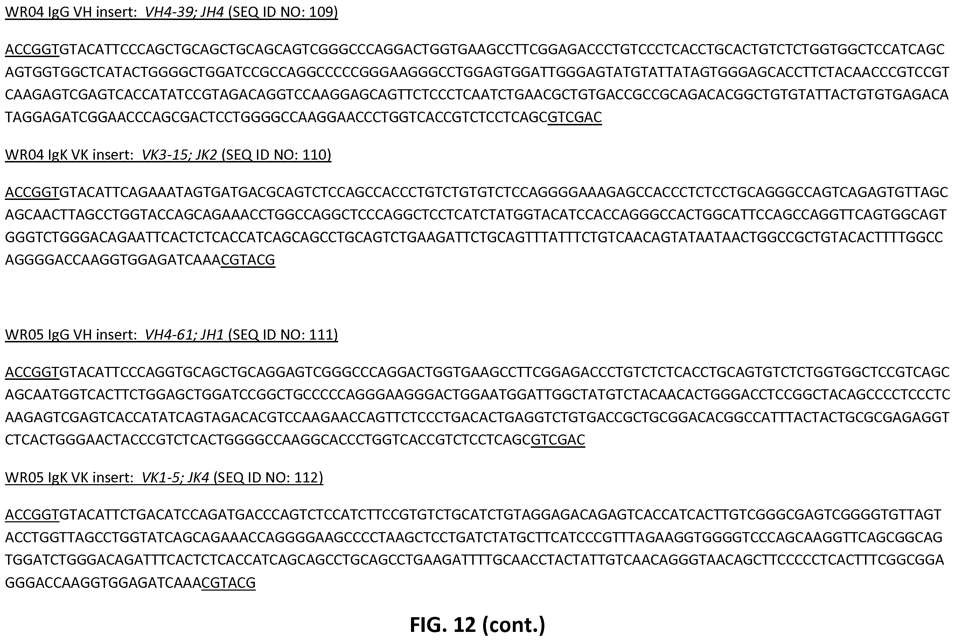

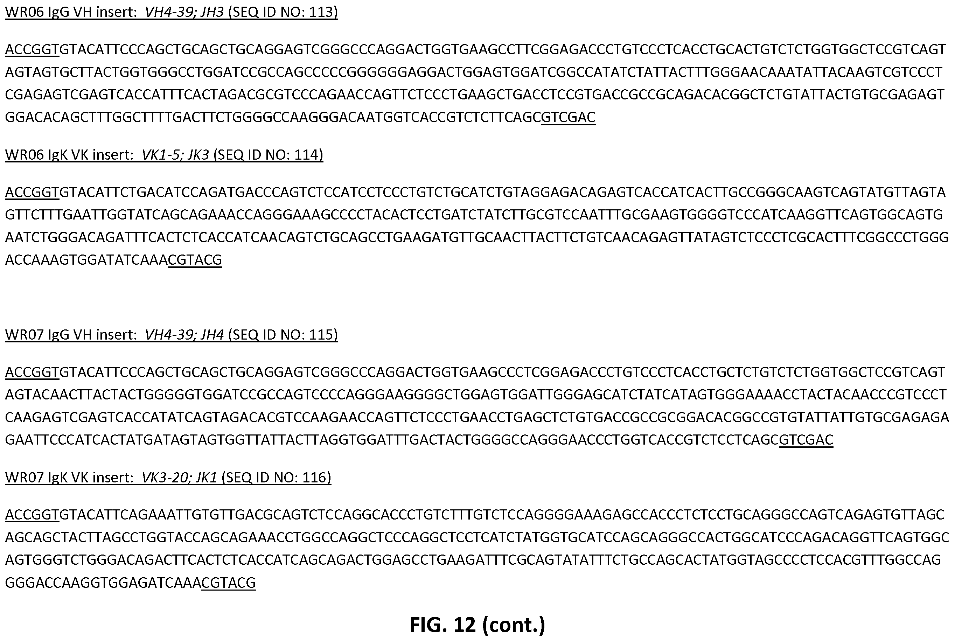

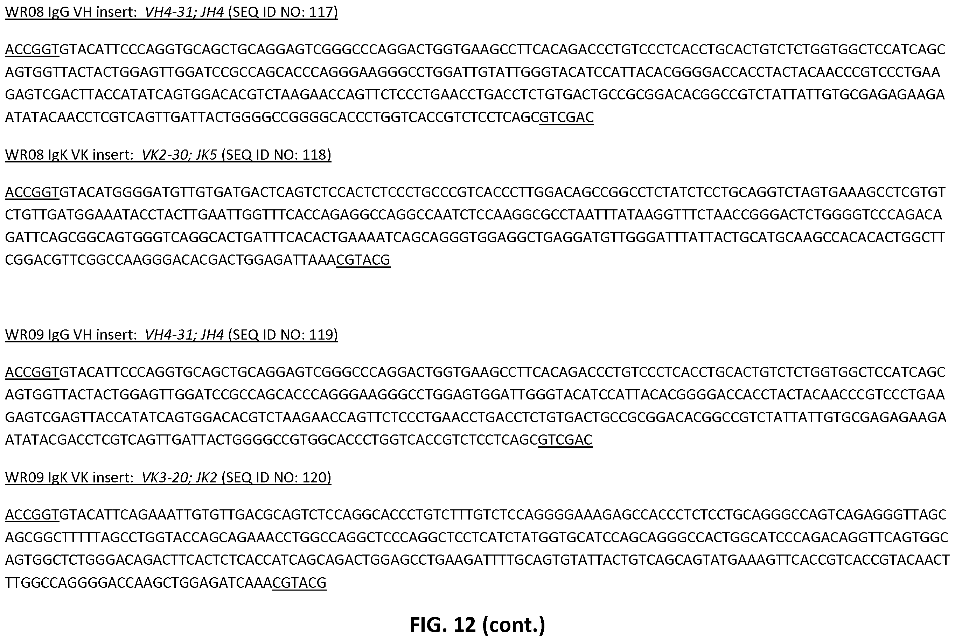

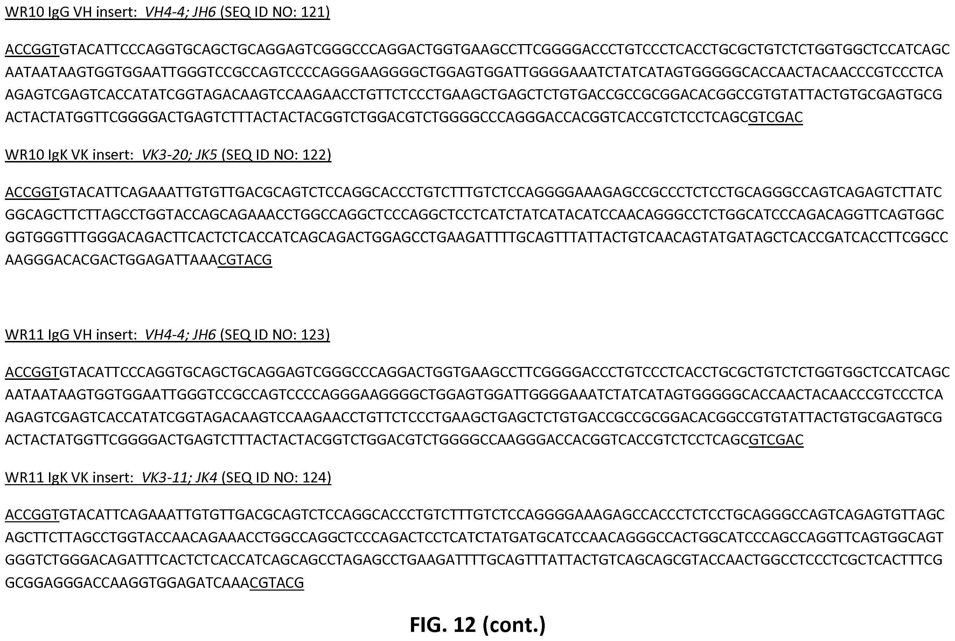

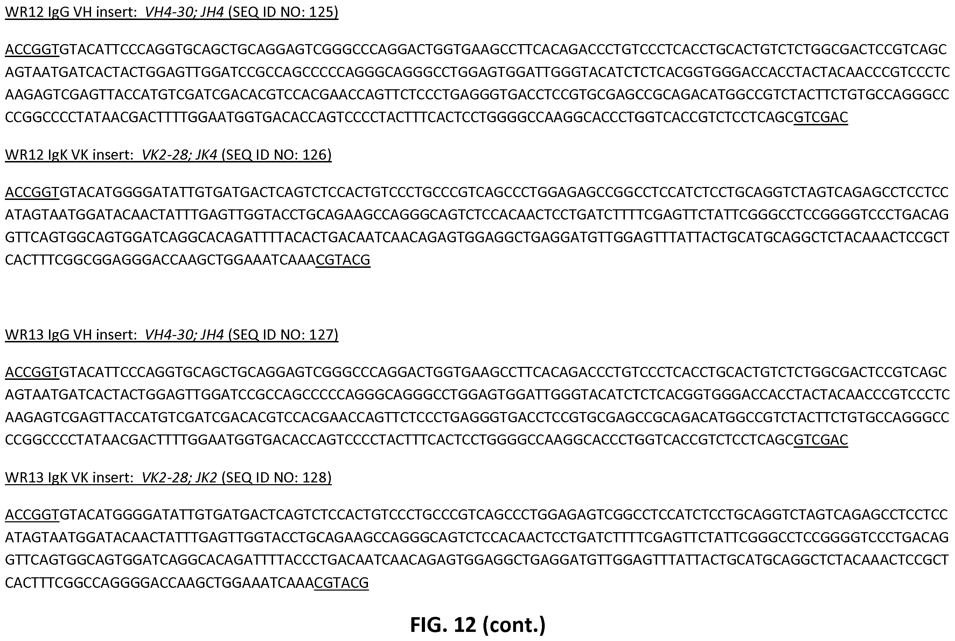

**FIG. 12****. Nucleic acid sequences for antibody variable regions.** Each consecutive pair of antibody sequences (heavy and light) present a single antibody. Underlined sequences are restriction sites.

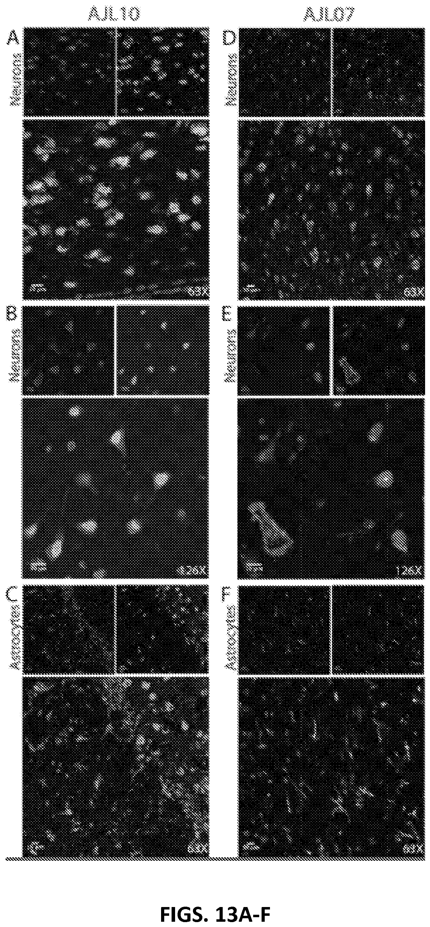

**FIGS. 13A-F****. IFC of AGS-enriched rhAbs targeting neuronal nuclei in both mouse and human MS-GM brain: AJL10 (ONCIS2) and AJL07 (ONCIS3).** Confocal images are shown at 63x magnification for the mouse tissue (FIGS. 13A and 13C; FIGS. 13D and 13F) and 126x for the human tissue (FIGS. 13B and 13E) with the colocalization marker for NeuN (for neuronal nuclei) shown as red (Alexa Fluor 594). The primary rhAb is shown as green (Alexa Fluor 488) and nuclei are counterstained blue (DAPI). The images are shown as independent red and green channels above the overlay including DAPI. Data are representative of six coronal sections per rhAb on mouse brain tissue and three sections per rhAb on MS-GM tissue. Scale bar represents 10 µm.

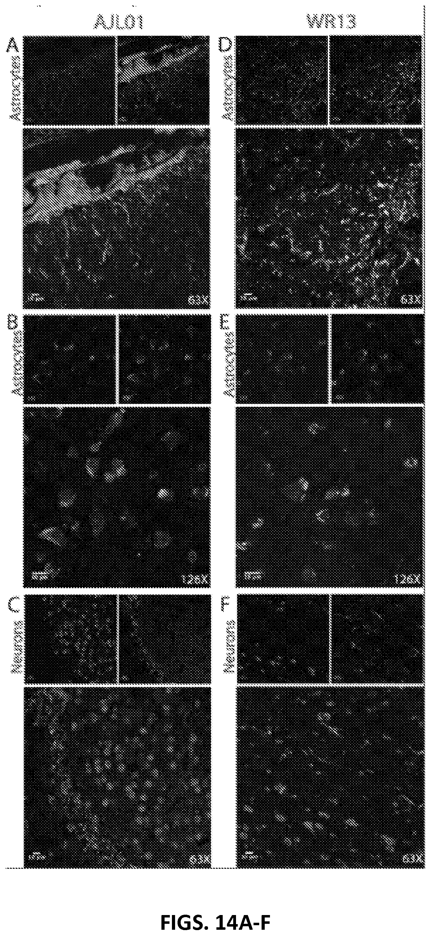

**FIGS. 14A-F****. IFC of AGS-enriched rhAbs targeting astrocytes in both mouse and human MS-GM brain: AJL01 (TMCIS4) and WR13 (ONCIS2).** Confocal images are shown at 63x magnification for the mouse tissue (FIGS. 14A and 14C; FIGS. 14D and 14F) and 126x for the human tissue (FIGS. 14B and 14E) with the colocalization marker for GFAP (for astrocytes) shown as red (Alexa Fluor 594). The primary rhAb is shown as green (Alexa Fluor 488) and nuclei are counterstained blue (DAPI). The images are shown as independent red and green channels above the overlay including DAPI. Data are representative of six coronal sections per rhAb on mouse brain tissue and three sections per rhAb on MS-GM tissue. Scale bar represents 10 µm.

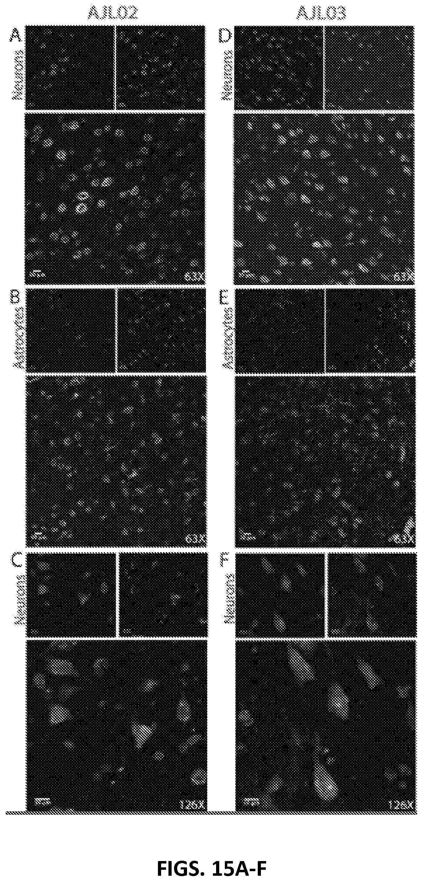

**FIGS. 15A-F****. CDMS derived AGS-enriched rhAbs demonstrate reactivity to neuronal nuclei and astrocytes: AJL02 and AJL03 (CDMS1).** Confocal images are shown at 63x magnification for the mouse tissue (FIGS. 15A-B; FIGS. 15D-E) and 126x for the human tissue (FIGS. 15C and 15F) with the colocalization marker for NeuN (neuronal nuclei) or GFAP (for astrocytes) shown as red (Alexa Fluor 594). The primary rhAb is shown as green (Alexa Fluor 488) and nuclei are counterstained blue (DAPI). The images are shown as independent red and green channels above the overlay including DAPI. Data are representative of six coronal sections per rhAb on mouse brain tissue and three sections per rhAb on MS-GM tissue. Scale bar represents 10 µm.

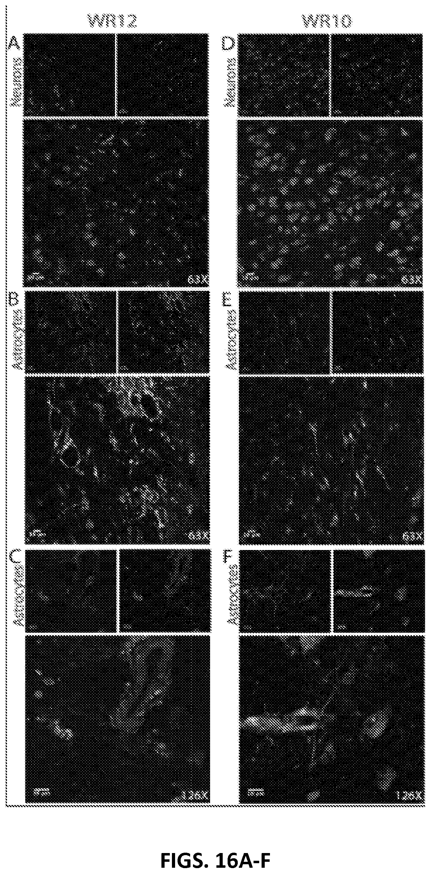

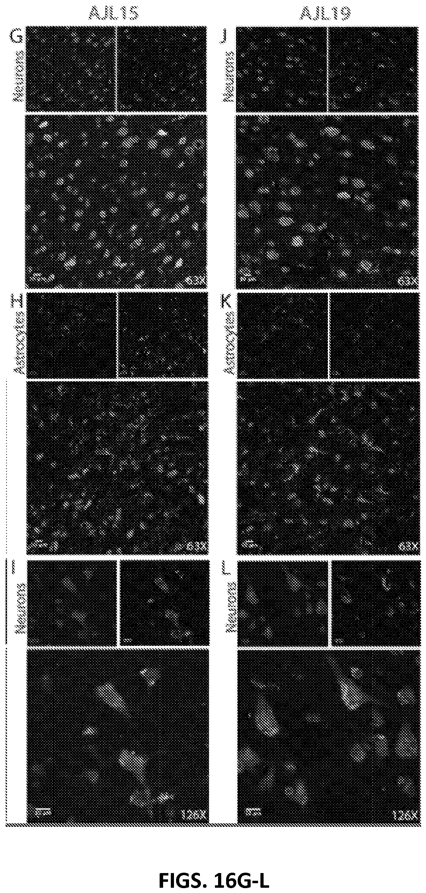

**FIGS. 16A-L****. CIS derived AGS-enriched rhAbs demonstrate reactivity to neuronal nuclei and astrocytes: WR12 (ONCIS2), WR10 (TMCIS4), AJL15 (TMCIS5), and AJL19 (TMCIS6).** Confocal images are shown at 63x magnification for the mouse tissue (FIGS. 16A, 16B, 16D, 16E, 16G, 16H, 16J, 16K) and 126x for the human tissue (FIGS. 16C, 16F, 16I, 16L) with the colocalization marker for NeuN (neuronal nuclei) or GFAP (for astrocytes) shown as red (Alexa Fluor 594). The primary rhAb is shown as green (Alexa Fluor 488) and nuclei are counterstained blue (DAPI). The images are shown as independent red and green channels above the overlay including DAPI. Data are representative of six coronal sections per rhAb on mouse brain tissue and three sections per rhAb on MS-GM tissue. Scale bar represents 10 µm.

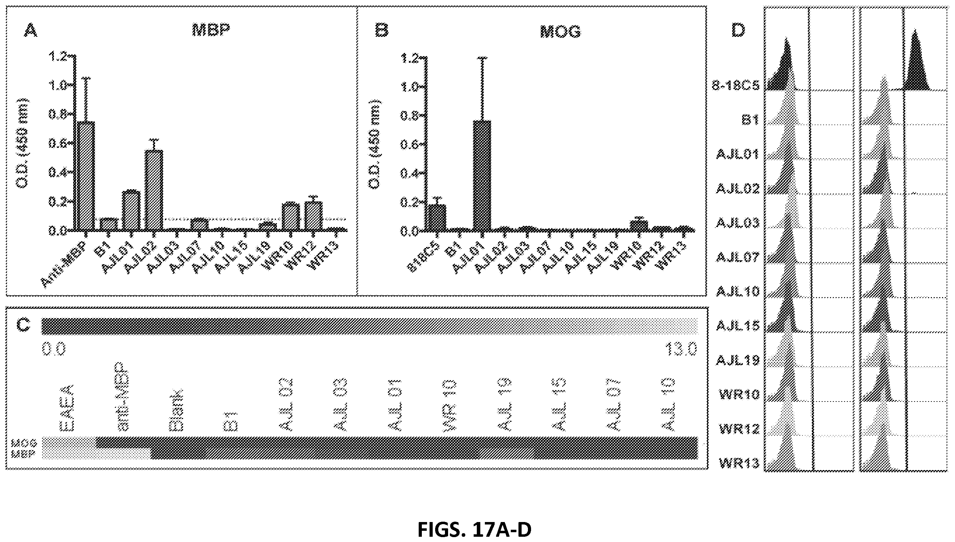

**FIGS. 17A-D****. AGS-enriched rhAbs do not bind strongly to myelin components.** (FIGS. 17A and B) Binding of 10 rhAbs to common myelin proteins MOG and MBP by ELISA. A dashed line represents the threshold for background signal, as observed with the negative control antibody B1. (FIG. 17C) Binding of 8 rhAbs to myelin-derived peptides demonstrate no reactivity compared to controls (EAEA and anti-MBP). (FIG. 17D) Binding of 10 rhAbs to HEK293 cells mock tranfected (left column) or transfected with MOG (right column) demonstrated no reactivity compared to control (8-18C5).

### DESCRIPTION OF ILLUSTRATIVE EMBODIMENTS

As discussed above, the inventor's laboratory has discovered a biomarker for conversion from CIS to clinically definite MS (CDMS) in the antibody genetics of V_{H}4-utilizing B cells in the CSF, termed the antibody gene signature (AGS) (Cameron *et al.,* 2009). She also found that B cells isolated from CNS lesions harbor the AGS (Ligocki *et al.,* 2010). This shared pattern of somatic hypermutation at 6 codons along the V_{H}4 gene implicates that the B cell pools are recognizing a shared set of antigens in the MS disease state that are not recognized by B cells in healthy individuals. Thus, the inventor hypothesized that AGS-enriched antibodies may bind to targets within the CNS. To address this hypothesis, she generated a panel of 32 full-length recombinant human antibodies (rhAbs) from single CSF B cells whose antibody genes contained AGS-targeted mutations. Surveying B cells and antibodies within the CSF is relevant to CNS disease because there are shared B cell clones between the same MS patient's CSF and CNS (Obermeier *et al.,* 2011), as well as between the meninges and CNS (Lovato *et al*., 2011). This panel of 32 rhAbs came from a diverse set of patients including CDMS and two initial CIS presentations (optic neuritis (ON_{CIS}) and transverse myelitis (TM_{CIS})). ON_{CIS} patients present with optic symptoms and lesions along the optic nerve, and TM_{CIS} patients exhibit sensory symptoms with lesions along short segments of the spinal cord. Regardless of either presentation of CIS, both patient types have CSF B cells pools enriched for AGS and are at high risk of converting to CDMS.

CNS targeting of the rhAb panel was determined using immunohistochemistry on both mouse and human brain tissue. Surprisingly, the AGS-enriched B cells targeted gray matter (GM) rather than the anticipated myelin-rich white matter (WM), which has been extensively studied in the MS field (Lassmann *et al.,* 2007). GM involvement in MS disease symptoms and advancement has been understudied even though the presence of cortical lesions correlates strongly with MS disease severity and progression as opposed to the more easily detected WM lesions (Bo *et al.,* 2007, Fisniku *et al.,* 2008 and Vercellino *et al.,* 2005). In fact, cortical GM demyelination is more extensive than WM (26.5% vs 6.5%) with the percentage of demyelination in the cortex increasing with disability and disease length (Bo *et al.,* 2003). Immunofluorescence confirmed GM targeting of the rhAbs to astrocyte bodies and processes, and neuronal nuclei in both human and mouse brain tissue. The inventor also found this targeting pattern in AGS-enriched rhAbs generated from both CIS presentations, ON_{CIS} and TM_{CIS}, as well as established CDMS. This is the first known description of MS derived antibodies sharing a mutational pattern that target GM and this previously unrecognized target of humoral immunity may elucidate the pathology and symptoms stemming from cortical damage in disease. These and other aspects of the disclosure are described in greater detail below.

### I. Multiple Sclerosis

### A. Multiple Sclerosis

Multiple Sclerosis (MS) is one of the most common diseases of the central nervous system (brain and spinal cord). It is an inflammatory condition associated with demyelination, or loss of the myelin sheath. Myelin, a fatty material that insulates nerves, acts as insulator in allowing nerves to transmit impulses from one point to another. In MS, the loss of myelin is accompanied by a disruption in the ability of the nerves to conduct electrical impulses to and from the brain and this produces the various symptoms of MS, such as impairments in vision, muscle coordination, strength, sensation, speech and swallowing, bladder control, sexuality and cognitive function. The plaques or lesions where myelin is lost appear as hardened, scar-like areas. These scars appear at different times and in different areas of the brain and spinal cord, hence the term "multiple" sclerosis, literally meaning many scars.

Currently, there is no single laboratory test, symptom, or physical finding that provides a conclusive diagnosis of MS. To complicate matters, symptoms of MS can easily be confused with a wide variety of other diseases such as acute disseminated encephalomyelitis, Lyme disease, HIV-associated myelopathy, HTLV-I-associated myelopathy, neurosyphilis, progressive multifocal leukoencephalopathy, systemic lupus erythematosus, polyarteritis nodosa, Sjogren's syndrome, Behcet's disease, sarcoidosis, paraneoplastic syndromes, subacute combined degeneration of cord, subacute myelo-optic neuropathy, adrenomyeloneuropathy, spinocerebellar syndromes, hereditary spastic paraparesis/primary lateral sclerosis, strokes, tumors, arteriovenous malformations, arachnoid cysts, Arnold-Chiari malformations, and cervical spondylosis. Consequently, the diagnosis of MS must be made by a process that demonstrates findings that are consistent with MS, and also rules out other causes.

Generally, diagnosis of MS relies on two criteria. First, there must have been two attacks at least one month apart. An attack, also known as an exacerbation, flare, or relapse, is a sudden appearance of or worsening of an MS symptom or symptoms which lasts at least 24 hours. Second, there must be more than one area of damage to central nervous system myelin sheath. Damage to sheath must have occurred at more than one point in time and not have been caused by any other disease that can cause demyelination or similar neurologic symptoms. MRI (magnetic resonance imaging) currently is the preferred method of imaging the brain to detect the presence of plaques or scarring caused by MS.

The diagnosis of MS cannot be made, however, solely on the basis of MRI. Other diseases can cause comparable lesions in the brain that resemble those caused by MS. Furthermore, the appearance of brain lesions by MRI can be quite heterogeneous in different patients, even resembling brain or spinal cord tumors in some. In addition, a normal MRI scan does not rule out a diagnosis of MS, as a small number of patients with confirmed MS do not show any lesions in the brain on MRI. These individuals often have spinal cord lesions or lesions which cannot be detected by MRI. As a result, it is critical that a thorough clinical exam also include a patient history and functional testing. This should cover mental, emotional, and language functions, movement and coordination, vision, balance, and the functions of the five senses. Sex, birthplace, family history, and age of the person when symptoms first began are also important considerations. Other tests, including evoked potentials (electrical diagnostic studies that may reveal delays in central nervous system conduction times), cerebrospinal fluid (seeking the presence of clonally-expanded immunoglobulin genes, referred to as oligoclonal bands), and blood (to rule out other causes), may be required in certain cases.

### B. Therapy and Prophylaxis

It may be that, on the basis of the diagnosis or prediction provided by the methods described herein, one will wish to begin, end or modify a therapeutic regimen. In particular, subjects diagnosed as having or at risk of developing MS may be started on a therapeutic regimen. The primary aims of therapy are returning function after an attack, preventing new attacks, and preventing disability. As with any medical treatment, medications used in the management of MS have several adverse effects, and many possible therapies are still under investigation.

During symptomatic attacks, administration of high doses of intravenous corticosteroids, such as methylprednisolone, is the routine therapy for acute relapses. The aim of this kind of treatment is to end the attack sooner and leave fewer lasting deficits in the patient. Although generally effective in the short term for relieving symptoms, corticosteroid treatments do not appear to have a significant impact on long-term recovery. Potential side effects include osteoporosis and impaired memory, the latter being reversible.

The earliest clinical presentation of relapsing-remitting MS (RRMS) is the clinically isolated syndrome (CIS). Several studies have shown that treatment with interferons during an initial attack can decrease the chance that a patient will develop MS. As of 2007, six disease-modifying treatments have been approved by regulatory agencies of different countries for relapsing-remitting MS. Three are interferons: two formulations of interferon beta-la (trade names Avonex and Rebif) and one of interferon β-1b (U.S. trade name Betaseron®, in Europe and Japan Betaferon). A fourth medication is glatiramer acetate (Copaxone®). The fifth medication, mitoxantrone, is an immunosuppressant also used in cancer chemotherapy, is approved only in the USA and largely for SPMS. Finally, the sixth is natalizumab (marketed as Tysabri®). All six medications are modestly effective at decreasing the number of attacks and slowing progression to disability, although they differ in their efficacy rate and studies of their long-term effects are still lacking. Comparisons between immunomodulators (all but mitoxantrone) show that the most effective is natalizumab, both in terms of relapse rate reduction and halting disability progression; it has also been shown to reduce the severity of MS. Mitoxantrone may be the most effective of them all; however, it is generally considered not as a long-term therapy as its use is limited by severe cardiotoxicity.

The interferons and glatiramer acetate are delivered by frequent injections, varying from once-per-day for glatiramer acetate to once-per-week (but intra-muscular) for Avonex. Natalizumab and mitoxantrone are given by IV infusion at monthly intervals. Treatment of progressive MS is more difficult than relapsing-remitting MS. Mitoxantrone has shown positive effects in patients with secondary progressive and progressive relapsing courses. It is moderately effective in reducing the progression of the disease and the frequency of relapses in patients in short-term follow-up. On the other hand no treatment has been proven to modify the course of primary progressive MS.

Disease-modifying treatments only reduce the progression rate of the disease but do not stop it. As multiple sclerosis progresses, the symptomatology tends to increase. The disease is associated with a variety of symptoms and functional deficits that result in a range of progressive impairments and handicap. Management of these deficits is therefore very important. Both drug therapy and neurorehabilitation have shown to ease the burden of some symptoms, even though neither influence disease progression. As for any patient with neurologic deficits, a multidisciplinary approach is key to limiting and overcoming disability; however there are particular difficulties in specifying a 'core team' because people with MS may need help from almost any health profession or service at some point. Similarly for each symptom there are different treatment options. Treatments should therefore be individualized depending both on the patient and the physician.

### II. Producing and Use of Monoclonal Antibodies

### A. General Methods

Antibodies according to the present disclosure may be produced by standard methods as are well known in the art (see, *e.g*., Antibodies: A Laboratory Manual, Cold Spring Harbor Laboratory, 1988; U.S. Patent 4,196,265). In brief, somatic cells with the potential for producing antibodies, specifically B lymphocytes (B cells), are selected. These cells may be obtained from biopsied spleens or lymph nodes, or from circulating blood. The antibody-producing B lymphocytes from the immunized animal are then fused with cells of an immortal myeloma cell, generally one of the same species as the animal that was immunized or human or human/mouse chimeric cells. Myeloma cell lines suited for use in hybridoma-producing fusion procedures preferably are non-antibody-producing, have high fusion efficiency, and enzyme deficiencies that render then incapable of growing in certain selective media which support the growth of only the desired fused cells (hybridomas).

Methods for generating hybrids of antibody-producing spleen or lymph node cells and myeloma cells usually comprise mixing somatic cells with myeloma cells in a 2:1 proportion, though the proportion may vary from about 20:1 to about 1:1, respectively, in the presence of an agent or agents (chemical or electrical) that promote the fusion of cell membranes. Fusion methods using Sendai virus have been described by Kohler and Milstein (1975; 1976), and those using polyethylene glycol (PEG), such as 37% (v/v) PEG, by Gefter *et al.* (1977). The use of electrically induced fusion methods also is appropriate (Goding, pp. 71-74, 1986).

Fusion procedures usually produce viable hybrids at low frequencies, about 1 × 10⁻⁶ to 1 × 10⁻⁸. However, this does not pose a problem, as the viable, fused hybrids are differentiated from the parental, infused cells (particularly the infused myeloma cells that would normally continue to divide indefinitely) by culturing in a selective medium. The selective medium is generally one that contains an agent that blocks the *de novo* synthesis of nucleotides in the tissue culture media. Exemplary and preferred agents are aminopterin, methotrexate, and azaserine. Aminopterin and methotrexate block *de novo* synthesis of both purines and pyrimidines, whereas azaserine blocks only purine synthesis. Where aminopterin or methotrexate is used, the media is supplemented with hypoxanthine and thymidine as a source of nucleotides (HAT medium). Where azaserine is used, the media is supplemented with hypoxanthine. Ouabain is added if the B cell source is an Epstein Barr virus (EBV) transformed human B cell line, in order to eliminate EBV transformed lines that have not fused to the myeloma.

The preferred selection medium is HAT or HAT with ouabain. Only cells capable of operating nucleotide salvage pathways are able to survive in HAT medium. The myeloma cells are defective in key enzymes of the salvage pathway, *e.g*., hypoxanthine phosphoribosyl transferase (HPRT), and they cannot survive. The B cells can operate this pathway, but they have a limited life span in culture and generally die within about two weeks. Therefore, the only cells that can survive in the selective media are those hybrids formed from myeloma and B cells. When the source of B cells used for fusion is a line of EBV-transformed B cells, as here, ouabain is also used for drug selection of hybrids as EBV-transformed B cells are susceptible to drug killing, whereas the myeloma partner used is chosen to be ouabain resistant.

Culturing provides a population of hybridomas from which specific hybridomas are selected. Typically, selection of hybridomas is performed by culturing the cells by single-clone dilution in microtiter plates, followed by testing the individual clonal supernatants (after about two to three weeks) for the desired reactivity. The assay should be sensitive, simple and rapid, such as radioimmunoassays, enzyme immunoassays, cytotoxicity assays, plaque assays dot immunobinding assays, and the like.

The selected hybridomas are then serially diluted or single-cell sorted by flow cytometric sorting and cloned into individual antibody-producing cell lines, which clones can then be propagated indefinitely to provide mAbs. The cell lines may be exploited for MAb production in two basic ways. A sample of the hybridoma can be injected (often into the peritoneal cavity) into an animal (*e.g*., a mouse). Optionally, the animals are primed with a hydrocarbon, especially oils such as pristane (tetramethylpentadecane) prior to injection. When human hybridomas are used in this way, it is optimal to inject immunocompromised mice, such as SCID mice, to prevent tumor rejection. The injected animal develops tumors secreting the specific monoclonal antibody produced by the fused cell hybrid. The body fluids of the animal, such as serum or ascites fluid, can then be tapped to provide MAbs in high concentration. The individual cell lines could also be cultured *in vitro,* where the MAbs are naturally secreted into the culture medium from which they can be readily obtained in high concentrations. Alternatively, human hybridoma cells lines can be used *in vitro* to produce immunoglobulins in cell supernatant. The cell lines can be adapted for growth in serum-free medium to optimize the ability to recover human monoclonal immunoglobulins of high purity.

MAbs produced by either means may be further purified, if desired, using filtration, centrifugation and various chromatographic methods such as FPLC or affinity chromatography. Fragments of the monoclonal antibodies of the present disclosure can be obtained from the purified monoclonal antibodies by methods which include digestion with enzymes, such as pepsin or papain, and/or by cleavage of disulfide bonds by chemical reduction. Alternatively, monoclonal antibody fragments can be synthesized using an automated peptide synthesizer.

It also is contemplated that a molecular cloning approach may be used to generate monoclonals. For this, RNA can be isolated from the hybridoma line and the antibody genes obtained by RT-PCR and cloned into an immunoglobulin expression vector. Alternatively, combinatorial immunoglobulin phagemid libraries are prepared from RNA isolated from the cell lines and phagemids expressing appropriate antibodies are selected by panning using viral antigens. The advantages of this approach over conventional hybridoma techniques are that approximately 10⁴ times as many antibodies can be produced and screened in a single round, and that new specificities are generated by H and L chain combination which further increases the chance of finding appropriate antibodies.

Other U.S. patents that teach the production of antibodies include U.S. Patent 5,565,332, which describes the production of chimeric antibodies using a combinatorial approach; U.S. Patent 4,816,567 which describes recombinant immunoglobulin preparations; and U.S. Patent 4,867,973 which describes antibody-therapeutic agent conjugates.

### B. Antibodies

The normal immune system has the ability to generate millions of antibodies with different antigen binding abilities. The diversity is brought about by the complexities of constructing immunoglobulin molecules. These molecules consist of paired polypeptide chains (heavy and light) each containing a constant and a variable region. The structures of the variable regions of the heavy and light chains are specified by immunoglobulin V genes. The heavy chain variable region is derived from three gene segments known as VH, D and JH. In humans there are about 100 different VH segments, over 20 D segments and six JH segments. The light chain genes have only two segments, the VL and JL segments. Antibody diversity is the result of random combinations of VH/D/JH segments with VUJL components superimposed on which are several mechanisms including junctional diversity and somatic mutation.

The germline VH genes can be separated into at least six families (VH1 through VH6) based on DNA nucleotide sequence identity of the first 95 to 101 amino acids. Members of the same family typically have 80% or more sequence identity, whereas members of different families have less than 70% identity. These families range in size from one VH6 gene to an estimated greater than 45 VH3 genes. In addition, many pseudogenes exist. Recent studies have nearly completed a physical map of the VH locus on chromosome 14q32.13.15. It has now been estimated that the human VH repertoire is represented by approximately 50 functional VH segments with about an equal number of pseudogenes. These studies estimate the size of the VH locus to be approximately 1100 kb. The VH4 family of genes contains 9 different members: 4-04, 4-28, 4-30, 4-31, 4-34, 4-39, 4-59, 4-61, 4-B4.

The present disclosure relates in part to antibodies exhibiting a "signature" in the VH4 sequences of certain B cells. The sequence signature typically comprises residues 31B, 56 and/or 81, but also can include one or more of residues 32, 40, 57, 60 and 89. By examining the sequence at these positions, and identifying mutations at one or more of the positions, it can be determined that a subject is at risk of developing MS and, in the presence of additional factors, has MS. Antibody protein sequences are shown in FIG. 11, and antibody nucleic acid sequences are shown in FIG. 12. The following table lists the AGS related changes for each of the identified antibodies.

**TABLE 1 - ANTIBODIES WITH AGS SIGNATURE CHANGES**

| **rhAb** | **# AGS** | **31B** | **40** | **56** | **57** | **81** | **89** |
|---|---|---|---|---|---|---|---|
| AJL01 | 3 | | | S→R --c; aga | T→A a--; gcc | K→N --g; aac | |
| AJL02 | 2 | | H→S ca-; tcc | | | K→R -a-; agg | |
| AJL03 | 2 | S→R a--; cgt | | | | K→N --g; aac | |
| AJL04 | 3 | 3 | P→S c--; tcc | S→G a--; ggc | T→I -c-; atc | | |
| AJL05 | 4 | S→N -g-; aat | | | T→A a--; gcc | K→N --g; aat | V→F g-g; ttt |
| AJL06 | 3 | G→N gg-; aat | | | | K→R -ag; aga | V→I g-g; atc |
| AJL07 | 3 | | | S→N -gc; aat | T→I -cc; att | K→N --g; aac | |
| AJL08 | 3 | | | S→T -gc; act | | K→N --g; aac | V→F g-g; ttt |
| AJL09 | 2 | | P→S c--; tcc | | | K→N --g; aac | |
| AJL10 | 4 | | P→S c--; tcc | S→T -gc; act | | K→R -a-; agg | V→I g-g; ata |
| AJL11 | 3 | | P→S c--; tcc | S→N -g-; aac | | K→M -a-; atg | |
| AJL12 | 3 | S→D ag-; gat | | S→N -g-; aac | | K→N --g; aac | |
| AJL13 | 3 | | | S→N -gc; aat | T→I -cc; att | K→N --g; aac | |
| AJL14 | 3 | S→N -g-; aat | | S→Y ag-; tac | | | V→I g-g; atc |
| AJL15 | 4 | S→N -g-; aat | P→S c--; tcc | | T→D ac-; gac | K→R -a-; agg | |
| AJL16 | 3 | S→P ag-; cct | | S→H ag-; cac | | K→R aa-; cgg | |
| AJL18 | 5 | S→K -gt; aaa | H→L -a-; ctc | S→T -g-; acc | | K→R -a-; agg | V→R gtg; cgc |
| AJL19 | 3 | | | S→D agc; gat | T→A a--; gcc | | V→L g-g; tta |
| AJL20 | 3 | | | S→T -g-; acc | | K→R -a-; agg | V→I g-g; ata |
| WR01 | 2 | | | | T→P a--; ccc | K→R -a-; agg | |
| WR02 | 2 | | | S→G a--; ggc | T→A a-c; gcg | | |
| WR03 | 3 | | | S→G a--; ggc | T→A a--; gcc | K→N --g; aac | |
| WR04 | 3 | S→G a-t; ggc | P→A c--; gcc | | | K→N --g; aat | |
| WR05 | 4 | S→G a--; ggt | | S→T -g-; acc | T→S a--; tcc | K→T -ag; aca | V→I g-g; att |
| WR06 | 3 | S→A ag-; gct | | S→N -g-; aac | T→K -cc; aaa | | |
| WR07 | 4 | S→T -g-; act | P→S c--; tcc | S→K -gc; aaa | | K→N --g; aac | |
| WR08 | 2 | | | S→T -g-; acc | | K→N --g; aac | |
| WR09 | 2 | | | S→T -g-; acc | | K→N --g; aac | |
| WR10 | 2 | | P→S c--; tcc | S→G a--; ggc | | | |
| WR11 | 2 | | P→S c--; tcc | S→G a--; ggc | | | |
| WR12 | 2 | | | S→T -g-; acc | | K→R -a-; agg | |
| WR13 | 2 | | | S→T -g-; acc | | K→R -a-; agg | |

In certain embodiments, the antibodies or binding fragments thereof do not specifically bind to myelin antigens (*e.g*., myelin basic protein (MBP), myelin oligodendrocyte glycoprotein (MOG)). For example, the antibodies or binding fragments thereof bind to a myelin antigen (MBP and/or MOG) with a K_{D} greater than 10⁻⁵ M or greater than 10⁻⁴ M.

Furthermore, the antibodies sequences may vary from the sequences provided above, optionally using methods discussed in greater detail below. For example, amino sequences may vary from those set out above in that (a) the variable regions may be segregated away from the constant domains of the light chains, (b) the amino acids may vary from those set out above while not drastically affecting the chemical properties of the residues thereby (so-called conservative substitutions), (c) the amino acids may vary from those set out above by a given percentage, *e.g.,* 80%, 85%, 90%, 91%, 92%, 93%, 94%, 95%, 96%, 97%, 98% or 99% homology. Alternatively, the nucleic acids encoding the antibodies may (a) be segregated away from the constant domains of the light chains, (b) vary from those set out above while not changing the residues coded thereby, (c) may vary from those set out above by a given percentage, *e.g.,* 70%, 75%, 80%, 85%, 90%, 91%, 92%, 93%, 94%, 95%, 96%, 97%, 98% or 99% homology, or (d) vary from those set out above by virtue of the ability to hybridize under high stringency conditions, as exemplified by low salt and/or high temperature conditions, such as provided by about 0.02 M to about 0.15 M NaCl at temperatures of about 50°C to about 70°C.

In making conservative changes in amino acid sequence, the hydropathic index of amino acids may be considered. The importance of the hydropathic amino acid index in conferring interactive biologic function on a protein is generally understood in the art (Kyte and Doolittle, 1982). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like.

It also is understood in the art that the substitution of like amino acids can be made effectively on the basis of hydrophilicity. U.S. Patent 4,554,101 states that the greatest local average hydrophilicity of a protein, as governed by the hydrophilicity of its adjacent amino acids, correlates with a biological property of the protein. As detailed in U.S. Patent 4,554,101, the following hydrophilicity values have been assigned to amino acid residues: basic amino acids: arginine (+3.0), lysine (+3.0), and histidine (-0.5); acidic amino acids: aspartate (+3.0 ± 1), glutamate (+3.0 ± 1), asparagine (+0.2), and glutamine (+0.2); hydrophilic, nonionic amino acids: serine (+0.3), asparagine (+0.2), glutamine (+0.2), and threonine (-0.4), sulfur containing amino acids: cysteine (-1.0) and methionine (-1.3); hydrophobic, nonaromatic amino acids: valine (-1.5), leucine (-1.8), isoleucine (-1.8), proline (-0.5 ± 1), alanine (-0.5), and glycine (0); hydrophobic, aromatic amino acids: tryptophan (-3.4), phenylalanine (-2.5), and tyrosine (-2.3).

It is understood that an amino acid can be substituted for another having a similar hydrophilicity and produce a biologically or immunologically modified protein. In such changes, the substitution of amino acids whose hydrophilicity values are within ± 2 is preferred, those that are within ± 1 are particularly preferred, and those within ± 0.5 are even more particularly preferred.

As outlined above, amino acid substitutions generally are based on the relative similarity of the amino acid side-chain substituents, for example, their hydrophobicity, hydrophilicity, charge, size, and the like. Exemplary substitutions that take into consideration the various foregoing characteristics are well known to those of skill in the art and include: arginine and lysine; glutamate and aspartate; serine and threonine; glutamine and asparagine; and valine, leucine and isoleucine.

### C. Engineering of Antibody Sequences

In various embodiments, one may choose to engineer sequences of the identified antibodies for a variety of reasons, such as improved expression, improved cross-reactivity, diminished off-target binding or abrogation of one or more natural effector functions, such as activation of complement or recruitment of immune cells (*e.g.,* T cells). In particular, IgM antibodies may be converted to IgG antibodies. The following is a general discussion of relevant techniques for antibody engineering.

Hybridomas may be cultured, then cells lysed, and total RNA extracted. Random hexamers may be used with RT to generate cDNA copies of RNA, and then PCR performed using a multiplex mixture of PCR primers expected to amplify all human variable gene sequences. PCR product can be cloned into pGEM-T Easy vector, then sequenced by automated DNA sequencing using standard vector primers. Assay of binding and neutralization may be performed using antibodies collected from hybridoma supernatants and purified by FPLC, using Protein G columns. Recombinant full length IgG antibodies can be generated by subcloning heavy and light chain Fv DNAs from the cloning vector into a Lonza pConIgG1 or pConK2 plasmid vector, transfected into 293 Freestyle cells or Lonza CHO cells, and collected and purified from the CHO cell supernatant.

The rapid availability of antibody produced in the same host cell and cell culture process as the final cGMP manufacturing process has the potential to reduce the duration of process development programs. Lonza has developed a generic method using pooled transfectants grown in CDACF medium, for the rapid production of small quantities (up to 50 g) of antibodies in CHO cells. Although slightly slower than a true transient system, the advantages include a higher product concentration and use of the same host and process as the production cell line. Example of growth and productivity of GS-CHO pools, expressing a model antibody, in a disposable bioreactor: in a disposable bag bioreactor culture (5 L working volume) operated in fed-batch mode, a harvest antibody concentration of 2 g/L was achieved within 9 weeks of transfection.

pCon Vectors™ are an easy way to re-express whole antibodies. The constant region vectors are a set of vectors offering a range of immunoglobulin constant region vectors cloned into the pEE vectors. These vectors offer easy construction of full length antibodies with human constant regions and the convenience of the GS System™.

Antibody molecules will comprise fragments (such as F(ab'), F(ab')₂) that are produced, for example, by the proteolytic cleavage of the mAbs, or single-chain immunoglobulins producible, for example, via recombinant means. Such antibody derivatives are monovalent. In one embodiment, such fragments can be combined with one another, or with other antibody fragments or receptor ligands to form "chimeric" binding molecules. Significantly, such chimeric molecules may contain substituents capable of binding to different epitopes of the same molecule.

In related embodiments, the antibody is a derivative of the disclosed antibodies, *e.g*., an antibody comprising the CDR sequences identical to those in the disclosed antibodies (*e.g.,* a chimeric, humanized or CDR-grafted antibody). In yet a further embodiment, the antibody is a fully human recombinant antibody.

The present disclosure also contemplates isotype modification. By modifying the Fc region to have a different isotype, different functionalities can be achieved. For example, changing to IgG₄ can reduce immune effector functions associated with other isotypes.

Modified antibodies may be made by any technique known to those of skill in the art, including expression through standard molecular biological techniques, or the chemical synthesis of polypeptides. Methods for recombinant expression are addressed elsewhere in this document.

### D. Expression

Nucleic acids according to the present disclosure will encode antibodies, optionally linked to other protein-encoding sequences. As used in this application, the term "a nucleic acid encoding an antibody" refers to a nucleic acid molecule that has been isolated free of total cellular nucleic acid. The disclosure also provides a nucleic acid encoding SEQ ID NOS:1-64, or a nucleic acid selected from SEQ ID NOS: 65-128.

**TABLE 2 - CODONS**

| **Amino Acids** | | | **Codons** |
|---|---|---|---|
| Alanine | Ala | A | GCA GCC GCG GCU |
| Cysteine | Cys | C | UGC UGU |
| Aspartic acid | Asp | D | GAC GAU |
| Glutamic acid | Glu | E | GAA GAG |
| Phenylalanine | Phe | F | UUC UUU |
| Glycine | Gly | G | GGA GGC GGG GGU |
| Histidine | His | H | CAC CAU |
| Isoleucine | Ile | I | AUA AUC AUU |
| Lysine | Lys | K | AAA AAG |
| Leucine | Leu | L | UUA UUG CUA CUC CUGCUU |
| Methionine | Met | M | AUG |
| Asparagine | Asn | N | AAC AAU |
| Proline | Pro | P | CCA CCC CCG CCU |
| Glutamine | Gln | Q | CAA CAG |
| Arginine | Arg | R | AGA AGG CGA CGC CGGCGU |
| Serine | Ser | S | AGCAGU UCA UCC UCG UCU |
| Threonine | Thr | T | ACA ACC ACG ACU |
| Valine | Val | V | GUA GUC GUG GUU |
| Tryptophan | Trp | W | UGG |
| Tyrosine | Tyr | Y | UAC UAU |

The DNA segments of the present disclosure include those encoding biologically functional equivalent proteins and peptides of the sequences described above. Such sequences may arise as a consequence of codon redundancy and amino acid functional equivalency that are known to occur naturally within nucleic acid sequences and the proteins thus encoded. Alternatively, functionally equivalent proteins or peptides may be created via the application of recombinant DNA technology, in which changes in the protein structure may be engineered, based on considerations of the properties of the amino acids being exchanged. Changes designed by man may be introduced through the application of site-directed mutagenesis techniques or may be introduced randomly and screened later for the desired function, as described below.

Within certain embodiments, expression vectors are employed in order to produce the polypeptide. In other embodiments, the expression vectors are used in gene therapy. Expression requires that appropriate signals be provided in the vectors, and which include various regulatory elements, such as enhancers/promoters from both viral and mammalian sources that drive expression of the genes of interest in host cells. Elements designed to optimize messenger RNA stability and translatability in host cells also are defined. The conditions for the use of a number of dominant drug selection markers for establishing permanent, stable cell clones expressing the products are also provided, as is an element that links expression of the drug selection markers to expression of the polypeptide.

Throughout this application, the term "expression construct" is meant to include any type of genetic construct containing a nucleic acid coding for a gene product in which part or all of the nucleic acid encoding sequence is capable of being transcribed. The transcript may be translated into a protein, but it need not be. In certain embodiments, expression includes both transcription of a gene and translation of mRNA into a gene product. In other embodiments, expression only includes transcription of the nucleic acid encoding a gene of interest.

The term "vector" is used to refer to a carrier nucleic acid molecule into which a nucleic acid sequence can be inserted for introduction into a cell where it can be replicated. A nucleic acid sequence can be "exogenous," which means that it is foreign to the cell into which the vector is being introduced or that the sequence is homologous to a sequence in the cell but in a position within the host cell nucleic acid in which the sequence is ordinarily not found. Vectors include plasmids, cosmids, viruses (bacteriophage, animal viruses, and plant viruses), and artificial chromosomes (*e.g*., YACs). One of skill in the art would be well equipped to construct a vector through standard recombinant techniques, which are described in Sambrook *et al.* (1989) and Ausubel *et al.* (1994).

The term "expression vector" refers to a vector containing a nucleic acid sequence coding for at least part of a gene product capable of being transcribed. In some cases, RNA molecules are then translated into a protein, polypeptide, or peptide. In other cases, these sequences are not translated, for example, in the production of antisense molecules or ribozymes. Expression vectors can contain a variety of "control sequences," which refer to nucleic acid sequences necessary for the transcription and possibly translation of an operably linked coding sequence in a particular host organism. In addition to control sequences that govern transcription and translation, vectors and expression vectors may contain nucleic acid sequences that serve other functions as well and are described *infra.*

### 1. Regulatory Elements

A "promoter" is a control sequence that is a region of a nucleic acid sequence at which initiation and rate of transcription are controlled. It may contain genetic elements at which regulatory proteins and molecules may bind such as RNA polymerase and other transcription factors. The phrases "operatively positioned," "operatively linked," "under control," and "under transcriptional control" mean that a promoter is in a correct functional location and/or orientation in relation to a nucleic acid sequence to control transcriptional initiation and/or expression of that sequence. A promoter may or may not be used in conjunction with an "enhancer," which refers to a cis-acting regulatory sequence involved in the transcriptional activation of a nucleic acid sequence.

A promoter may be one naturally-associated with a gene or sequence, as may be obtained by isolating the 5' non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment.

A recombinant or heterologous enhancer refers also to an enhancer not normally associated with a nucleic acid sequence in its natural environment. Such promoters or enhancers may include promoters or enhancers of other genes, and promoters or enhancers isolated from any other prokaryotic, viral, or eukaryotic cell, and promoters or enhancers not "naturally-occurring," *i.e.,* containing different elements of different transcriptional regulatory regions, and/or mutations that alter expression. In addition to producing nucleic acid sequences of promoters and enhancers synthetically, sequences may be produced using recombinant cloning and/or nucleic acid amplification technology, including PCR™, in connection with the compositions disclosed herein (see U.S. Patent 4,683,202, U.S. Patent 5,928,906). Furthermore, it is contemplated the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the cell type, organelle, and organism chosen for expression. Those of skill in the art of molecular biology generally know the use of promoters, enhancers, and cell type combinations for protein expression, for example, see Sambrook *et al.* (1989). The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

Table 3 lists several elements/promoters that may be employed, in the context of the present invention, to regulate the expression of a gene. This list is not intended to be exhaustive of all the possible elements involved in the promotion of expression but, merely, to be exemplary thereof. Table 4 provides examples of inducible elements, which are regions of a nucleic acid sequence that can be activated in response to a specific stimulus.

| **TABLE 3** | |
|---|---|
| **Promoter and/or Enhancer** | |
| Promoter/Enhancer | References |
| Immunoglobulin Heavy Chain | Banerji *et al.,* 1983; Gilles *et al.*, 1983; Grosschedl *et al.*, 1985; Atchinson *et al.*, 1986, 1987; Imler *et al.*, 1987; Weinberger *et al.*, 1984; Kiledjian *et al.*, 1988; Porton *et al.*; 1990 |
| Immunoglobulin Light Chain | Queen *et al.*,1983; Picard *et al.*, 1984 |
| T-Cell Receptor | Luria *et al.*, 1987; Winoto *et al.*, 1989; Redondo *et al.*; 1990 |
| HLA DQ a and/or DQ β | Sullivan *et al.*, 1987 |
| β-Interferon | Goodbourn *et al.*, 1986; Fujita *et al.*, 1987; Goodbourn *et al.*, 1988 |
| Interleukin-2 | Greene *et al*., 1989 |
| Promoter/Enhancer | References |
| Interleukin-2 Receptor | Greene *et al.*, 1989; Lin *et al.*, 1990 |
| MHC Class II 5 | Koch *et al.*, 1989 |
| MHC Class II HLA-DRa | Sherman *et al.*, 1989 |
| β-Actin | Kawamoto *et al.*, 1988; Ng *et al.*; 1989 |
| Muscle Creatine Kinase (MCK) | Jaynes *et al.*, 1988; Horlick *et al.*, 1989; Johnson *et al.*, 1989 |
| Prealbumin (Transthyretin) | Costa *et al.*, 1988 |
| Elastase I | Ornitz *et al.*, 1987 |
| Metallothionein (MTII) | Karin *et al.*, 1987; Culotta *et al.*, 1989 |
| Collagenase | Pinkert *et al.*, 1987; Angel *et al.*, 1987 |
| Albumin | Pinkert *et al.*, 1987; Tronche *et al.*, 1989, 1990 |
| α-Fetoprotein | Godbout *et al.*, 1988; Campere *et al.*, 1989 |
| t-Globin | Bodine *et al.*, 1987; Perez-Stable *et al.*, 1990 |
| β-Globin | Trudel *et al.*, 1987 |
| c-fos | Cohen *et al.*, 1987 |
| *c-HA-ras* | Triesman, 1986; Deschamps *et al.*, 1985 |
| Insulin | Edlund *et al.*, 1985 |
| Neural Cell Adhesion Molecule (NCAM) | Hirsh *et al.*, 1990 |
| α₁-Antitrypain | Latimer *et al.*, 1990 |
| H2B (TH2B) Histone | Hwang *et al.*, 1990 |
| Mouse and/or Type I Collagen | Ripe *et al.*, 1989 |
| Glucose-Regulated Proteins (GRP94 and GRP78) | Chang *et al.*, 1989 |
| Rat Growth Hormone | Larsen *et al.*, 1986 |
| Human Serum Amyloid A (SAA) | Edbrooke *et al.*, 1989 |
| Troponin I (TN I) | Yutzey *et al.*, 1989 |
| Platelet-Derived Growth Factor (PDGF) | Pech *et al.*, 1989 |

| Promoter/Enhancer | References |
|---|---|
| Duchenne Muscular Dystrophy | Klamut *et al.*, 1990 |
| SV40 | Banerji *et al.*, 1981; Moreau *et al.*, 1981; Sleigh *et al.*, 1985; Firak *et al.*, 1986; Herr *et al.*, 1986; Imbra *et al.*, 1986; Kadesch *et al.*, 1986; Wang *et al.*, 1986; Ondek *et al.*, 1987; Kuhl *et al.*, 1987; Schaffner *et al.*, 1988 |
| Polyoma | Swartzendruber *et al.*, 1975; Vasseur *et al.*, 1980; Katinka *et al.*, 1980, 1981; Tyndell *et al.*, 1981; Dandolo *et al.*, 1983; de Villiers *et al.*, 1984; Hen *et al.*, 1986; Satake *et al.*, 1988; Campbell and/or Villarreal, 1988 |
| Retroviruses | Kriegler *et al.*, 1982, 1983; Levinson *et al.*, 1982; Kriegler *et al.*, 1983, 1984a, b, 1988; Bosze *et al.*, 1986; Miksicek *et al.*, 1986; Celander *et al.*, 1987; Thiesen *et al.*, 1988; Celander *et al.*, 1988; Choi *et al.*, 1988; Reisman *et al.*, 1989 |
| Papilloma Virus | Campo *et al.*, 1983; Lusky *et al.*, 1983; Spandidos and/or Wilkie, 1983; Spalholz *et al.*, 1985; Lusky *et al.*, 1986; Cripe *et al.*, 1987; Gloss *et al.*, 1987; Hirochika *et al.*, 1987; Stephens *et al.*, 1987; Glue *et al.*, 1988 |
| Hepatitis B Virus | Bulla *et al.*, 1986; Jameel *et al.*, 1986; Shaul *et al.*, 1987; Spandau *et al.*, 1988; Vannice *et al.*, 1988 |
| Human Immunodeficiency Virus | Muesing *et al.*, 1987; Hauber *et al.*, 1988; Jakobovits *et al.*, 1988; Feng *et al.*, 1988; Takebe *et al.*, 1988; Rosen *et al.*, 1988; Berkhout *et al.*, 1989; Laspia *et al.*, 1989; Sharp *et al.*, 1989; Braddock *et al.*, 1989 |
| Cytomegalovirus (CMV) | Weber *et al.*, 1984; Boshart *et al.*, 1985; Foecking *et al.*, 1986 |
| Gibbon Ape Leukemia Virus | Holbrook *et al.*, 1987; Quinn *et al.*, 1989 |

| **TABLE 4** | | |
|---|---|---|
| **Inducible Elements** | | |
| Element | Inducer | References |
| MT II | Phorbol Ester (TFA) Heavy metals | Palmiter *et al.*, 1982; Haslinger *et al.*, 1985; Searle *et al.*, 1985; Stuart *et al.*, 1985; Imagawa *et al.*, 1987, Karin *et al.*, 1987; Angel *et al.*, 1987b; McNeall *et al.*, 1989 |
| MMTV (mouse mammary tumor virus) | Glucocorticoids | Huang *et al.*, 1981; Lee *et al.*, 1981; Majors *et al.*, 1983; Chandler *et al.*, 1983; Lee *et al.*, 1984; Ponta *et al.*, 1985; Sakai *et al.*, 1988 |
| β-Interferon | poly(rI)x poly(rc) | Tavernier *et al.*, 1983 |
| Adenovirus 5 E2 | ElA | Imperiale *et al.*, 1984 |
| Collagenase | Phorbol Ester (TPA) | Angel *et al.*, 1987a |
| Stromelysin | Phorbol Ester (TPA) | Angel *et al.*, 1987b |
| SV40 | Phorbol Ester (TPA) | Angel *et al.*, 1987b |
| Murine MX Gene | Interferon, Newcastle Disease Virus | Hug *et al.*, 1988 |
| GRP78 Gene | A23187 | Resendez *et al.*, 1988 |
| α-2-Macroglobulin | IL-6 | Kunz *et al.*, 1989 |
| Vimentin | Serum | Rittling *et al.*, 1989 |
| MHC Class I Gene H-2κb | Interferon | Blanar *et al.*, 1989 |
| HSP70 | ElA, SV40 Large T Antigen | Taylor *et al.*, 1989, 1990a, 1990b |
| Proliferin | Phorbol Ester-TPA | Mordacq *et al.*, 1989 |
| Tumor Necrosis Factor | PMA | Hensel *et al.*, 1989 |
| Thyroid Stimulating Hormone α Gene | Thyroid Hormone | Chatterjee *et al.*, 1989 |

The identity of tissue-specific promoters or elements, as well as assays to characterize their activity, is well known to those of skill in the art. Examples of such regions include the human LIMK2 gene (Nomoto *et al.,* 1999), the somatostatin receptor 2 gene (Kraus *et al.,* 1998), murine epididymal retinoic acid-binding gene (Lareyre *et al.,* 1999), human CD4 (Zhao-Emonet *et al.,* 1998), mouse alpha2 (XI) collagen (Tsumaki *et al.,* 1998), D1A dopamine receptor gene (Lee *et al.,* 1997), insulin-like growth factor II (Wu *et al.,* 1997), human platelet endothelial cell adhesion molecule-1 (Almendro *et al.,* 1996).

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

### 2. IRES

Internal ribosome entry sites (IRES) elements are used to create multigene, or polycistronic, messages. IRES elements are able to bypass the ribosome scanning model of 5'-methylated Cap dependent translation and begin translation at internal sites (Pelletier and Sonenberg, 1988). IRES elements from two members of the picornavirus family (polio and encephalomyocarditis) have been described (Pelletier and Sonenberg, 1988), as well an IRES from a mammalian message (Macejak and Sarnow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patents 5,925,565 and 5,935,819).

### 3. Multi-Purpose Cloning Sites

Vectors can include a multiple cloning site (MCS), which is a nucleic acid region that contains multiple restriction enzyme sites, any of which can be used in conjunction with standard recombinant technology to digest the vector. See Carbonelli *et al.* (1999); Levenson *et al.* (1998); and Cocea (1997). "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

### 4. Splicing Sites

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression (see Chandler *et al.,* 1997).

### 5. Termination Signals

The vectors or constructs of the present disclosure will generally comprise at least one termination signal. A "termination signal" or "terminator" is comprised of the DNA sequences involved in specific termination of an RNA transcript by an RNA polymerase. Thus, in certain embodiments a termination signal that ends the production of an RNA transcript is contemplated. A terminator may be necessary *in vivo* to achieve desirable message levels.

In eukaryotic systems, the terminator region may also comprise specific DNA sequences that permit site-specific cleavage of the new transcript so as to expose a polyadenylation site. This signals a specialized endogenous polymerase to add a stretch of about 200 A residues (polyA) to the 3' end of the transcript. RNA molecules modified with this polyA tail appear to more stable and are translated more efficiently. Thus, in other embodiments involving eukaryotes, it is preferred that that terminator comprises a signal for the cleavage of the RNA, and it is more preferred that the terminator signal promotes polyadenylation of the message. The terminator and/or polyadenylation site elements can serve to enhance message levels and/or to minimize read through from the cassette into other sequences.

Terminators contemplated for use in the invention include any known terminator of transcription described herein or known to one of ordinary skill in the art, including but not limited to, for example, the termination sequences of genes, such as for example the bovine growth hormone terminator or viral termination sequences, such as for example the SV40 terminator. In certain embodiments, the termination signal may be a lack of transcribable or translatable sequence, such as due to a sequence truncation.

### 6. Polyadenylation Signals

In expression, particularly eukaryotic expression, one will typically include a polyadenylation signal to effect proper polyadenylation of the transcript. The nature of the polyadenylation signal is not believed to be crucial to the successful practice of the invention, and/or any such sequence may be employed. Preferred embodiments include the SV40 polyadenylation signal and/or the bovine growth hormone polyadenylation signal, convenient and/or known to function well in various target cells. Polyadenylation may increase the stability of the transcript or may facilitate cytoplasmic transport.

### 7. Origins of Replication

In order to propagate a vector in a host cell, it may contain one or more origins of replication sites (often termed "ori"), which is a specific nucleic acid sequence at which replication is initiated. Alternatively an autonomously replicating sequence (ARS) can be employed if the host cell is yeast.

### 8. Selectable and Screenable Markers

In certain embodiments of the invention, cells containing a nucleic acid construct of the present invention may be identified *in vitro* or *in vivo* by including a marker in the expression vector. Such markers would confer an identifiable change to the cell permitting easy identification of cells containing the expression vector. Generally, a selectable marker is one that confers a property that allows for selection. A positive selectable marker is one in which the presence of the marker allows for its selection, while a negative selectable marker is one in which its presence prevents its selection. An example of a positive selectable marker is a drug resistance marker.

Usually the inclusion of a drug selection marker aids in the cloning and identification of transformants, for example, genes that confer resistance to neomycin, puromycin, hygromycin, DHFR, GPT, zeocin and histidinol are useful selectable markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes such as herpes simplex virus thymidine kinase (*tk*) or chloramphenicol acetyltransferase (CAT) may be utilized. One of skill in the art would also know how to employ immunologic markers, possibly in conjunction with FACS analysis. The marker used is not believed to be important, so long as it is capable of being expressed simultaneously with the nucleic acid encoding a gene product. Further examples of selectable and screenable markers are well known to one of skill in the art.

### 9. Viral Vectors

The capacity of certain viral vectors to efficiently infect or enter cells, to integrate into a host cell genome and stably express viral genes, have led to the development and application of a number of different viral vector systems (Robbins *et al.,* 1998). Viral systems are currently being developed for use as vectors for *ex vivo* and *in vivo* gene transfer. For example, adenovirus, herpes-simplex virus, retrovirus and adeno-associated virus vectors are being evaluated currently for treatment of diseases such as cancer, cystic fibrosis, Gaucher disease, renal disease and arthritis (Robbins and Ghivizzani, 1998; Imai *et al.,* 1998; U.S. Patent 5,670,488). The various viral vectors described below, present specific advantages and disadvantages, depending on the particular gene-therapeutic application.

**Adenoviral Vectors.** In particular embodiments, an adenoviral expression vector is contemplated for the delivery of expression constructs. "Adenovirus expression vector" is meant to include those constructs containing adenovirus sequences sufficient to (a) support packaging of the construct and (b) to ultimately express a tissue or cell-specific construct that has been cloned therein.

Adenoviruses comprise linear, double-stranded DNA, with a genome ranging from 30 to 35 kb in size (Reddy *et al.,* 1998; Morrison *et al.,* 1997; Chillon *et al.,* 1999). An adenovirus expression vector comprises a genetically engineered form of the adenovirus. Advantages of adenoviral gene transfer include the ability to infect a wide variety of cell types, including non-dividing cells, a mid-sized genome, ease of manipulation, high infectivity and the ability to be grown to high titers (Wilson, 1996). Further, adenoviral infection of host cells does not result in chromosomal integration because adenoviral DNA can replicate in an episomal manner, without potential genotoxicity associated with other viral vectors. Adenoviruses also are structurally stable (Marienfeld *et al.,* 1999) and no genome rearrangement has been detected after extensive amplification (Parks *et al.,* 1997; Bett *et al.,* 1993).

Salient features of the adenovirus genome are an early region (E1, E2, E3 and E4 genes), an intermediate region (pIX gene, Iva2 gene), a late region (LI, L2, L3, L4 and L5 genes), a major late promoter (MLP), inverted-terminal-repeats (ITRs) and a ψ sequence (Zheng, *et al.,* 1999; Robbins *et al.,* 1998; Graham and Prevec, 1995). The early genes E1, E2, E3 and E4 are expressed from the virus after infection and encode polypeptides that regulate viral gene expression, cellular gene expression, viral replication, and inhibition of cellular apoptosis. Further on during viral infection, the MLP is activated, resulting in the expression of the late (L) genes, encoding polypeptides required for adenovirus encapsidation. The intermediate region encodes components of the adenoviral capsid. Adenoviral inverted terminal repeats (ITRs; 100-200 bp in length), are *cis* elements, and function as origins of replication and are necessary for viral DNA replication. The ψ sequence is required for the packaging of the adenoviral genome.

A common approach for generating adenoviruses for use as a gene transfer vectors is the deletion of the E1 gene (E1⁻), which is involved in the induction of the E2, E3 and E4 promoters (Graham and Prevec, 1995). Subsequently, a therapeutic gene or genes can be inserted recombinantly in place of the E1 gene, wherein expression of the therapeutic gene(s) is driven by the E1 promoter or a heterologous promoter. The E1⁻, replication-deficient virus is then proliferated in a "helper" cell line that provides the E1 polypeptides *in trans* (*e.g.,* the human embryonic kidney cell line 293). Thus, in the present invention it may be convenient to introduce the transforming construct at the position from which the E1-coding sequences have been removed. However, the position of insertion of the construct within the adenovirus sequences is not critical. Alternatively, the E3 region, portions of the E4 region or both may be deleted, wherein a heterologous nucleic acid sequence under the control of a promoter operable in eukaryotic cells is inserted into the adenovirus genome for use in gene transfer (U.S. Patent 5,670,488; U.S. Patent 5,932,210).

Although adenovirus based vectors offer several unique advantages over other vector systems, they often are limited by vector immunogenicity, size constraints for insertion of recombinant genes and low levels of replication. The preparation of a recombinant adenovirus vector deleted of all open reading frames, comprising a full length dystrophin gene and the terminal repeats required for replication (Haecker *et al.,* 1996) offers some potentially promising advantages to the above mentioned adenoviral shortcomings. The vector was grown to high titer with a helper virus in 293 cells and was capable of efficiently transducing dystrophin in mdx mice, in myotubes *in vitro* and muscle fibers *in vivo.* Helper-dependent viral vectors are discussed below.

A major concern in using adenoviral vectors is the generation of a replication-competent virus during vector production in a packaging cell line or during gene therapy treatment of an individual. The generation of a replication-competent virus could pose serious threat of an unintended viral infection and pathological consequences for the patient. Armentano *et al.* (1990), describe the preparation of a replication-defective adenovirus vector, claimed to eliminate the potential for the inadvertent generation of a replication-competent adenovirus (U.S. Patent 5,824,544). The replication-defective adenovirus method comprises a deleted E1 region and a relocated protein IX gene, wherein the vector expresses a heterologous, mammalian gene.

Other than the requirement that the adenovirus vector be replication defective, or at least conditionally defective, the nature of the adenovirus vector is not believed to be crucial to the successful practice of the invention. The adenovirus may be of any of the 42 different known serotypes and/or subgroups A-F. Adenovirus type 5 of subgroup C is the preferred starting material in order to obtain the conditional replication-defective adenovirus vector for use in the present invention. This is because adenovirus type 5 is a human adenovirus about which a great deal of biochemical and genetic information is known, and it has historically been used for most constructions employing adenovirus as a vector.

As stated above, the typical vector according to the present disclosure is replication defective and will not have an adenovirus E1 region. Adenovirus growth and manipulation is known to those of skill in the art, and exhibits broad host range *in vitro* and *in vivo* (U.S. Patent 5,670,488; U.S. Patent 5,932,210; U.S. Patent 5,824,544). This group of viruses can be obtained in high titers, *e.g.,* 10⁹ to 10¹¹ plaque-forming units per ml, and they are highly infective. The life cycle of adenovirus does not require integration into the host cell genome. The foreign genes delivered by adenovirus vectors are episomal and, therefore, have low genotoxicity to host cells. Many experiments, innovations, preclinical studies and clinical trials are currently under investigation for the use of adenoviruses as gene delivery vectors. For example, adenoviral gene delivery-based gene therapies are being developed for liver diseases (Han *et al.,* 1999), psychiatric diseases (Lesch, 1999), neurological diseases (Smith, 1998; Hermens and Verhaagen, 1998), coronary diseases (Feldman *et al.,* 1996), muscular diseases (Petrof, 1998), gastrointestinal diseases (Wu, 1998) and various cancers such as colorectal (Fujiwara and Tanaka, 1998; Dorai *et al.,* 1999), pancreatic, bladder (Irie *et al.,* 1999), head and neck (Blackwell *et al.,* 1999), breast (Stewart *et al.,* 1999), lung (Batra *et al.,* 1999) and ovarian (Vanderkwaak *et al.,* 1999).

**Retroviral Vectors.** In certain embodiments of the invention, the uses of retroviruses for gene delivery are contemplated. Retroviruses are RNA viruses comprising an RNA genome. When a host cell is infected by a retrovirus, the genomic RNA is reverse transcribed into a DNA intermediate which is integrated into the chromosomal DNA of infected cells. This integrated DNA intermediate is referred to as a provirus. A particular advantage of retroviruses is that they can stably infect dividing cells with a gene of interest (*e.g.,* a therapeutic gene) by integrating into the host DNA, without expressing immunogenic viral proteins. Theoretically, the integrated retroviral vector will be maintained for the life of the infected host cell, expressing the gene of interest.

The retroviral genome and the proviral DNA have three genes: gag, pol, and env, which are flanked by two long terminal repeat (LTR) sequences. The gag gene encodes the internal structural (matrix, capsid, and nucleocapsid) proteins; the pol gene encodes the RNA-directed DNA polymerase (reverse transcriptase) and the env gene encodes viral envelope glycoproteins. The 5' and 3' LTRs serve to promote transcription and polyadenylation of the virion RNAs. The LTR contains all other cis-acting sequences necessary for viral replication.

A recombinant retrovirus may be genetically modified in such a way that some of the structural, infectious genes of the native virus have been removed and replaced instead with a nucleic acid sequence to be delivered to a target cell (U.S. Patent 5,858,744; U.S. Patent 5,739,018). After infection of a cell by the virus, the virus injects its nucleic acid into the cell and the retrovirus genetic material can integrate into the host cell genome. The transferred retrovirus genetic material is then transcribed and translated into proteins within the host cell. As with other viral vector systems, the generation of a replication-competent retrovirus during vector production or during therapy is a major concern. Retroviral vectors suitable for use in the present invention are generally defective retroviral vectors that are capable of infecting the target cell, reverse transcribing their RNA genomes, and integrating the reverse transcribed DNA into the target cell genome, but are incapable of replicating within the target cell to produce infectious retroviral particles (*e.g.,* the retroviral genome transferred into the target cell is defective in gag, the gene encoding virion structural proteins, and/or in pol, the gene encoding reverse transcriptase). Thus, transcription of the provirus and assembly into infectious virus occurs in the presence of an appropriate helper virus or in a cell line containing appropriate sequences enabling encapsidation without coincident production of a contaminating helper virus.

The growth and maintenance of retroviruses is known in the art (U.S. Patent 5,955,331; U.S. Patent 5,888,502). Nolan *et al.* describe the production of stable high titre, helper-free retrovirus comprising a heterologous gene (U.S. Patent 5,830,725). Methods for constructing packaging cell lines useful for the generation of helper-free recombinant retroviruses with amphoteric or ecotrophic host ranges, as well as methods of using the recombinant retroviruses to introduce a gene of interest into eukaryotic cells *in vivo* and *in vitro* are contemplated in the present invention (U.S. Patent 5,955,331).

Currently, the majority of all clinical trials for vector-mediated gene delivery use murine leukemia virus (MLV)-based retroviral vector gene delivery (Robbins *et al.,* 1998; Miller *et al.,* 1993). Disadvantages of retroviral gene delivery include a requirement for ongoing cell division for stable infection and a coding capacity that prevents the delivery of large genes. However, recent development of vectors such as lentivirus (*e.g*., HIV), simian immunodeficiency virus (SIV) and equine infectious-anemia virus (EIAV), which can infect certain non-dividing cells, potentially allow the *in vivo* use of retroviral vectors for gene therapy applications (Amado and Chen, 1999; Klimatcheva *et al*., 1999; White *et al.,* 1999; Case *et al.,* 1999). For example, HIV-based vectors have been used to infect non-dividing cells such as neurons (Miyatake *et al.,* 1999), islets (Leibowitz *et al.,* 1999) and muscle cells (Johnston *et al.,* 1999). The therapeutic delivery of genes *via* retroviruses are currently being assessed for the treatment of various disorders such as inflammatory disease (Moldawer *et al.,* 1999), AIDS (Amado and Chen, 1999; Engel and Kohn, 1999), cancer (Clay *et al.,* 1999), cerebrovascular disease (Weihl *et al.,* 1999) and hemophilia (Kay, 1998).

**Herpesviral Vectors.** Herpes simplex virus (HSV) type I and type II contain a double-stranded, linear DNA genome of approximately 150 kb, encoding 70-80 genes. Wild type HSV are able to infect cells lytically and to establish latency in certain cell types (*e.g.,* neurons). Similar to adenovirus, HSV also can infect a variety of cell types including muscle (Yeung *et al.,* 1999), ear (Derby *et al.,* 1999), eye (Kaufman *et al.,* 1999), tumors (Yoon *et al.,* 1999; Howard *et al.,* 1999), lung (Kohut *et al.,* 1998), neuronal (Garrido *et al.,* 1999; Lachmann and Efstathiou, 1999), liver (Miytake *et al.,* 1999; Kooby *et al.,* 1999) and pancreatic islets (Rabinovitch *et al.,* 1999).

HSV viral genes are transcribed by cellular RNA polymerase II and are temporally regulated, resulting in the transcription and subsequent synthesis of gene products in roughly three discernable phases or kinetic classes. These phases of genes are referred to as the Immediate Early (IE) or α genes, Early (E) or β genes and Late (L) or γ genes. Immediately following the arrival of the genome of a virus in the nucleus of a newly infected cell, the IE genes are transcribed. The efficient expression of these genes does not require prior viral protein synthesis. The products of IE genes are required to activate transcription and regulate the remainder of the viral genome.

For use in therapeutic gene delivery, HSV must be rendered replication-defective. Protocols for generating replication-defective HSV helper virus-free cell lines have been described (U.S. Patent 5,879,934; U.S. Patent 5,851,826). One IE protein, ICP4, also known as α4 or Vmw175, is absolutely required for both virus infectivity and the transition from IE to later transcription. Thus, due to its complex, multifunctional nature and central role in the regulation of HSV gene expression, ICP4 has typically been the target of HSV genetic studies.

Phenotypic studies of HSV viruses deleted of ICP4 indicate that such viruses will be potentially useful for gene transfer purposes (Krisky *et al.,* 1998a). One property of viruses deleted for ICP4 that makes them desirable for gene transfer is that they only express the five other IE genes: ICP0, ICP6, ICP27, ICP22 and ICP47 (DeLuca *et al.,* 1985), without the expression of viral genes encoding proteins that direct viral DNA synthesis, as well as the structural proteins of the virus. This property is desirable for minimizing possible deleterious effects on host cell metabolism or an immune response following gene transfer. Further deletion of IE genes ICP22 and ICP27, in addition to ICP4, substantially improve reduction of HSV cytotoxicity and prevented early and late viral gene expression (Krisky *et al.,* 1998b).

The therapeutic potential of HSV in gene transfer has been demonstrated in various *in vitro* model systems and *in vivo* for diseases such as Parkinson's (Yamada *et al.,* 1999), retinoblastoma (Hayashi *et al.,* 1999), intracerebral and intradermal tumors (Moriuchi *et al.,* 1998), B-cell malignancies (Suzuki *et al.,* 1998), ovarian cancer (Wang *et al.,* 1998) and Duchenne muscular dystrophy (Huard *et al.,* 1997).

**Adeno-Associated Viral Vectors.** Adeno-associated virus (AAV), a member of the parvovirus family, is a human virus that is increasingly being used for gene delivery therapeutics. AAV has several advantageous features not found in other viral systems. First, AAV can infect a wide range of host cells, including non-dividing cells. Second, AAV can infect cells from different species. Third, AAV has not been associated with any human or animal disease and does not appear to alter the biological properties of the host cell upon integration. For example, it is estimated that 80-85% of the human population has been exposed to AAV. Finally, AAV is stable at a wide range of physical and chemical conditions which lends itself to production, storage and transportation requirements.

The AAV genome is a linear, single-stranded DNA molecule containing 4681 nucleotides. The AAV genome generally comprises an internal non-repeating genome flanked on each end by inverted terminal repeats (ITRs) of approximately 145 bp in length. The ITRs have multiple functions, including origins of DNA replication, and as packaging signals for the viral genome. The internal non-repeated portion of the genome includes two large open reading frames, known as the AAV replication (rep) and capsid (cap) genes. The rep and cap genes code for viral proteins that allow the virus to replicate and package the viral genome into a virion. A family of at least four viral proteins is expressed from the AAV rep region, Rep 78, Rep 68, Rep 52, and Rep 40, named according to their apparent molecular weight. The AAV cap region encodes at least three proteins, VP 1, VP2, and VP3.

AAV is a helper-dependent virus requiring co-infection with a helper virus (*e.g*., adenovirus, herpesvirus or vaccinia) in order to form AAV virions. In the absence of co-infection with a helper virus, AAV establishes a latent state in which the viral genome inserts into a host cell chromosome, but infectious virions are not produced. Subsequent infection by a helper virus "rescues" the integrated genome, allowing it to replicate and package its genome into infectious AAV virions. Although AAV can infect cells from different species, the helper virus must be of the same species as the host cell (*e.g*., human AAV will replicate in canine cells co-infected with a canine adenovirus).

AAV has been engineered to deliver genes of interest by deleting the internal non-repeating portion of the AAV genome and inserting a heterologous gene between the ITRs. The heterologous gene may be functionally linked to a heterologous promoter (constitutive, cell-specific, or inducible) capable of driving gene expression in target cells. To produce infectious recombinant AAV (rAAV) containing a heterologous gene, a suitable producer cell line is transfected with a rAAV vector containing a heterologous gene. The producer cell is concurrently transfected with a second plasmid harboring the AAV rep and cap genes under the control of their respective endogenous promoters or heterologous promoters. Finally, the producer cell is infected with a helper virus.

Once these factors come together, the heterologous gene is replicated and packaged as though it were a wild-type AAV genome. When target cells are infected with the resulting rAAV virions, the heterologous gene enters and is expressed in the target cells. Because the target cells lack the rep and cap genes and the adenovirus helper genes, the rAAV cannot further replicate, package or form wild-type AAV.

The use of helper virus, however, presents a number of problems. First, the use of adenovirus in a rAAV production system causes the host cells to produce both rAAV and infectious adenovirus. The contaminating infectious adenovirus can be inactivated by heat treatment (56°C. for 1 hour). Heat treatment, however, results in approximately a 50% drop in the titer of functional rAAV virions. Second, varying amounts of adenovirus proteins are present in these preparations. For example, approximately 50% or greater of the total protein obtained in such rAAV virion preparations is free adenovirus fiber protein. If not completely removed, these adenovirus proteins have the potential of eliciting an immune response from the patient. Third, AAV vector production methods which employ a helper virus require the use and manipulation of large amounts of high titer infectious helper virus, which presents a number of health and safety concerns, particularly in regard to the use of a herpesvirus. Fourth, concomitant production of helper virus particles in rAAV virion producing cells diverts large amounts of host cellular resources away from rAAV virion production, potentially resulting in lower rAAV virion yields.

**Lentiviral Vectors.** Lentiviruses are complex retroviruses, which, in addition to the common retroviral genes *gag, pol,* and *env,* contain other genes with regulatory or structural function. The higher complexity enables the virus to modulate its life cycle, as in the course of latent infection. Some examples of lentivirus include the Human Immunodeficiency Viruses: HIV-1, HIV-2 and the Simian Immunodeficiency Virus: SIV. Lentiviral vectors have been generated by multiply attenuating the HIV virulence genes, for example, the genes *env, vif, vpr, vpu* and *nef* are deleted making the vector biologically safe.

Recombinant lentiviral vectors are capable of infecting non-dividing cells and can be used for both *in vivo* and *ex vivo* gene transfer and expression of nucleic acid sequences. The lentiviral genome and the proviral DNA have the three genes found in retroviruses: *gag, pol* and *env,* which are flanked by two long terminal repeat (LTR) sequences. The *gag* gene encodes the internal structural (matrix, capsid and nucleocapsid) proteins; the *pol* gene encodes the RNA-directed DNA polymerase (reverse transcriptase), a protease and an integrase; and the *env* gene encodes viral envelope glycoproteins. The 5' and 3' LTR's serve to promote transcription and polyadenylation of the virion RNA's. The LTR contains all other *cis*-acting sequences necessary for viral replication. Lentiviruses have additional genes including *vif, vpr, tat, rev, vpu, nef* and *vpx.*

Adjacent to the 5' LTR are sequences necessary for reverse transcription of the genome (the tRNA primer binding site) and for efficient encapsidation of viral RNA into particles (the *Psi* site). If the sequences necessary for encapsidation (or packaging of retroviral RNA into infectious virions) are missing from the viral genome, the *cis* defect prevents encapsidation of genomic RNA. However, the resulting mutant remains capable of directing the synthesis of all virion proteins.

Lentiviral vectors are known in the art, see Naldini *et al.,* (1996); Zufferey *et al.,* (1997); U.S. Patents 6,013,516; and 5,994,136. In general, the vectors are plasmid-based or virus-based, and are configured to carry the essential sequences for incorporating foreign nucleic acid, for selection and for transfer of the nucleic acid into a host cell. The *gag, pol* and *env* genes of the vectors of interest also are known in the art. Thus, the relevant genes are cloned into the selected vector and then used to transform the target cell of interest.

Recombinant lentivirus capable of infecting a non-dividing cell wherein a suitable host cell is transfected with two or more vectors carrying the packaging functions, namely gag, pol and env, as well as rev and tat is described in U.S. Patent 5,994,136. This describes a first vector that can provide a nucleic acid encoding a viral *gag* and a *pol* gene and another vector that can provide a nucleic acid encoding a viral *env* to produce a packaging cell. Introducing a vector providing a heterologous gene, such as the STAT-la gene in this invention, into that packaging cell yields a producer cell which releases infectious viral particles carrying the foreign gene of interest. The *env* preferably is an amphotropic envelope protein which allows transduction of cells of human and other species.

One may target the recombinant virus by linkage of the envelope protein with an antibody or a particular ligand for targeting to a receptor of a particular cell-type. By inserting a sequence (including a regulatory region) of interest into the viral vector, along with another gene which encodes the ligand for a receptor on a specific target cell, for example, the vector is now target-specific.

The vector providing the viral env nucleic acid sequence is associated operably with regulatory sequences, *e.g*., a promoter or enhancer. The regulatory sequence can be any eukaryotic promoter or enhancer, including for example, the Moloney murine leukemia virus promoter-enhancer element, the human cytomegalovirus enhancer or the vaccinia P7.5 promoter. In some cases, such as the Moloney murine leukemia virus promoter-enhancer element, the promoter-enhancer elements are located within or adjacent to the LTR sequences.

The heterologous or foreign nucleic acid sequence, such as the STAT-la encoding polynucleotide sequence herein, is linked operably to a regulatory nucleic acid sequence. Preferably, the heterologous sequence is linked to a promoter, resulting in a chimeric gene. The heterologous nucleic acid sequence may also be under control of either the viral LTR promoter-enhancer signals or of an internal promoter, and retained signals within the retroviral LTR can still bring about efficient expression of the transgene. Marker genes may be utilized to assay for the presence of the vector, and thus, to confirm infection and integration. The presence of a marker gene ensures the selection and growth of only those host cells which express the inserts. Typical selection genes encode proteins that confer resistance to antibiotics and other toxic substances, *e.g*., histidinol, puromycin, hygromycin, neomycin, methotrexate, *etc.,* and cell surface markers.

The vectors are introduced via transfection or infection into the packaging cell line. The packaging cell line produces viral particles that contain the vector genome. Methods for transfection or infection are well known by those of skill in the art. After cotransfection of the packaging vectors and the transfer vector to the packaging cell line, the recombinant virus is recovered from the culture media and titered by standard methods used by those of skill in the art. Thus, the packaging constructs can be introduced into human cell lines by calcium phosphate transfection, lipofection or electroporation, generally together with a dominant selectable marker, such as neo, DHFR, Gln synthetase or ADA, followed by selection in the presence of the appropriate drug and isolation of clones. The selectable marker gene can be linked physically to the packaging genes in the construct.

Lentiviral transfer vectors Naldini *et al.* (1996), have been used to infect human cells growth-arrested *in vitro* and to transduce neurons after direct injection into the brain of adult rats. The vector was efficient at transferring marker genes in vivo into the neurons and long term expression in the absence of detectable pathology was achieved. Animals analyzed ten months after a single injection of the vector showed no decrease in the average level of transgene expression and no sign of tissue pathology or immune reaction (Blomer *et al.,* 1997). Thus, in the present invention, one may graft or transplant cells infected with the recombinant lentivirus *ex vivo,* or infect cells *in vivo.*

**Other Viral Vectors.** The development and utility of viral vectors for gene delivery is constantly improving and evolving. Other viral vectors such as poxvirus; *e.g*., vaccinia virus (Gnant *et al.,* 1999; Gnant *et al.,* 1999), alpha virus; *e.g.,* sindbis virus, Semliki forest virus (Lundstrom, 1999), reovirus (Coffey *et al.,* 1998) and influenza A virus (Neumann *et al.,* 1999) are contemplated for use in the present invention and may be selected according to the requisite properties of the target system.

In certain embodiments, vaccinia viral vectors are contemplated for use in the present invention. Vaccinia virus is a particularly useful eukaryotic viral vector system for expressing heterologous genes. For example, when recombinant vaccinia virus is properly engineered, the proteins are synthesized, processed and transported to the plasma membrane. Vaccinia viruses as gene delivery vectors have recently been demonstrated to transfer genes to human tumor cells, *e.g.,* EMAP-II (Gnant *et al.,* 1999), inner ear (Derby *et al.,* 1999), glioma cells, *e.g.,* p53 (Timiryasova *et al.,* 1999) and various mammalian cells, *e.g.,* P₄₅₀ (U.S. Patent 5,506,138). The preparation, growth and manipulation of vaccinia viruses are described in U.S. Patent 5,849,304 and U.S. Patent 5,506,138.

In other embodiments, sindbis viral vectors are contemplated for use in gene delivery. Sindbis virus is a species of the alphavirus genus (Garoff and Li, 1998) which includes such important pathogens as Venezuelan, Western and Eastern equine encephalitis viruses (Sawai *et al.,* 1999; Mastrangelo *et al.,* 1999). *In vitro,* sindbis virus infects a variety of avian, mammalian, reptilian, and amphibian cells. The genome of sindbis virus consists of a single molecule of single-stranded RNA, 11,703 nucleotides in length. The genomic RNA is infectious, is capped at the 5' terminus and polyadenylated at the 3' terminus, and serves as mRNA. Translation of a vaccinia virus 26S mRNA produces a polyprotein that is cleaved co- and post-translationally by a combination of viral and presumably host-encoded proteases to give the three virus structural proteins, a capsid protein (C) and the two envelope glycoproteins (E1 and PE2, precursors of the virion E2).

Three features of Sindbis virus suggest that it would be a useful vector for the expression of heterologous genes. First, it has a wide host range, both in nature and in the laboratory. Second, gene expression occurs in the cytoplasm of the host cell and is rapid and efficient. Third, temperature-sensitive mutations in RNA synthesis are available that may be used to modulate the expression of heterologous coding sequences by simply shifting cultures to the non-permissive temperature at various time after infection. The growth and maintenance of sindbis virus is known in the art (U.S. Patent 5,217,879).

**Chimeric Viral Vectors.** Chimeric or hybrid viral vectors are being developed for use in therapeutic gene delivery and are contemplated for use in the present invention. Chimeric poxviral/retroviral vectors (Holzer *et al.,* 1999), adenoviral/retroviral vectors (Feng *et al.,* 1997; Bilbao *et al.,* 1997; Caplen *et al.,* 1999) and adenoviral/adeno-associated viral vectors (Fisher *et al.,* 1996; U.S. Patent 5,871,982) have been described.

These "chimeric" viral gene transfer systems can exploit the favorable features of two or more parent viral species. For example, Wilson *et al.,* provide a chimeric vector construct which comprises a portion of an adenovirus, AAV 5' and 3' ITR sequences and a selected transgene, described below (U.S. Patent 5,871,983).

The adenovirus/AAV chimeric virus uses adenovirus nucleic acid sequences as a shuttle to deliver a recombinant AAV/transgene genome to a target cell. The adenovirus nucleic acid sequences employed in the hybrid vector can range from a minimum sequence amount, which requires the use of a helper virus to produce the hybrid virus particle, to only selected deletions of adenovirus genes, which deleted gene products can be supplied in the hybrid viral production process by a selected packaging cell. At a minimum, the adenovirus nucleic acid sequences employed in the pAdA shuttle vector are adenovirus genomic sequences from which all viral genes are deleted and which contain only those adenovirus sequences required for packaging adenoviral genomic DNA into a preformed capsid head. More specifically, the adenovirus sequences employed are the cis-acting 5' and 3' inverted terminal repeat (ITR) sequences of an adenovirus (which function as origins of replication) and the native 5' packaging/enhancer domain, that contains sequences necessary for packaging linear Ad genomes and enhancer elements for the E1 promoter. The adenovirus sequences may be modified to contain desired deletions, substitutions, or mutations, provided that the desired function is not eliminated.

The AAV sequences useful in the above chimeric vector are the viral sequences from which the rep and cap polypeptide encoding sequences are deleted. More specifically, the AAV sequences employed are the cis-acting 5' and 3' inverted terminal repeat (ITR) sequences. These chimeras are characterized by high titer transgene delivery to a host cell and the ability to stably integrate the transgene into the host cell chromosome (U.S. Patent 5,871,983). In the hybrid vector construct, the AAV sequences are flanked by the selected adenovirus sequences discussed above. The 5' and 3' AAV ITR sequences themselves flank a selected transgene sequence and associated regulatory elements, described below. Thus, the sequence formed by the transgene and flanking 5' and 3' AAV sequences may be inserted at any deletion site in the adenovirus sequences of the vector. For example, the AAV sequences are desirably inserted at the site of the deleted E1a/E1b genes of the adenovirus. Alternatively, the AAV sequences may be inserted at an E3 deletion, E2a deletion, and so on. If only the adenovirus 5' ITR/packaging sequences and 3' ITR sequences are used in the hybrid virus, the AAV sequences are inserted between them.

The transgene sequence of the vector and recombinant virus can be a gene, a nucleic acid sequence or reverse transcript thereof, heterologous to the adenovirus sequence, which encodes a protein, polypeptide or peptide fragment of interest. The transgene is operatively linked to regulatory components in a manner which permits transgene transcription. The composition of the transgene sequence will depend upon the use to which the resulting hybrid vector will be put. For example, one type of transgene sequence includes a therapeutic gene which expresses a desired gene product in a host cell. These therapeutic genes or nucleic acid sequences typically encode products for administration and expression in a patient *in vivo* or *ex vivo* to replace or correct an inherited or non-inherited genetic defect or treat an epigenetic disorder or disease.

### 10. Non-Viral Transformation

Suitable methods for nucleic acid delivery for transformation of an organelle, a cell, a tissue or an organism for use with the current invention are believed to include virtually any method by which a nucleic acid (*e.g.,* DNA) can be introduced into an organelle, a cell, a tissue or an organism, as described herein or as would be known to one of ordinary skill in the art. Such methods include, but are not limited to, direct delivery of DNA such as by injection (U.S. Patents 5,994,624, 5,981,274, 5,945,100, 5,780,448, 5,736,524, 5,702,932, 5,656,610, 5,589,466 and 5,580,859), including microinjection (Harland and Weintraub, 1985; U.S. Patent 5,789,215); by electroporation (U.S. Patent 5,384,253); by calcium phosphate precipitation (Graham and Van Der Eb, 1973; Chen and Okayama, 1987; Rippe *et al.,* 1990); by using DEAE-dextran followed by polyethylene glycol (Gopal, 1985); by direct sonic loading (Fechheimer *et al.,* 1987); by liposome mediated transfection (Nicolau and Sene, 1982; Fraley *et al.,* 1979; Nicolau *et al.,* 1987; Wong *et al.,* 1980; Kaneda *et al.,* 1989; Kato *et al.,* 1991); by microprojectile bombardment (PCT Application Nos. WO 94/09699 and 95/06128; U.S. Patents 5,610,042; 5,322,783, 5,563,055, 5,550,318, 5,538,877 and 5,538,880); by agitation with silicon carbide fibers (Kaeppler *et al.,* 1990; U.S. Patents 5,302,523 and 5,464,765); or by PEG-mediated transformation of protoplasts (Omirulleh *et al.,* 1993; U.S. Patents 4,684,611 and 4,952,500); by desiccation/inhibition-mediated DNA uptake (Potrykus *et al.,* 1985). Through the application of techniques such as these, organelle(s), cell(s), tissue(s) or organism(s) may be stably or transiently transformed.

**Injection.** In certain embodiments, a nucleic acid may be delivered to an organelle, a cell, a tissue or an organism via one or more injections (*i.e.,* a needle injection), such as, for example, either subcutaneously, intradermally, intramuscularly, intervenously or intraperitoneally. Methods of injection of vaccines are well known to those of ordinary skill in the art (*e.g.,* injection of a composition comprising a saline solution). Further embodiments of the present disclosure include the introduction of a nucleic acid by direct microinjection. Direct microinjection has been used to introduce nucleic acid constructs into Xenopus oocytes (Harland and Weintraub, 1985).

**Electroporation.** In certain embodiments of the present invention, a nucleic acid is introduced into an organelle, a cell, a tissue or an organism via electroporation. Electroporation involves the exposure of a suspension of cells and DNA to a high-voltage electric discharge. In some variants of this method, certain cell wall-degrading enzymes, such as pectin-degrading enzymes, are employed to render the target recipient cells more susceptible to transformation by electroporation than untreated cells (U.S. Patent 5,384,253). Alternatively, recipient cells can be made more susceptible to transformation by mechanical wounding.

Transfection of eukaryotic cells using electroporation has been quite successful. Mouse pre-B lymphocytes have been transfected with human κ-immunoglobulin genes (Potter *et al.,* 1984), and rat hepatocytes have been transfected with the chloramphenicol acetyltransferase gene (Tur-Kaspa *et al.,* 1986) in this manner.

To effect transformation by electroporation in cells such as, for example, plant cells, one may employ either friable tissues, such as a suspension culture of cells or embryogenic callus or alternatively one may transform immature embryos or other organized tissue directly. In this technique, one would partially degrade the cell walls of the chosen cells by exposing them to pectin-degrading enzymes (pectolyases) or mechanically wounding in a controlled manner. Examples of some species which have been transformed by electroporation of intact cells include maize (U.S. Patent 5,384,253; Rhodes *et al.,* 1995; D'Halluin *et al.,* 1992), wheat (Zhou *et al.,* 1993), tomato (Hou and Lin, 1996), soybean (Christou *et al.,* 1987) and tobacco (Lee *et al.,* 1989).

One also may employ protoplasts for electroporation transformation of plant cells (Bates, 1994; Lazzeri, 1995). For example, the generation of transgenic soybean plants by electroporation of cotyledon-derived protoplasts is described by Dhir and Widholm in International Patent Application No. WO 92/17598. Other examples of species for which protoplast transformation has been described include barley (Lazerri, 1995), sorghum (Battraw *et al.,* 1991), maize (Bhattacharjee *et al.,* 1997), wheat (He *et al.,* 1994) and tomato (Tsukada, 1989).

**Calcium Phosphate.** In other embodiments of the present invention, a nucleic acid is introduced to the cells using calcium phosphate precipitation. Human KB cells have been transfected with adenovirus 5 DNA (Graham and Van Der Eb, 1973) using this technique. Also in this manner, mouse L(A9), mouse C127, CHO, CV-1, BHK, NIH3T3 and HeLa cells were transfected with a neomycin marker gene (Chen and Okayama, 1987), and rat hepatocytes were transfected with a variety of marker genes (Rippe *et al.,* 1990).

DEAE-Dextran: In another embodiment, a nucleic acid is delivered into a cell using DEAE-dextran followed by polyethylene glycol. In this manner, reporter plasmids were introduced into mouse myeloma and erythroleukemia cells (Gopal, 1985).

**Sonication Loading.** Additional embodiments of the present invention include the introduction of a nucleic acid by direct sonic loading. LTK⁻ fibroblasts have been transfected with the thymidine kinase gene by sonication loading (Fechheimer *et al.,* 1987).

**Liposome-Mediated Transfection.** In a further embodiment of the invention, a nucleic acid may be entrapped in a lipid complex such as, for example, a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh and Bachhawat, 1991). Also contemplated is an nucleic acid complexed with Lipofectamine (Gibco BRL) or Superfect (Qiagen).

Liposome-mediated nucleic acid delivery and expression of foreign DNA *in vitro* has been very successful (Nicolau and Sene, 1982; Fraley *et al.,* 1979; Nicolau *et al.,* 1987). The feasibility of liposome-mediated delivery and expression of foreign DNA in cultured chick embryo, HeLa and hepatoma cells has also been demonstrated (Wong *et al.,* 1980).

In certain embodiments of the invention, a liposome may be complexed with a hemagglutinating virus (HVJ). This has been shown to facilitate fusion with the cell membrane and promote cell entry of liposome-encapsulated DNA (Kaneda *et al.,* 1989). In other embodiments, a liposome may be complexed or employed in conjunction with nuclear non-histone chromosomal proteins (HMG-1) (Kato *et al.,* 1991). In yet further embodiments, a liposome may be complexed or employed in conjunction with both HVJ and HMG-1. In other embodiments, a delivery vehicle may comprise a ligand and a liposome.

**Receptor-Mediated Transfection.** Still further, a nucleic acid may be delivered to a target cell via receptor-mediated delivery vehicles. These take advantage of the selective uptake of macromolecules by receptor-mediated endocytosis that will be occurring in a target cell. In view of the cell type-specific distribution of various receptors, this delivery method adds another degree of specificity.

Certain receptor-mediated gene targeting vehicles comprise a cell receptor-specific ligand and a nucleic acid-binding agent. Others comprise a cell receptor-specific ligand to which the nucleic acid to be delivered has been operatively attached. Several ligands have been used for receptor-mediated gene transfer (Wu and Wu, 1987; Wagner *et al.,* 1990; Perales *et al.,* 1994; Myers, EPO 0273085), which establishes the operability of the technique. Specific delivery in the context of another mammalian cell type has been described (Wu and Wu, 1993). In certain aspects of the present invention, a ligand will be chosen to correspond to a receptor specifically expressed on the target cell population.

In other embodiments, a nucleic acid delivery vehicle component of a cell-specific nucleic acid targeting vehicle may comprise a specific binding ligand in combination with a liposome. The nucleic acid(s) to be delivered are housed within the liposome and the specific binding ligand is functionally incorporated into the liposome membrane. The liposome will thus specifically bind to the receptor(s) of a target cell and deliver the contents to a cell. Such systems have been shown to be functional using systems in which, for example, epidermal growth factor (EGF) is used in the receptor-mediated delivery of a nucleic acid to cells that exhibit upregulation of the EGF receptor.

In still further embodiments, the nucleic acid delivery vehicle component of a targeted delivery vehicle may be a liposome itself, which will preferably comprise one or more lipids or glycoproteins that direct cell-specific binding. For example, lactosyl-ceramide, a galactose-terminal asialganglioside, have been incorporated into liposomes and observed an increase in the uptake of the insulin gene by hepatocytes (Nicolau *et al.,* 1987). It is contemplated that the tissue-specific transforming constructs of the present invention can be specifically delivered into a target cell in a similar manner.

### 11. Expression Systems

Numerous expression systems exist that comprise at least a part or all of the compositions discussed above. Prokaryote- and/or eukaryote-based systems can be employed for use with the present invention to produce nucleic acid sequences, or their cognate polypeptides, proteins and peptides. Many such systems are commercially and widely available.

The insect cell/baculovirus system can produce a high level of protein expression of a heterologous nucleic acid segment, such as described in U.S. Patents 5,871,986 and 4,879,236 and which can be bought, for example, under the name MaxBac® 2.0 from Invitrogen® and BacPack™ Baculovirus Expression System From Clontech®.

Other examples of expression systems include Stratagene®'s Complete Control™ Inducible Mammalian Expression System, which involves a synthetic ecdysone-inducible receptor, or its pET Expression System, an *E. coli* expression system. Another example of an inducible expression system is available from Invitrogen®, which carries the T-Rex™ (tetracycline-regulated expression) System, an inducible mammalian expression system that uses the full-length CMV promoter. Invitrogen® also provides a yeast expression system called the *Pichia methanolica* Expression System, which is designed for high-level production of recombinant proteins in the methylotrophic yeast *Pichia methanolica.* One of skill in the art would know how to express a vector, such as an expression construct, to produce a nucleic acid sequence or its cognate polypeptide, protein, or peptide.

Primary mammalian cell cultures may be prepared in various ways. In order for the cells to be kept viable while *in vitro* and in contact with the expression construct, it is necessary to ensure that the cells maintain contact with the correct ratio of oxygen and carbon dioxide and nutrients but are protected from microbial contamination. Cell culture techniques are well documented.

One embodiment of the foregoing involves the use of gene transfer to immortalize cells for the production of proteins. The gene for the protein of interest may be transferred as described above into appropriate host cells followed by culture of cells under the appropriate conditions. The gene for virtually any polypeptide may be employed in this manner. The generation of recombinant expression vectors, and the elements included therein, are discussed above. Alternatively, the protein to be produced may be an endogenous protein normally synthesized by the cell in question.

Examples of useful mammalian host cell lines are Vero and HeLa cells and cell lines of Chinese hamster ovary, W138, BHK, COS-7, 293, HepG2, NIH3T3, RIN and MDCK cells. In addition, a host cell strain may be chosen that modulates the expression of the inserted sequences, or modifies and process the gene product in the manner desired. Such modifications (*e.g.,* glycosylation) and processing (*e.g.,* cleavage) of protein products may be important for the function of the protein. Different host cells have characteristic and specific mechanisms for the post-translational processing and modification of proteins. Appropriate cell lines or host systems can be chosen to insure the correct modification and processing of the foreign protein expressed.

A number of selection systems may be used including, but not limited to, HSV thymidine kinase, hypoxanthine-guanine phosphoribosyltransferase and adenine phosphoribosyltransferase genes, in *tk-, hgprt-* or *aprt-* cells, respectively. Also, antimetabolite resistance can be used as the basis of selection for *dhfr,* that confers resistance to; *gpt,* that confers resistance to mycophenolic acid; *neo,* that confers resistance to the aminoglycoside G418; and *hygro,* that confers resistance to hygromycin.

### 12. Preparation of Transgenic Animals

In an embodiment of the invention, a transgenic animal is produced by the integration of an antibody transgene into the genome in a manner that permits the expression of the transgene. Methods for producing transgenic animals are generally described by Wagner and Hoppe (U.S. Patent 4,873,191), and Brinster *et al.* (1985).

Typically, a gene flanked by genomic sequences is transferred by microinjection into a fertilized egg. The microinjected eggs are implanted into a host female, and the progeny are screened for the expression of the transgene. Transgenic animals may be produced from the fertilized eggs from a number of animals including, but not limited to reptiles, amphibians, birds, mammals, and fish.

DNA clones for microinjection can be prepared by any means known in the art. For example, DNA clones for microinjection can be cleaved with enzymes appropriate for removing the bacterial plasmid sequences, and the DNA fragments electrophoresed on 1% agarose gels in TBE buffer, using standard techniques. The DNA bands are visualized by staining with ethidium bromide, and the band containing the expression sequences is excised. The excised band is then placed in dialysis bags containing 0.3 M sodium acetate, pH 7.0. DNA is electroeluted into the dialysis bags, extracted with a 1:1 phenol:chloroform solution and precipitated by two volumes of ethanol. The DNA is redissolved in 1 ml of low salt buffer (0.2 M NaCl, 20 mM Tris, pH 7.4, and 1 mM EDTA) and purified on an Elutip-D™ column. The column is first primed with 3 ml of high salt buffer (1 M NaCl, 20 mM Tris, pH 7.4, and 1 mM EDTA) followed by washing with 5 ml of low salt buffer. The DNA solutions are passed through the column three times to bind DNA to the column matrix. After one wash with 3 ml of low salt buffer, the DNA is eluted with 0.4 ml high salt buffer and precipitated by two volumes of ethanol. DNA concentrations are measured by absorption at 260 nm in a UV spectrophotometer. For microinjection, DNA concentrations are adjusted to 3 µg/ml in 5 mM Tris, pH 7.4 and 0.1 mM EDTA. Other methods for purification of DNA for microinjection are described in in Palmiter *et al.* (1982); and in Sambrook *et al.* (2001).

In an exemplary microinjection procedure, female mice six weeks of age are induced to superovulate with a 5 IU injection (0.1 cc, ip) of pregnant mare serum gonadotropin (PMSG; Sigma) followed 48 hours later by a 5 IU injection (0.1 cc, ip) of human chorionic gonadotropin (hCG; Sigma). Females are placed with males immediately after hCG injection. Twenty-one hours after hCG injection, the mated females are sacrificed by C02 asphyxiation or cervical dislocation and embryos are recovered from excised oviducts and placed in Dulbecco's phosphate buffered saline with 0.5% bovine serum albumin (BSA; Sigma). Surrounding cumulus cells are removed with hyaluronidase (1 mg/ml). Pronuclear embryos are then washed and placed in Earle's balanced salt solution containing 0.5% BSA (EBSS) in a 37.5 °C. incubator with a humidified atmosphere at 5% CO₂, 95% air until the time of injection. Embryos can be implanted at the two-cell stage.

Randomly cycling adult female mice are paired with vasectomized males. C57BL/6 or Swiss mice or other comparable strains can be used for this purpose. Recipient females are mated at the same time as donor females. At the time of embryo transfer, the recipient females are anesthetized with an intraperitoneal injection of 0.015 ml of 2.5% avertin per gram of body weight. The oviducts are exposed by a single midline dorsal incision. An incision is then made through the body wall directly over the oviduct. The ovarian bursa is then torn with watchmakers forceps. Embryos to be transferred are placed in DPBS (Dulbecco's phosphate buffered saline) and in the tip of a transfer pipet (about 10 to 12 embryos). The pipet tip is inserted into the infundibulum and the embryos transferred. After the transfer, the incision is closed by two sutures.

### 13. Interfereing RNAs

RNA interference (also referred to as "RNA-mediated interference" or RNAi) is a mechanism by which gene expression can be reduced or eliminated. Double-stranded RNA (dsRNA) has been observed to mediate the reduction, which is a multi-step process. dsRNA activates post-transcriptional gene expression surveillance mechanisms that appear to function to defend cells from virus infection and transposon activity (Fire *et al.,* 1998; Grishok *et al.,* 2000; Ketting *et al.,* 1999; Lin and Avery *et al.,* 1999; Montgomery *et al.,* 1998; Sharp and Zamore, 2000; Tabara *et al.,* 1999). Activation of these mechanisms targets mature, dsRNA-complementary mRNA for destruction. RNAi offers major experimental advantages for study of gene function. These advantages include a very high specificity, ease of movement across cell membranes, and prolonged down-regulation of the targeted gene (Fire *et al.,* 1998; Grishok *et al.,* 2000; Ketting *et al.,* 1999; Lin and Avery *et al.,* 1999; Montgomery *et al.,* 1998; Sharp *et al.,* 1999; Sharp and Zamore, 2000; Tabara *et al.,* 1999). Moreover, dsRNA has been shown to silence genes in a wide range of systems, including plants, protozoans, fungi, *C*. *elegans, Trypanasoma, Drosophila,* and mammals (Grishok *et al.,* 2000; Sharp *et al.,* 1999; Sharp and Zamore, 2000; Elbashir *et al.,* 2001). It is generally accepted that RNAi acts post-transcriptionally, targeting RNA transcripts for degradation. It appears that both nuclear and cytoplasmic RNA can be targeted (Bosher and Labouesse, 2000).

siRNAs must be designed so that they are specific and effective in suppressing the expression of the genes of interest. Methods of selecting the target sequences, *i.e.,* those sequences present in the gene or genes of interest to which the siRNAs will guide the degradative machinery, are directed to avoiding sequences that may interfere with the siRNA's guide function while including sequences that are specific to the gene or genes. Typically, siRNA target sequences of about 21 to 23 nucleotides in length are most effective. This length reflects the lengths of digestion products resulting from the processing of much longer RNAs as described above (Montgomery *et al.,* 1998).

The making of siRNAs has been mainly through direct chemical synthesis; through processing of longer, double-stranded RNAs through exposure to *Drosophila* embryo lysates; or through an *in vitro* system derived from S2 cells. Use of cell lysates or *in vitro* processing may further involve the subsequent isolation of the short, 21-23 nucleotide siRNAs from the lysate, *etc.,* making the process somewhat cumbersome and expensive. Chemical synthesis proceeds by making two single-stranded RNA-oligomers followed by the annealing of the two single-stranded oligomers into a double-stranded RNA. Methods of chemical synthesis are diverse. Non-limiting examples are provided in U.S. Patents 5,889,136, 4,415,723, and 4,458,066 and in Wincott *et al.* (1995).

Several further modifications to siRNA sequences have been suggested in order to alter their stability or improve their effectiveness. It is suggested that synthetic complementary 21-mer RNAs having di-nucleotide overhangs (*i.e.,* 19 complementary nucleotides + 3' non-complementary dimers) may provide the greatest level of suppression. These protocols primarily use a sequence of two (2'-deoxy) thymidine nucleotides as the di-nucleotide overhangs. These dinucleotide overhangs are often written as dTdT to distinguish them from the typical nucleotides incorporated into RNA. The literature has indicated that the use of dT overhangs is primarily motivated by the need to reduce the cost of the chemically synthesized RNAs. It is also suggested that the dTdT overhangs might be more stable than UU overhangs, though the data available shows only a slight (< 20%) improvement of the dTdT overhang compared to an siRNA with a UU overhang.

Chemically synthesized siRNAs are found to work optimally when they are in cell culture at concentrations of 25-100 nM, but concentrations of about 100 nM have achieved effective suppression of expression in mammalian cells. siRNAs have been most effective in mammalian cell culture at about 100 nM. In several instances, however, lower concentrations of chemically synthesized siRNA have been used (Caplen *et al.,* 2000; Elbashir *et al.,* 2001).

WO 99/32619 and WO 01/68836 suggest that RNA for use in siRNA may be chemically or enzymatically synthesized. The enzymatic synthesis contemplated in these references is by a cellular RNA polymerase or a bacteriophage RNA polymerase *(e.g.,* T3, T7, SP6) via the use and production of an expression construct as is known in the art. For example, see U.S. Patent 5,795,715. The contemplated constructs provide templates that produce RNAs that contain nucleotide sequences identical to a portion of the target gene. The length of identical sequences provided by these references is at least 25 bases, and may be as many as 400 or more bases in length. An important aspect of this reference is that the authors contemplate digesting longer dsRNAs to 21-25mer lengths with the endogenous nuclease complex that converts long dsRNAs to siRNAs *in vivo.* They do not describe or present data for synthesizing and using *in vitro* transcribed 21-25mer dsRNAs. No distinction is made between the expected properties of chemical or enzymatically synthesized dsRNA in its use in RNA interference.

Similarly, WO 00/44914 suggests that single strands of RNA can be produced enzymatically or by partial/total organic synthesis. Preferably, single-stranded RNA is enzymatically synthesized from the PCR products of a DNA template, preferably a cloned cDNA template and the RNA product is a complete transcript of the cDNA, which may comprise hundreds of nucleotides. WO 01/36646 places no limitation upon the manner in which the siRNA is synthesized, providing that the RNA may be synthesized *in vitro* or *in vivo*, using manual and/or automated procedures. This reference also provides that *in vitro* synthesis may be chemical or enzymatic, for example using cloned RNA polymerase (*e.g.*, T3, T7, SP6) for transcription of the endogenous DNA (or cDNA) template, or a mixture of both. Again, no distinction in the desirable properties for use in RNA interference is made between chemically or enzymatically synthesized siRNA.

U.S. Patent 5,795,715 reports the simultaneous transcription of two complementary DNA sequence strands in a single reaction mixture, wherein the two transcripts are immediately hybridized. The templates used are preferably of between 40 and 100 base pairs, and which is equipped at each end with a promoter sequence. The templates are preferably attached to a solid surface. After transcription with RNA polymerase, the resulting dsRNA fragments may be used for detecting and/or assaying nucleic acid target sequences.

### E. Purification

In certain embodiments, the antibodies of the present disclsoure may be purified. The term "purified," as used herein, is intended to refer to a composition, isolatable from other components, wherein the protein is purified to any degree relative to its naturally-obtainable state. A purified protein therefore also refers to a protein, free from the environment in which it may naturally occur. Where the term "substantially purified" is used, this designation will refer to a composition in which the protein or peptide forms the major component of the composition, such as constituting about 50%, about 60%, about 70%, about 80%, about 90%, about 95% or more of the proteins in the composition.

Protein purification techniques are well known to those of skill in the art. These techniques involve, at one level, the crude fractionation of the cellular milieu to polypeptide and non-polypeptide fractions. Having separated the polypeptide from other proteins, the polypeptide of interest may be further purified using chromatographic and electrophoretic techniques to achieve partial or complete purification (or purification to homogeneity). Analytical methods particularly suited to the preparation of a pure peptide are ion-exchange chromatography, exclusion chromatography; polyacrylamide gel electrophoresis; isoelectric focusing. Other methods for protein purification include, precipitation with ammonium sulfate, PEG, antibodies and the like or by heat denaturation, followed by centrifugation; gel filtration, reverse phase, hydroxylapatite and affinity chromatography; and combinations of such and other techniques.

In purifying an antibody of the present disclosure, it may be desirable to express the polypeptide in a prokaryotic or eukaryotic expression system and extract the protein using denaturing conditions. The polypeptide may be purified from other cellular components using an affinity column, which binds to a tagged portion of the polypeptide. As is generally known in the art, it is believed that the order of conducting the various purification steps may be changed, or that certain steps may be omitted, and still result in a suitable method for the preparation of a substantially purified protein or peptide.

Commonly, complete antibodies are fractionated utilizing agents (*i.e*., protein A) that bind the Fc portion of the antibody. Alternatively, antigens may be used to simultaneously purify and select appropriate antibodies. Such methods often utilize the selection agent bound to a support, such as a column, filter or bead. The antibodies are bound to a support, contaminants removed (*e.g*., washed away), and the antibodies released by applying conditions (salt, heat, *etc*.).

Various methods for quantifying the degree of purification of the protein or peptide will be known to those of skill in the art in light of the present disclosure. These include, for example, determining the specific activity of an active fraction, or assessing the amount of polypeptides within a fraction by SDS/PAGE analysis. Another method for assessing the purity of a fraction is to calculate the specific activity of the fraction, to compare it to the specific activity of the initial extract, and to thus calculate the degree of purity. The actual units used to represent the amount of activity will, of course, be dependent upon the particular assay technique chosen to follow the purification and whether or not the expressed protein or peptide exhibits a detectable activity.

It is known that the migration of a polypeptide can vary, sometimes significantly, with different conditions of SDS/PAGE (Capaldi *et al.,* 1977). It will therefore be appreciated that under differing electrophoresis conditions, the apparent molecular weights of purified or partially purified expression products may vary.

### F. Single Chain/Single Domain Antibodies

A Single Chain Variable Fragment (scFv) is a fusion of the variable regions of the heavy and light chains of immunoglobulins, linked together with a short (usually serine, glycine) linker. This chimeric molecule, also known as a single domain antibody, retains the specificity of the original immunoglobulin, despite removal of the constant regions and the introduction of a linker peptide. This modification usually leaves the specificity unaltered. These molecules were created historically to facilitate phage display where it is highly convenient to express the antigen binding domain as a single peptide. Alternatively, scFv can be created directly from subcloned heavy and light chains derived from a hybridoma. Single domain or single chain variable fragments lack the constant Fc region found in complete antibody molecules, and thus, the common binding sites (*e.g.*, protein A/G) used to purify antibodies (single chain antibodies include the Fc region). These fragments can often be purified/immobilized using Protein L since Protein L interacts with the variable region of kappa light chains.

Flexible linkers generally are comprised of helix- and turn-promoting amino acid residues such as alaine, serine and glycine. However, other residues can function as well. Tang *et al.* (1996) used phage display as a means of rapidly selecting tailored linkers for single-chain antibodies (scFvs) from protein linker libraries. A random linker library was constructed in which the genes for the heavy and light chain variable domains were linked by a segment encoding an 18-amino acid polypeptide of variable composition. The scFv repertoire (approx. 5 × 10⁶ different members) was displayed on filamentous phage and subjected to affinity selection with hapten. The population of selected variants exhibited significant increases in binding activity but retained considerable sequence diversity. Screening 1054 individual variants subsequently yielded a catalytically active scFv that was produced efficiently in soluble form. Sequence analysis revealed a conserved proline in the linker two residues after the V_{H} C terminus and an abundance of arginines and prolines at other positions as the only common features of the selected tethers.

The recombinant antibodies of the present disclosure may also involve sequences or moieties that permit dimerization or multimerization of the receptors. Such sequences include those derived from IgA, which permit formation of multimers in conjunction with the J-chain. Another multimerization domain is the Gal4 dimerization domain. In other embodiments, the chains may be modified with agents such as biotin/avidin, which permit the combination of two antibodies.

In a separate embodiment, a single-chain antibody can be created by joining receptor light and heavy chains using a non-peptide linker or chemical unit. Generally, the light and heavy chains will be produced in distinct cells, purified, and subsequently linked together in an appropriate fashion (*i.e*., the N-terminus of the heavy chain being attached to the C-terminus of the light chain via an appropriate chemical bridge).

Cross-linking reagents are used to form molecular bridges that tie functional groups of two different molecules, *e.g*., a stablizing and coagulating agent. However, it is contemplated that dimers or multimers of the same analog or heteromeric complexes comprised of different analogs can be created. To link two different compounds in a step-wise manner, hetero-bifunctional cross-linkers can be used that eliminate unwanted homopolymer formation.

An exemplary hetero-bifunctional cross-linker contains two reactive groups: one reacting with primary amine group (*e.g.*, N-hydroxy succinimide) and the other reacting with a thiol group (*e.g.*, pyridyl disulfide, maleimides, halogens, *etc*.). Through the primary amine reactive group, the cross-linker may react with the lysine residue(s) of one protein (*e.g.*, the selected antibody or fragment) and through the thiol reactive group, the cross-linker, already tied up to the first protein, reacts with the cysteine residue (free sulfhydryl group) of the other protein (*e.g.*, the selective agent).

It is preferred that a cross-linker having reasonable stability in blood will be employed. Numerous types of disulfide-bond containing linkers are known that can be successfully employed to conjugate targeting and therapeutic/preventative agents. Linkers that contain a disulfide bond that is sterically hindered may prove to give greater stability *in vivo*, preventing release of the targeting peptide prior to reaching the site of action. These linkers are thus one group of linking agents.

Another cross-linking reagent is SMPT, which is a bifunctional cross-linker containing a disulfide bond that is "sterically hindered" by an adjacent benzene ring and methyl groups. It is believed that steric hindrance of the disulfide bond serves a function of protecting the bond from attack by thiolate anions such as glutathione which can be present in tissues and blood, and thereby help in preventing decoupling of the conjugate prior to the delivery of the attached agent to the target site.

The SMPT cross-linking reagent, as with many other known cross-linking reagents, lends the ability to cross-link functional groups such as the SH of cysteine or primary amines (*e.g*., the epsilon amino group of lysine). Another possible type of cross-linker includes the hetero-bifunctional photoreactive phenylazides containing a cleavable disulfide bond such as sulfosuccinimidyl-2-(p-azido salicylamido) ethyl-1,3'-dithiopropionate. The N-hydroxy-succinimidyl group reacts with primary amino groups and the phenylazide (upon photolysis) reacts non-selectively with any amino acid residue.

In addition to hindered cross-linkers, non-hindered linkers also can be employed in accordance herewith. Other useful cross-linkers, not considered to contain or generate a protected disulfide, include SATA, SPDP and 2-iminothiolane (Wawrzynczak & Thorpe, 1987). The use of such cross-linkers is well understood in the art. Another embodiment involves the use of flexible linkers.

U.S. Patent 4,680,338, describes bifunctional linkers useful for producing conjugates of ligands with amine-containing polymers and/or proteins, especially for forming antibody conjugates with chelators, drugs, enzymes, detectable labels and the like. U.S. Patents 5,141,648 and 5,563,250 disclose cleavable conjugates containing a labile bond that is cleavable under a variety of mild conditions. This linker is particularly useful in that the agent of interest may be bonded directly to the linker, with cleavage resulting in release of the active agent. Particular uses include adding a free amino or free sulfhydryl group to a protein, such as an antibody, or a drug.

U.S. Patent 5,856,456 provides peptide linkers for use in connecting polypeptide constituents to make fusion proteins, *e.g*., single chain antibodies. The linker is up to about 50 amino acids in length, contains at least one occurrence of a charged amino acid (preferably arginine or lysine) followed by a proline, and is characterized by greater stability and reduced aggregation. U.S. Patent 5,880,270 discloses aminooxy-containing linkers useful in a variety of immunodiagnostic and separative techniques.

### III. Pharmaceutical Formulations and Treatment of MS

### A. Multiple Sclerosis Therapies

In accordance with the present disclosure, the inventor proposes that the inhibition of antibodies described herein, *i.e*., those having the VH4 signature and binding to gray matter antigens, can be inhibited as part of an MS therapy. There are several different embodiments by which this can be achieved.

First, one may ablate that B cells populations that produce the antibodies described herein. The most well know example of a B cell ablative therapy is the use of an anti-CD20 antibody that non-selectively attacks B cells. This type of approach has been used to treat B cell malignancies and certain autoimmune disorders. Another option would be to tailore a specific agent that could physically ablate only the specific B cells described herein, such as an anti-idiotypic antibody, or one that could "silence" the expression of the antibodies, such as an siRNA directed to the specific message produced by these B cells.

Another approach would be to limit the activity of the antibodies described herein by providing an inhibitory factor. A factor with this capability could be an anti-idiotypic antibody that binds the AGS antibody, a peptide that reflects an epitope to which the AGS antibody binds, or an antibody fragment having the same specificity as the AGS antibody but lacking effector functions (*i.e*., lacking Fc structures).

### B. Formulation and Administration

The present disclosure provides pharmaceutical compositions comprising antibody inhibitory substances. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Other suitable pharmaceutical excipients include starch, glucose, lactose, sucrose, saline, dextrose, gelatin, malt, rice, flour, chalk, silica gel, sodium stearate, glycerol monostearate, talc, sodium chloride, dried skim milk, glycerol, propylene glycol, water, ethanol and the like.

The compositions can be formulated as neutral or salt forms. Pharmaceutically acceptable salts include those formed with anions such as those derived from hydrochloric, phosphoric, acetic, oxalic, tartaric acids, *etc*., and those formed with cations such as those derived from sodium, potassium, ammonium, calcium, ferric hydroxides, isopropylamine, triethylamine, 2-ethylamino ethanol, histidine, procaine, *etc.*

The antibodies of the present disclosure may include classic pharmaceutical preparations. Administration of these compositions will be via any common route so long as the target tissue is available via that route. This includes oral, nasal, buccal, rectal, vaginal or topical. Alternatively, administration may be by intradermal, subcutaneous, intramuscular, intraperitoneal or intravenous injection. Such compositions would normally be administered as pharmaceutically acceptable compositions, described *supra.* Of particular interest is direct intratumoral administration, perfusion of a tumor, or admininstration local or regional to a tumor, for example, in the local or regional vasculature or lymphatic system, or in a resected tumor bed.

The active compounds may also be administered parenterally or intraperitoneally. Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

### C. Combination Therapies

In the context of the present invention, it also is contemplated that the siRNA described herein could be used similarly in conjunction with more traditional MS treatments. These compositions or therapies would be provided in a combined amount effective to treat the disease. This process may involve administration of the siRNA according to the present invention and the other agent or therapy at the same time. This may be achieved by administering a single composition or pharmacological formulation that includes both agents, or by contacting the cell with two distinct compositions or formulations, at the same time, wherein one composition includes the siRNA according to the present invention and the other includes the other agent.

Alternatively, the therapy according to the present invention may precede or follow the other agent/treatment by intervals ranging from minutes to weeks. In embodiments where the other agent is administered seperately, one would generally ensure that a significant period of time did not expire between the time of each delivery, such that the agents would still be able to exert an advantageously combined effect. In such instances, it is contemplated that one would administer both modalities within about 12-24 hours of each other and, more preferably, within about 6-12 hours of each other, with a delay time of only about 12 hours being most preferred. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

It also is conceivable that more than one administration of either agent/therapy will be desired. Various combinations may be employed, where an agent according to the present invention therapy is "A" and the other agent/therapy is "B", as exemplified below:

| | | | | | | | |
|---|---|---|---|---|---|---|---|
| A/B/A | B/A/B | B/B/A | A/A/B | B/A/A | A/B/B | B/B/B/A | B/B/A/B |
| A/A/B/B | A/B/A/B | A/B/B/A | B/B/A/A | | B/A/B/A | B/A/A/B | B/B/B/A |
| A/A/A/B | B/A/A/A | A/B/A/A | A/A/B/A | | A/B/B/B | B/A/B/B | B/B/A/B |

Other combinations are contemplated. Again, to achieve cell killing, both agents are delivered to a cell in a combined amount effective to kill the cell.

Traditional therapeutic MS agents or factors suitable for combination are those described above in the discussion of MS therapy and prophylaxis.

### IV. Antibody Conjugates

Antibodies may be linked to at least one agent to form an antibody conjugate. In order to increase the efficacy of antibody molecules as diagnostic or therapeutic agents, it is conventional to link or covalently bind or complex at least one desired molecule or moiety. Such a molecule or moiety may be, but is not limited to, at least one effector or reporter molecule. Effector molecules comprise molecules having a desired activity, e.g., immunosuppression/anti-inflammation. Non-limiting examples of such molecules are set out above. Such molecules are optionally attached via cleavable linkers designed to allow the molecules to be released at or near the target site.

By contrast, a reporter molecule is defined as any moiety which may be detected using an assay. Non-limiting examples of reporter molecules which have been conjugated to antibodies include enzymes, radiolabels, haptens, fluorescent labels, phosphorescent molecules, chemiluminescent molecules, chromophores, photoaffinity molecules, colored particles or ligands, such as biotin.

Antibody conjugates are generally preferred for use as diagnostic agents. Antibody diagnostics generally fall within two classes, those for use in *in vitro* diagnostics, such as in a variety of immunoassays, and those for use *in vivo* diagnostic protocols, generally known as "antibody-directed imaging." Many appropriate imaging agents are known in the art, as are methods for their attachment to antibodies (see, for *e.g*., U.S. Patents 5,021,236, 4,938,948, and 4,472,509). The imaging moieties used can be paramagnetic ions, radioactive isotopes, fluorochromes, NMR-detectable substances, and X-ray imaging agents.

In the case of paramagnetic ions, one might mention by way of example ions such as chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) and/or erbium (III), with gadolinium being particularly preferred. Ions useful in other contexts, such as X-ray imaging, include but are not limited to lanthanum (III), gold (III), lead (II), and especially bismuth (III).

In the case of radioactive isotopes for therapeutic and/or diagnostic application, one might mention astatine²¹¹, ¹⁴carbon, ⁵¹chromium, ³⁶chlorine, ⁵⁷cobalt, ⁵⁸cobalt, copper⁶⁷, ¹⁵²Eu, gallium⁶⁷, ³hydrogen, iodine¹²³, iodine¹²⁵, iodine¹³¹, indium¹¹¹, ⁵⁹iron , ³²phosphorus, rhenium¹⁸⁶, rhenium¹⁸⁸, ⁷⁵selenium, ³⁵sulphur, technicium^{99m} and/or yttrium⁹⁰. ¹²⁵I is often being preferred for use in certain embodiments, and technicium^{99m} and/or indium¹¹¹ are also often preferred due to their low energy and suitability for long range detection. Radioactively labeled monoclonal antibodies may be produced according to well-known methods in the art. For instance, monoclonal antibodies can be iodinated by contact with sodium and/or potassium iodide and a chemical oxidizing agent such as sodium hypochlorite, or an enzymatic oxidizing agent, such as lactoperoxidase. Monoclonal antibodies may be labeled with technetium^{99m} by ligand exchange process, for example, by reducing pertechnate with stannous solution, chelating the reduced technetium onto a Sephadex column and applying the antibody to this column. Alternatively, direct labeling techniques may be used, *e.g*., by incubating pertechnate, a reducing agent such as SNCl₂, a buffer solution such as sodium-potassium phthalate solution, and the antibody. Intermediary functional groups are often used to bind radioisotopes to antibody and exist as metallic ions are diethylenetriaminepentaacetic acid (DTPA) or ethylene diaminetetracetic acid (EDTA).

Among the fluorescent labels contemplated for use as conjugates include Alexa 350, Alexa 430, AMCA, BODIPY 630/650, BODIPY 650/665, BODIPY-FL, BODIPY-R6G, BODIPY-TMR, BODIPY-TRX, Cascade Blue, Cy3, Cy5,6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, TAMRA, TET, Tetramethylrhodamine, and/or Texas Red.

Another type of antibody conjugates contemplated are those intended primarily for use *in vitro*, where the antibody is linked to a secondary binding ligand and/or to an enzyme (an enzyme tag) that will generate a colored product upon contact with a chromogenic substrate. Examples of suitable enzymes include urease, alkaline phosphatase, (horseradish) hydrogen peroxidase or glucose oxidase. Preferred secondary binding ligands are biotin and avidin and streptavidin compounds. The use of such labels is well known to those of skill in the art and is described, for example, in U.S. Patents 3,817,837, 3,850,752, 3,939,350, 3,996,345, 4,277,437, 4,275,149 and 4,366,241.

Yet another known method of site-specific attachment of molecules to antibodies comprises the reaction of antibodies with hapten-based affinity labels. Essentially, hapten-based affinity labels react with amino acids in the antigen binding site, thereby destroying this site and blocking specific antigen reaction. However, this may not be advantageous since it results in loss of antigen binding by the antibody conjugate.

Molecules containing azido groups may also be used to form covalent bonds to proteins through reactive nitrene intermediates that are generated by low intensity ultraviolet light (Potter and Haley, 1983). In particular, 2- and 8-azido analogues of purine nucleotides have been used as site-directed photoprobes to identify nucleotide binding proteins in crude cell extracts (Owens & Haley, 1987; Atherton *et al.*, 1985). The 2- and 8-azido nucleotides have also been used to map nucleotide binding domains of purified proteins (Khatoon *et al.*, 1989; King *et al.,* 1989; Dholakia *et al.,* 1989) and may be used as antibody binding agents.

Several methods are known in the art for the attachment or conjugation of an antibody to its conjugate moiety. Some attachment methods involve the use of a metal chelate complex employing, for example, an organic chelating agent such a diethylenetriaminepentaacetic acid anhydride (DTPA); ethylenetriaminetetraacetic acid; N-chloro-p-toluenesulfonamide; and/or tetrachloro-3α-6α-diphenylglycouril-3 attached to the antibody (U.S. Patents 4,472,509 and 4,938,948). Monoclonal antibodies may also be reacted with an enzyme in the presence of a coupling agent such as glutaraldehyde or periodate. Conjugates with fluorescein markers are prepared in the presence of these coupling agents or by reaction with an isothiocyanate. In U.S. Patent 4,938,948, imaging of breast tumors is achieved using monoclonal antibodies and the detectable imaging moieties are bound to the antibody using linkers such as methyl-p-hydroxybenzimidate or N-succinimidyl-3-(4-hydroxyphenyl)propionate.

In other embodiments, derivatization of immunoglobulins by selectively introducing sulfhydryl groups in the Fc region of an immunoglobulin, using reaction conditions that do not alter the antibody combining site are contemplated. Antibody conjugates produced according to this methodology are disclosed to exhibit improved longevity, specificity and sensitivity (U.S. Patent 5,196,066). Site-specific attachment of effector or reporter molecules, wherein the reporter or effector molecule is conjugated to a carbohydrate residue in the Fc region have also been disclosed in the literature (O'Shannessy *et al.,* 1987). This approach has been reported to produce diagnostically and therapeutically promising antibodies which are currently in clinical evaluation.

### V. Immunodetection Methods

In still further embodiments, there are immunodetection methods using the antibodies of the present disclosure. Some immunodetection methods include enzyme linked immunosorbent assay (ELISA), radioimmunoassay (RIA), immunoradiometric assay, fluoroimmunoassay, chemiluminescent assay, bioluminescent assay, and Western blot to mention a few. In particular, a competitive assay for the detection and quantitation of antibodies also is provided. The steps of various useful immunodetection methods have been described in the scientific literature, such as, e.g., Doolittle and Ben-Zeev (1999), Gulbis and Galand (1993), De Jager *et al.* (1993), and Nakamura *et al.* (1987). In general, the immunobinding methods include obtaining a sample and contacting the sample with a first antibody in accordance with embodiments discussed herein, as the case may be, under conditions effective to allow the formation of immunocomplexes.

Contacting the chosen biological sample with the antibody under effective conditions and for a period of time sufficient to allow the formation of immune complexes (primary immune complexes) is generally a matter of simply adding the antibody composition to the sample and incubating the mixture for a period of time long enough for the antibodies to form immune complexes with, *i.e*., to bind to any antigen present. After this time, the sample-antibody composition, such as a tissue section, ELISA plate, dot blot or Western blot, will generally be washed to remove any non-specifically bound antibody species, allowing only those antibodies specifically bound within the primary immune complexes to be detected.

In general, the detection of immunocomplex formation is well known in the art and may be achieved through the application of numerous approaches. These methods are generally based upon the detection of a label or marker, such as any of those radioactive, fluorescent, biological and enzymatic tags. Patents concerning the use of such labels include U.S. Patents 3,817,837, 3,850,752, 3,939,350, 3,996,345, 4,277,437, 4,275,149 and 4,366,241. Of course, one may find additional advantages through the use of a secondary binding ligand such as a second antibody and/or a biotin/avidin ligand binding arrangement, as is known in the art.

The antibody employed in the detection may itself be linked to a detectable label, wherein one would then simply detect this label, thereby allowing the amount of the primary immune complexes in the composition to be determined. Alternatively, the first antibody that becomes bound within the primary immune complexes may be detected by means of a second binding ligand that has binding affinity for the antibody. In these cases, the second binding ligand may be linked to a detectable label. The second binding ligand is itself often an antibody, which may thus be termed a "secondary" antibody. The primary immune complexes are contacted with the labeled, secondary binding ligand, or antibody, under effective conditions and for a period of time sufficient to allow the formation of secondary immune complexes. The secondary immune complexes are then generally washed to remove any non-specifically bound labeled secondary antibodies or ligands, and the remaining label in the secondary immune complexes is then detected.

Further methods include the detection of primary immune complexes by a two step approach. A second binding ligand, such as an antibody that has binding affinity for the antibody, is used to form secondary immune complexes, as described above. After washing, the secondary immune complexes are contacted with a third binding ligand or antibody that has binding affinity for the second antibody, again under effective conditions and for a period of time sufficient to allow the formation of immune complexes (tertiary immune complexes). The third ligand or antibody is linked to a detectable label, allowing detection of the tertiary immune complexes thus formed. This system may provide for signal amplification if this is desired.

One method of immunodetection uses two different antibodies. A first biotinylated antibody is used to detect the target antigen, and a second antibody is then used to detect the biotin attached to the complexed biotin. In that method, the sample to be tested is first incubated in a solution containing the first step antibody. If the target antigen is present, some of the antibody binds to the antigen to form a biotinylated antibody/antigen complex. The antibody/antigen complex is then amplified by incubation in successive solutions of streptavidin (or avidin), biotinylated DNA, and/or complementary biotinylated DNA, with each step adding additional biotin sites to the antibody/antigen complex. The amplification steps are repeated until a suitable level of amplification is achieved, at which point the sample is incubated in a solution containing the second step antibody against biotin. This second step antibody is labeled, as for example with an enzyme that can be used to detect the presence of the antibody/antigen complex by histoenzymology using a chromogen substrate. With suitable amplification, a conjugate can be produced which is macroscopically visible.

Another known method of immunodetection takes advantage of the immuno-PCR (Polymerase Chain Reaction) methodology. The PCR method is similar to the Cantor method up to the incubation with biotinylated DNA, however, instead of using multiple rounds of streptavidin and biotinylated DNA incubation, the DNA/biotin/streptavidin/antibody complex is washed out with a low pH or high salt buffer that releases the antibody. The resulting wash solution is then used to carry out a PCR reaction with suitable primers with appropriate controls. At least in theory, the enormous amplification capability and specificity of PCR can be utilized to detect a single antigen molecule.

### A. Immunohistochemistry

The antibodies may also be used in conjunction with both fresh-frozen and/or formalin-fixed, paraffin-embedded tissue blocks prepared for study by immunohistochemistry (IHC). The method of preparing tissue blocks from these particulate specimens has been successfully used in previous IHC studies of various prognostic factors, and is well known to those of skill in the art (Brown *et al.*, 1990; Abbondanzo *et al*., 1990; Allred *et al*., 1990).

Briefly, frozen-sections may be prepared by rehydrating 50 ng of frozen "pulverized" tissue at room temperature in phosphate buffered saline (PBS) in small plastic capsules; pelleting the particles by centrifugation; resuspending them in a viscous embedding medium (OCT); inverting the capsule and/or pelleting again by centrifugation; snap-freezing in -70°C isopentane; cutting the plastic capsule and/or removing the frozen cylinder of tissue; securing the tissue cylinder on a cryostat microtome chuck; and/or cutting 25-50 serial sections from the capsule. Alternatively, whole frozen tissue samples may be used for serial section cuttings.

Permanent-sections may be prepared by a similar method involving rehydration of the 50 mg sample in a plastic microfuge tube; pelleting; resuspending in 10% formalin for 4 hours fixation; washing/pelleting; resuspending in warm 2.5% agar; pelleting; cooling in ice water to harden the agar; removing the tissue/agar block from the tube; infiltrating and/or embedding the block in paraffin; and/or cutting up to 50 serial permanent sections. Again, whole tissue samples may be substituted.

### B. Immunodetection Kits

In still further embodiments, there are kits for use with the immunodetection methods described above. The immunodetection kits will thus comprise, in suitable container means, a first antibody and, optionally, an immunodetection reagent.

In certain embodiments, the antibody may be pre-bound to a solid support, such as a column matrix and/or well of a microtitre plate. The immunodetection reagents of the kit may take any one of a variety of forms, including those detectable labels that are associated with or linked to the given antibody. Detectable labels that are associated with or attached to a secondary binding ligand are also contemplated. Exemplary secondary ligands are those secondary antibodies that have binding affinity for the first antibody.

Further suitable immunodetection reagents for use in the present kits include the two-component reagent that comprises a secondary antibody that has binding affinity for the first antibody, along with a third antibody that has binding affinity for the second antibody, the third antibody being linked to a detectable label. As noted above, a number of exemplary labels are known in the art and all such labels may be employed in connection with embodiments discussed herein.

The kits may further comprise a suitably aliquoted composition of the antibody, whether labeled or unlabeled, as may be used to prepare a standard curve for a detection assay. The kits may contain antibody-label conjugates either in fully conjugated form, in the form of intermediates, or as separate moieties to be conjugated by the user of the kit. The components of the kits may be packaged either in aqueous media or in lyophilized form.

The container means of the kits will generally include at least one vial, test tube, flask, bottle, syringe or other container means, into which the antibody may be placed, or preferably, suitably aliquoted. The kits will also include a means for containing the antibody and any other reagent containers in close confinement for commercial sale. Such containers may include injection or blow-molded plastic containers into which the desired vials are retained.

### VI. Examples

The following examples are included to demonstrate preferred embodiments. It should be appreciated by those of skill in the art that the techniques disclosed in the examples which follow represent techniques discovered by the inventors to function well in the practice of embodiments, and thus can be considered to constitute preferred modes for its practice.

### EXAMPLE 1 - METHODS

**Patient sample acquisition and processing.** CSF was obtained by lumbar puncture from patients recruited into the study in accordance with The University of Texas Southwestern Medical Center (UTSWMC) Institutional Review Board (IRB). This study includes patient samples as previously published by the inventor's group (Cameron *et al.,* 2009 and Ligocki *et al.,* 2013) containing patients with clinically definite multiple sclerosis (CDMS), clinically isolated syndrome optic neuritis (ON_{CIS}), and clinically isolated syndrome transverse myelitis (TM_{CIS}). The samples were stained with fluorescently labeled antibodies and sorted for single CD19⁺ B cells through a CD45⁺ lymphocyte gate as previously described into 96-well plates using either the BD FACSAria flowcytometer (Becton Dickinson, San Jose, CA) or the MoFlo High-Performance Cell Sorter (Cytomation, Ft Collins, CO) (Ligocki *et al*., 2013).

**Single-cell PCR and genetic analysis of V_{H} and Vκ genes.** After the single cell sort and cell lysis, either gDNA was amplified for the CDMS patient samples or cDNA was generated for the ON_{CIS} and TM_{CIS} patient samples as previously described (Ligocki *et al.,* 2013). Multi-plexed nested PCR was performed to amplify and the Immunoglobulin (Ig) heavy chain and corresponding Ig kappa light chain from each individually sorted CSF B cell. The products were purified, sequenced, catalogued, and analyzed for gene and mutation characteristics (Ligocki *et al*., 2013).

Germline rearrangements were identified using the IMGT/V-OUEST Ig blasting tool (world-wide-web at imgt.org/IMGT_vquest/share/textes/). Antibody variable heavy (V_{H}) and variable kappa (Vκ) sequences were analyzed and compiled using a Perl program developed at UTSWMC (Ligocki *et al*., 2010 and Ligocki *et al*., 2013) using IMGT/V-QUEST as the initial source for sequence alignment.

**Cloning of full-length recombinant human IgG antibodies (rhAbs).** Sequences from CDMS, ON_{CIS}, and TM_{CIS} patients were chosen as candidates for cloning into full-length expression vectors based on their V_{H} genetics. The criteria was: expressing a V_{H}4 gene and have 2 or more of the 6 AGS codons mutated (Cameron *et al*., 2009 and Ligocki *et al.,* 2010). 60% were also clonally expanded by identifying another V_{H} sequence within the same patient with identical amino acids in the CDR3 region. The corresponding Vκ sequence was amplified from the same well as the V_{H} sequence to identify the antibody binding region of the single CSF B cell. Sequence and patient details for each selection are shown in Table 5 and Supplemental Table 6. Additional rounds of PCR were done to add restriction enzyme sites to both the 5' and 3' ends of the original PCR products to allow for insertion into the expression vectors using modifications of previously published primers (Yurasov *et al.,* 2005). Some heavy and light chain rearrangement sequences were purchased from Integrated DNA Technologies (IDT, IA, USA) for extraction into the expression vectors. Dr. Michel Nussenzweig provided the backbone expression vectors for both the IgG and IgK chains. These vectors and the procedure have been extensively described for the production of monoclonal human IgG₁(Tiller *et al*.*,* 2008). Briefly, AgeI was used as the 5' restriction enzyme site for both the V_{H} and Vκ inserts and plasmid backbone and SalI and BsiWI were used as the 3' restriction enzyme site for the V_{H} and Vκ respectively (NEB, MA, USA). After digestion, ligation of both the insert and the corresponding expression vector backbone was performed using T4 ligase (NEB). DH5α cells were transformed with a plasmid and individual colonies from the plate were grown for miniprep (Qiagen, CA, USA). The vectors were sequenced in order to confirm that the insert matched the original patient heavy and light chain rearrangements captured by PCR and that the coding region remained in frame. Midiprep DNA (Qiagen) was used for transformation and production of rhAbs in culture. Sequences were validated after each growth.

Two control rhAbs were provided that were cloned from systemic lupus erythematosus (SLE) patient derived B cells. B1 has been shown to not bind to mouse brain and G11 has been shown to bind to NMDARs in the mouse brain as well as dsDNA (Zhang *et al*.*,* 2009). These two antibodies have been studied and published and were used as controls for the full-length IgG₁ rhAb construct in all the experiments presented in this current study.

**Production of monoclonal rhAbs.** Human embryonic kidney fibroblast (HEK) 293T cells were grown to 50-80% confluency in a 10 cm dish in DMEM media supplemented with FCS (Gibco, Life Technologies). The cotransfection of paired cloning vectors corresponding to the IgK and the IgH of a rhAb were mixed (12.5 µg total DNA) with JetPEI solution (Polyplus transfection) and added dropwise to the cells. The plates were incubated in a 5% CO₂ water-jacketed incubator (Nuaire, MN, USA) at 37°C in 20 ml DMEM media supplemented with ultra-low IgG FCS media (Gibco). Supernatant was harvested and fresh media added on days 3, 5, 7, and 10. ELISAs were used to determine the yield and the concentration of the rhAbs produced in culture. Goat anti-human IgG Fc antibody (Santa Cruz. TX, USA) was used as the coating antibody and serially diluted samples were incubated for 2 hrs at room temperature. Plates were probed with goat anti-human IgG Fc HRP-conjugated antibody (Santa Cruz) for 1 hr and developed using tetramethylbenzidine (TMB) substrate solution (Ebioscience, CA, USA) and stopped with 1 M HCl. The plates were read at 450 nm using the Epoch Nano (Biotek, VT, USA). Standard curves and rhAb concentrations were interpolated using GraphPad Prism 6 (CA, USA). Supernatants were concentrated using the 10kDa MWCO Amcion Ultra centrifugal filter units (Millipore, MA, USA) following manufacturer's recommendations. A second ELISA was performed on the concentrated stocks of rhAbs and then aliquoted and stored at -80°C. Additionally, a non-transfected cell culture supernatant was confirmed to not contain any IgG above ELISA detection. These concentrated rhAbs were used as primary antibodies for all mouse brain immunohistochemistry.

**Biotinylation of monoclonal rhAbs.** A set of ten AGS rhAbs and 2 control rhAbs were purified by passing supernatant through a column with a bed of protein G sepharose beads followed by dialysis in PBS and DPBS (Life Technologies). Purity and yield were determined by SDS-page gel stained with coomasie blue and ELISA as described above. Each rhAb was biotinylated using 100 µg of column-purified product and following manufacturer's instructions for the Thermo Scientific EZ-Link Micro NHS-PEG4-Biotinylation kit (Thermo Scientific, MA, USA). These biotinylated rhAbs were used as primary antibodies for all human brain immunohistochemistry.

**Processing of frozen brain tissue.** Mice were sacrificed 2-3 days post stroke induction as previously described (Stowe *et al*.*,* 2011) and perfused with 4% paraformaldehyde. The brains were extracted and preserved in 4% paraformaldehyde for 48 hrs at 4°C followed by cryoprotection in sequential 15% and 30% sucrose solutions. Post-mortem human brain samples were provided by the Human Brain and Spinal Fluid Resource Center (UCLA, Los Angeles, CA). Three samples were used for the studies: white matter (WM) from a healthy control without neurological complications (HC), white matter plaque from a patient with clinically definite MS (MS-P), normal appearing WM from the same MS patient (MS-WM), and normal appearing gray matter (MS-GM). Mean time to sampling from time of death was 16 hrs. Upon removing from -80°C, they were preserved similarly to mouse brains with 4% paraformaldehyde for 48 hrs at 4°C followed by cryoprotection in sequential 15% and 30% sucrose solutions. All tissues were embedded in O.C.T freezing compound and stored at -20°C until cryosectioned. Tissue sections (12-16 µm) were cut and attached to charged glass slides using a cryostat (Thermo Scientific MICROM) and frozen at -20°C. Tissues were stained with cresyl violet to validate the integrity of the preservation of the tissue.

**Diaminobenzidine (DAB)-immunohistochemistry (IHC) staining of mouse tissue.** Tissue sections were subjected to antigen retrieval for 2 min using low pH Antigen Unmasking Solution (Vector Laboratories, Burlingame, CA, USA). Endogenous biotin was blocked using 3% H₂O₂ solution for 5 min at room temperature and then washed. The sections were blocked with 3% normal goat serum in PBS for 10 min at room temperature, washed with PBS, and then were incubated overnight at 4°C with 1 µg rhAb (10 ng/µl) per brain slice. The next day, sections were washed and DAB staining was conducted following the manufacturer's instructions using a biotinylated secondary goat anti-human IgG Fc antibody (Vector Laboratories, Burlingame, CA, USA). The slides were dehydrated and cleared with sequential washes in increasing percentages of EtOH, from 70% to 100%, with two final washes in xylenes. Slides were mounted with a permount:xylene solution and imaged using a 40x brightfield lens on the NanoZoomer (Hammatsu, Japan). Images were visualized using NDP.view software (Hammatsu, Japan) and 20x images were exported for visualization and adjustments to brightness and contrast were done with ImageJ software (NIH, USA).

**DAB-IHC staining of human tissue.** Initial processing of the human brain tissue sections remained the same as the mouse tissue. After blocking with 3% normal goat serum in PBS for 10 min at room temperature, an additional blocking step was performed with BloxAll for 10 min at room temperature (Vector Laboratories, Burlingame, CA, USA). Tissues were incubated overnight at 4°C with 1 µg biotinylated-rhAb (10 ng/µl) per brain slice. The next day, these biotinylated-rhAbs were detected without a secondary antibody and instead with ABC reagent alone (Vector Laboratories, Burlingame, CA, USA). Dehydration, clearing, mounting, and visualization of the human tissue followed the same procedure as the mouse tissue.

**Immunofluorescence (IFC) staining of mouse tissue.** Ten AGS rhAbs and 2 control rhAbs from the DAB panel were selected for further experiments using IFC (Table 5). Tissue sections were subjected to antigen retrieval for 2 min using low pH Antigen Unmasking Solution (Vector Laboratories, Burlingame, CA, USA). The sections were blocked with 1% normal goat serum and 1% Tween-20 in PBS for 1 hr at room temperature. Due to the presence of IgG deposits even in healthy brain and as an artifact of post-mortem tissue preparation, the set of 10 rhAbs and the 2 control rhAbs used in the mouse brain IFC were biotinylated to eliminate the need for a species specific secondary antibody. Most of the rhAbs were diluted in blocking solution. Pierce Immunostain Enhancer (Thermo Scientific) was used as the diluent for the primary rhAb incubation as well as the secondary Alexa Fluor488 for the following two rhAbs: AJL03, AJL15. Slides were washed with PBS, and then incubated overnight at 4°C with 1 µg rhAb (10 ng/µl) per brain slice. Next day, the sections were washed and incubated for 1 hr at room temperature with the secondary antibody Alexa Fluor 488 goat anti-human IgG Fc (Life Technologies). Then a colocalization marker, either GFAP (Abcam) or NeuN (Chemicon) were used at 1:1000 and 1:100 dilutions respectively, was incubated for 1 hr at room temperature and then incubated for an additional hour with the appropriate secondary antibody Alexa Fluor 594 anti-rabbit IgG Fc for GFAP or Alexa Fluor 594 anti-mouse IgG Fc for NeuN detection (Life Technologies). Next, the stained tissue sections were incubated for three minutes with DAPI (1:1000) as a counterstain for nuclei (Life Technologies). The sections were washed and wet mounted with Fluoro-Gel (Electron Microscopy Diatome). Slides were viewed with a fluorescent Leica TCS SP5 confocal microscope (Leica microsystems) and viewed and adjusted in brightness and contrast using ImageJ software (NIH, USA).

**IFC staining of human tissue.** Initial processing of the human brain tissue sections remained the same as the mouse tissue above. After the initial blocking, endogenous biotin was blocked per manufacturer's instructions using the streptavidin-biotin blocking kit (Vector Laboratories, Burlingame, CA, USA). Pierce Immunostain Enhancer (Thermo Scientific) was used as the diluent for the primary rhAb incubation as well as the secondary Alexa Fluor 488 for all human tissue IFC. Slides were washed with PBS, and then incubated overnight at 4 °C with 2 µg rhAb (20 ng/µl) per brain slice. Next day, the sections were washed, and incubated for 1.5 hrs at room temperature with the secondary antibody Alexa Fluor 488 goat anti-streptavidin (Life Technologies). The colocalization with either GFAP or NeuN, DAPI counterstain, mounting and visualization followed the same procedure as the mouse brain tissue.

### EXAMPLE 2 - RESULTS

**rhAb selection.** The inventor has previously shown that CIS patients at risk to convert and those with CDMS harbor B cells in the CSF with a unique mutational pattern in their V_{H}4 repertoires termed the Antibody Gene Signature (AGS) (Cameron *et al.,* 2009 and Ligocki *et al*.*,* 2010). A shared pattern of increased replacement mutations at 6 specific codons within V_{H}4 genes suggests selection and recognition of a shared set of antigens. Therefore, she sought to determine the biological significance of this mutational pattern by testing the binding ability of AGS-enriched antibodies to brain tissue. Using single cell sorting of CSF derived B cells from CDMS, ON_{CIS}, and TM_{CIS} patients, the inventor was able to determine the exact antibody-binding variable regions from PCR amplified V_{H}J_{H} and VκJκ sequences. Using a full-length recombinant human IgG₁ antibody expression vector system, variable regions were cloned and expressed for further study. Only those B cells expressing a V_{H}4 family gene with mutations in 2 or more of the 6 AGS codons were considered for this analysis.

A total of 32 rhAbs were chosen for expression. The details of 10 rhAbs are shown in Table 5 and the remaining 22 in Table 6. Briefly, all rhAbs contained mutations at 2 or more of the 6 AGS codons, and the majority (66%) contained 3 or more AGS mutations. Additionally, 60% were also clonally expanded, suggesting an antigen driven process. These rhAbs were cloned from 10 CSF patient repertoires: 9 rhAbs from 4 CDMS patients, 14 rhAbs from 3 ON_{CIS} patients, and 9 rhAbs from 3 TM_{CIS} patients. Two control rhAbs cloned from SLE patient B cells were provided by Dr. Betty Diamond as controls for the human IgG construct. The expected binding of this control set has been extensively published using mouse tissue. B1, the negative control, does not react to brain, whereas G11, the positive control, reacts to NMDARs in the brain as well as dsDNA (Zhang *et al.,* 2009).

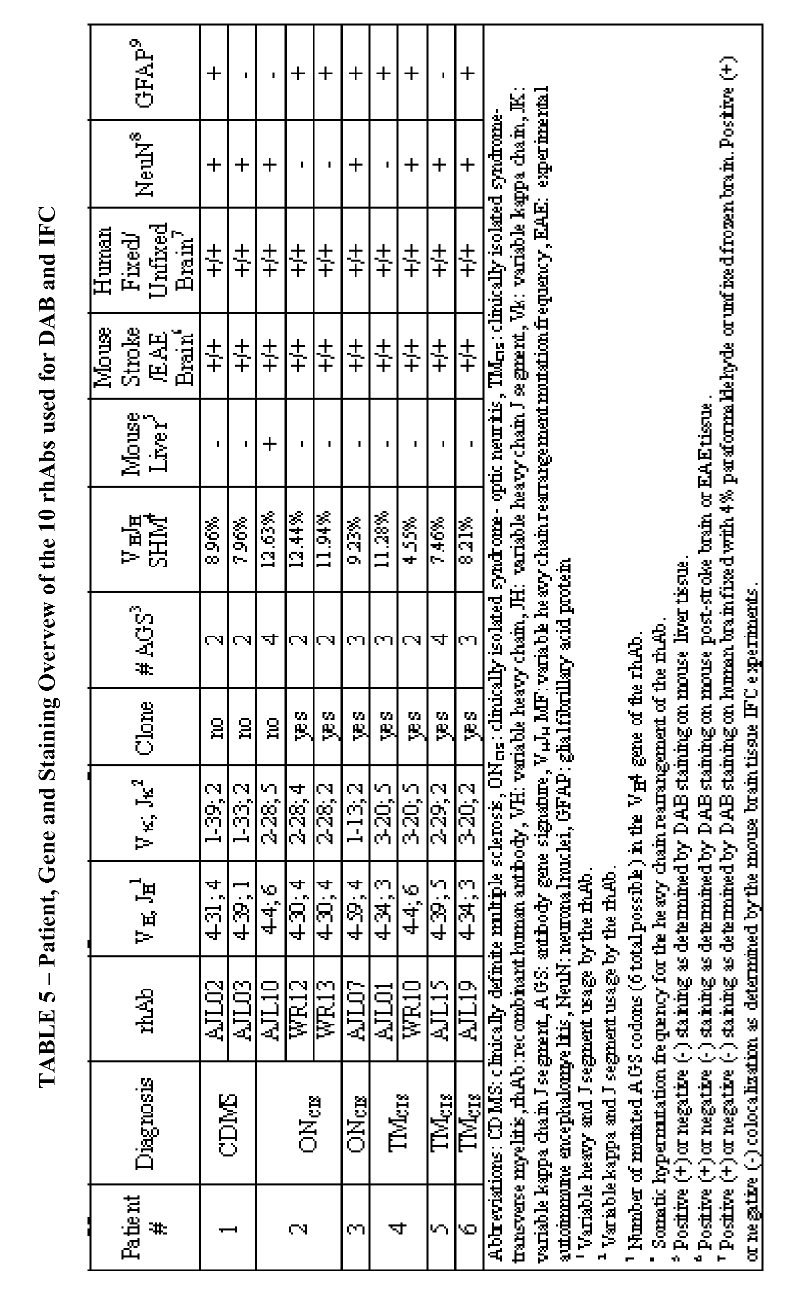

| **Table 6 - Patient, gene, and staining overview of the 22 rhAbs used only for mouse brain tissue DAB.¹** | | | | |
|---|---|---|---|---|
| Patient # | Diagnosis | rhAb | Clone | # AGS² |
| 2 | ON_{CIS} | AJL11 | no | 3 |
| 3 | ON_{CIS} | AJL06 | no | 3 |
| | | AJL08 | yes | 3 |
| | | AJL09 | yes | 2 |
| | | AJL13 | yes | 3 |
| 4 | TM_{CIS} | WR11 | yes | 2 |
| 5 | TM_{CIS} | AJL14 | no | 3 |
| | | AJL16 | yes | 3 |
| 6 | TM_{CIS} | AJL18 | yes | 5 |
| | | AJL20 | no | 3 |
| 7 | CDMS | WR01 | yes | 2 |
| | | WR02 | yes | 2 |
| 8 | CDMS | WR03 | no | 3 |
| | | WR04 | no | 3 |
| | | WR05 | no | 4 |
| | | WR06 | no | 3 |
| 9 | CDMS | WR07 | no | 4 |
| 10 | ON_{CIS} | AJL04 | yes | 3 |
| | | AJL05 | yes | 4 |
| | | AJL12 | no | 3 |
| | | WR08 | yes | 2 |
| | | WR09 | yes | 2 |
| Abbreviations: CDMS: clinically definite multiple sclerosis, ON_{CIS}: clinically isolated syndrome- optic neuritis, TM_{CIS}: clinically isolated syndrome-transverse myelitis, rhAb: recombinant human antibody, AGS: antibody gene signature | | | | |
| ¹ See DAB images of the rhAbs listed here | | | | |
| ² Number of mutated AGS codons (6 total possible) in the V_{H}4 gene of the rhAb. | | | | |

**AGS-enriched rhAbs bind to mouse brain tissue.** The panel of 32 experimental AGS-enriched and clonally expanded rhAbs as well as the 2 control rhAbs was first tested for targeting to mouse brain tissue using DAB. This methodology offers sensitive detection of primary rhAb binding to the tissue. Due to previous work from other laboratories demonstrating a lack of binding of antibodies cloned from CDMS CSF B cells to normal brain tissue or WM (Owens *et al*.*,* 2009 and von Budingen *et al*.*,* 2008), the inventor chose to utilize brain tissue from a mouse model of transient stroke as a source of inflammation (Stowe *et al*.*,* 2011). This provided generalized non-antigen directed inflammation to minimize bias of any specific CNS antigen. The secondary antibody used for detection, goat anti-human IgG, did not react to mouse brain alone (FIG. 1A). As expected, the negative construct control rhAb B1 also did not react to mouse brain, while the positive control rhAb G11 showed recognition to brain and thus validated the assay (FIG. 1A).

The full panel of 32 rhAbs was tested on mouse brain by DAB. There was a wide range of staining intensity. However, all but two of the 32 rhAbs, WR01 and WR11, displayed binding to brain tissue (FIGS. 1A-D and FIGS. 6A-C). There was no difference of rhAb staining patterns amongst the three patient groups used to derive the rhAbs. A common feature was that the binding appears cellular in the cortex and midbrain and was either absent or sparse along the heavily myelinated corpus callosum.

**AGS-enriched rhAbs bind to human brain tissue.** Four sources of human brain were used for the DAB staining experiments: MS normal appearing gray matter (MS-GM) (FIGS. 2A-D), MS normal appearing white matter (MS-WM) (FIGS. 7A-D), MS white matter plaque (MS-P) (FIGS. 8A-D), and healthy control normal appearing (NA) WM (HC-WM) (FIGS. 9A-D). Due to the rhAb staining pattern found in the mouse brain, the tissue source of most interest was the MS-GM. The two negative controls, ABC reagent alone and rhAb B1, did not bind to MS-GM whereas the positive control rhAb G11 bound MS-GM (FIG. 2A). The ten AGS-enriched rhAbs from 3 different disease types representing 6 different patients all showed binding to human MS-GM (FIGS. 2B-D). As with the mouse brain DAB (FIGS. 1A-D), the staining appeared to be cellular and exhibited similar staining patterns in the human brain DAB (FIGS. 2A-D).

In contrast, the rhAbs demonstrated poor recognition to MS-WM (FIGS. 7A-D). Plaque tissue from the MS patient showed evidence of damage, and the binding was diminished or absent in MS-P tissue (FIGS. 8A-D). The rhAbs also had weak or no binding to HC-WM (FIGS. 9A-D). A common feature shared by the rhAbs was that testing on all sources of WM tissue resulted in weaker staining patterns than was seen in GM tissue.

To confirm the paucity of rhAb reactivity to myelinated tracts, the inventor evaluated binding of the subset of rhAbs to myelin basic protein (MBP) and myelin oligodendrocyte glycoprotein (MOG), two myelin components of considerable interest in MS.²¹ With the exception of AJL01, which was reactive to both MBP and MOG, all of the rhAbs tested negative to binding MBP and MOG by ELISA (FIGS. 17A-B). In addition, none of the subset rhAbs reacted to MBP or MOG in a myelin array that has been previously described (FIG. 17C).²² Finally, none of these rhAbs reacted to MOG expressed on the cell surface of HeLa cells as assessed by flow cytometry (FIG. 17D).

**AGS-enriched rhAbs target neurons and astrocytes in both mouse and human brain tissue.** Due to the location and appearance of the DAB staining, the inventor hypothesized that the rhAbs were binding to either of two major cell types in the brain, neurons and/or astrocytes. Therefore, IFC colocalization experiments were performed on 10 of the rhAbs from the initial cohort of 32. These 10 were chosen to sample all three disease subtypes and the same source of mouse brain tissue was utilized in these experiments. B1 and G11 were again used as negative and positive controls for the assay respectively. B1 did not recognize brain tissue (FIGS. 2A, 3A), but G11 did recognize mouse brain tissue (FIGS. 2B, 3B) as evidenced in both the NeuN and the GFAP colocalization experiments. Similar staining pattern of both B1 and G11 was confirmed in human MS-GM (FIGS. 10A, 10B).

Three rhAbs, one from each patient type, colocalized with neuronal nuclei identified by NeuN in both mouse and human brain. AJL03 from patient CDMS1 showed similar staining of neuronal nuclei in both species tissue type (FIGS. 3C, 3D). AJL10, from patient ON_{CIS}2, showed colocalization with neuronal nuclei with the human targeting being very concentrated in the nucleus compared to a more diffuse staining pattern in mouse (FIGS. 3E, 3F). Additionally, there were areas of AJL10 binding that were independent of NeuN but were still associated with nuclei as marked by DAPI (FIG. 3E). AJL15 from patient TM_{CIS}5 also colocalized with neuronal nuclei in both mouse and human tissue (FIGS. 3G, 3H). AGS-enriched B cells that recognize neuronal nuclei in the GM are found in all disease presentations and have conserved recognition between mouse and human species.

In order to see if targeting to astrocytes was present, the other major cell type in the GM in addition to neurons, IFC with GFAP was tested. WR12 and WR13 from the same patient, ON_{CIS}2, colocalized with astrocytes and their processes in mouse tissue (FIGS. 4C, 4E). In human tissue, both rhAbs recognized GFAP-positive astrocyte bodies with additional vessel staining seen with WR12 (FIGS. 4D, 4F). AJL01 from patient TM_{CIS}4 predominately colocalized with astrocytes with additional staining along the periventricular lining in mouse (FIG. 4G). AJL01 colocalized to astrocyte endfeet within a vessel in addition to the astrocyte bodies in MS-GM shown (FIG. 4H). In addition to AGS-enriched B cells that recognize neurons, this mutational pattern also imparts recognition to astrocytes.

Four AGS-enriched rhAbs from all 3 patient types displayed dual-cell targeting. AJL02 from patient CDMS1 predominantly colocalized with NeuN in a ring-like fashion within the nuclei (FIG. 5A) with minor colocalization with GFAP astrocyte processes (FIG. 5B). This ring-like staining pattern around neuronal nuclei was also seen in human tissue (FIG. 5C). AJL07 was cloned from patient ON_{CIS}3, and displayed dual-cell targeting to both neurons and astrocytes in mouse brain (FIGS. 5D, 5E). In MS-GM AJL07 principally stained neuronal nuclei and also a vessel (FIG. 5F), keeping with the dual-cell targeting observed in mouse. From patient TM_{CIS}4, WR10 displayed dual-cell targeting with colocalization to both the edges of neuronal nuclei and astrocyte processes in mouse brain (FIGS. 5G, 5H). WR10 stained astrocyte bodies, endfeet around a vessel, as well as a neuron seen at the top of the panel in MS-GM (FIG. 5I). AJL19 from patient TM_{CIS}6 reacted similarly to WR10 with dual-cell targeting as it colocalized with both neuronal nuclei and astrocytes (FIGS. 5J, 5K). Colocalization of AJL19 with neuronal nuclei was also seen in MS-GM (FIG. 5L).

Due to the focused cellular binding of these rhAbs by DAB, the inventor hypothesized that the rhAbs were binding to either neurons and/or astrocytes in the gray matter. Therefore, IFC colocalization experiments were performed on the subset of rhAbs. In both mouse and human brain tissues, NeuN was utilized as a marker for neuronal nuclei and GFAP was utilized as a marker for astrocytes. FIGS. 13A-F and FIGS. 14A-F feature four of these rhAbs and the remaining six are summarized in FIGS. 15A-F (CDMS derived rhAbs) and FIGS. 16A-L (CIS derived rhAbs).

AJL10 is a rhAb cloned from patient ON_{CIS}2 that had an AGS score of 6.07 and converted to MS 2.5 years after sampling. This antibody utilizes the V_{H}4-4 gene paired with a J_{H}6 segment and also uses a V_{K} light chain. AJL10 has replacement SHM at 4 of the 6 AGS codons (40, 56, 81, and 89) with an overall high mutation frequency of 12.63% (Table 5). No additional clones of this B cell were detected in the patient's CSF. As shown in FIGS. 13A-F, AJL10 demonstrated co-localization with neuronal nuclei in both mouse (FIG. 13A), and human (FIG. 13B) gray matter. AJL10 did not cross-react with astrocytes as demonstrated by the lack of co-localization with the astrocyte specific antibody, GFAP (FIG. 13C). AJL07 is a rhAb cloned from a different CIS patient, ON_{CIS}3, that had an AGS score of 10.68 and converted to MS 1 month after sampling. This antibody utilizes the V_{H}4-59 gene paired with a J_{H}4 segment and also uses a V_{K} light chain. AJL07 has replacement SHM at 3 of the 6 AGS codons (56, 57, and 81) with an overall high mutation frequency of 9.23% (Table 5). Three additional clones of this B cell were detected in the patient's CSF. AJL07 co-localized with NeuN in both mouse and human gray matter (FIGS. 13D-E) and also cross-reacted with astrocytes as demonstrated by GFAP co-localization (FIG. 13F). Interestingly, AJL07 also bound to the vasculature in human gray matter (FIG. 13E).

Next, the inventor wanted to see if CIS patients presenting with TM would also display gray matter binding by their AGS-enriched rhAbs (FIGS. 14A-F). AJL01 is a rhAb cloned from patient TM_{CIS}4 that had an AGS score of 17.90 and converted to MS 8 months after sampling. This antibody utilizes the V_{H}4-34 gene paired with a J_{H}3 segment and also uses a V_{K} light chain. AJL01 has replacement SHM at 3 of the 6 AGS codons (56, 57, and 81) with an overall high mutation frequency of 11.28% (Table 5). This was detected as a four member clone within the patient's CSF. AJL01 colocalized to astrocyte endfeet within the blood-brain barrier in addition to the astrocyte bodies shown in both mouse and human GM (FIGS. 14A-B). AJL01 did not cross-react with neurons in mouse GM (FIG. 14C). Another AGS-enriched rhAb, WR13, also bound to astrocytes. This rhAb was cloned from the same ON_{CIS}2 patient as AJL10. This antibody utilizes the V_{H}4-30 gene paired with a J_{H}4 segment and also uses a V_{K} light chain. WR13 has replacement SHM at 2 of the 6 AGS codons (56 and 81) with an overall mutation frequency of 11.94% (Table 5). Similar to AJL01 from a TM_{CIS} patient, WR13 from an ON_{CIS} patient co-localized to astrocytes in both mouse and human GM (FIGS. 14D-E) and did not cross-react with neurons (FIG. 14F). A clonally related rhAb also detected in this patient's CSF, WR12, was tested and demonstrated the same staining pattern (FIGS. 16A-L).

### EXAMPLE 3 - DISCUSSION

Utilization of the AGS as a molecular diagnostic tool to identify patients that will subsequently develop CDMS does not address the impact of AGS-enriched B cells on disease pathogenesis. Determining the target specificity of AGS-enriched antibodies from CSF B cells of MS patients (early CIS and established CDMS) is the first step towards dissecting the potential role of AGS-enriched antibodies in the pathogenesis of MS. Using AGS enrichment as a genetic marker of antigen driven selection represented in MS disease, the inventor cloned 32 rhAbs from singly sorted CSF B cells to recapitulate the antibody that was present on the surface of the cell at the time of sample collection. Regardless of initial presentation of disease (ON_{CIS} vs TM_{CIS}), early or established disease (CIS vs CDMS), these rhAbs are recognizing neuronal nuclei and/or astrocytes in both mouse and human brain tissue. This AGS is present at all initial disease presentations and durations indicating that the stage of disease does not significantly alter the cellular targeting of the AGS-enriched B cell repertoire. Significant immune system activation antecedes the initial clinical presentation of disease and complements the finding that rhAbs cloned from ON_{CIS} and TM_{CIS} patients already display recognition to brain targets. CDMS derived rhAbs also maintain recognition of brain tissue, which corroborates recent findings by others that intrathecal IgG from MS patients recognize similar peptides over time (Yu *et al*.*,* 2011).

DAB staining on entire coronal sections from mice allowed for both sensitive detection and a large sampling of multiple brain regions for the initial rhAb testing. Surprisingly the rhAbs showed targeting to cellular targets in the cortex and midbrain with only mild/rare staining along the corpus callosum in mouse tissue. If these rhAbs were strongly targeting WM, there would be accumulation of staining in the corpus callosum that is composed of highly myelinated axonal tracts. Instead, the inventor saw staining in the GM, which has gained appreciation for involvement in CDMS symptoms and disease progression (Bo *et al.,* 2007, Fisniku *et al.,* 2008, Vercellino *et al.,* 2005 and Bo *et al.,* 2003. WM lesions are readily detected with gadolinium enhancement on MRI and luxol fast blue staining of postmortem tissue, whereas GM lesions are more difficult to detect via traditional MRI and IHC, explaining why GM pathology was historically under-recognized (Pirko *et al.,* 2007). Progressive GM loss over time occurs at both CIS and MS stages (Valsasina et al., 2005 and Chard *et al.,* 2004), which suggests that the underlying pathology responsible for the loss is not restricted to later disease stages.

Due to the cortical and cellular appearance of the DAB staining, colocalization with NeuN for neuronal nuclei and GFAP for astrocytes was tested using IFC. The inventor found that cloned rhAbs from AGS-enriched B cells isolated from CDMS, ON_{CIS}, and TM_{CIS} exhibit targeting to neuronal nuclei and astrocytes, with four rhAbs displaying dual-cell recognition. Three of the rhAbs representing all three patient subtypes displayed colocalization with only neuronal nuclei. Targeting of neurons fits well with findings of neuronal loss and degeneration present in MS. Proton magnetic resonance spectroscopy (MRS) measurement of N-acetyl aspartate (NAA), a neuronal marker, is significantly reduced in MS CNS (Kapeller *et al.,* 2001), especially as patients progress through disease stages (Davie *et al.,* 1997), supporting that a loss of neurons is associated with progressive neurodegeneration. Furthermore, extensive subpial demyelination with a gradient of neuronal loss outwards from the meninges is associated with ectopic B cell aggregates (Magliozzi *et al.,* 2010), which are found in patients with a more severe disease course and are adjacent to cortical lesions (Howell *et al.,* 2011). Since the rhAbs described in the present study were cloned from B cells in the CSF, which is in close contact with the meninges, these AGS-enriched B cells may be strategically located to contribute to GM neuronal damage. Neurofilaments comprise the axonal/neuronal cytoskeleton and have also been identified as autoantigen targets from MS CSF (Fialovaw *et al.,* 2013), and titers are correlated with atrophy, axonal damage, as well as clinical disability (Eikelenboom *et al.,* 2003). Immunizing mice with neurofilament induces GM damage as well as deposition of IgG within neuronal cell bodies and axons (Huizinga *et al.,* 2007), highlighting the ability of an intracellular neuronal target to elicit disease. Additionally, the myelin sheaths contained degenerating or dead axons (Bitsch *et al.,* 2000) supporting the notion that the classical demyelination seen in MS can also occur secondary to axonal and neuronal damage, as axonal transection is a common feature in MS lesions (Trapp *et al.,* 1998).

The integrity of the blood brain barrier (BBB) is maintained by astrocytes and could be disrupted by rhAbs targeting them for damage or altering their functionality. It is well accepted that the BBB is compromised in CIS and MS patients (de Vries *et al.,* 2012), and any damage that perpetuates this could allow for further influx of inflammatory cells and mediators into the already inflamed CNS. Brain targeting antibodies can be detected in healthy human sera (Levin *et al.,* 2010) and could gain access to the CNS once the BBB is compromised. The binding of three rhAbs in human and mouse GM clearly shows that these rhAbs can bind to astrocytes by colocalizing with GFAP. Additonally, four rhAbs displayed both astrocyte and neuronal nuclei targeting. IFC on MS-GM showed rhAbs targeting architecture surrounding vessels, which includes astrocyte endfeet as well as endothelial cells, and could contribute to MS pathology by disturbing the BBB. Serum IgG from active MS demonstrates recognition of brain microvascular endothelial cells, which also correlated with BBB disruption (Trojano *et al.,* 1996). The difference in appearance of GFAP positive astrocyte immunostaining between mouse and human brain in this study could be due to the planer orientation of the sectioning with the mouse coronal sections having abundant processes and rare astrocytes cell bodies and the human GM having more immunostained cell bodies and rarer processes (Pham *et al.,* 2009). There is a gaining appreciation of the extensive functions of astrocytes in health and disease beyond maintaining the BBB (Brosnan and Raine, 2013), and thus pathology could arise if the bound antibody alters the astrocytes' functions.

Another critical role for astrocytes is to support remyelination of lesions by providing support and signals to oligodendrocyte precursor cells (Talbott *et al.,* 2005), which could potentially be hindered by antibody targeting. In fact, failure to remyelinate lesions is a feature of progression in CDMS (Bramow *et al.,* 2010). In the well-studied neurological autoimmune disease neuromyelitis optica (NMO), pathology is attributed to antibodies targeting aquaporin-4 on astrocytes, which leads to both cell death and altered functionality resulting in BBB disruption, cellular dysfunction, and loss of support to neurons. Anti-astrocyte potassium channel antibodies have been found in approximately half of tested MS patient sera (Srivastava *et al.,* 2012), which could lead to a decreased ability of astrocytes to maintain proper extracelluar ion levels, thereby promoting cellular damage. More recently, the intracellular astrocyte protein contactin-2/TAG-1 was identified as an autoantigen recognized by MS CSF and the induced disease in mice exhibited inflammation in both WM and GM regions (Derfuss *et al.,* 2009). These findings highlight the importance of an immune reaction directed at intracellular and extracellular astrocyte GM antigens in the pathogenesis and symptoms of MS that predominantly contributes to the AGS enriched B cell pool of these patients.

It is unclear from the current study if these antibodies are pathogenic since several of the rhAbs recognize antigen(s) in the nuclei of neurons rather than an easily accessible cell surface antigen. Furthermore, it is unclear if the astrocyte binding rhAbs recognize an extracellular or intracellular target. Intracellular targets are associated with many autoimmune diseases, especially systemic lupus erythematosus (SLE) and Sjogren's. Antibodies to intracellular and intranuclear antigens of neurons can be endocytosed and gain access to their cognate antigens within the cell or nucleus (Reichlin, 1998) and may contribute to disease pathology. Antibodies targeting aquaporin-4 on astrocytes contribute to NMO pathology and bind to extracelluar targets (Bennett *et al.,* 2009), though more recently a group found that a majority of serum tested from NMO patients recognize intracellular portions of the molecule (Kampylafka *et al.,* 2011) which overlap with the dominant T cell epitope (Arellano *et al.,* 2012). The B cells that recognize the intracellular portions can acquire and present this epitope to T cells and receive cognate help and cytokines to drive a concerted cellular and humoral immune response to this antigen. Recognition and immune responses towards both intracellular and extracellular antigens are present in various autoimmune diseases and is a shared feature with MS.

In testing the panel of AGS-enriched rhAbs, the inventor discovered that the rhAbs had similar staining patterns in both human and mouse brain tissue. This species-preserved homology of important CNS elements provides promise addressing the pathogenic potential of these rhAbs, particularly in the context of GM pathology, which is gaining appreciation for its pathological significance in addition to the traditional WM focus. It is unknown whether these rhAbs harbor the ability to initiate pathology or if they perpetuate damage due to exposed antigens. Regardless, either mechanism could contribute significantly to both neuron and astrocyte viability and functionality. Since this AGS-enrichment within the B cell pool and shared targeting to both neurons and astrocytes are present at both presentations of initial disease, ON_{CIS} and TM_{CIS}, this highlights the importance of elucidating the targets that drive the mutational signature as it is an early biomarker of MS disease and conversion.

All of the compositions and methods disclosed and claimed herein can be made and executed without undue experimentation in light of the present disclosure.

### VII. REFERENCES

U.S. Patent 3,817,837
U.S. Patent 3,850,752
U.S. Patent 3,939,350
U.S. Patent 3,996,345
U.S. Patent 4,196,265
U.S. Patent 4,275,149
U.S. Patent 4,277,437
U.S. Patent 4,366,241
U.S. Patent 4,472,509
U.S. Patent 4,554,101
U.S. Patent 4,680,338
U.S. Patent 4,683,202
U.S. Patent 4,684,611
U.S. Patent 4,816,567
U.S. Patent 4,867,973
U.S. Patent 4,879,236
U.S. Patent 4,938,948
U.S. Patent 4,952,500
U.S. Patent 5,021,236
U.S. Patent 5,141,648
U.S. Patent 5,196,066
U.S. Patent 5,217,879
U.S. Patent 5,302,523
U.S. Patent 5,322,783
U.S. Patent 5,384,253
U.S. Patent 5,464,765
U.S. Patent 5,506,138
U.S. Patent 5,538,877
U.S. Patent 5,538,880
U.S. Patent 5,550,318
U.S. Patent 5,563,055
U.S. Patent 5,563,250
U.S. Patent 5,565,332
U.S. Patent 5,580,859
U.S. Patent 5,589,466
U.S. Patent 5,610,042
U.S. Patent 5,656,610
U.S. Patent 5,670,488
U.S. Patent 5,702,932
U.S. Patent 5,736,524
U.S. Patent 5,739,018
U.S. Patent 5,780,448
U.S. Patent 5,789,215
U.S. Patent 5,824,544
U.S. Patent 5,830,725
U.S. Patent 5,849,304
U.S. Patent 5,851,826
U.S. Patent 5,856,456
U.S. Patent 5,858,744
U.S. Patent 5,871,982
U.S. Patent 5,871,983
U.S. Patent 5,871,986
U.S. Patent 5,879,934
U.S. Patent 5,880,270
U.S. Patent 5,888,502
U.S. Patent 5,925,565
U.S. Patent 5,928,906
U.S. Patent 5,932,210
U.S. Patent 5,935,819
U.S. Patent 5,945,100
U.S. Patent 5,955,331
U.S. Patent 5,981,274
U.S. Patent 5,994,136
U.S. Patent 5,994,624
U.S. Patent 6,013,516
Abbondanzo et al., Am. J. Pediatr. Hematol. Oncol., 12(4), 480-489, 1990.
Allred et al., Arch. Surg., 125(1), 107-113, 1990.
Almendro et al., J. Immunol., 157(12):5411-5421, 1996.
Amado and Chen, Science, 285(5428):674-676, 1999.
Angel et al., Cell, 49:729, 1987a.
Angel et al., Cell, 49:729, 1987b.
Arellano et al., Arch Neurol., Sep;69(9):1125-31, 2012.
Armentano et al., Proc. Natl. Acad. Sci. USA, 87(16):6141-6145, 1990.
Atherton et al., Biol. of Reproduction, 32, 155-171, 1985.
Ausubel et al., Current Protocols in Molecular Biology, Greene Publishing Associates and Wiley Interscience, N.Y., 1994.
Banerji et al., Cell, 27:299, 1981.
Banerji et al., Cell, 33(3):729-740, 1983.
Bates, Mol. Biotechnol., 2(2):135-145, 1994.
Batra et al., Am. J. Respir. Cell Mol. Biol., 21(2):238-245, 1999.
Battraw and Hall, Theor. App. Genet., 82(2):161-168, 1991.
Bennett et al., Ann Neurol., Nov;66(5):617-29, 2009.
Berkhout et al., Cell, 59:273-282, 1989.
Bett et al., J. Virololgy, 67(10):5911-5921, 1993.
Bhattacharjee et al., J. Plant Bioch. Biotech., 6(2):69-73. 1997.
Bilbao et al., FASEB J., 11(8):624-634, 1997.
Bitsch et al., Brain, Jun;123 (Pt 6):1174-83, 2013.
Blackwell et al., Arch. Otolaryngol Head Neck Surg., 125(8):856-863, 1999.
Blanar et al., EMBO J., 8:1139, 1989.
Blomer et al., J. Virol., 71(9):6641-6649, 1997.
Bo et al., Arch Neurol., Jan;64(1):76-80, 2007.
Bo et al., J Neuropathol Exp Neurol., Jul;62(7):723-32, 2003.
Bodine and Ley, EMBO J., 6:2997, 1987.
Boshart et al., Cell, 41:521, 1985.
Bosze et al., EMBO J., 5(7):1615-1623, 1986.
Braddock et al., Cell, 58:269, 1989.
Bramow et al, Brain., Oct;133(10):2983-98, 2010.
Brosnan et al., Glia., Apr;61(4):453-65, 2013.
Brown et al., J. Immunol. Meth., 12;130(1), :111-121, 1990.
Bulla and Siddiqui, J. Virol., 62:1437, 1986.
Cameron et al., Journal of Neuroimmunology, 213:123-30, 2009.
Campbell and Villarreal, Mol. Cell. Biol., 8:1993, 1988.
Campere and Tilghman, Genes and Dev., 3:537, 1989.
Campo et al., Nature, 303:77, 1983.
Capaldi et al., Biochem. Biophys. Res. Comm., 74(2):425-433, 1977.
Caplen et al., Gene Ther., 6(3):454-459, 1999.
Carbonelli et al., FEMS Microbiol. Lett., 177(1):75-82, 1999.
Case et al., Proc. Natl. Acad. Sci. USA, 96(6):2988-2993, 1999.
Celander and Haseltine, J. Virology, 61:269, 1987.
Celander et al., J. Virology, 62:1314, 1988.
Cepok et al., Brain., Jul;128(Pt 7):1667-76, 2005.
Chandler et al., Proc. Natl. Acad. Sci. USA, 94(8):3596-601, 1997.
Chang et al., Mol. Cell. Biol., 9:2153, 1989.
Chard et al., Mult Scler., Aug;10(4):387-91, 2004.
Chatterjee et al., Proc. Natl. Acad. Sci. USA, 86:9114, 1989.
Chen and Okayama, Mol. Cell Biol., 7(8):2745-2752, 1987.
Chillon et al., J. Virol., 73(3):2537-2540, 1999.
Choi et al., J. Mol. Biol., 262(2):151-167, 1996.
Christou et al., Proc. Natl. Acad. Sci. USA, 84(12):3962-3966, 1987.
Clay et al., J. Immunol., 162:1749, 1999.
Cocea, Biotechniques, 23(5):814-816, 1997.
Coffey et al., Science, 282(5392):1332-1334, 1998.
Cohen et al., J. Cell. Physiol., 5:75, 1987.
Costa et al., Mol. Cell. Biol., 8:81-90, 1988.
Cripe et al., EMBO J., 6:3745, 1987.
Culotta and Hamer, Mol. Cell. Biol., 9:1376-1380, 1989.
D'Halluin et al., Plant Cell, 4(12):1495-1505, 1992.
Dandolo et al., J. Virology, 47:55-64, 1983.
Davi et al., J Neurol Neurosurg Psychiatry, Dec;63(6):736-42, 1997.
De Jager et al., Semin. Nucl. Med. 23(2), 165-179, 1993.
de Vries et al., Epilepsia., Nov;53 Suppl 6:45-52, 2012.
DeLuca et al., J. Virol., 56(2):558-570, 1985.
Derby et al., Hear Res, 134(1-2):1-8, 1999.
Derfuss et al., Proc Natl Acad Sci USA., May 19;106(20):8302-7, 2009.
Deschamps et al., Science, 230:1174-1177, 1985.
Dholakia et al., J. Biol. Chem., 264, 20638-20642, 1989.
Doolittle and Ben-Zeev, Methods Mol. Biol., 109, :215-237, 1999.
Dorai et al., Int. J. Cancer, 82(6):846-52, 1999.
Duraisamy et al., Gene, 373:28-34, 2006.
Edbrooke et al., Mol. Cell. Biol., 9:1908-1916, 1989.
Edlund et al., Science, 230:912-916, 1985.
Eikelenboom et al., Neurology, Jan 28;60(2):219-23, 2003.
Engel and Kohn, Front Biosci, 4:e26-33, 1999.
EPO 0273085
Fechheimer et al., Proc Natl. Acad. Sci. USA, 84:8463-8467, 1987.
Feldman et al., Cardiovasc. Res., 32(2):194-207, 1996.
Feldman et al., Semin. Interv. Cardiol., 1(3):203-208,1996.
Feng and Holland, Nature, 334:6178, 1988.
Feng et al., Nat. Biotechnol., 15(9):866-870, 1997.
Fialova et al., J Neuroimmunol, Apr 27, 2013.
Firak and Subramanian, Mol. Cell. Biol., 6:3667, 1986.
Fisher et al., Hum. Gene Ther., 7(17):2079-2087, 1996.
Fisniku et al., Ann Neurol., Sep;64(3):247-54, 2008.
Foecking and Hofstetter, Gene, 45(1):101-105, 1986.
Fraley et al., Proc. Natl. Acad. Sci. USA, 76:3348-3352, 1979.
Fujita et al., Cell, 49:357, 1987.
Fujiwara and Tanaka, Nippon Geka Gakkai Zasshi, 99(7):463-468, 1998.
Garoff and Li, Curr. Opin. Biotechnol., 9(5):464-469, 1998.
Garrido et al., J. Neurovirol., 5(3):280-288, 1999.
Gefter et al., Somatic Cell Genet., 3:231-236, 1977.
Ghosh and Bachhawat, In: Liver Diseases, Targeted Diagnosis and Therapy Using Specific Receptors and Ligands, Wu et al. (Eds.), Marcel Dekker, NY, 87-104, 1991.
Gloss et al., EMBO J., 6:3735, 1987.
Gnant et al., Cancer Res., 59(14):3396-403, 1999.
Gnant et al., J. Natl. Cancer Inst., 91(20):1744-1750, 1999.
Godbout et al., Mol. Cell. Biol., 8:1169, 1988.
Goodbourn et al., Cell, 45:601, 1986.
Gopal, Mol. Cell Biol., 5:1188-1190, 1985.
Graham and Prevec, Mol Biotechnol, 3(3):207-220, 1995.
Graham and Van Der Eb, Virology, 52:456-467, 1973.
Greene et al., Immunology Today, 10:272, 1989.
Grosschedl and Baltimore, Cell, 41:885, 1985.
Gulbis and Galand, Hum. Pathol. 24(12), 1271-1285, 1993.
Haecker et al., Hum. Gene Ther., 7(15):1907-1914, 1996.
Han et al., J. Infect. Dis., 179:230-233, 1999.
Harland and Weintraub, J. Cell Biol., 101(3): 1094-1099, 1985.
Haslinger and Karin, Proc. Natl. Acad. Sci. USA, 82:8572, 1985.
Hauber and Cullen, J. Virology, 62:673, 1988.
Hayashi et al., Neurosci. Lett., 267(1):37-40, 1999.
He et al., Plant Cell Reports, 14 (2-3): 192-196, 1994.
Hen et al., Nature, 321:249, 1986.
Hensel et al., Lymphokine Res., 8:347, 1989.
Hermens and Verhaagen, Prog. Neurobiol., 55(4):399-432, 1998.
Herr and Clarke, Cell, 45:461, 1986.
Hirochika et al., J. Virol., 61:2599, 1987.
Holbrook et al., Virology, 157:211, 1987.
Holzer et al. Virology, 253(1):107-114, 1999.
Horlick and Benfield, Mol. Cell. Biol., 9:2396, 1989.
Hou and Lin, Plant Physiology, 111:166, 1996.
Howard et al., Ann. NY Acad. Sci., 880:352-365, 1999.
Howell et al., Brain, Sep;134(Pt 9):2755-71, 2011.
Huang et al., Cell, 27:245, 1981.
Huard et al., Neuromuscul Disord, 7(5):299-313, 1997.
Hug et al., Mol. Cell. Biol., 8:3065-3079, 1988.
Huizinga et al., J Neuropathol Exp Neurol., Apr;66(4):295-304, 2007.
Hwang et al., Mol. Cell. Biol., 10:585, 1990.
Imagawa et al., Cell, 51:251, 1987.
Imai et al., Nephrologie, 19(7):397-402, 1998.
Imbra and Karin, Nature, 323:555, 1986.
Imler et al., Mol. Cell. Biol., 7:2558, 1987.
Imperiale and Nevins, Mol. Cell. Biol., 4:875, 1984.
Irie et al., Antisense Nucleic Acid Drug Dev., 9(4):341-349, 1999.
Jakobovits et al., Mol. Cell. Biol., 8:2555, 1988.
Jameel and Siddiqui, Mol. Cell. Biol., 6:710, 1986.
Jaynes et al., Mol. Cell. Biol., 8:62, 1988.
Johnson et al., Mol. Cell. Biol., 9(8):3393-3399, 1989.
Johnston et al., J. Virol., 73(6):4991-5000, 1999.
Kadesch and Berg, Mol. Cell. Biol., 6:2593, 1986.
Kaeppler et al., Plant Cell Rep., 8:415-418, 1990.
Kampylafka et al., J Autoimmun., May;36(3-4):221-7, 2011.
Kaneda et al., Science, 243:375-378, 1989.
Kapeller et al., J Neurol., Feb;248(2):131-8, 2001.
Karin et al., Mol. Cell. Biol., 7:606, 1987.
Katinka et al., Cell, 20:393, 1980.
Katinka et al., Nature, 290:720, 1981.
Kato et al, J. Biol. Chem., 266:3361-3364, 1991.
Kaufman et al., Arch. Ophthalmol., 117(7):925-928, 1999.
Kawamoto et al., Mol. Cell. Biol., 8:267, 1988.
Kay, Haemophilia, 4(4):389-392, 1998.
Keegan et al., Lancet., Aug 13-19;366(9485):579-82, 2005.
Khatoon et al., Ann. of Neurology, 26, 210-219, 1989.
Kiledjian et al., Mol. Cell. Biol., 8:145, 1988.
King et al., J. Biol. Chem., 269, 10210-10218, 1989.
Klamut et al., Mol. Cell. Biol., 10:193, 1990.
Klimatcheva et al., Front Biosci, 4:D481-496, 1999.
Koch et al., Mol. Cell. Biol., 9:303, 1989.
Kohler and Milstein, Eur. J. Immunol., 6, 511-519, 1976.
Kohler and Milstein, Nature, 256, 495-497, 1975.
Kohut et al., Am. J. Physiol., 275(6Pt1):L1089-1094, 1998.
Kooby et al., FASEB J, 13(11):1325-34, 1999.
Kraus et al. FEBS Lett., 428(3):165-170, 1998.
Kriegler and Botchan, Mol. Cell. Biol., 3:325, 1983.
Kriegler et al., Cell, 38:483, 1984a.
Kriegler et al., In: Cancer Cells 2/Oncogenes and Viral Genes, Van de Woude et al. eds, Cold Spring Harbor, Cold Spring Harbor Laboratory, 1984b.
Krisky et al., Gene Ther, 5(11):1517-1530, 1998a.
Krisky et al., Gene Ther, 5(12):1593-1603, 1998b.
Kuhl et al., Cell, 50:1057, 1987.
Kunz et al., Nucl. Acids Res., 17:1121, 1989.
Kyte and Doolittle, J. Mol. Biol., 157(1): 105-132, 1982.
Lachmann and Efstathiou, Curr. Opin. Mol. Ther., 1(5):622-632, 1999..
Lareyre et al., J. Biol. Chem., 274(12):8282-8290, 1999.
Larsen et al., Proc. Natl. Acad. Sci. USA, 83:8283, 1986.
Laspia et al., Cell, 59:283, 1989.
Lassmann et al., Brain Pathol., Apr;17(2):210-8, 2007.
Latimer et al., Mol. Cell. Biol., 10:760, 1990.
Lazzeri, Methods Mol. Biol., 49:95-106, 1995.
Lee et al., DNA Cell Biol., 16(11):1267-1275, 1997.
Lee et al., Environ. Mol. Mutagen., 13(1):54-59, 1989.
Lee et al., Nature, 294:228, 1981.
Lee et al., Nucleic Acids Res., 12:4191-206, 1984.
Leibowitz et al., Diabetes, 48(4):745-753, 1999.
Lesch, Biol Psychiatry, 45(3):247-253, 1999.
Levenson et al., Hum. Gene Ther., 9(8):1233-1236, 1998.
Levin et al., Brain Res., Jul 23:1345:221-32, 2010.
Li et al., Cancer Biol. Ther., 2:187-193, 2003b.
Ligocki et al., J Neuroimmunol., Sep 14;226(1-2):192-3, 2010.
Ligocki, et al., Genes Immun., Apr 18, 2013.
Lin et al., Mol. Cell. Biol., 10:850, 1990.
Lovato et al., Brain., Jan 7, 2011.
Lucchinetti et al., Ann Neurol., Jun;47(6):707-17, 2000.
Lundstrom, J. Recept Signal Transduct. Res., 19(1-4):673-686, 1999.
Luria et al., EMBO J., 6:3307, 1987.
Lusky and Botchan, Proc. Natl. Acad. Sci. USA, 83:3609, 1986.
Lusky et al., Mol. Cell. Biol., 3:1108, 1983.
Macejak and Sarnow, Nature, 353:90-94, 1991.
Magliozzi et al., Ann Neurol., Oct;68(4):477-93, 2010.
Majors and Varmus, Proc. Natl. Acad. Sci. USA, 80:5866, 1983.
Marienfeld et al., Gene Ther., 6(6):1101-1113, 1999.
Mastrangelo et al., Biotechnol. Bioeng., 65(3):298-305, 1999.
McNeall et al., Gene, 76:81, 1989.
Miksicek et al., Cell, 46:203, 1986.
Miller et al., J. Pharmacol. Exp. Ther., 264:11-16, 1993.
Miyatake et al., Gene Ther., 6:564-572, 1999.
Moldawer et al., Shock, 12(2):83-101, 1999.
Mordacq and Linzer, Genes and Dev., 3:760, 1989.
Moreau et al., Nucl. Acids Res., 9:6047, 1981.
Moriuchi et al., Cancer Res, 58(24):5731-5737, 1998.
Morrison et al., J. Gen. Virol., 78(Pt 4):873-878, 1997.
Morrison, Science, 229(4719):1202-1207, 1985.
Muesing et al., Cell, 48:691, 1987.
Nakamura et al., In: Enzyme Immunoassays: Heterogeneous and Homogeneous Systems, Chapter 27, 1987.
Naldini et al., Science, 272(5259):263-267, 1996.
Neuberger et al., Nucleic Acids Res., 16(14B):6713-6724, 1988.
Neumann et al., Proc. Natl. Acad. Sci. USA, 96(16):9345-9350, 1999.
Ng et al., Nuc. Acids Res., 17:601, 1989.
Nicolau and Sene, Biochim. Biophys. Acta, 721:185-190, 1982.
Nicolau et al., Methods Enzymol., 149:157-176, 1987.
Nomoto et al., Gene, 236(2):259-271, 1999.
Obermeier et al., J Neuroimmunol., Apr;233(1-2):245-8, 2011.
Omirulleh et al., Plant Mol. Biol., 21(3):415-428, 1993.
Ondek et al., EMBO J., 6:1017, 1987.
O'Shannessy et al., J. Immun. Meth., 99, 153-161, 1987.
Owens and Haley, J. Biol. Chem., 259, 14843-14848, 1987.
Owens et al., Ann Neurol., Jun;65(6):639-49, 2009.
Palmiter et al., Cell, 29:701, 1982.
Parks et al., J. Virol., 71(4):3293-8, 1997.
PCT Appln. WO 92/17598
PCT Appln. WO 94/09699
PCT Appln. WO 95/06128
Pech et al., Mol. Cell. Biol., 9:396, 1989.
Pelletier and Sonenberg, Nature, 334(6180):320-325, 1988.
Perales et al., Proc. Natl. Acad. Sci. USA, 91:4086-4090, 1994.
Perez-Stable and Constantini, Mol. Cell. Biol., 10:1116, 1990.
Petrof, Eur Respir J, 11(2):492-497, 1998.
Pham et al., J Neuroimmunol., Mar 31;208(1-2):30-9, 2009.
Picard and Schaffner, Nature, 307:83, 1984.
Pinkert et al., Genes and Dev., 1:268, 1987.
Pirko et al., Neurology, Feb 27;68(9):634-42, 2007.
Ponta et al., Proc. Natl. Acad. Sci. USA, 82:1020, 1985.
Potrykus et al., Mol. Gen. Genet., 199(2):169-177, 1985.
Potter and Haley, Meth. Enzymol., 91, 613-633, 1983.
Potter et al., Proc. Natl. Acad. Sci. USA, 81:7161-7165, 1984.
Queen and Baltimore, Cell, 35:741, 1983.
Quinn et al., Mol. Cell. Biol., 9:4713, 1989.
Reddy et al., Virology, 251(2):414-26, 1998.
Redondo et al., Science, 247:1225, 1990.
Reichlin, J Autoimmun., Oct;11(5):557-61, 1998.
Reisman and Rotter, Mol. Cell. Biol., 9:3571, 1989.
Resendez Jr. et al., Mol. Cell. Biol., 8:4579, 1988.
Rhodes et al., Methods Mol. Biol., 55:121-131, 1995.
Rippe et al., Mol. Cell. Biol., 9(5):2224-22277, 1989.
Rippe, et al., Mol. Cell Biol., 10:689-695, 1990.
Rittling et al., Nucl. Acids Res., 17:1619, 1989.
Robbins and Ghivizzani, Pharmacol Ther, 80(1):35-47, 1998.
Robbins et al., Trends Biotechnol., 16(1):35-40, 1998.
Sambrook et al., In: Molecular cloning: a laboratory manual, 2nd Ed., Cold Spring Harbor Laboratory Press, Cold Spring Harbor, NY, 1989.
Sawai et al. Mol. Genet. Metab., 67(1):36-42, 1999.
Schaffner et al., J. Mol. Biol., 201:81, 1988.
Searle et al., Mol. Cell. Biol., 5:1480, 1985.
Sellebjerg et al., J Neuroimmunol., Aug 1;108(1-2):207-15, 2000.
Sellebjerg et al., J Neurol Sci., May 7;157(2):168-74, 1998.
Sharp and Marciniak, Cell, 59:229, 1989.
Shaul and Ben-Levy, EMBO J., 6:1913, 1987.
Sherman et al., Mol. Cell. Biol., 9:50, 1989.
Sleigh and Lockett, J. EMBO, 4:3831, 1985.
Smith et al., Neuron., 20:1093-1102, 1998.
Spalholz et al., Cell, 42:183, 1985.
Spandau and Lee, J. Virology, 62:427, 1988.
Spandidos and Wilkie, EMBO J., 2:1193, 1983.
Srivastava et al., N Engl J Med., Jul 12;367(2):115-23, 2012.
Stephens and Hentschel, Biochem. J., 248:1, 1987.
Stewart et al., Arch. Biochem. Biophys. 365:71-74; 1999.
Stowe et al., Ann Neurol., Jun;69(6):975-85, 2011.
Stuart et al., Nature, 317:828, 1985.
Sullivan and Peterlin, Mol. Cell. Biol., 7:3315, 1987.
Sun et al., J. Steroid Biochem., 26(1):83-92, 1987.
Suzuki et al., Biochem Biophys Res Commun, 252(3):686-90, 1998.
Swartzendruber and Lehman, J. Cell. Physiology, 85:179, 1975.
Takebe et al., Mol. Cell. Biol., 8:466, 1988.
Talbott et al., Exp Neurol., Mar;192(1):11-24, 2005.
Tavernier et al., Nature, 301:634, 1983.
Taylor and Kingston, Mol. Cell. Biol., 10:165, 1990a.
Taylor and Kingston, Mol. Cell. Biol., 10:176, 1990b.
Taylor et al., J. Biol. Chem., 264:15160, 1989.
Thiesen et al., J. Virology, 62:614, 1988.
Tiller et al., J Immunol Methods., Jan 1;329(1-2):112-24, 2008.
Timiryasova et al., Int. J. Oncol., 14(5):845-854, 1999.
Trapp et al., N Engl J Med., Jan 29;338(5):278-85, 1998.
Trojano et al., J Neurol Sci., Nov;143(1-2):107-13, 1996.
Tronche et al., Mol. Biol. Med., 7:173, 1990.
Tronche et al., Mol. Cell. Biol., 9:4759, 1989.
Trudel and Constantini, Genes and Dev., 6:954, 1987.
Tsukada et al., Plant Cell Physiol., 30(4)599-604, 1989.
Tsumaki et al., J. Biol. Chem., 273(36):22861-22864, 1998.
Tur-Kaspa et al., Mol. Cell Biol., 6:716-718, 1986.
Valsasina et al., Neurology., Oct 11;65(7):1126-8, 2005.
Vanderkwaak et al., Gynecol Oncol, 74(2):227-234, 1999.
Vasseur et al., Proc. Natl. Acad. Sci. USA, 77:1068, 1980.
Vercellino et al., J Neuropathol Exp Neurol., Dec;64(12):1101-7, 2005.
Verhoeyen et al., Science, 239(4847):1534-1536, 1988.
von Budingen et al., Eur J Immunol., Jul;38(7):2014-23, 2008.
Wagner et al., Proc. Natl. Acad. Sci. USA 87(9):3410-3414, 1990.
Wang and Calame, Cell, 47:241, 1986.
Wang et al., Infect. Immun., 66:4193-202, 1998.
Wawrzynczak & Thorpe, Cancer Treat Res., 37:239-51, 1988.
Weber et al., Cell, 36:983, 1984.
Weihl et al., Neurosurgery, 44(2):239-252, 1999.
White et al. J. Virol., 73(4):2832-2840, 1999.
Wilson, J. Clin. Invest., 98(11):2435, 1996.
Winoto and Baltimore, Cell, 59:649, 1989.
Wong et al., Gene, 10:87-94, 1980.
Wood et al., J. Clin. Lab. Immunol., 17(4):167-171, 1985.
Wu and Wu, Adv. Drug Delivery Rev., 12:159-167, 1993.
Wu and Wu, J. Biol. Chem., 262:4429-4432, 1987.
Wu et al., Biochem. Biophys. Res. Commun., 233(1):221-226, 1997.
Wu et al., Cancer Res., 58(8): 1605-8, 1998.
Yamada et al., Brain Res., 833(2):302-307, 1999.
Yeung et al., Gene Ther., 6(9):1536-1544, 1999.
Yoon et al., J. Gastrointest. Surg., 3(1):34-48, 1999.
Yu et al., J Neuroimmunol., Dec 15;240-241:129-36, 2011.
Yurasov et al., J Exp Med., Mar 7;201(5):703-11, 2005.
Yutzey et al. Mol. Cell. Biol., 9:1397, 1989.
Zhang et al., J Autoimmun., Nov-Dec;33(3-4):270-4, 2009.
Zhao-Emonet et al., Biochim. Biophys. Acta, 1442(2-3):109-119, 1998.
Zheng et al., J. Gen. Virol., 80(Pt 7):1735-1742, 1999.
Zhou et al., Nature, 361(6412):543-547 , 1993.
Zufferey et al., Nat. Biotechnol., 15(9):871-875, 1997.

## Claims

1. An siRNA that reduces the amount of a VH4 antibody, wherein said VH4 antibody binds targets within the Central Nervous System (CNS), for use in a method of treating multiple sclerosis (MS) or clinically isolated syndrome in a patient, wherein the method comprises administering to said subject said siRNA, and wherein the VH4 antibody has at least two substitutions with respect to the germline sequence at codon positions selected from 31B, 32, 40, 56, 57, 60, 81, and 89.

2. The siRNA for use of claim 1, wherein said siRNA is administered systemically in said method.

3. The siRNA for use of claim 1, wherein said siRNA is administered through a route that targets neuronal tissue in said method.

4. The siRNA for use of any one of claims 1 to 3, wherein the siRNA reduces the amount of a VH4 antibody having a substitution with respect to the germline sequence at codon positions selected from 31B, 56, and 81.

5. The siRNA for use of any one of claims 1 to 3, wherein the siRNA reduces the amount of a VH4 antibody having heavy chain CDRs selected from SEQ ID NOS: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61 and 63, and light chain CDRs selected from SEQ ID NOS: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62 and 64, respectively.

6. The siRNA for use of claims 1 to 5, the method further comprising administering to said subject one or more traditional MS therapies.

## Patentansprüche

1. Eine siRNA, die die Menge eines VH4-Antikörpers verringert, wobei besagter VH4-Antikörper Ziele innerhalb des Zentralnervensystems (ZNS) bindet, zur Verwendung in einem Verfahren zur Behandlung Multipler Sklerose (MS) oder eines klinisch isolierten Syndroms in einem Patienten, wobei das Verfahren Verabreichen besagter siRNA an besagte Person umfasst und wobei der VH4-Antikörper wenigstens zwei Substitutionen bezogen auf die Keimbahn-Sequenz an Codonpositionen, ausgewählt aus 31B, 32, 40, 56, 57, 60, 81 und 89, besitzt.

2. Die siRNA zur Verwendung nach Anspruch 1, wobei besagte siRNA in besagtem Verfahren systemisch verabreicht wird.

3. Die siRNA zur Verwendung nach Anspruch 1, wobei besagte siRNA über einen Weg, der auf neuronales Gewebe gerichtet ist, in besagtem Verfahren verabreicht wird.

4. Die siRNA zur Verwendung nach einem der Ansprüche 1 bis 3, wobei die siRNA die Menge eines VH4-Antikörpers mit einer Substitution bezogen auf die Keimbahn-Sequenz an Codonpositionen, ausgewählt aus 31B, 56 und 81, verringert.

5. Die siRNA zur Verwendung nach einem der Ansprüche 1 bis 3, wobei die siRNA die Menge eines VH4-Antikörpers mit schwerkettigen CDRs, ausgewählt aus SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61 und 63, beziehungsweise leichtkettigen CDRs, ausgewählt aus SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62 und 64, verringert.

6. Die siRNA zur Verwendung nach einem der Ansprüche 1 bis 5, wobei das Verfahren außerdem Verabreichen einer oder mehrerer herkömmlichen MS-Therapien an besagte Person umfasst.

## Revendications

1. ARNsi qui réduit la quantité d'un anticorps VH4, dans lequel ledit anticorps VH4 se lie à des cibles dans le système nerveux central (SNC), pour une utilisation dans une méthode de traitement de la sclérose en plaques (SEP) ou d'un syndrome cliniquement isolé chez un patient, dans lequel la méthode comprend l'administration audit sujet dudit ARNsi, et dans lequel l'anticorps VH4 a au moins deux substitutions par rapport à la séquence de lignée germinale aux positions de codon choisies parmi 31B, 32, 40, 56, 57, 60, 81 et 89.

2. ARNsi pour une utilisation selon la revendication 1, lequel ARNsi est administré par voie systémique dans ladite méthode.

3. ARNsi pour une utilisation selon la revendication 1, lequel ARNsi est administré par une voie qui cible un tissu neuronal dans ladite méthode.

4. ARNsi pour une utilisation selon l'une quelconque des revendications 1 à 3, lequel ARNsi réduit la quantité d'un anticorps VH4 ayant une substitution par rapport à la séquence de lignée germinale aux positions de codon choisies parmi 31B, 56 et 81.

5. ARNsi pour une utilisation selon l'une quelconque des revendications 1 à 3, lequel ARNsi réduit la quantité d'un anticorps VH4 ayant des CDR de chaîne lourde choisies parmi les SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61 et 63, et les CDR de chaîne légère choisies parmi les SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62 et 64, respectivement.

6. ARNsi pour une utilisation selon les revendications 1 à 5, dans lequel la méthode comprend en outre l'administration audit sujet d'un ou plusieurs traitements traditionnels de la SEP.
